(19) Europäisches Patentamt
European Patent Office
Office européen des brevets

(11) EP 3 843 728 B1

(12) EUROPEAN PATENT SPECIFICATION

(45) Date of publication and mention of the grant of the patent:
**30.04.2025 Bulletin 2025/18**

(21) Application number: **19856339.7**

(22) Date of filing: **29.08.2019**

(51) International Patent Classification (IPC):
***C07D 471/04*** $^{(2006.01)}$ ***C07D 519/00*** $^{(2006.01)}$
***A61K 31/4353*** $^{(2006.01)}$ ***A61K 31/4375*** $^{(2006.01)}$
***A61P 11/00*** $^{(2006.01)}$ ***A61P 13/12*** $^{(2006.01)}$
***A61P 35/00*** $^{(2006.01)}$

(52) Cooperative Patent Classification (CPC):
**A61P 11/00; A61P 13/12; A61P 35/00; C07D 471/04; C07D 519/00**

(86) International application number:
**PCT/US2019/048737**

(87) International publication number:
**WO 2020/047207 (05.03.2020 Gazette 2020/10)**

(54) **INHIBITORS OF (ALPHA-V)(BETA-6) INTEGRIN**

INHIBITOREN VON (ALPHA-V)(BETA-6)-INTEGRIN

INHIBITEURS DE L’INTÉGRINE (ALPHA-V)(BÊTA -6)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.08.2018 US 201862724400 P**

(43) Date of publication of application:
**07.07.2021 Bulletin 2021/27**

(73) Proprietor: **Morphic Therapeutic, Inc.**
**Waltham, MA 02451 (US)**

(72) Inventors:
- **HARRISON, Bryce, A.** Framingham, MA 01701 (US)
- **DOWLING, James, E.** Lexington, MA 02420 (US)
- **GERASYUTO, Aleksey I.** Flemington, NJ 08822 (US)
- **BURSAVICH, Matthew, G.** Needham, MA 02494 (US)
- **TROAST, Dawn, M.** Bedford, MA 01730 (US)
- **LIPPA, Blaise, S.** Newton, MA 02459 (US)
- **ROGERS, Bruce, N.** Belmont, MA 02478 (US)
- **ZHONG, Cheng** Belmont, MA 02478 (US)
- **QIAO, Qi** Natick, MA 01760 (US)
- **LIN, Fu-Yang** Sudbury, MA 01776 (US)
- **SOSA, Brian** Cambridge, MA 02139 (US)
- **BORTOLATO, Angrea** Metuchen, NJ 08840 (US)
- **SVENSSON, Mats, A.** New York, NY 10001 (US)
- **HICKEY, Eugene** Danbury, CT 06811 (US)
- **DAY, Tyler** New York, NY 10036 (US)
- **KIM, Byungchan** West New York, NJ 07093 (US)
- **KONZE, Kyle, D.** Brooklyn, NY 11215 (US)

(74) Representative: **Carpmaels & Ransford LLP**
**One Southampton Row**
**London WC1B 5HA (GB)**

(56) References cited:
**WO-A1-2017/162572**
**WO-A1-2018/089355**
**WO-A1-2018/119087**
**WO-A1-98/46220**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

WO-A1-99/30709
WO-A1-99/31061
WO-A2-2018/160521
US-A- 6 017 926
US-A1- 2016 280 705
US-A1- 2017 290 817
US-A1- 2018 244 648

- PERKINS JAMES J ET AL: "Non-peptide [alpha] v [beta] 3 antagonists: Identification of potent, chain-shortened RGD mimetics that incorporate a central pyrrolidinone constraint", BIOORGANIC & MEDICINAL CHEMISTRY LETTERS, vol. 13, no. 24, December 2003 (2003-12-01), AMSTERDAM, NL, pages 4285 - 4288, XP055892480, ISSN: 0960-894X, DOI: 10.1016/j.bmcl.2003.09.055

## Description

### RELATED APPLICATION

This application claims the benefit of priority to U.S. Provisional Patent Application No. 62/724,400, filed August 29, 2018.

### TECHNICAL FIELD

This disclosure relates to novel chemical compounds and methods useful for inhibiting αvβ6 integrin.

### BACKGROUND

The heterodimeric integrin family of receptors modulate cellular shape and cell adhesion to the extracellular matrix in response to extrinsic and intrinsic cues.

Integrin signaling controls cell survival, cell cycle progression, cell differentiation, and cell migration.

The integrin receptor exclusively can signal a cell bi-directionally, both "inside-out" and "outside-in." Thus, they mediate cell migration by transmitting forces from the extracellular matrix to the cytoskeleton and regulate cytoskeletal organization to achieve shape changes needed during cell migration. RGD-binding integrins can bind to and activate TGF-β, and have recently been implicated in fibrotic disease.

Integrins are expressed on the surface of most of human cells. Their pathology contributes to a diverse set of human diseases, including platelet disorders, atherosclerosis, cancer, osteoporosis, fibrosis, diabetic neuropathy of the kidney, macular degeneration and various autoimmune and chronic inflammation diseases.

The role of integrins as drug targets has long been recognized, and a total of six injectable integrin inhibitors have been approved by the Food and Drug Administration for the treatment of various therapeutic indications: inflammatory bowel disease (Entyvio®, Tysabri®), multiple sclerosis (Tysabri®), psoriasis (Raptiva®), and acute coronary syndrome (Reopro®, Aggrastat®, Integrilin®). However, there has been a notable absence of therapeutic success with orally bioavailable integrin inhibitors.

Of the 24 known integrin heterodimers, as least half have relevance in inflammation, fibrosis, oncology and vascular disease. There exists a need for new classes of integrin inhibitors. However, there remains a need for a small molecule integrin inhibitor of αvβ6 suitable for oral administration. The oral administration route is preferred for small-molecule delivery as it allows a wide range of doses to be administered, allows convenient patient self-administration, is adaptable to varying dosage regimens and needs no special equipment. Therefore, it is important to identify of αvβ6 integrin inhibitor compounds that are not only potent at the intended biological target, but are also demonstrating other characteristics relating to the ability of the compound to be absorbed in the body (e.g, after oral delivery) in a therapeutically effective manner. For example, αvβ6 integrin inhibitor compounds can be selected based on both potency and based on performance in an in vitro permeability assay (e.g., evaluating the ability of compounds to cross a layer of Madin-Darby Canine Kidney (MDCK) cells from the apical to basolateral side (A->B)).

WO 1999/030709 A1 discloses compounds and derivatives thereof as vitronectin receptor antagonists.

Perkins James J et al., Bioorganic & Medicinal Chemistry Letters, vol. 13, no. 24, pages 4285-4288 (2003*)* disclose a series of pyrrolidinone-containing αvβ3 receptor antagonists for use in reducing bone resorption.

US 6 017 926 A discloses compounds and derivatives thereof as integrin receptor antagonists for use in inhibiting bone resorption.

WO 2018/089355 A1 discloses compounds as antagonists to αv-containing integrins for use in treating conditions such as pathological fibrosis and transplant rejection.

WO 2018/160521 A2 discloses small molecule inhibitors of αvβ6 integrin for use in treating conditions such as idiopathic pulmonary fibrosis and diabetic nephropathy.

### SUMMARY

Applicants have discovered novel αvβ6 integrin inhibitor compounds and evaluated the posession, performance and utility of represeentative examples of such compounds, both for biochemical potency (e.g., using the assay of Example 12 to evaluate fluorescence polarization assays of compounds for αvβ6 binding) and in vitro permeability properties (e.g., using the assay of Example 13 to evaluate MDCK permeability).

Generally, the present disclosure is concerned with a compound represented by formula (I):

A-B-C (I)

wherein:

A is

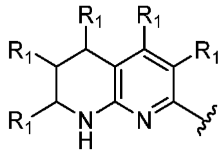

or

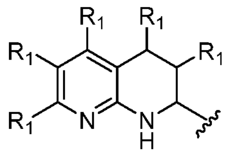

;

B is alkylene, -alkylene-(O); -alkylene-N(R)C(O)-, -alkylene-(heterocyclyl)-C(O)-, -alkylene-C(O)N(R)-, -alkylene-C(O)-, -alkylene-N(R)-, -alkylene-N(R)C(O)N(R)-, - alkylene-N(R)$SO_2$-, -alkylene-(aryl)-, -alkylene-(heterocyclyl)-, -alkylene-(heterocyclyl)-alkylene-, -aryl-alkylene-N(R)C(O)-; -aryl-C(O)N(R)-, -aryl-N(R)C(O)-, -(heterocyclyl)-alkylene-, -heterocyclyl-alkylene-N(R)C(O)-; -heterocyclyl-C(O)N(R)-, -O-heterocyclyl-; - alkylene-O-; -heterocyclyl-C(O)-; -cycloalkylene; -cycloalkylene-O-; -alkylene-C*(H)(F)-, - alkylene-C*$(F)_2$-, -alkylene-C(H)(F)C*$H_2$-, or -alkylene-C$(F)_2$C*$H_2$-;

C is

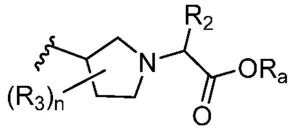

;

R is H, alkyl, or aryl;

$R_1$ is independently H, alkyl, halide, alkoxy, $CF_3$, OH, alkylene-OH, $NO_2$, - N(H)R, or $NH_2$;

$R_2$ is substituted or unsubstituted bicyclic heterocyclyl, wherein at least one ring atom of the bicyclic heterocyclyl is O;

$R_3$ is independently selected from H, halide, $CF_3$, alkyl, alkylene-alkoxy, aryl, hydroxyl, and alkoxy;

$R_a$ is H, $(C_1\text{-}C_6)$alkyl, -$(C_1\text{-}C_6)$alkylene-O-$(C_1\text{-}C_6)$alkyl, or -$(C_1\text{-}C_6)$alkylene-O-C(O)O$(C_1\text{-}C_6)$alkyl;

n is 0, 1, 2, 3, or 4;

* denotes the point of attachment of B to C; and

the absolute configuration at any stereocenter is R, S, or a mixture thereof;

or a pharmaceutically acceptable salt thereof.

Specifically, the invention relates to the specific compounds defined in the claims.

In certain embodiments, the invention relates to a compound selected from the group consisting of:

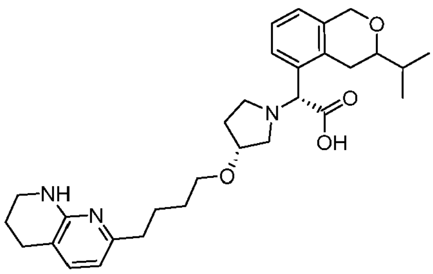

,

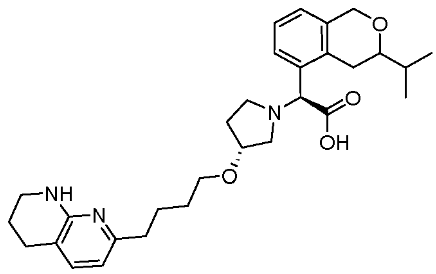

,

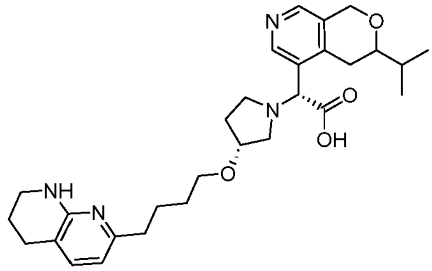

,

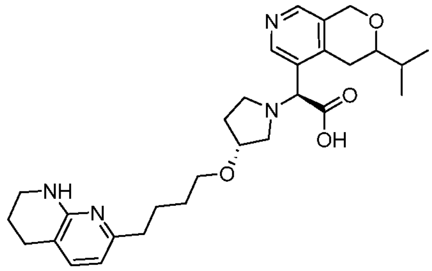

,

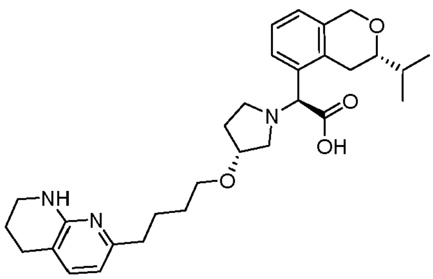 , 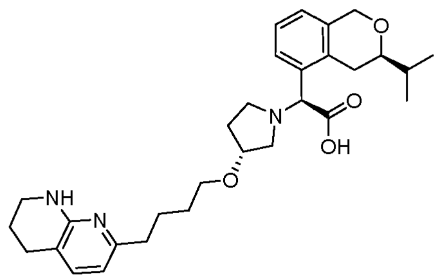 ,

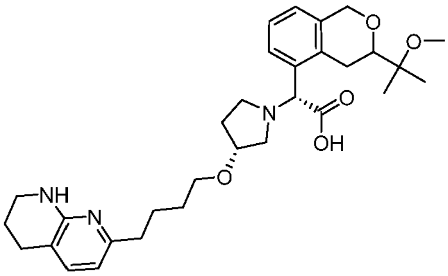 , and 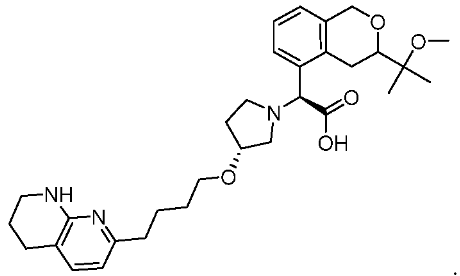 .

In certain embodiments, the invention relates to a compound as described herein for use in a method of treating a disease or a condition selected from the group consisting of idiopathic pulmonary fibrosis, diabetic nephropathy, focal segmental glomerulosclerosis, chronic kidney disease, nonalcoholic steatohepatitis, primary biliary cholangitis, primary sclerosing cholangitis, solid tumors, hematological tumors, organ transplant, Alport syndrome, interstitial lung disease radiation-induced fibrosis, bleomycin-induced fibrosis, asbestos-induced fibrosis, flu-induced fibrosis, coagulation-induced fibrosis, vascular injury-induced fibrosis, aortic stenosis, and cardiac fibrosis comprising the step of: administering to a subject in need thereof a therapeutically effective amount of any one of the compounds described herein.

## BRIEF DESCRIPTION OF THE FIGURES

**Figure 1** depicts a table summarizing inhibition of αvβ6 integrin by exemplary compounds measured in a fluorescence polarization assay.

**Figure 2** depicts a table summarizing permeability properties of exemplary compounds measured in a MDCK *in vitro* assay of Example 13.

## DETAILED DESCRIPTION OF THE INVENTION

In certain embodiments, the invention relates to compounds that inhibit αvβ6 integrin. In certain embodiments, the compounds are selective for αvβ6 integrin.

The compounds will be useful for the treatment of idiopathic pulmonary fibrosis, diabetic nephropathy, focal segmental glomerulosclerosis, chronic kidney disease, nonalcoholic steatohepatitis, primary biliary cholangitis, primary sclerosing cholangitis, solid tumors, hematological tumors, organ transplant, Alport syndrome, interstitial lung disease, radiation-induced fibrosis, bleomycin-induced fibrosis, asbestos-induced fibrosis, flu-induced fibrosis, coagulation-induced fibrosis, vascular injury-induced fibrosis, aortic stenosis, or cardiac fibrosis.

### DEFINITIONS

For convenience, before further description of the present invention, certain terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

In order for the present invention to be more readily understood, certain terms and phrases are defined below and throughout the specification.

The articles "a" and "an" are used herein to refer to one or to more than one (i.e., to at least one) of the grammatical object of the article. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods disclosed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

Certain compounds contained in compositions of the present invention may exist in particular geometric or stereoisomeric forms. In addition, polymers of the present invention may also be optically active. The present invention contemplates all such compounds, including cis- and trans-isomers, *R*- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

Structures depicted herein are also meant to include compounds that differ only in the presence of one or more isotopically enriched atoms. For example, compounds produced by the replacement of a hydrogen with deuterium or tritium, or of a carbon with a $^{13}C$- or $^{14}C$-enriched carbon are within the scope of this invention.

The term "prodrug" as used herein encompasses compounds that, under physiological conditions, are converted into therapeutically active agents. A common method for making a prodrug is to include selected moieties that are hydrolyzed under physiological conditions to reveal the desired molecule. In other embodiments, the prodrug is converted by an enzymatic activity of the host animal.

The phrase "pharmaceutically acceptable excipient" or "pharmaceutically acceptable carrier" as used herein means a pharmaceutically acceptable material, composition or vehicle, such as a liquid or solid filler, diluent, excipient, solvent or encapsulating material, involved in carrying or transporting the subject chemical from one organ or portion of the body, to another organ or portion of the body. Each carrier must be "acceptable" in the sense of being compatible with the

other ingredients of the formulation, not injurious to the patient, and substantially non-pyrogenic. Some examples of materials which can serve as pharmaceutically acceptable carriers include: (1) sugars, such as lactose, glucose, and sucrose; (2) starches, such as corn starch and potato starch; (3) cellulose, and its derivatives, such as sodium carboxymethyl cellulose, ethyl cellulose, and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) glycols, such as propylene glycol; (11) polyols, such as glycerin, sorbitol, mannitol, and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations. In certain embodiments, pharmaceutical compositions of the present invention are non-pyrogenic, i.e., do not induce significant temperature elevations when administered to a patient.

The term "pharmaceutically acceptable salts" refers to the relatively non-toxic, inorganic and organic acid addition salts of the compound(s). These salts can be prepared in situ during the final isolation and purification of the compound(s), or by separately reacting a purified compound(s) in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, naphthylate, mesylate, glucoheptonate, lactobionate, and laurylsulphonate salts. (See, for example, Berge et al. (1977) "Pharmaceutical Salts", J. Pharm. Sci. 66:1-19.)

In other cases, the compounds useful in the present invention may contain one or more acidic functional groups and, thus, are capable of forming pharmaceutically acceptable salts with pharmaceutically acceptable bases. The term "pharmaceutically acceptable salts" in these instances refers to the relatively non-toxic inorganic and organic base addition salts of a compound(s). These salts can likewise be prepared in situ during the final isolation and purification of the compound(s), or by separately reacting the purified compound(s) in its free acid form with a suitable base, such as the hydroxide, carbonate, or bicarbonate of a pharmaceutically acceptable metal cation, with ammonia, or with a pharmaceutically acceptable organic primary, secondary, or tertiary amine. Representative alkali or alkaline earth salts include the lithium, sodium, potassium, calcium, magnesium, and aluminum salts. Representative organic amines useful for the formation of base addition salts include ethylamine, diethylamine, ethylenediamine, ethanolamine, diethanolamine and piperazine (see, for example, Berge et al., *supra*).

A "therapeutically effective amount" (or "effective amount") of a compound with respect to use in treatment, refers to an amount of the compound in a preparation which, when administered as part of a desired dosage regimen (to a mammal, preferably a human) alleviates a symptom, ameliorates a condition, or slows the onset of disease conditions according to clinically acceptable standards for the disorder or condition to be treated or the cosmetic purpose, e.g., at a reasonable benefit/risk ratio applicable to any medical treatment.

The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic, (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

The term "patient" refers to a mammal in need of a particular treatment. In certain embodiments, a patient is a primate, canine, feline, or equine. In certain embodiments, a patient is a human.

An aliphatic chain comprises the classes of alkyl, alkenyl and alkynyl defined below. A straight aliphatic chain is limited to unbranched carbon chain moieties. As used herein, the term "aliphatic group" refers to a straight chain, branched-chain, or cyclic aliphatic hydrocarbon group and includes saturated and unsaturated aliphatic groups, such as an alkyl group, an alkenyl group, or an alkynyl group.

"Alkyl" refers to a fully saturated cyclic or acyclic, branched or unbranched carbon chain moiety having the number of carbon atoms specified, or up to 30 carbon atoms if no specification is made. For example, alkyl of 1 to 8 carbon atoms refers to moieties such as methyl, ethyl, propyl, butyl, pentyl, hexyl, heptyl, and octyl, and those moieties which are positional isomers of these moieties. Alkyl of 10 to 30 carbon atoms includes decyl, undecyl, dodecyl, tridecyl, tetradecyl, pentadecyl, hexadecyl, heptadecyl, octadecyl, nonadecyl, eicosyl, heneicosyl, docosyl, tricosyl and tetracosyl. In certain embodiments, a straight chain or branched chain alkyl has 30 or fewer carbon atoms in its backbone (e.g., $C_1$-$C_{30}$ for straight chains, $C_3$-$C_{30}$ for branched chains), and more preferably 20 or fewer. Alkyl goups may be substituted or unsubstituted.

As used herein, the term "alkylene" refers to an alkyl group having the specified number of carbons, for example from 2 to 12 carbon atoms, that contains two points of attachment to the rest of the compound on its longest carbon chain. Non-limiting examples of alkylene groups include methylene $-(CH_2)-$, ethylene $-(CH_2CH_2)-$, n-propylene $-(CH_2CH_2CH_2)-$ and isopropylene $-(CH_2CH(CH_3))-$. Alkylene groups can be cyclic or acyclic, branched or unbranched carbon chain moiety, and may be optionally substituted with one or more substituents.

"Cycloalkyl" means mono- or bicyclic or bridged or spirocyclic, or polycyclic saturated carbocyclic rings, each having from 3 to 12 carbon atoms. Likewise, preferred cycloalkyls have from 3-10 carbon atoms in their ring structure, and more preferably have 3-6 carbons in the ring structure. Cycloalkyl groups may be substituted or unsubstituted.

Unless the number of carbons is otherwise specified, "lower alkyl," as used herein, means an alkyl group, as defined above, but having from one to ten carbons, more preferably from one to six carbon atoms in its backbone structure such as methyl, ethyl, n-propyl, isopropyl, n-butyl, isobutyl, sec-butyl, and tert-butyl. Likewise, "lower alkenyl" and "lower alkynyl" have similar chain lengths. Throughout the application, preferred alkyl groups are lower alkyls. In certain embodiments, a substituent designated herein as alkyl is a lower alkyl.

"Alkenyl" refers to any cyclic or acyclic, branched or unbranched unsaturated carbon chain moiety having the number of carbon atoms specified, or up to 26 carbon atoms if no limitation on the number of carbon atoms is specified; and having one or more double bonds in the moiety. Alkenyl of 6 to 26 carbon atoms is exemplified by hexenyl, heptenyl, octenyl, nonenyl, decenyl, undecenyl, dodenyl, tridecenyl, tetradecenyl, pentadecenyl, hexadecenyl, heptadecenyl, octadecenyl, nonadecenyl, eicosenyl, heneicosoenyl, docosenyl, tricosenyl, and tetracosenyl, in their various isomeric forms, where the unsaturated bond(s) can be located anywhere in the moiety and can have either the (Z) or the (E) configuration about the double bond(s).

"Alkynyl" refers to hydrocarbyl moieties of the scope of alkenyl, but having one or more triple bonds in the moiety.

The term "alkylthio" refers to an alkyl group, as defined above, having a sulfur moiety attached thereto. In certain embodiments, the "alkylthio" moiety is represented by one of -(S)-alkyl, -(S)-alkenyl, -(S)-alkynyl, and $-(S)-(CH_2)_m-R^1$, wherein m and $R^1$ are defined below. Representative alkylthio groups include methylthio and ethylthio. The terms "alkoxyl" or "alkoxy" as used herein refers to an alkyl group, as defined below, having an oxygen moiety attached thereto. Representative alkoxyl groups include methoxy, ethoxy, propoxy and tert-butoxy. An "ether" is two hydrocarbons covalently linked by an oxygen. Accordingly, the substituent of an alkyl that renders that alkyl an ether is or resembles an alkoxyl, such as can be represented by one of -O-alkyl, -O-alkenyl, -O-alkynyl, $-O-(CH_2)_m-R_{10}$, where m and $R_{10}$ are described below.

The terms "amine" and "amino" are art-recognized and refer to both unsubstituted and substituted amines, e.g., a moiety that can be represented by the formulae:

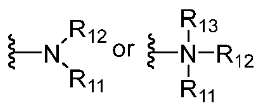

wherein $R_{11}$, $R_{12}$ and $R_{13}$ each independently represent a hydrogen, an alkyl, an alkenyl, $-(CH_2)_m-R_{10}$, or $R_{11}$ and $R_{12}$ taken together with the N atom to which they are attached complete a heterocycle having from 4 to 8 atoms in the ring structure; $R_{10}$ represents an alkenyl, aryl, cycloalkyl, a cycloalkenyl, a heterocyclyl, or a polycyclyl; and m is zero or an integer in the range of 1 to 8. In certain embodiments, only one of $R_{11}$ or $R_{12}$ can be a carbonyl, e.g., $R_{11}$, $R_{12}$, and the nitrogen together do not form an imide. In even more certain embodiments, $R_{11}$ and $R_{12}$ (and optionally $R_{13}$) each independently represent a hydrogen, an alkyl, an alkenyl, or $-(CH_2)_m-R_{10}$. Thus, the term "alkylamine" as used herein means an amine group, as defined above, having a substituted or unsubstituted alkyl attached thereto, i.e., at least one of $R_{11}$ and $R_{12}$ is an alkyl group. In certain embodiments, an amino group or an alkylamine is basic, meaning it has a conjugate acid with a $pK_a > 7.00$, i.e., the protonated forms of these functional groups have $pK_{aS}$ relative to water above about 7.00.

The term "amide", as used herein, refers to a group

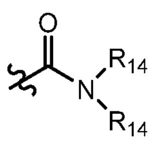

wherein each $R_{14}$ independently represent a hydrogen or hydrocarbyl group, or two $R_{14}$ are taken together with the N atom to which they are attached complete a heterocycle having from 4 to 8 atoms in the ring structure.

The term "aryl" as used herein includes 3- to 12-membered substituted or unsubstituted single-ring aromatic groups in which each atom of the ring is carbon (i.e., carbocyclic aryl) or where one or more atoms are heteroatoms (i.e., heteroaryl). Preferably, aryl groups include 5- to 12-membered rings, more preferably 6- to 10-membered rings The term "aryl" also includes polycyclic ring systems having two or more cyclic rings in which two or more carbons are common to two adjoining rings wherein at least one of the rings is aromatic, e.g., the other cyclic rings can be cycloalkyls, cycloalkenyls, cycloalkynyls, aryls, heteroaryls, and/or heterocyclyls. Carboycyclic aryl groups include benzene, naphthalene, phenanthrene, phenol and aniline. Heteroaryl groups include substituted or unsubstituted aromatic 3- to 12-membered ring

structures, more preferably 5- to 12-membered rings, more preferably 5- to 10-membered rings, whose ring structures include one to four heteroatoms. Heteroaryl groups include, for example, pyrrole, furan, thiophene, imidazole, oxazole, thiazole, triazole, pyrazole, pyridine, pyrazine, pyridazine and pyrimidine. Aryl and heteroaryl can be monocyclic, bicyclic, or polycyclic. Each instance of an aryl group may be independently optionally substituted, *i.e.,* unsubstituted (an "unsubstituted aryl") or substituted (a "substituted aryl") with one or more substituents; e.g., for instance from 1 to 5 substituents, 1 to 4 substituents, 1 to 3 substituents, 1 to 2 substituents or just 1 substituent. The aromatic ring may be substituted at one or more ring positions with one or more substituents, such as halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, fluoroalkyl (such as trifluromethyl), cyano, or the like. For example, in certain embodiments, the aryl group can be an unsubstituted $C_5$-$C_{12}$ aryl and in certain embodiments, the aryl group can be a substituted $C_5$-$C_{10}$ aryl.

The term "halo", "halide", or "halogen" as used herein means halogen and includes, for example, and without being limited thereto, fluoro, chloro, bromo and iodo, in both radioactive and non-radioactive forms. In a preferred embodiment, halo is selected from the group consisting of fluoro, chloro and bromo.

The terms "heterocyclyl" or "heterocyclic group" refer to 3- to 12-membered ring structures, more preferably 5- to 12-membered rings, more preferably 5- to 10-membered rings, whose ring structures include one to four heteroatoms. Heterocycles can be monocyclic, bicyclic, spirocyclic, or polycyclic. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams and sultones. The heterocyclic ring can be substituted at one or more positions with such substituents as described above, as for example, halogen, alkyl, aralkyl, alkenyl, alkynyl, cycloalkyl, hydroxyl, amino, nitro, sulfhydryl, imino, amido, phosphate, phosphonate, phosphinate, carbonyl, carboxyl, silyl, sulfamoyl, sulfinyl, ether, alkylthio, sulfonyl, ketone, aldehyde, ester, a heterocyclyl, an aromatic or heteroaromatic moiety, $-CF_3$, and -CN.

The term "carbonyl" is art-recognized and includes such moieties as can be represented by the formula:

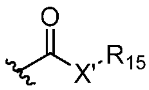
or
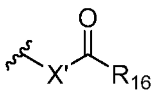

wherein X' is a bond or represents an oxygen or a sulfur, and $R_{15}$ represents a hydrogen, an alkyl, an alkenyl, $-(CH_2)_m-R_{10}$ or a pharmaceutically acceptable salt, $R_{16}$ represents a hydrogen, an alkyl, an alkenyl or $-(CH_2)_m-R_{10}$, where m and $R_{10}$ are as defined above. Where X' is an oxygen and $R_{15}$ or $R_{16}$ is not hydrogen, the formula represents an "ester." Where X' is an oxygen, and $R_{15}$ is as defined above, the moiety is referred to herein as a carboxyl group, and particularly when $R_{15}$ is a hydrogen, the formula represents a "carboxylic acid". Where X' is an oxygen, and $R_{16}$ is a hydrogen, the formula represents a "formate." In general, where the oxygen atom of the above formula is replaced by a sulfur, the formula represents a "thiocarbonyl" group. Where X' is a sulfur and $R_{15}$ or $R_{16}$ is not hydrogen, the formula represents a "thioester" group. Where X' is a sulfur and $R_{15}$ is a hydrogen, the formula represents a "thiocarboxylic acid" group. Where X' is a sulfur and $R_{16}$ is a hydrogen, the formula represents a "thioformate" group. On the other hand, where X' is a bond, and $R_{15}$ is not hydrogen, the above formula represents a "ketone" group. Where X' is a bond, and $R_{15}$ is a hydrogen, the above formula represents an "aldehyde" group.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc.

As used herein, the term "nitro" means $-NO_2$; the term "halogen" designates -F, -Cl, -Br, or -I; the term "sulfhydryl" means -SH; the term "hydroxyl" means -OH; the term "sulfonyl" means $-SO_2-$; the term "azido" means $-N_3$; the term "cyano" means -CN; the term "isocyanato" means -NCO; the term "thiocyanato" means -SCN; the term "isothiocyanato" means -NCS; and the term "cyanato" means -OCN.

The term "sulfamoyl" is art-recognized and includes a moiety that can be represented by the formula:

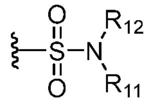

in which $R_{11}$ and $R_{12}$ are as defined above.

The term "sulfate" is art recognized and includes a moiety that can be represented by the formula:

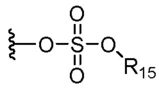

in which $R_{15}$ is as defined above.

The term "sulfonamide" is art recognized and includes a moiety that can be represented by the formula:

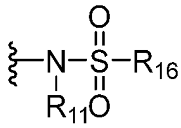

in which $R_{11}$ and $R_{16}$ are as defined above.

The term "sulfonate" is art-recognized and includes a moiety that can be represented by the formula:

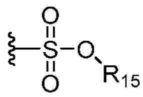

in which $R_{54}$ is an electron pair, hydrogen, alkyl, cycloalkyl, or aryl.

The terms "sulfoxido" or "sulfinyl", as used herein, refers to a moiety that can be represented by the formula:

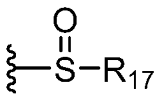

in which $R_{17}$ is selected from the group consisting of hydrogen, alkyl, alkenyl, alkynyl, cycloalkyl, heterocyclyl, aralkyl, or aryl.

The term "urea" is art-recognized and may be represented by the general formula

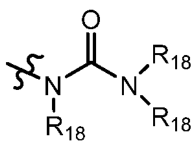

wherein each $R_{18}$ independently represents hydrogen or a hydrocarbyl, such as alkyl, or any occurrence of $R_{18}$ taken together with another and the intervening atom(s) complete a heterocycle having from 4 to 8 atoms in the ring structure.

As used herein, the definition of each expression, e.g., alkyl, m, n, etc., when it occurs more than once in any structure, is intended to be independent of its definition elsewhere in the same structure.

The term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms. Substituents can include any substituents described herein, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxy, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. In preferred embodiments, the substituents on substituted alkyls are selected from $C_{1-6}$ alkyl, $C_{3-6}$ cycloalkyl, halogen, carbonyl, cyano, or hydroxyl. In more preferred embodiments, the substituents on substituted alkyls are selected from fluoro, carbonyl, cyano, or hydroxyl. It will be understood by those skilled in the art that substituents can themselves be substituted, if appropriate. Unless specifically stated as "unsubstituted," references to chemical moieties herein are understood to include substituted variants. For example, reference to an "aryl" group or moiety implicitly includes both substituted and unsubstituted variants.

For purposes of this invention, the chemical elements are identified in accordance with the Periodic Table of the Elements, CAS version, Handbook of Chemistry and Physics, 67th Ed., 1986-87, inside cover.

EXEMPLARY COMPOUNDS OF THE DISCLOSURE

Generally, the disclosure relates to a compound of Formula I:

A-B-C (I)

wherein:

A is

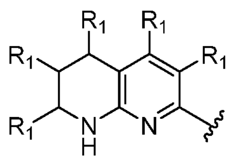 or 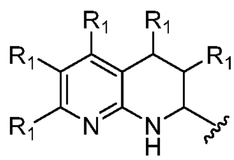 ;

B is alkylene, -alkylene-(O); -alkylene-N(R)C(O)-, -alkylene-(heterocyclyl)-C(O)-, -alkylene-C(O)N(R)-, -alkylene-C(O)-, -alkylene-N(R)-, -alkylene-N(R)C(O)N(R)-, - alkylene-N(R)$SO_2$-, -alkylene-(aryl)-, -alkylene-(heterocyclyl)-, -alkylene-(heterocyclyl)-alkylene-, -aryl-alkylene-N(R)C(O)-; -aryl-C(O)N(R)-, -aryl-N(R)C(O)-, -(heterocyclyl)-alkylene-, -heterocyclyl-alkylene-N(R)C(O)-; -heterocyclyl-C(O)N(R)-, -O-heterocyclyl-; - alkylene-O-; -heterocyclyl-C(O)-; -cycloalkylene; cycloalkylene-O-; -alkylene-C*(H)(F)-, - alkylene-C*$(F)_2$-, -alkylene-C(H)(F)C*$H_2$-, or -alkylene-C$(F)_2$C*$H_2$-;

C is

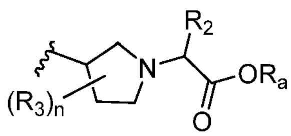 ;

R is H, alkyl, or aryl;

$R_1$ is independently H, alkyl, halide, alkoxy, $CF_3$, OH, alkylene-OH, $NO_2$, - N(H)R, or $NH_2$;

$R_2$ is substituted or unsubstituted bicyclic heterocyclyl, wherein at least one ring atom of the bicyclic heterocyclyl is O;

$R_3$ is independently selected from H, halide, $CF_3$, alkyl, alkylene-alkoxy, aryl, hydroxyl, and alkoxy;

$R_a$ is H, $(C_1\text{-}C_6)$alkyl, -$(C_1\text{-}C_6)$alkylene-O-$(C_1\text{-}C_6)$alkyl, or -$(C_1\text{-}C_6)$alkylene-O-C(O)O$(C_1\text{-}C_6)$alkyl;

n is 0, 1, 2, 3, or 4;

* denotes the point of attachment of B to C; and

the absolute configuration at any stereocenter is R, S, or a mixture thereof;

or a pharmaceutically acceptable salt thereof. The invention is defined by the claims.

Therefore, the invention relates particularly to the specific compounds of Formula (I) recited in the claims.

In certain embodiments, the invention relates to a compound selected from the group consisting of:

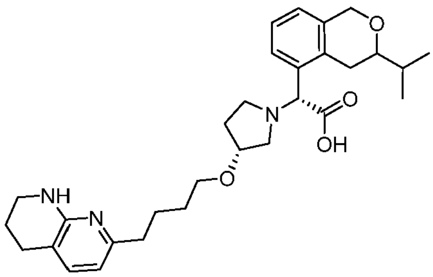
,
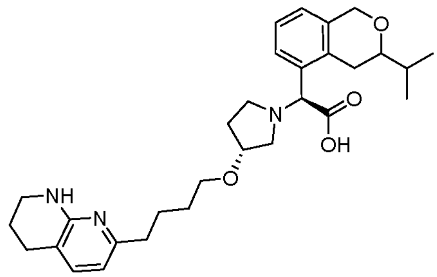
,
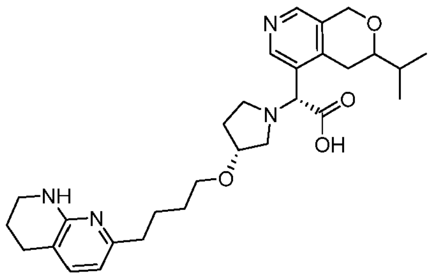
,
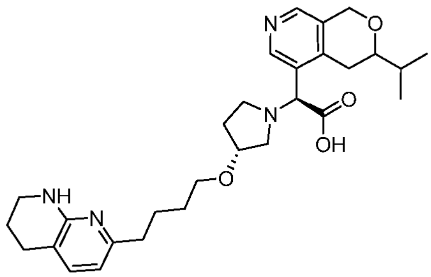
,
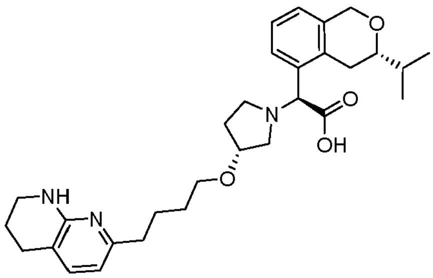
,
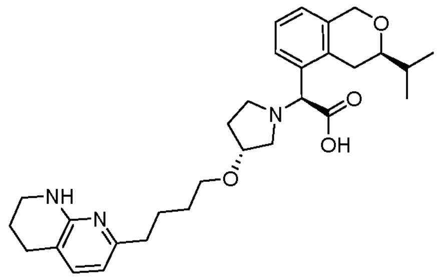
,
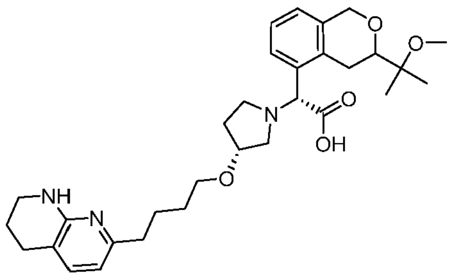
, and
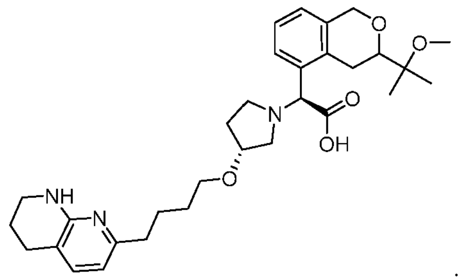
.

In certain embodiments, the invention relates to a compound selected from the group consisting of:

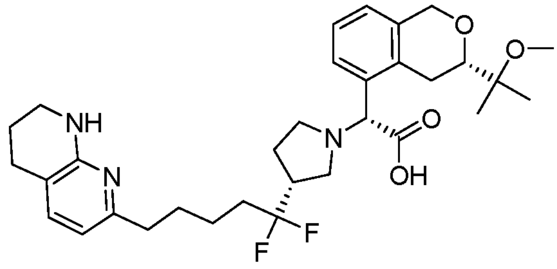,

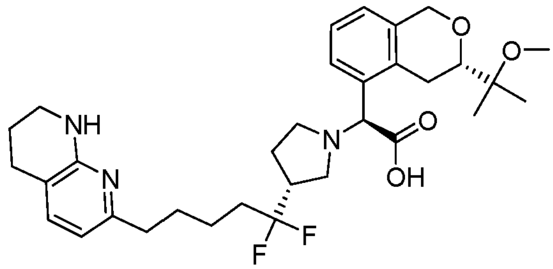, 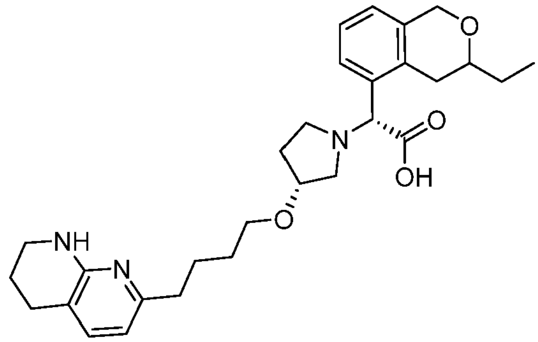,

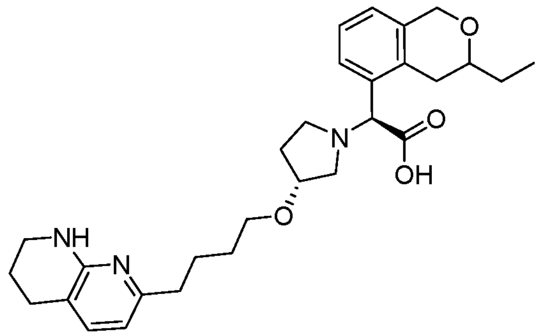, 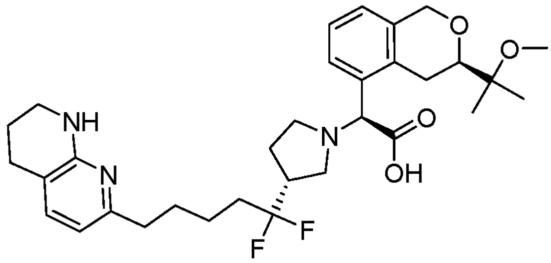,

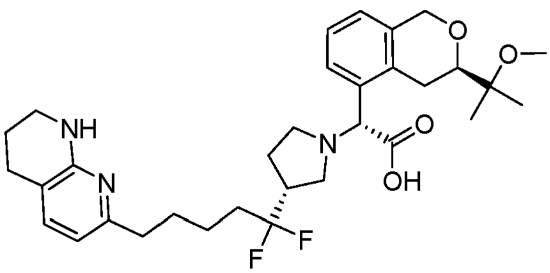, 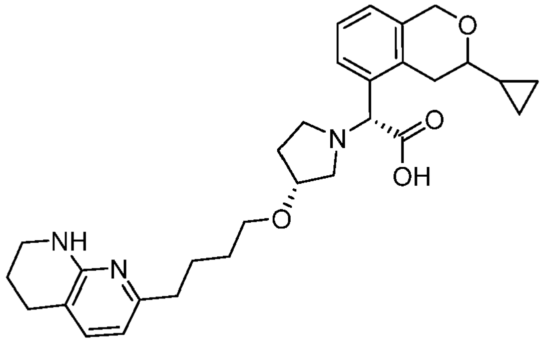,

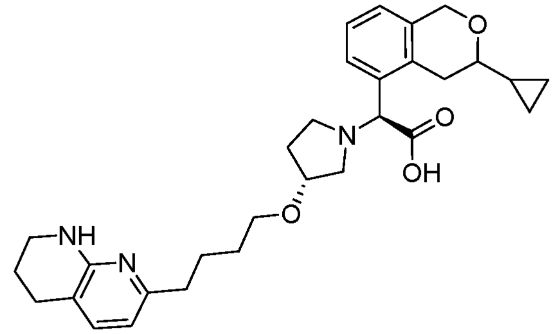
,
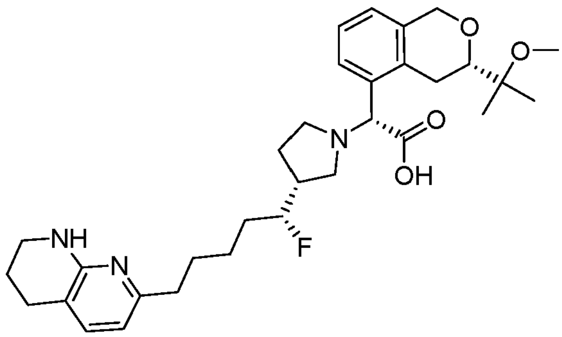
,
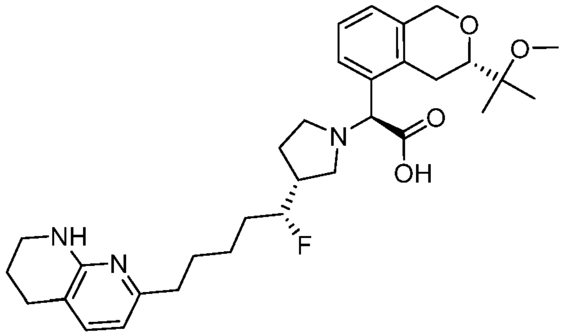
,
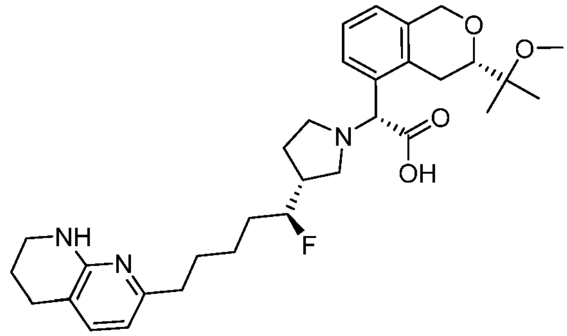
,
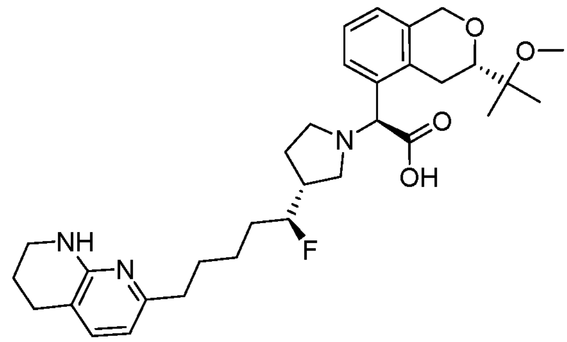
,
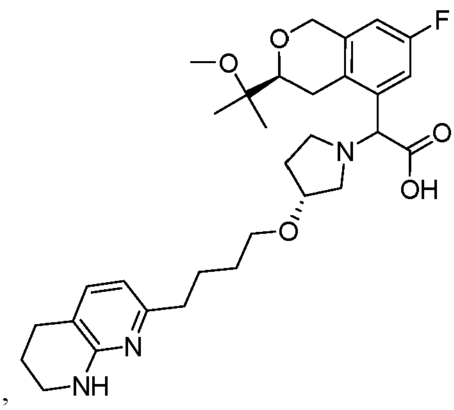
,
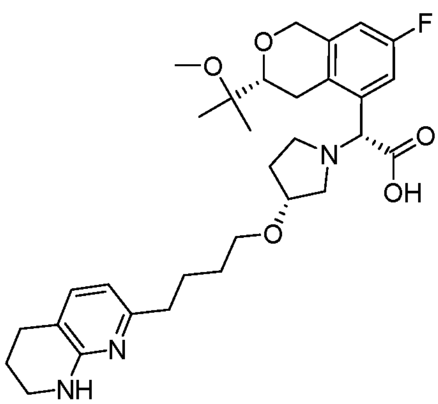
,
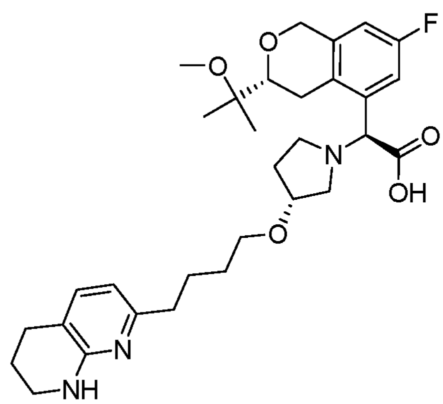
,

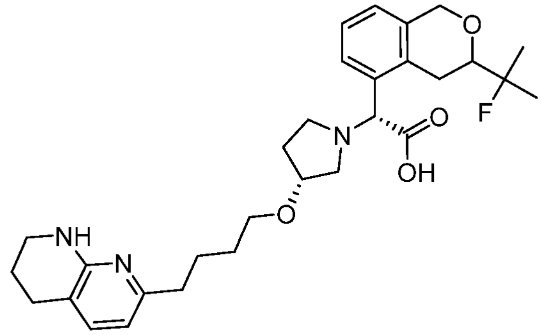

, and

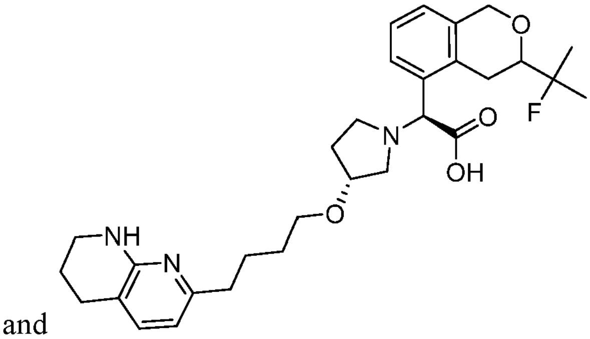

.

In certain embodiments, the invention relates to a compound selected from the group consisting of:

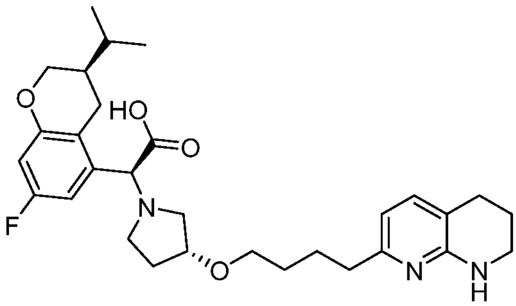

,

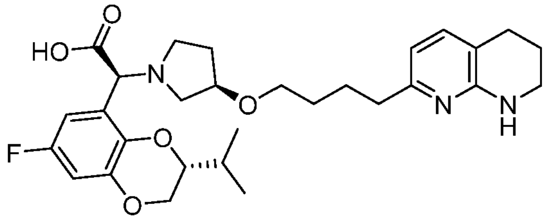

,

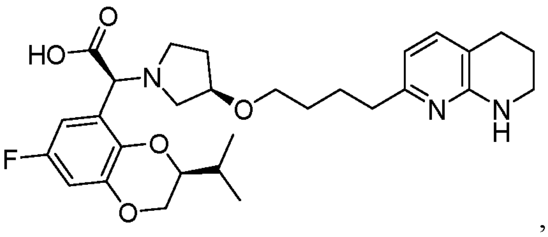

,

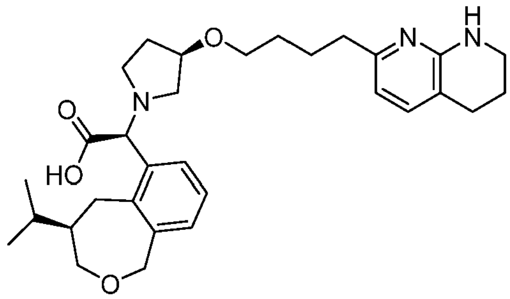

,

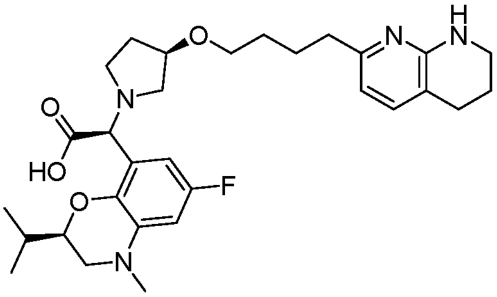

,

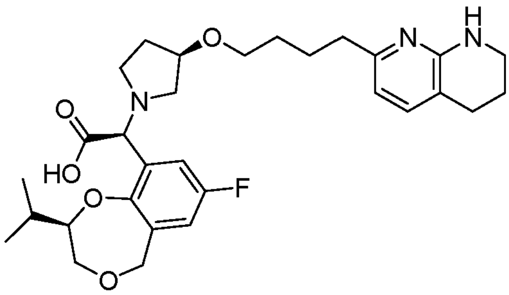

,

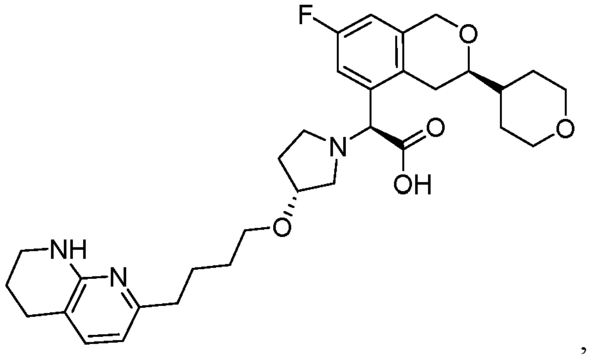

, and

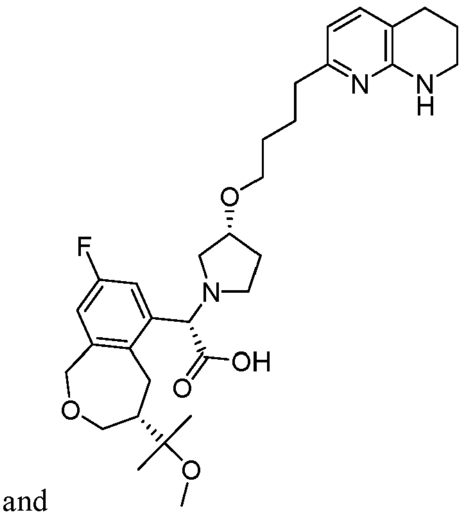

.

In certain embodiments, the invention relates to a compound selected from the group consisting of:

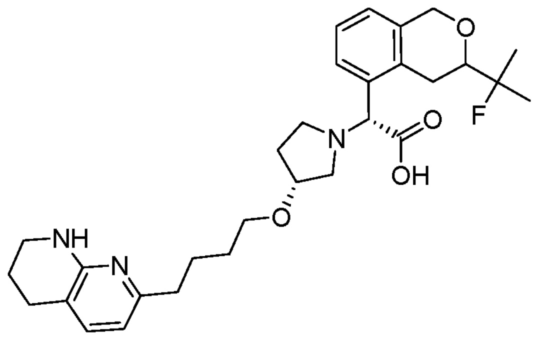

,

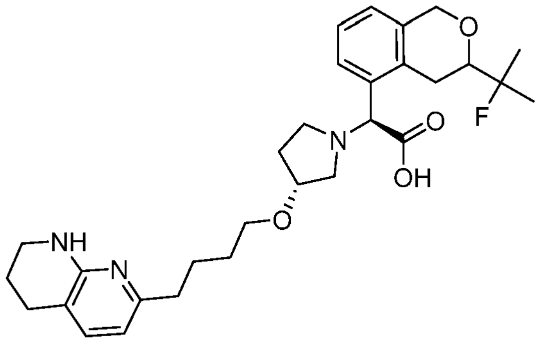

,

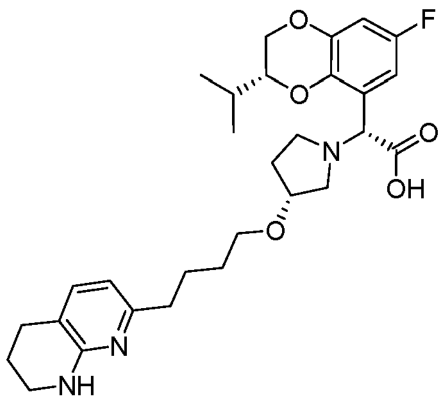

,

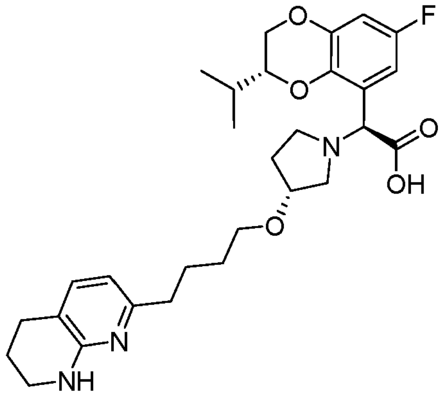,

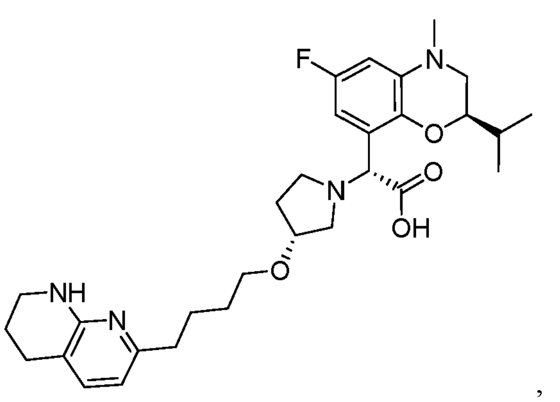,

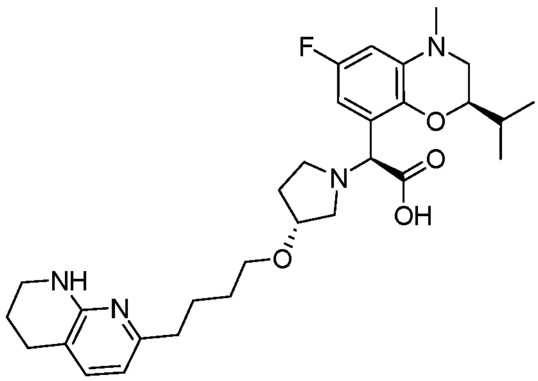,

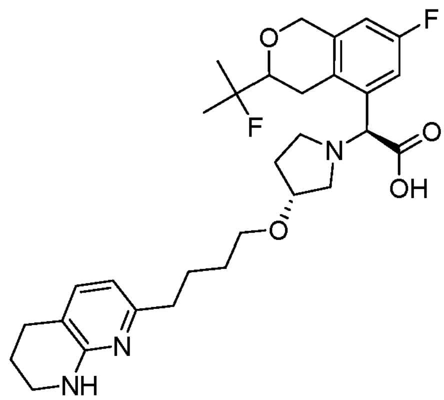,

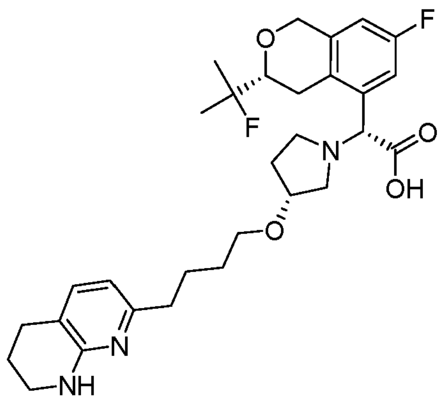,

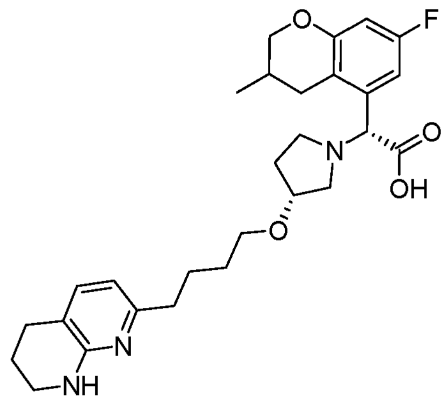,

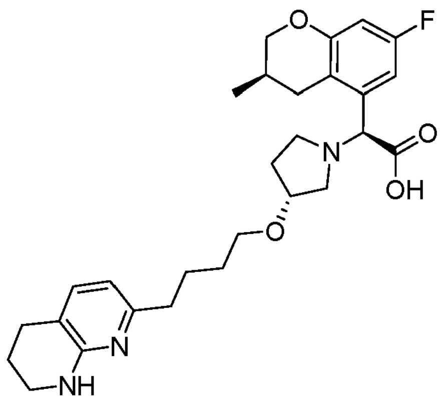,

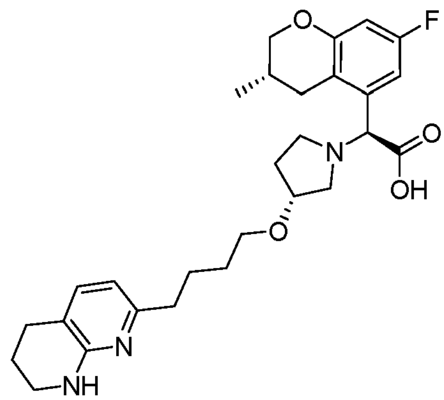,

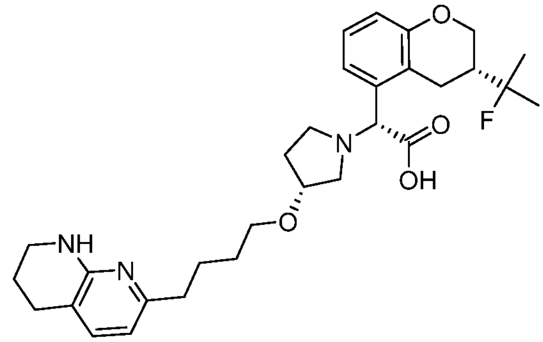,
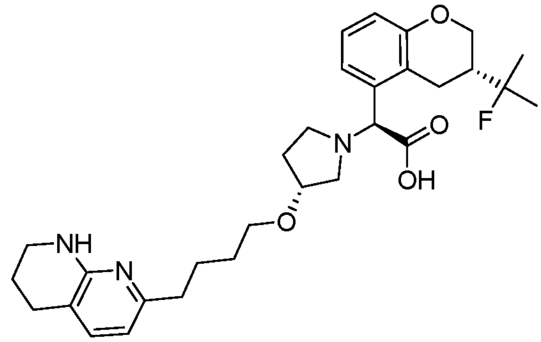,

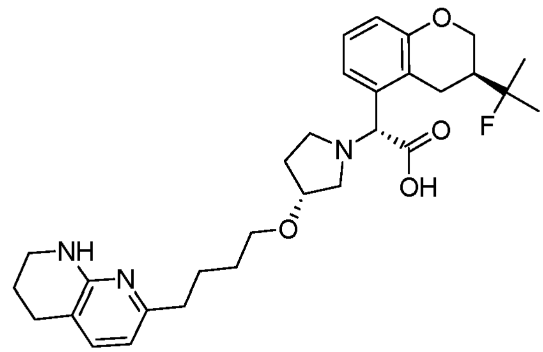,
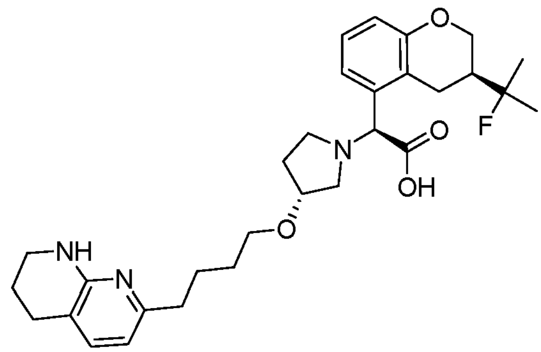,

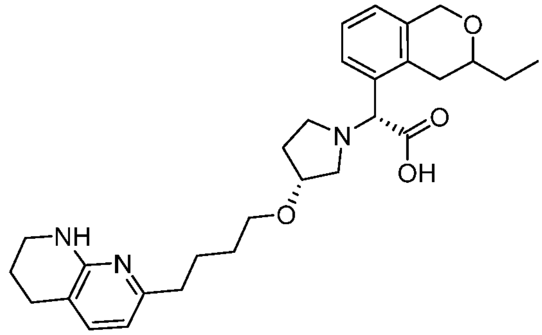,
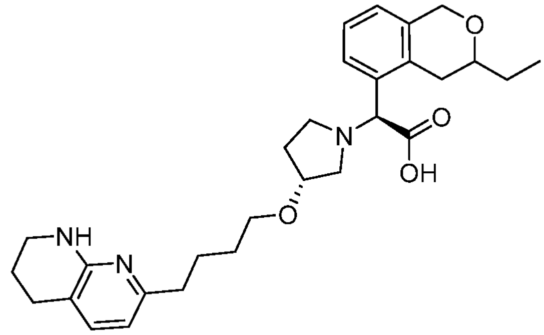,

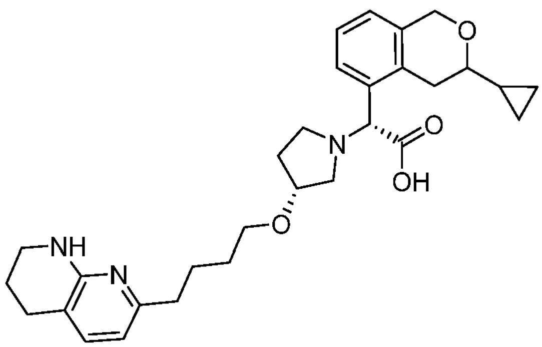,
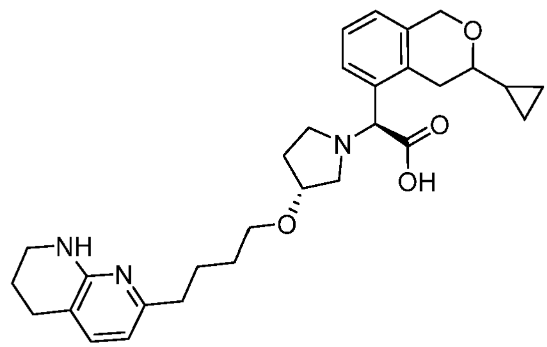,

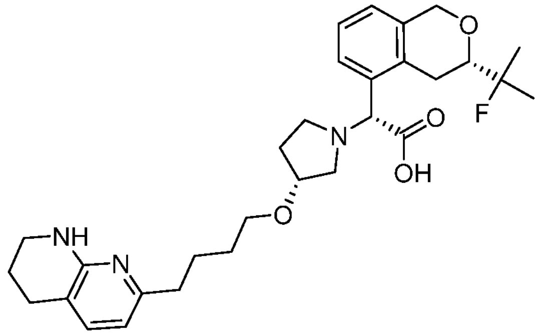,

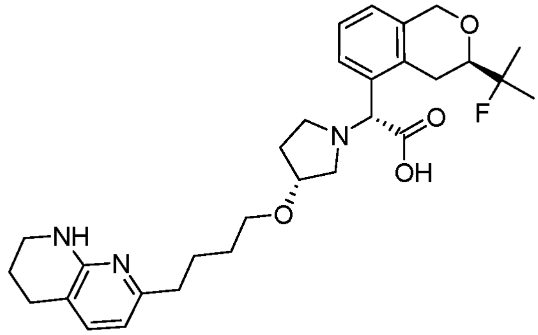,

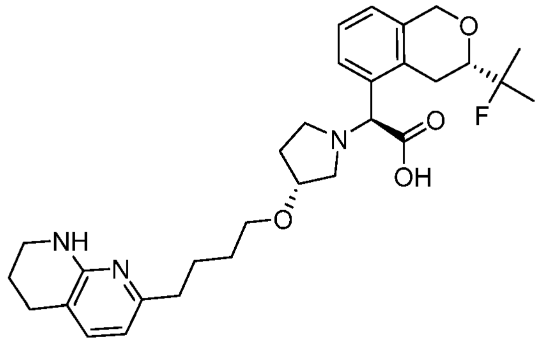,

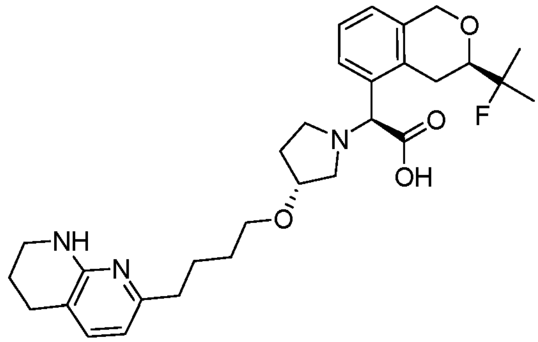,

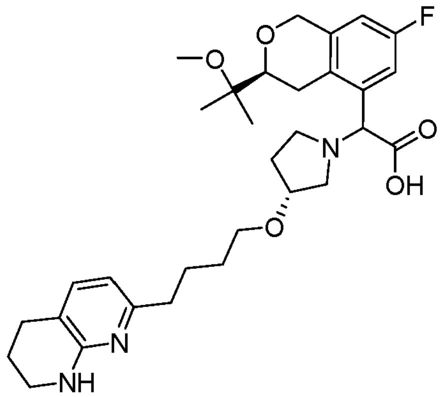,

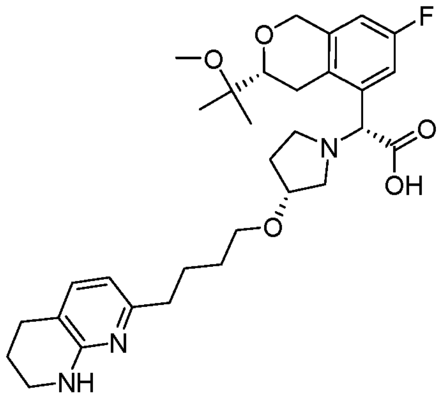,

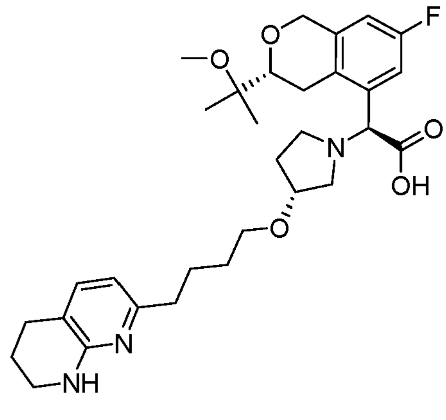,

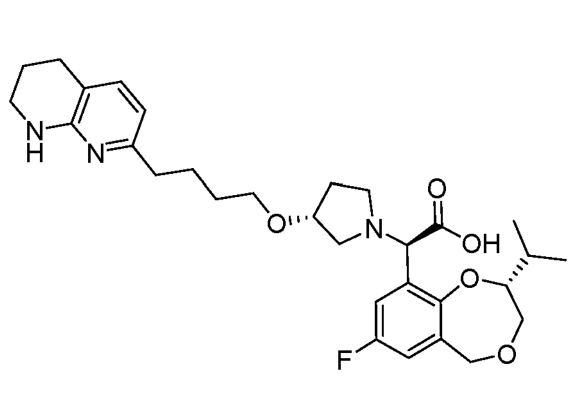,

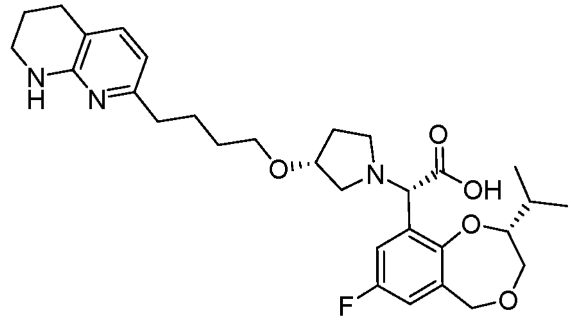
,
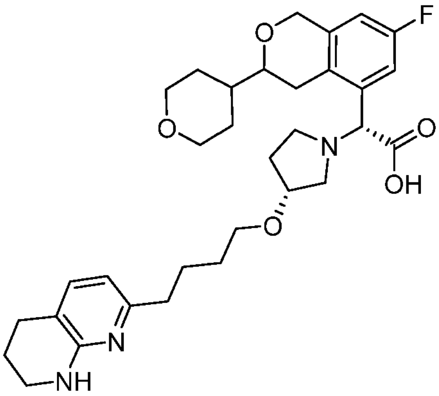
,
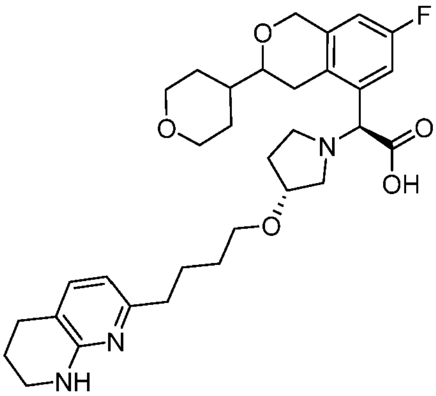
,
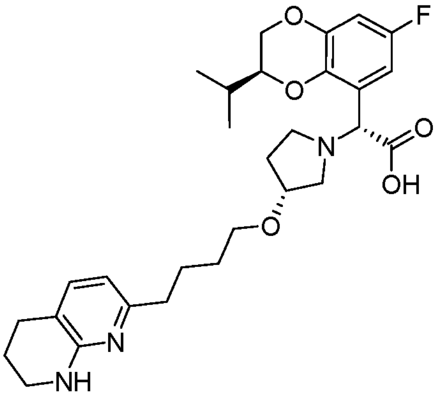
,
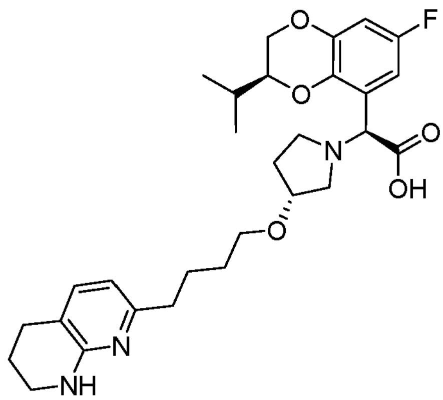
,
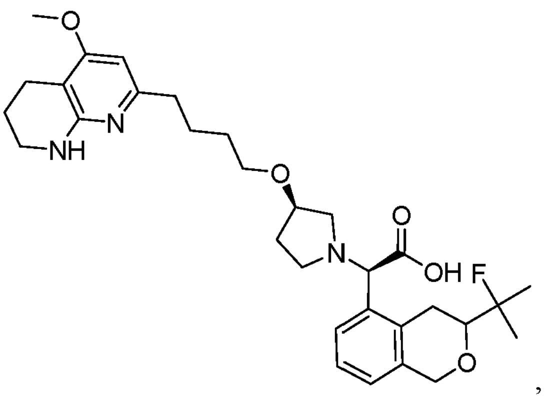
,
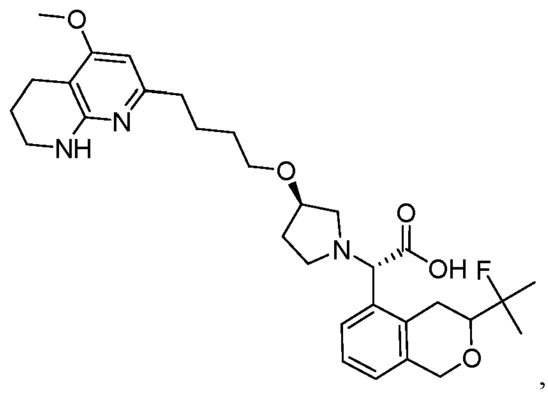
,
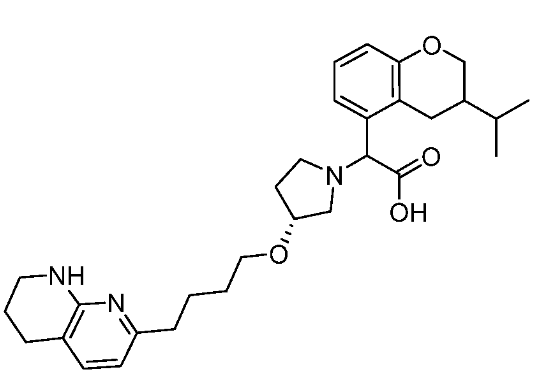
,

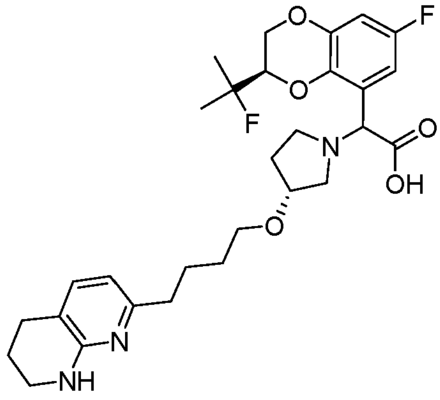
,
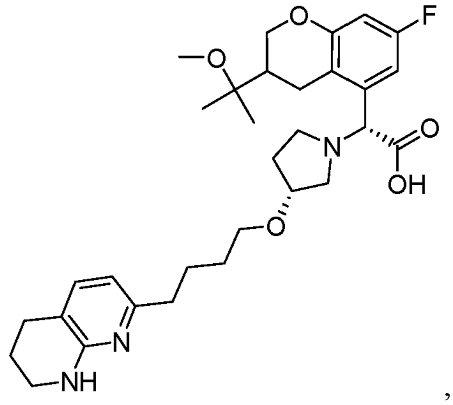
,
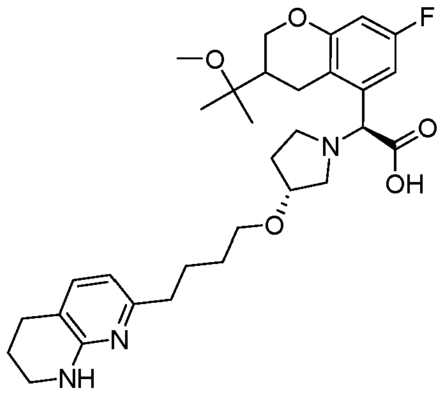
,
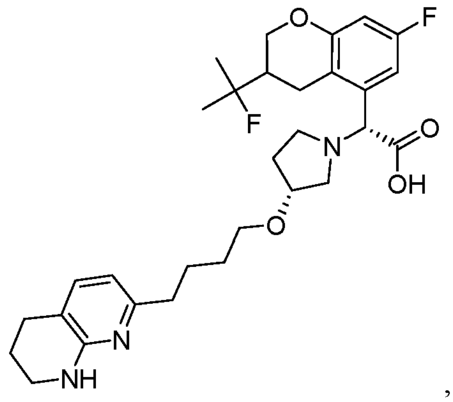
,
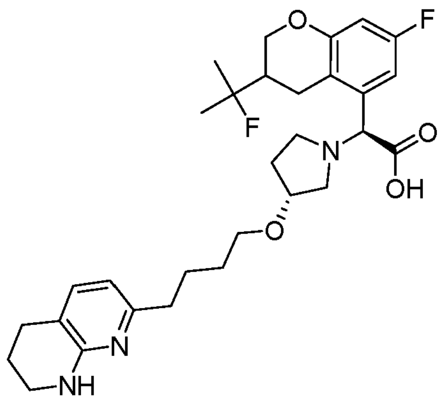
,
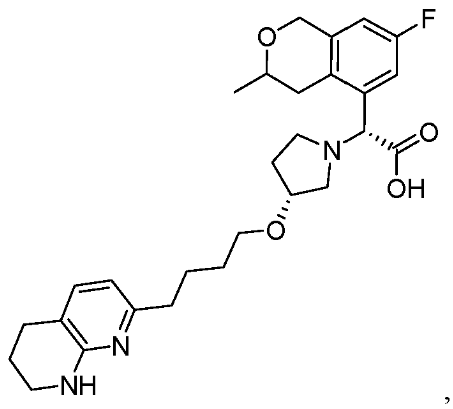
,
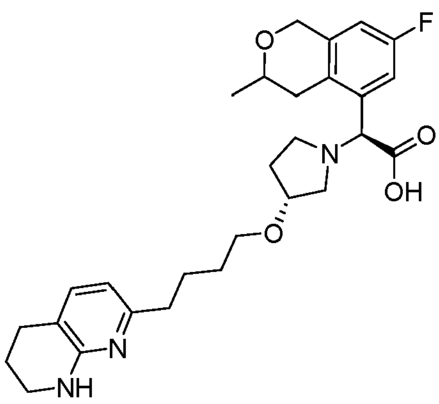
, and
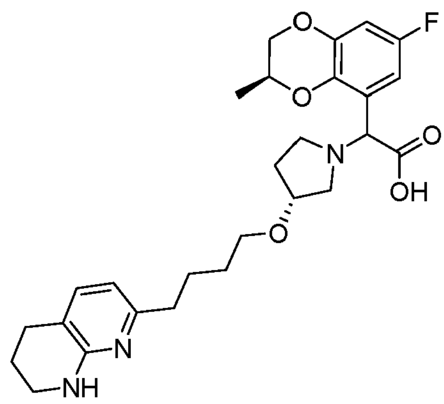
.

In certain embodiments, the invention relates to a compound selected from the group consisting of:

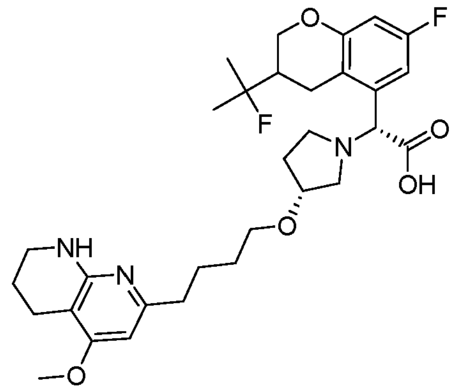
,
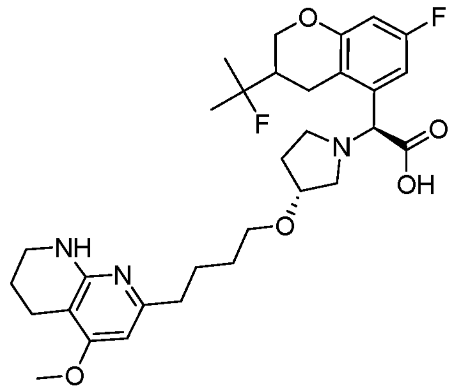
,
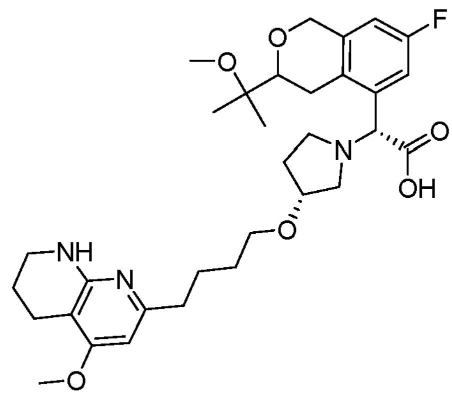
,
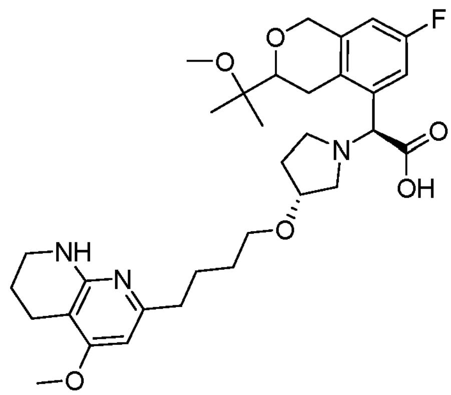
,
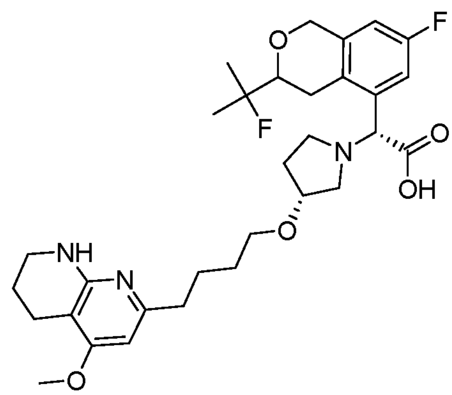
,
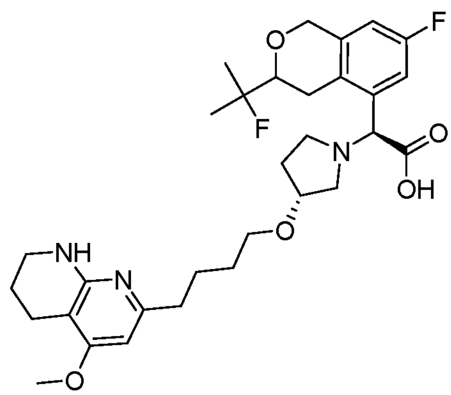
,
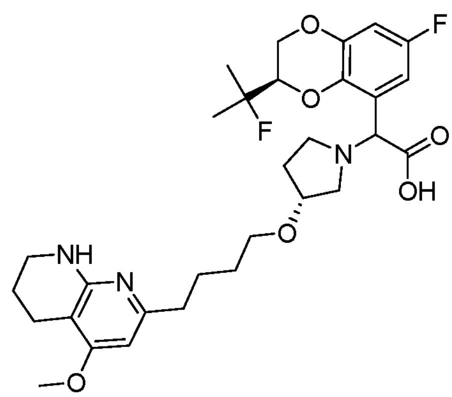
,
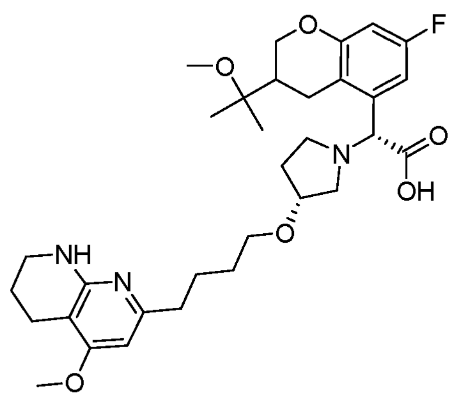
,

and

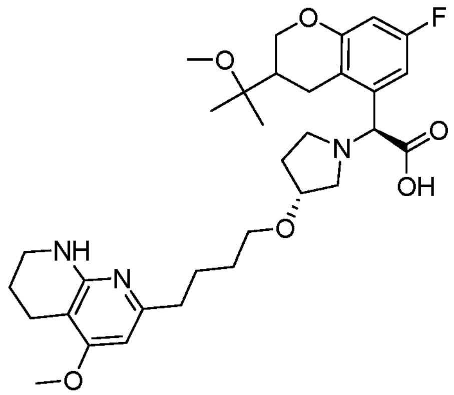

.

In certain embodiments, the invention relates to a compound selected from the group consisting of:

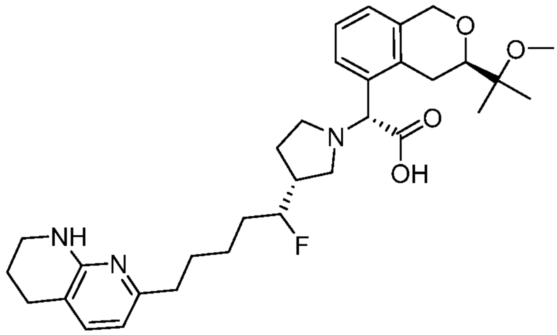

,

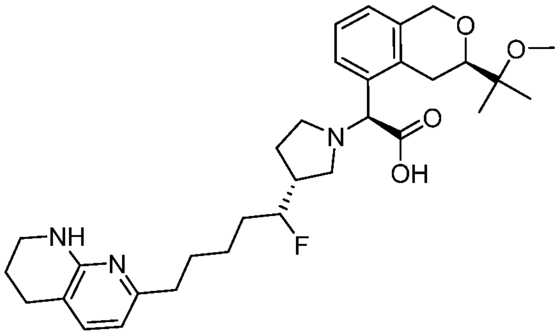

,

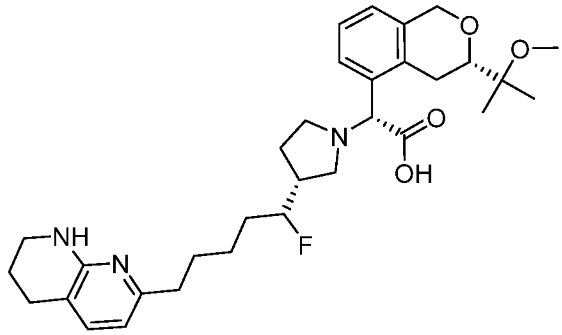

,

and

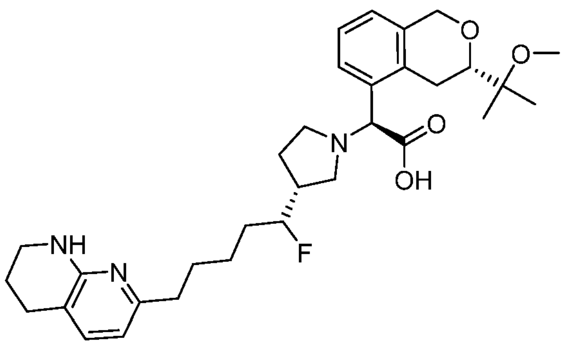

.

In certain embodiments, the invention relates to a compound selected from the group consisting of:

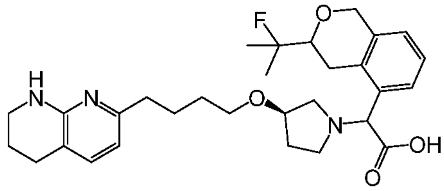,
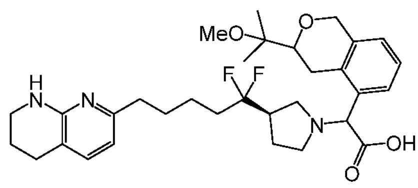,

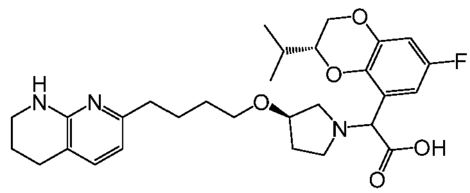,
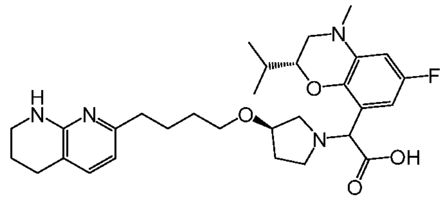,

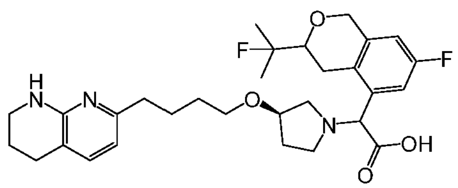,
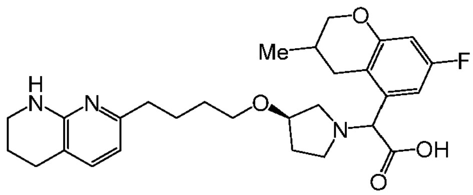,

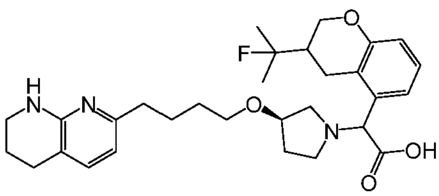, and
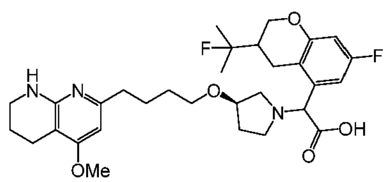.

In certain embodiments, the invention relates to a compound selected from the group consisting of:

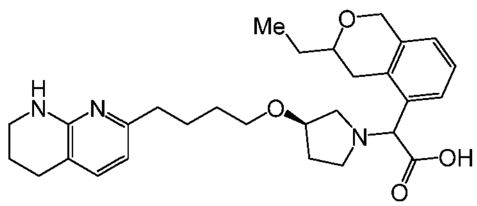,
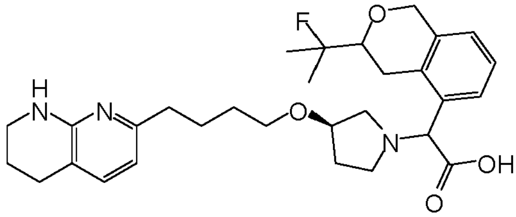,

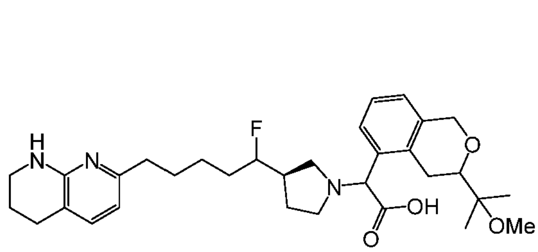,
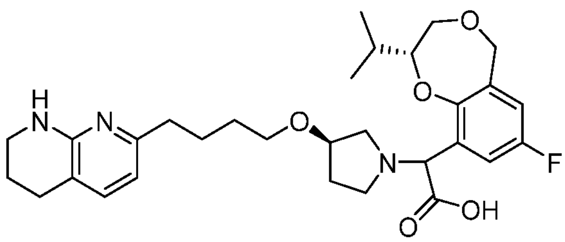,

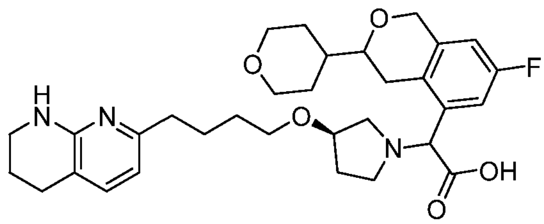, 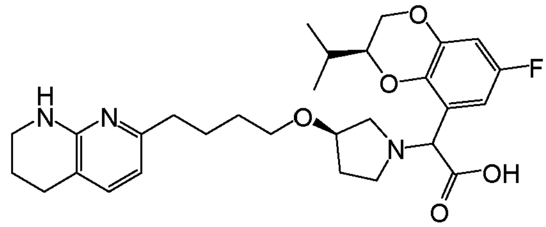,

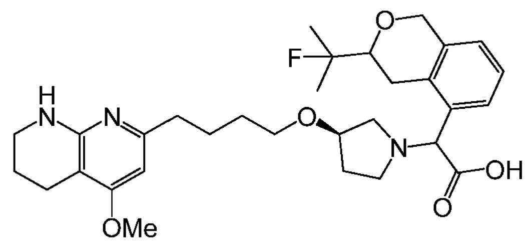, 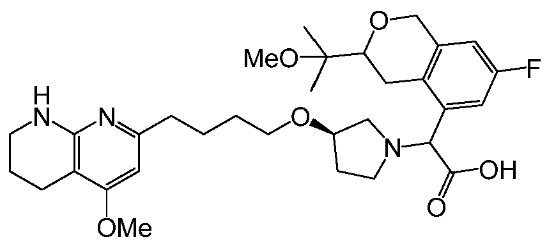,

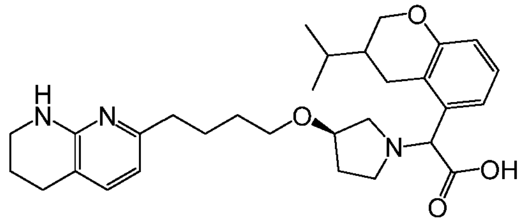, 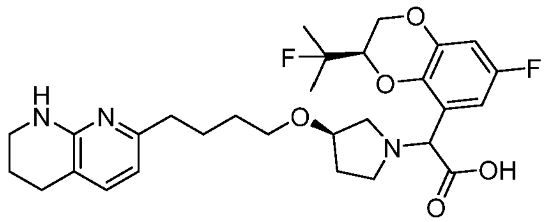,

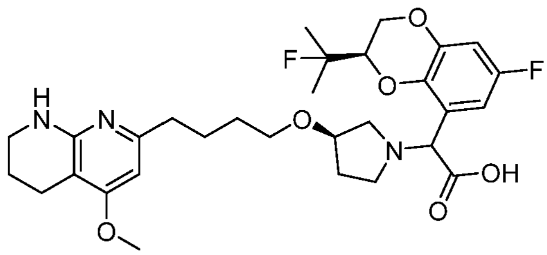, 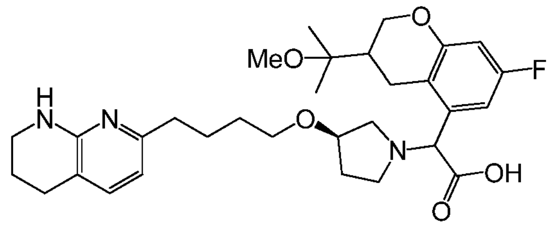,

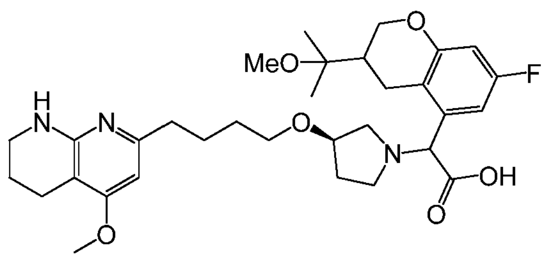, 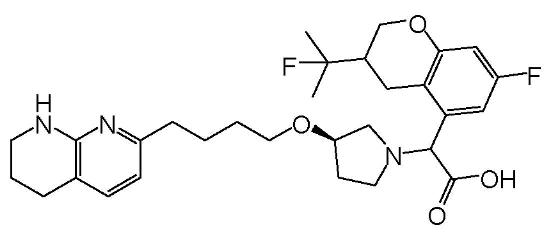,

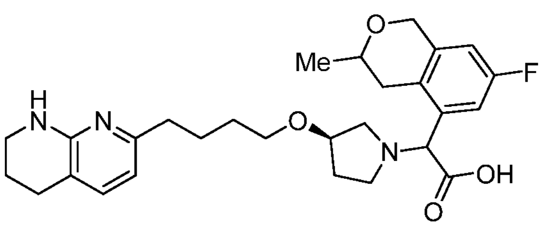, and 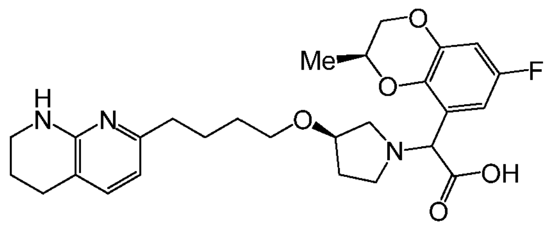.

In certain embodiments, the invention relates to a compound selected from the group consisting of:

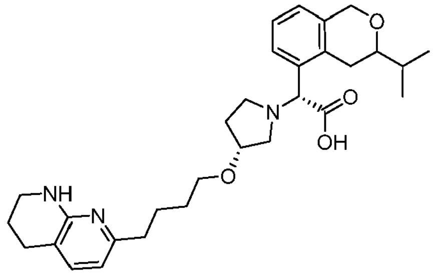
,
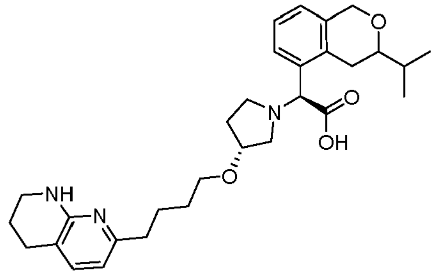
,
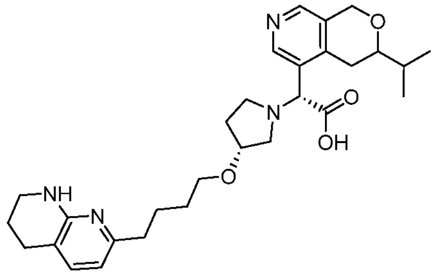
,
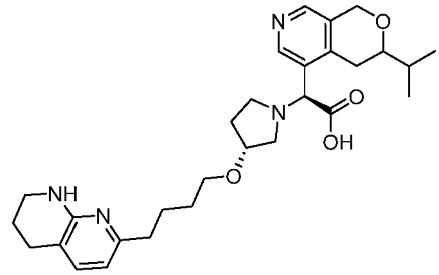
,
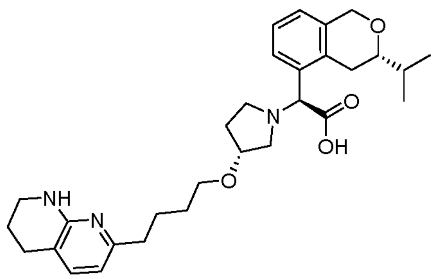
,
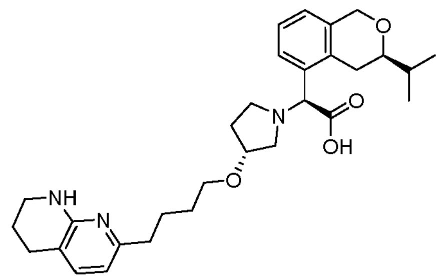
,
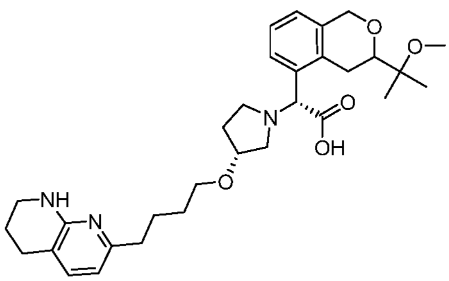
, and
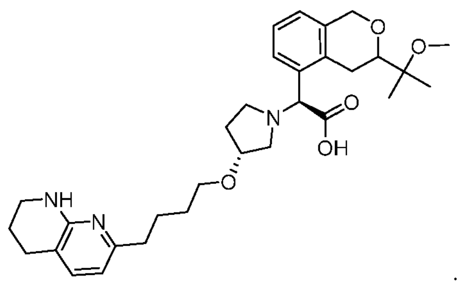
.

In certain embodiments, the invention relates to a compound selected from the group consisting of:

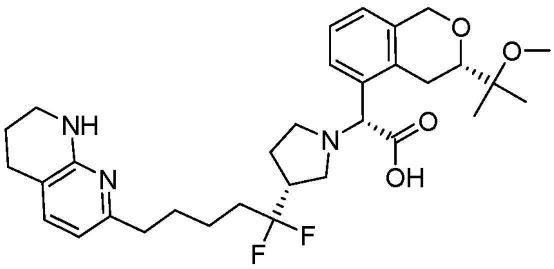
,

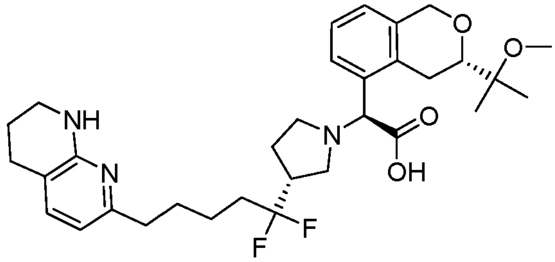

,

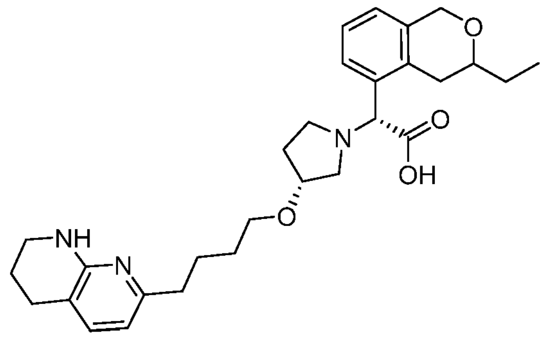

,

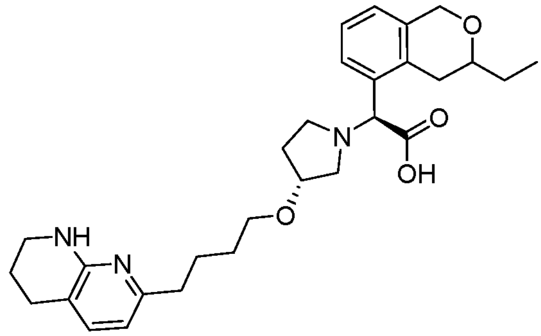

,

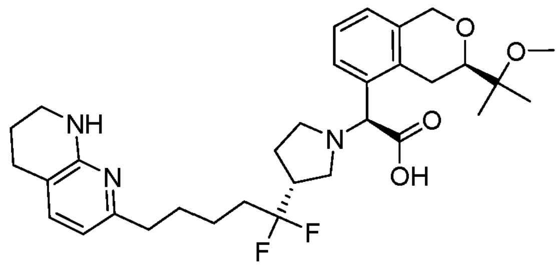

,

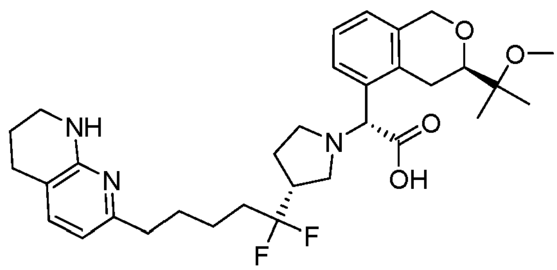

,

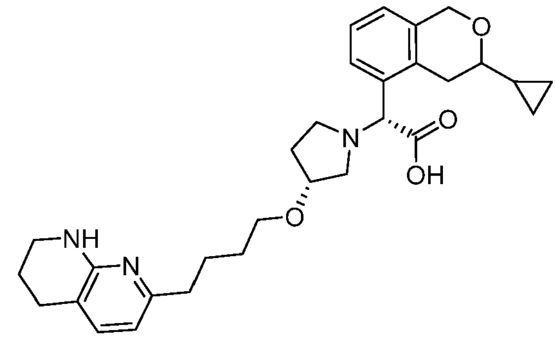

,

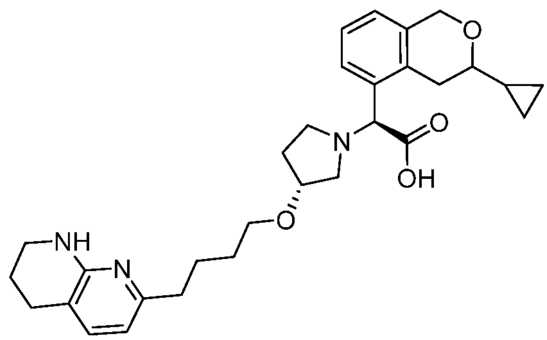

,

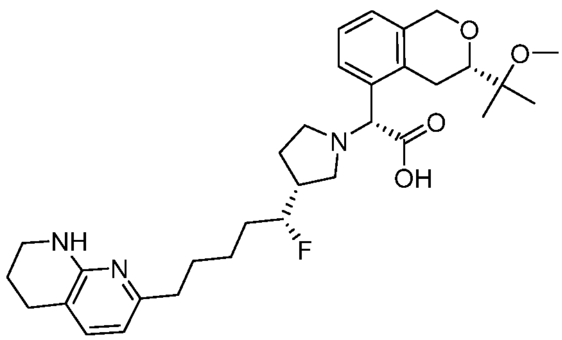

,

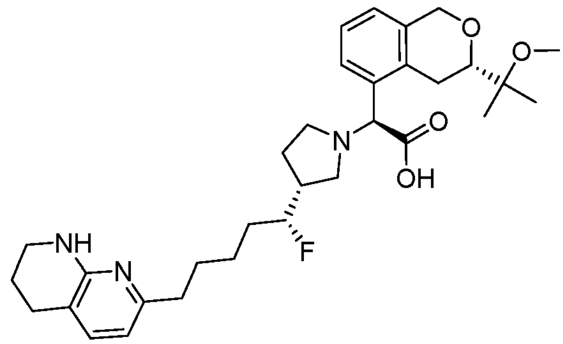

,

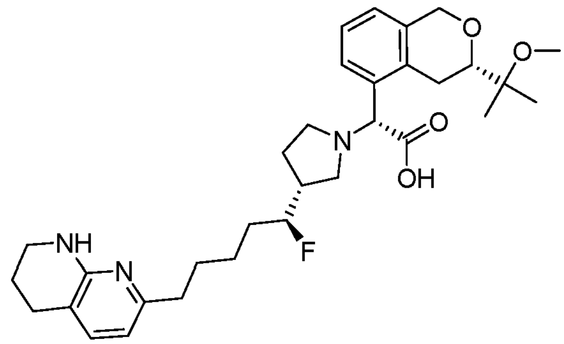

,

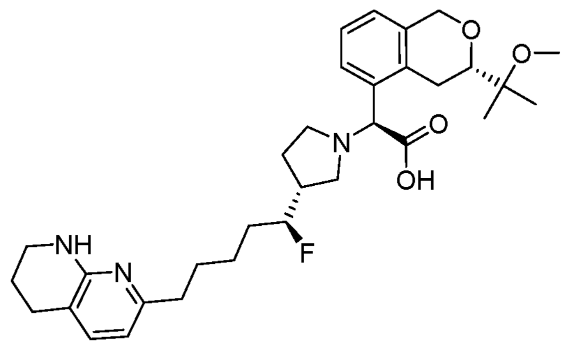

,

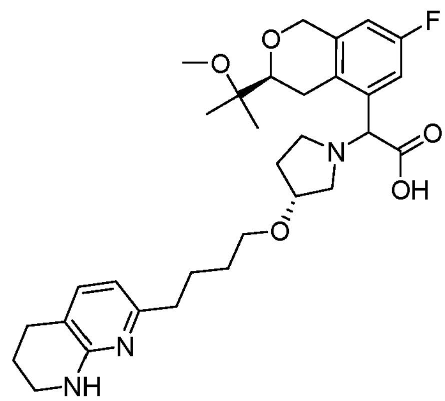

,

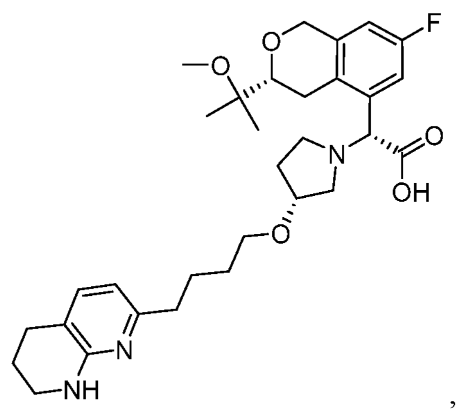

,

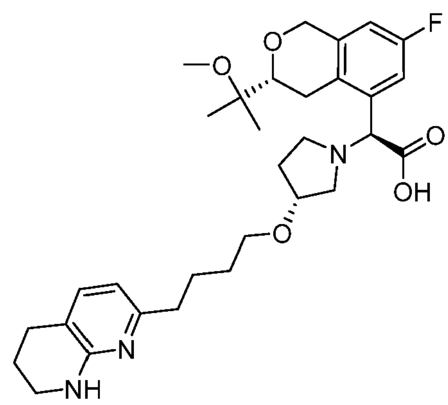

,

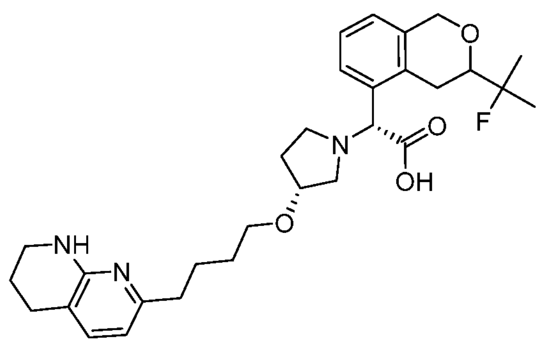

, and

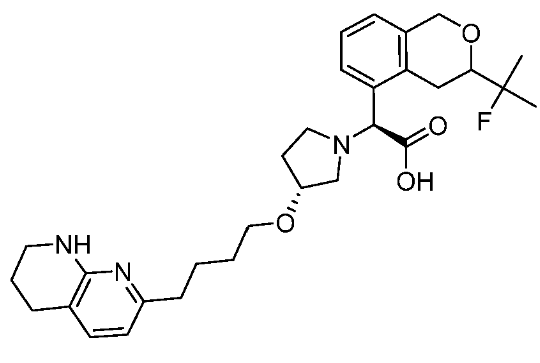

.

In certain embodiments, the invention relates to a compound selected from the group consisting of:

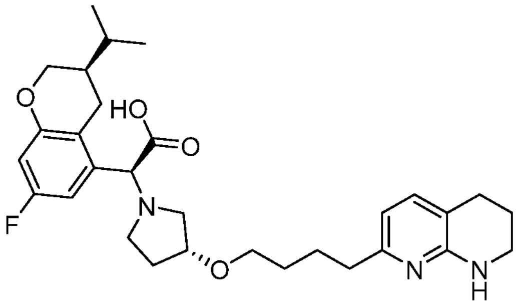 , 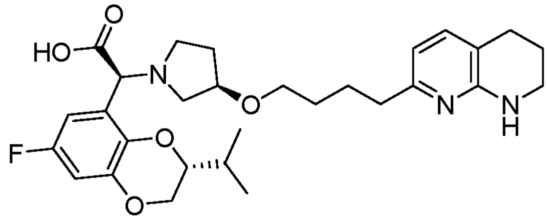 ,

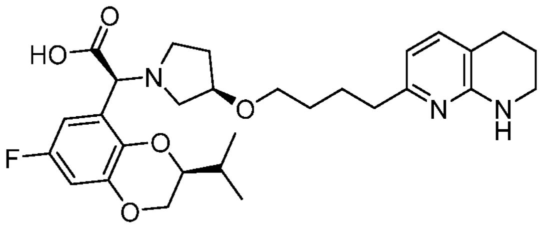 , 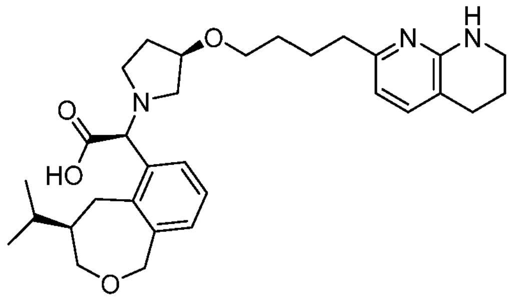 ,

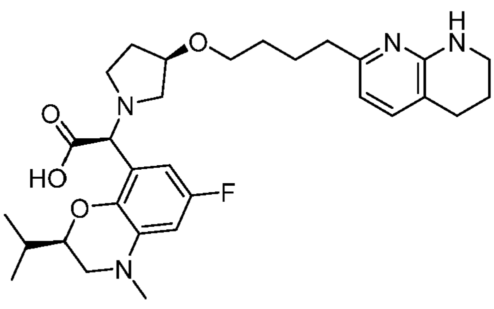 , 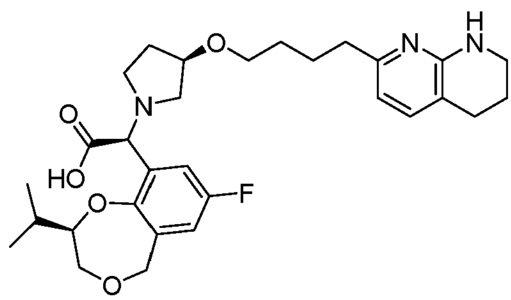 ,

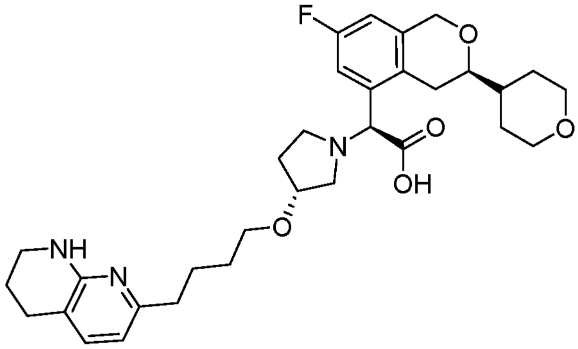 , and 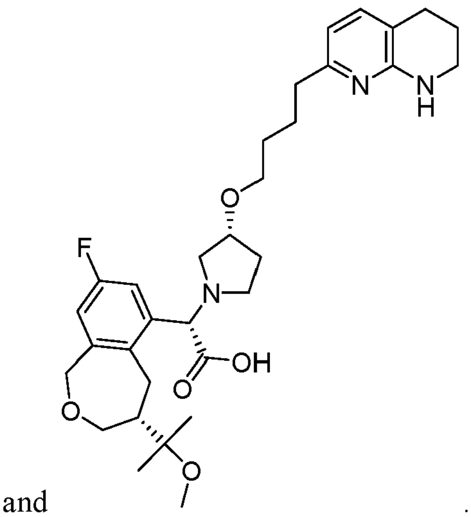 .

In certain embodiments, the invention relates to a compound selected from the group consisting of:

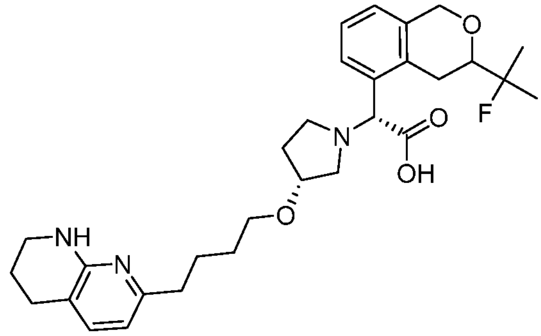

,

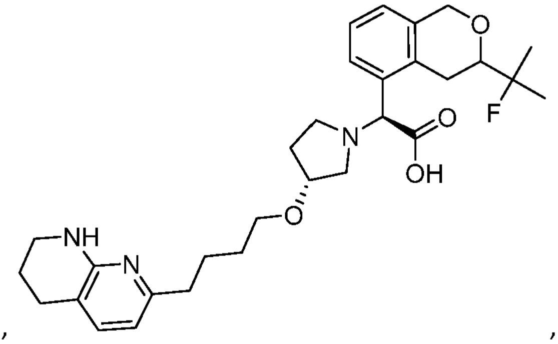

,

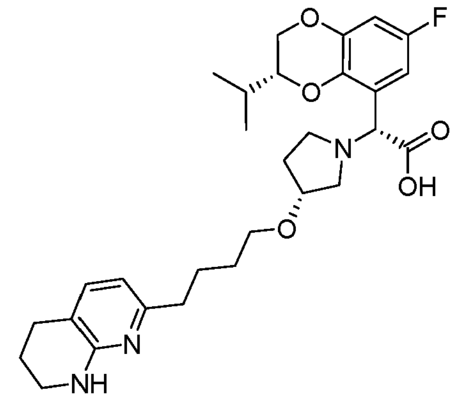

,

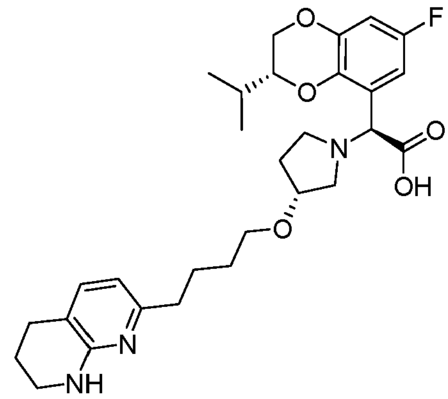

,

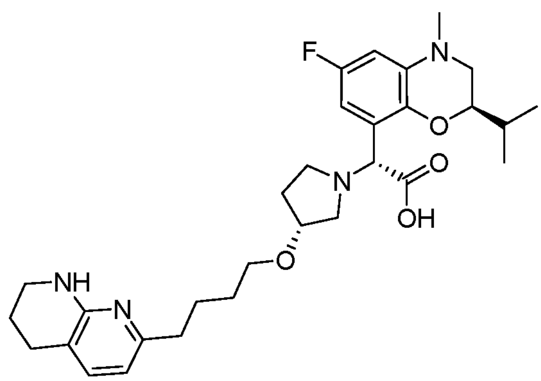

,

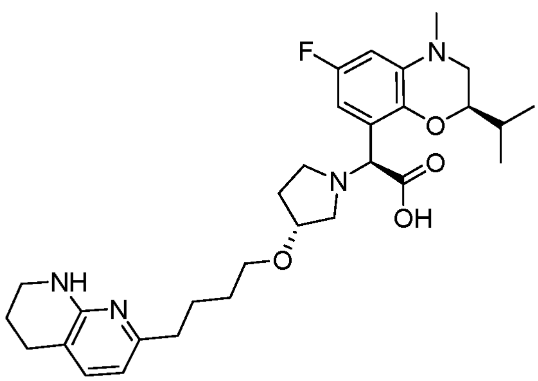

,

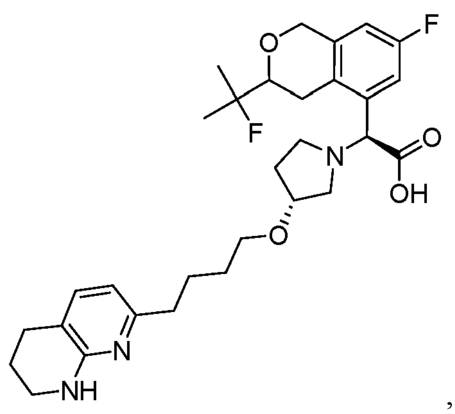

,

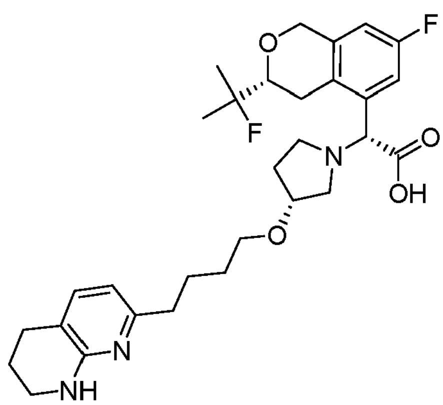

,

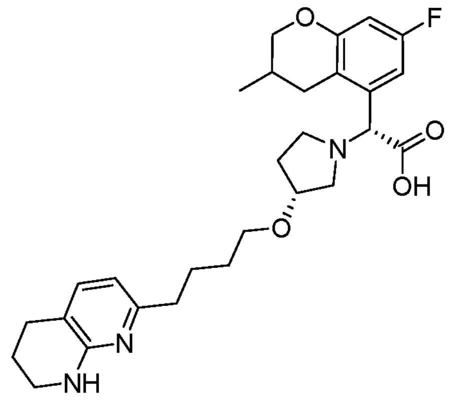

,

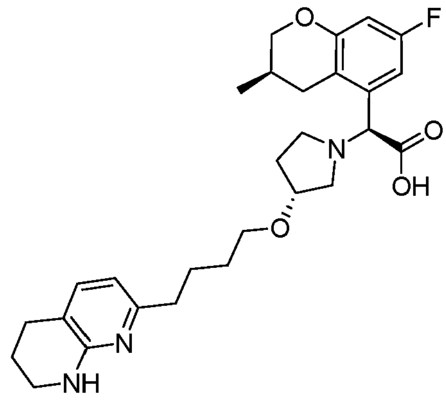

,

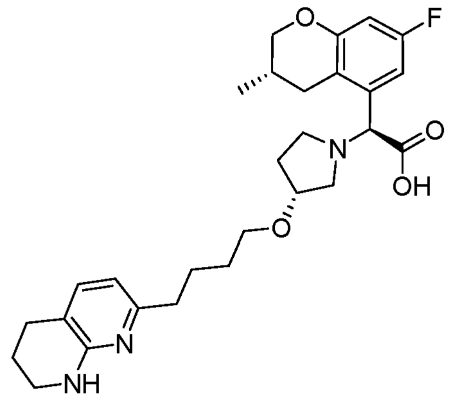

,

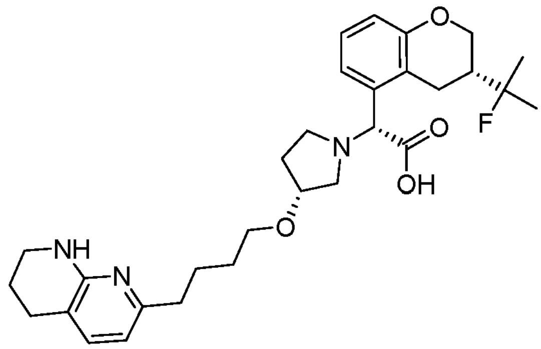

,

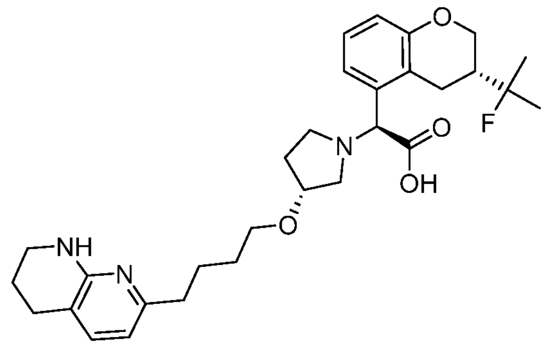

,

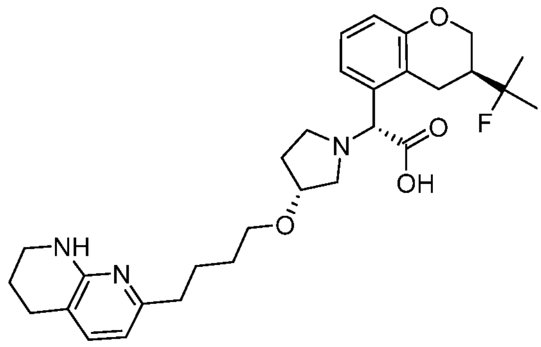

,

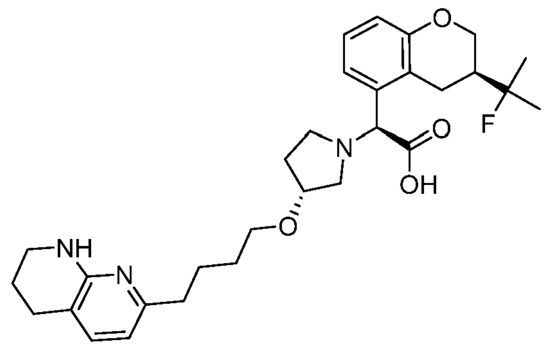

,

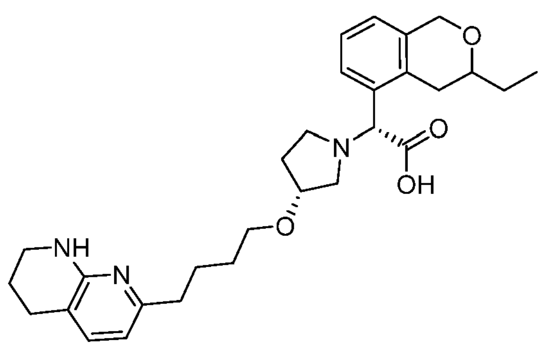

,

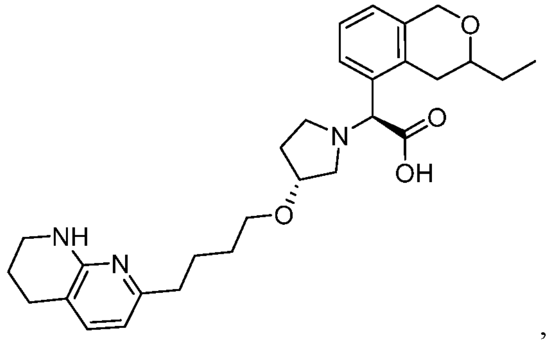
,

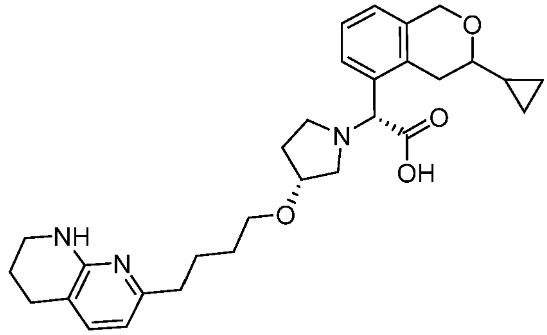
,

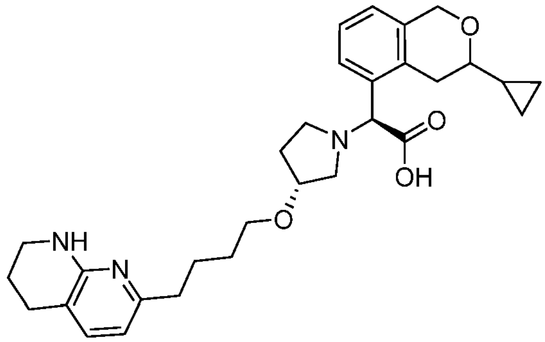
,

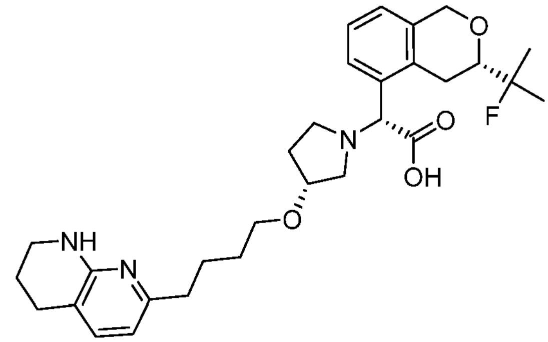
,

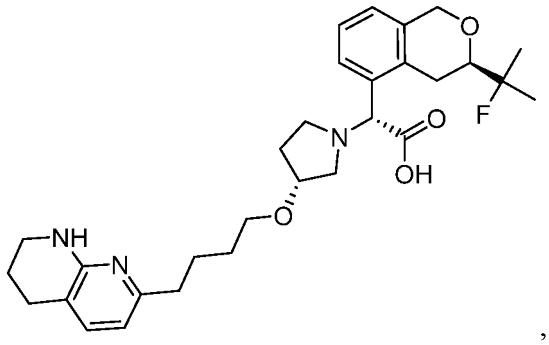
,

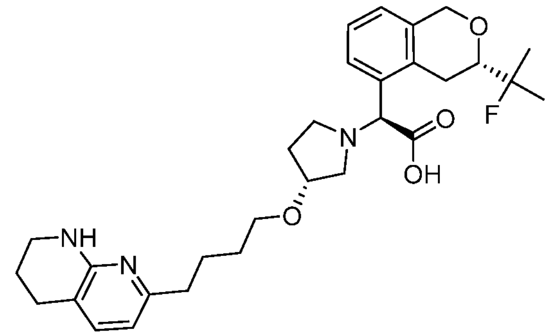
,

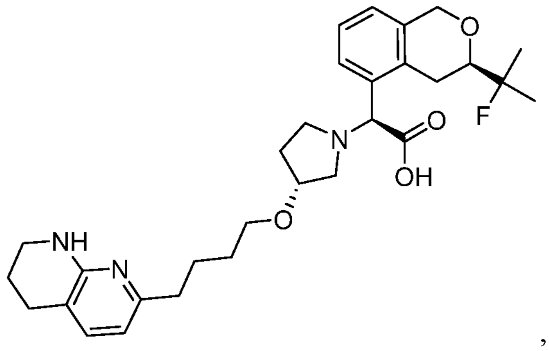
,

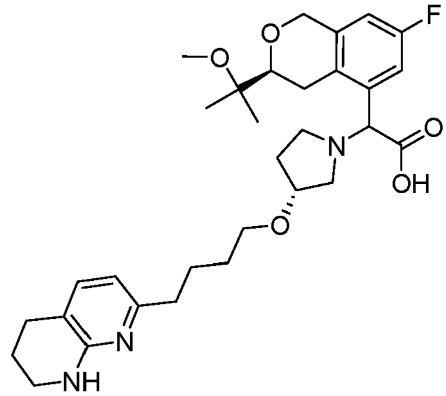
,

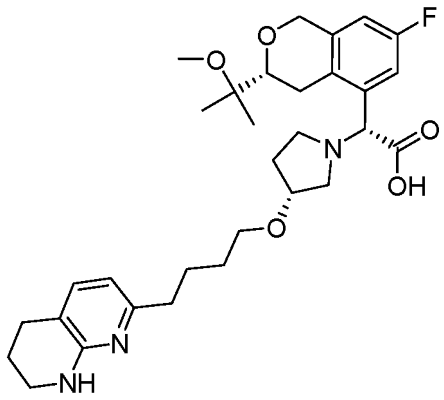

,

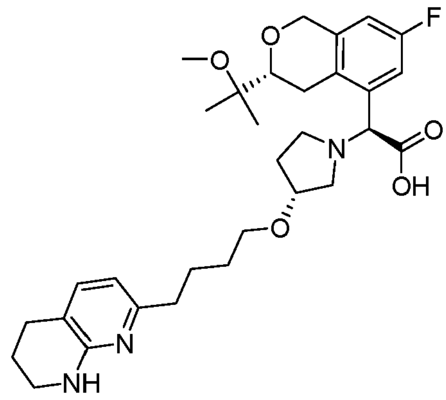

,

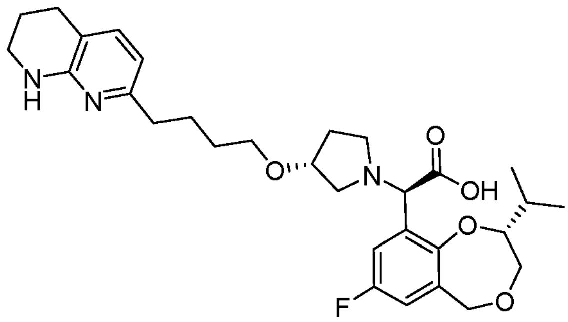

,

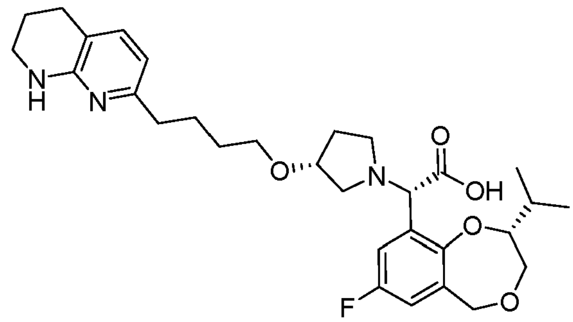

,

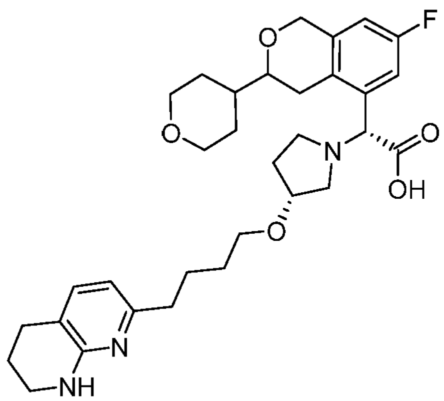

,

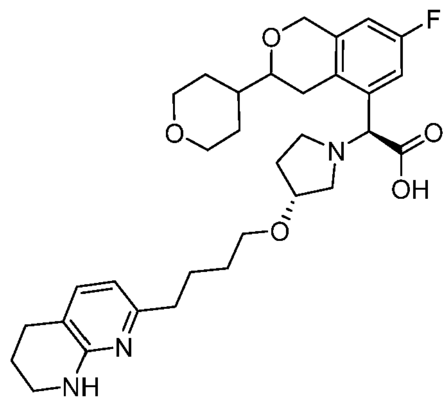

,

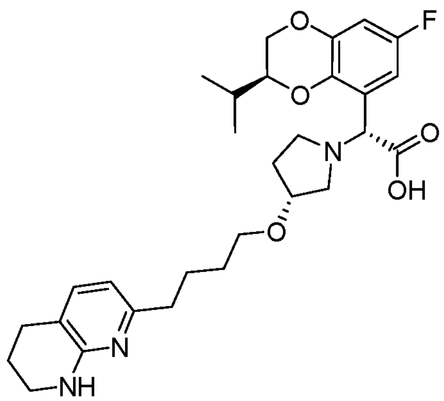

,

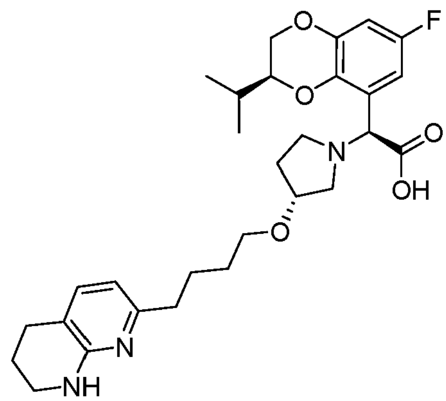

,

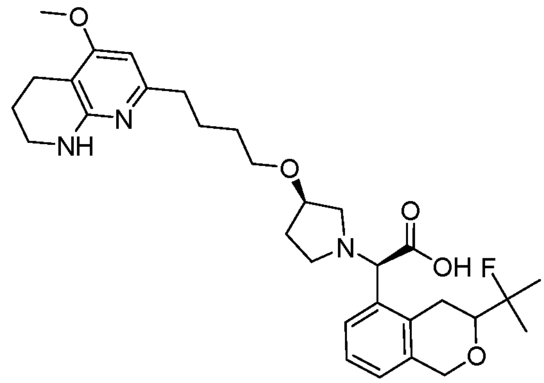
,
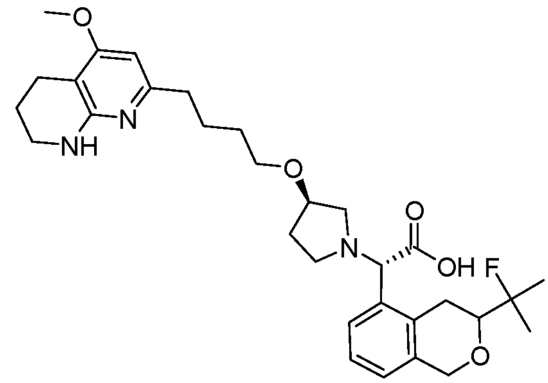
,
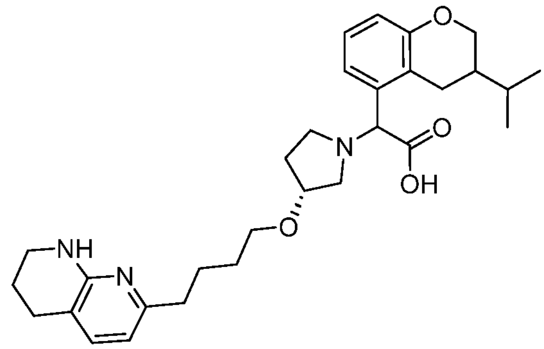
,
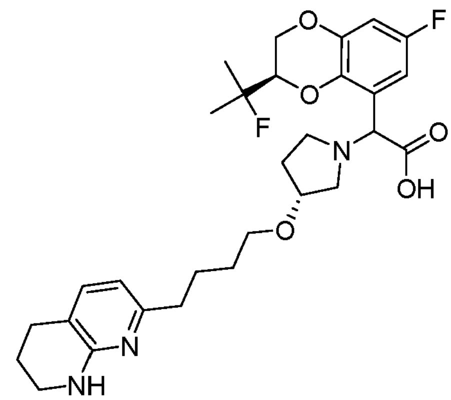
,
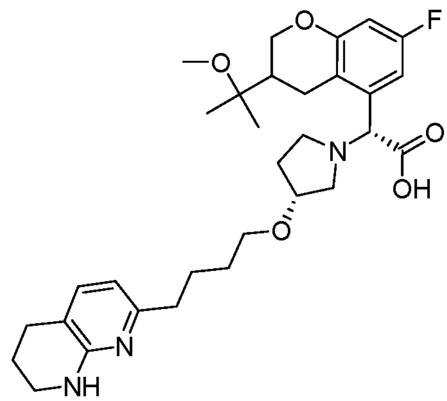
,
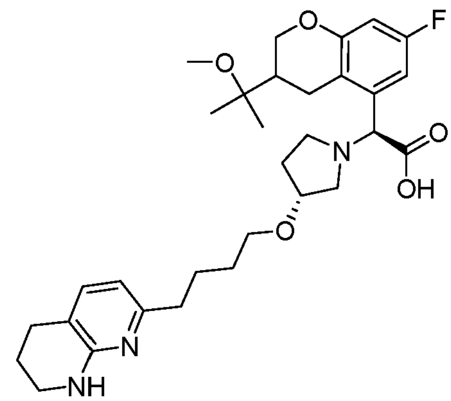
,
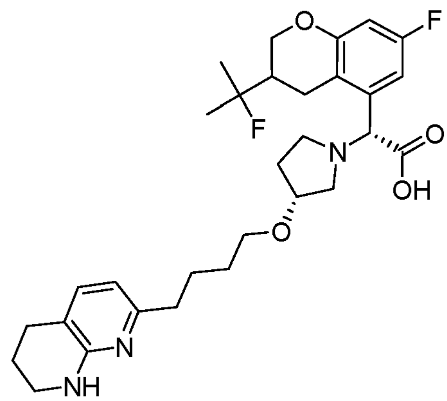
,
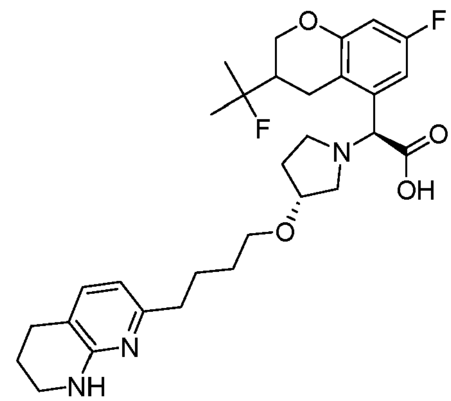
,

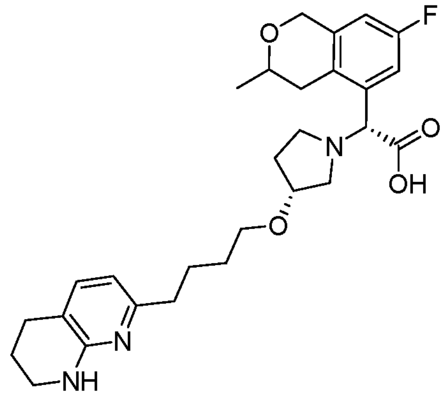,
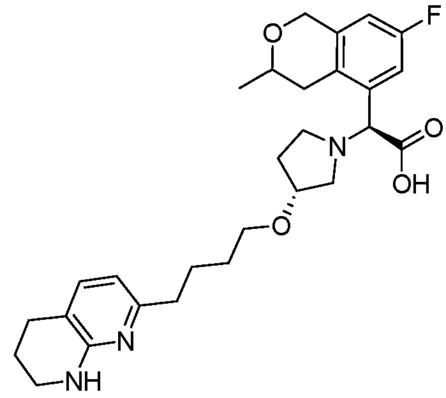,

and

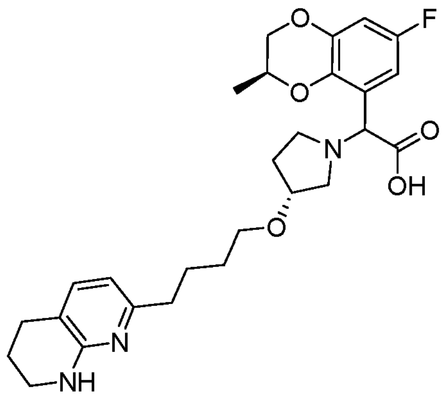.

In certain embodiments, the invention relates to a compound selected from the group consisting of:

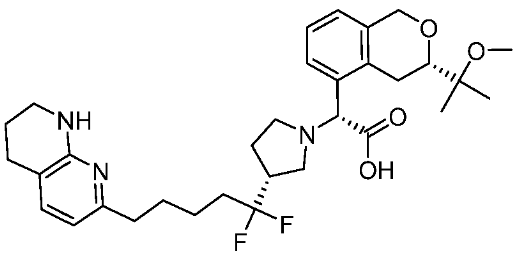,
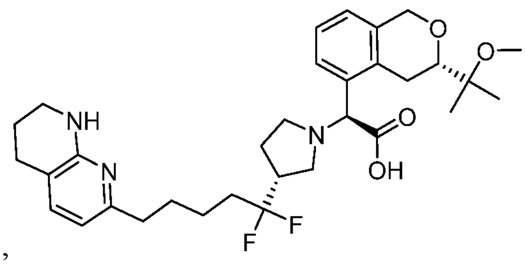,
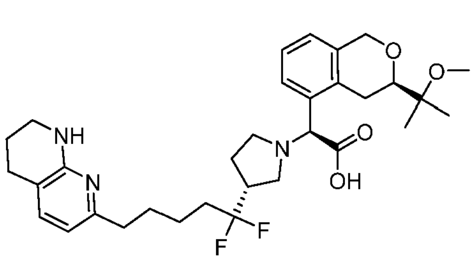, and
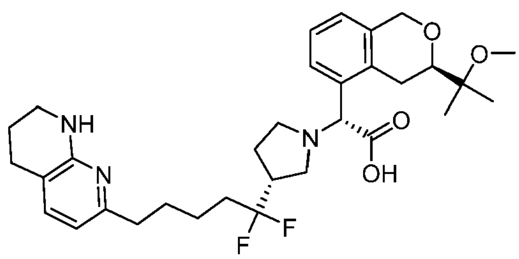.

In certain embodiments, the invention relates to a compound selected from the group consisting of:

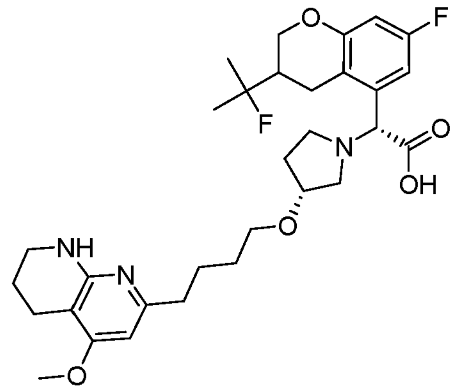
,

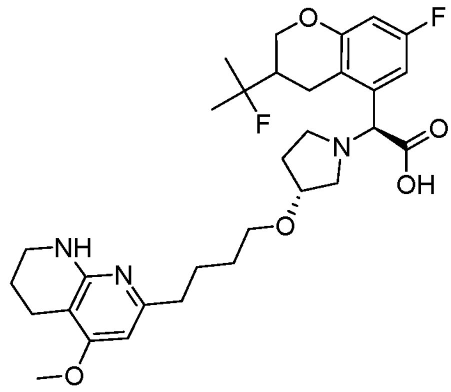
,

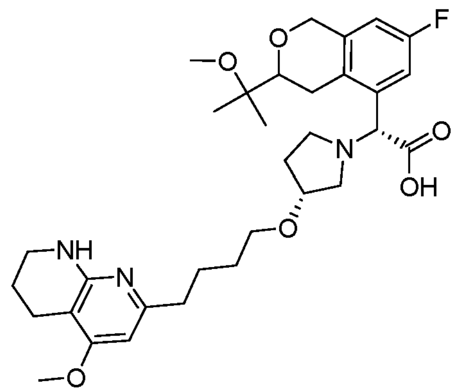
,

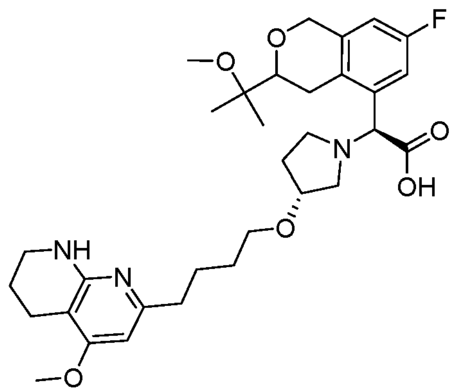
,

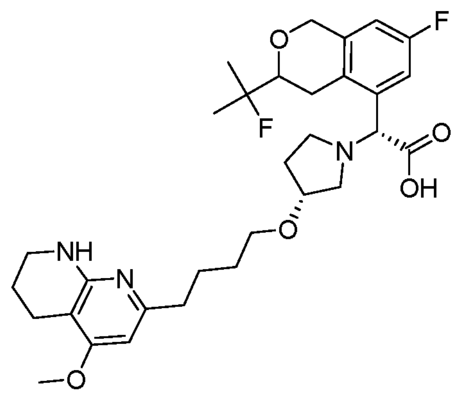
,

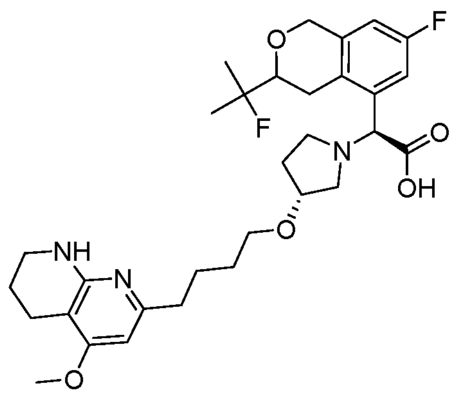
,

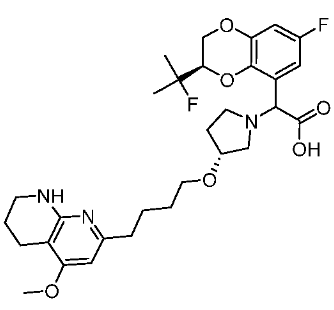
,

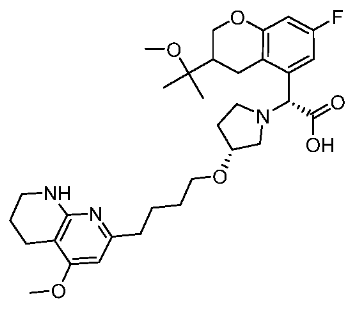
,

and

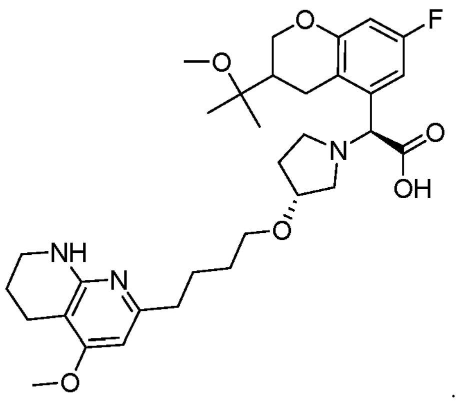

.

In certain embodiments, the invention relates to a compound selected from the group consisting of:

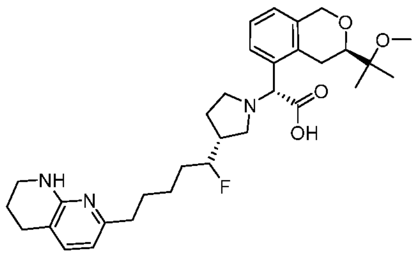

,

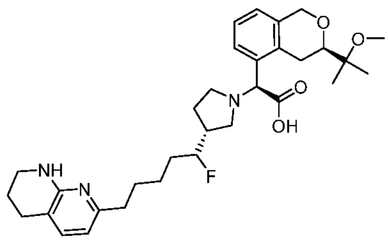

,

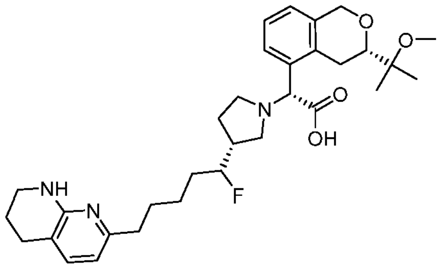

, and

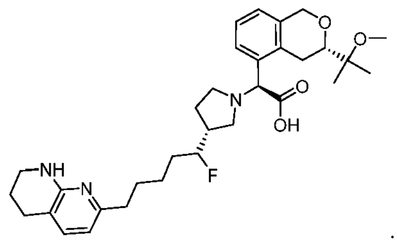

.

In certain embodiments, the invention relates to a compound selected from the group consisting of:

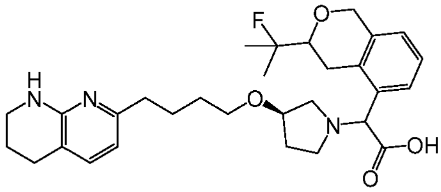

,

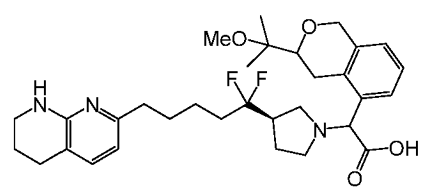

,

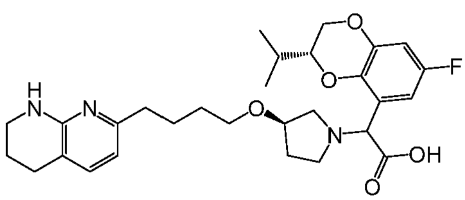

,

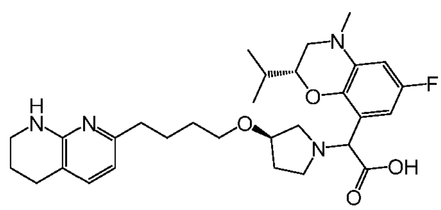

,

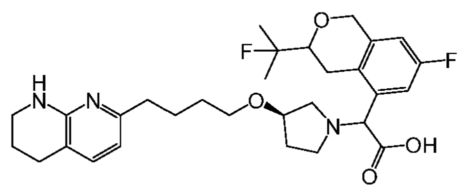,

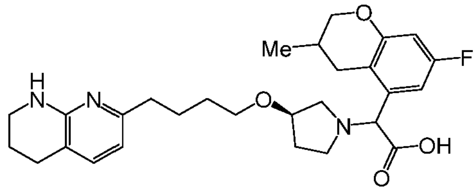,

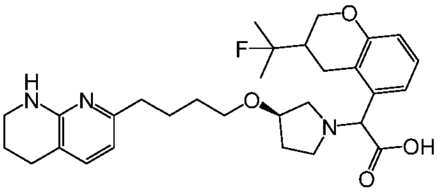, and 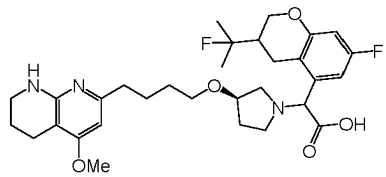.

In certain embodiments, the invention relates to a compound selected from the group consisting of:

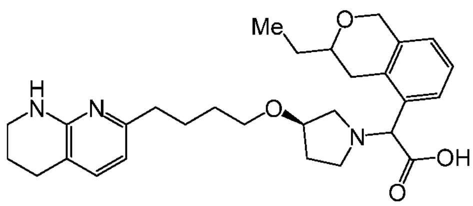,

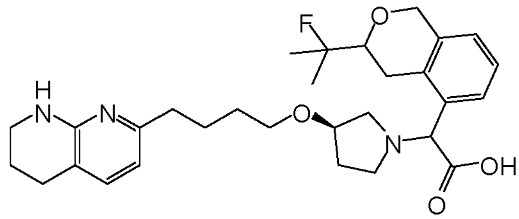,

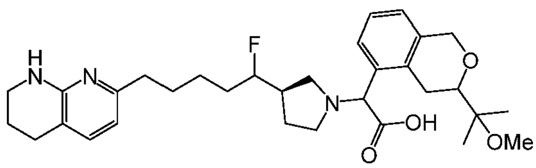,

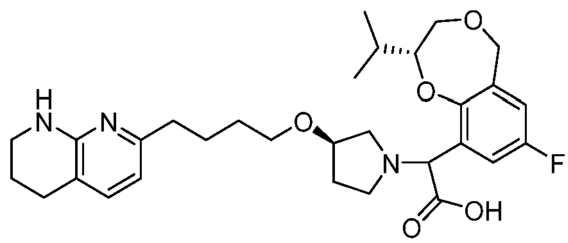,

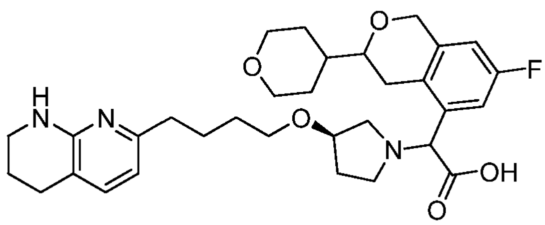,

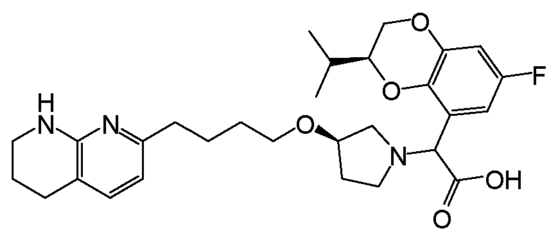,

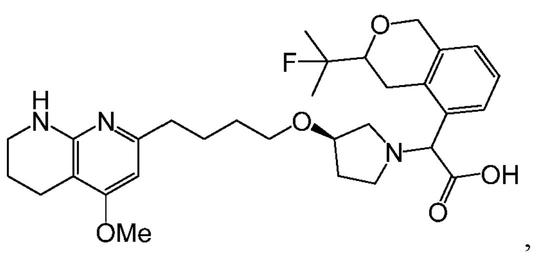,

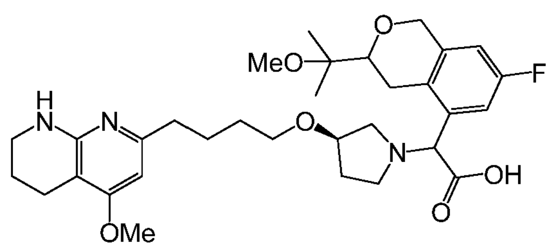,

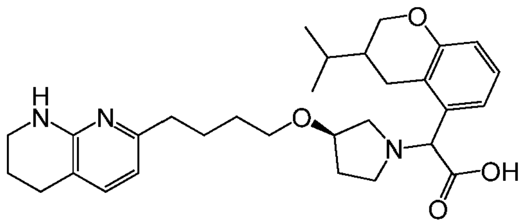,
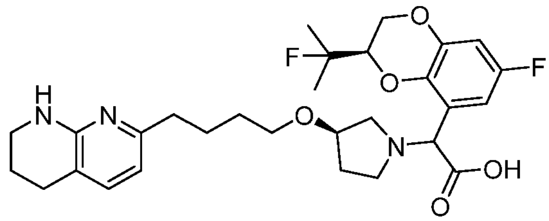,
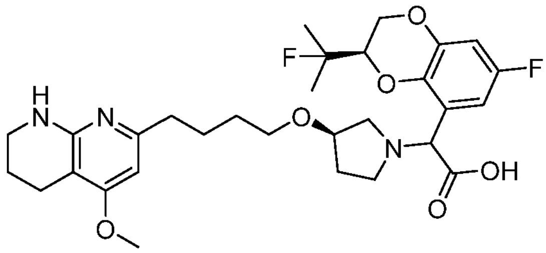,
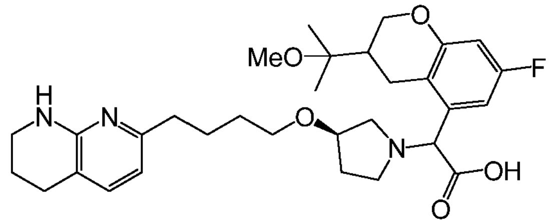,
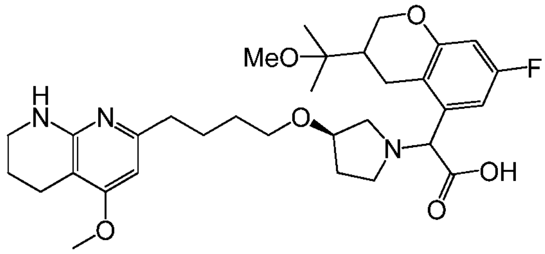,
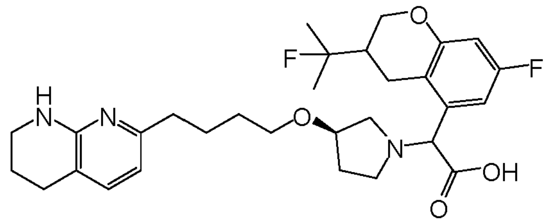,
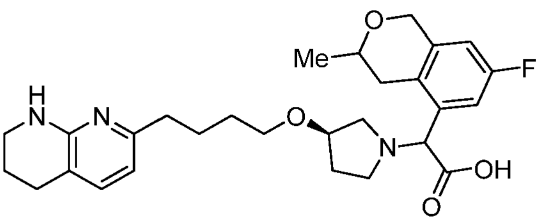, and
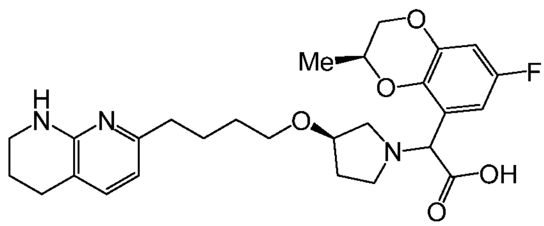.

In certain embodiments, the invention relates to any one of the aforementioned compounds, wherein the compound is a pharmaceutically acceptable salt.

## EXEMPLARY PHARMACEUTICAL COMPOSITIONS

**In** certain embodiments, the invention relates to a pharmaceutical composition comprising any one of the aforementioned compounds and a pharmaceutically acceptable carrier.

Patients, including but not limited to humans, can be treated by administering to the patient an effective amount of the active compound or a pharmaceutically acceptable prodrug or salt thereof in the presence of a pharmaceutically acceptable carrier or diluent. The active materials can be administered by any appropriate route, for example, orally, parenterally, intravenously, intradermally, subcutaneously, or topically, in liquid or solid form.

The concentration of active compound in the drug composition will depend on absorption, inactivation and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient can be administered at once, or can be divided into a number of smaller doses to be administered at varying intervals of time.

In certain embodiments, the mode of administration of the active compound is oral. Oral compositions will generally include an inert diluent or an edible carrier. They can be enclosed in gelatin capsules or compressed into tablets.

For the purpose of oral therapeutic administration, the active compound can be incorporated with excipients and used in the form of tablets, troches or capsules. Pharmaceutically compatible binding agents, and/or adjuvant materials can be included as part of the composition.

The tablets, pills, capsules and troches can contain any of the following ingredients, or compounds of a similar nature: a binder such as microcrystalline cellulose, gum tragacanth or gelatin; an excipient such as starch or lactose, a disintegrating agent such as alginic acid, Primogel or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, unit dosage forms can contain various other materials that modify the physical form of the dosage unit, for example, coatings of sugar, shellac, or other enteric agents.

The compound can be administered as a component of an elixir, suspension, syrup, wafer, chewing gum or the like. A syrup can contain, in addition to the active compound(s), sucrose or sweetener as a sweetening agent and certain preservatives, dyes and colorings and flavors.

The compound or a pharmaceutically acceptable prodrug or salts thereof can also be mixed with other active materials that do not impair the desired action, or with materials that supplement the desired action, such as antibiotics, antifungals, anti-inflammatories or other antivirals, including but not limited to nucleoside compounds. Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents, such as ethylenediaminetetraacetic acid; buffers, such as acetates, citrates or phosphates, and agents for the adjustment of tonicity, such as sodium chloride or dextrose. The parental preparation can be enclosed in ampoules, disposable syringes or multiple dose vials made of glass or plastic.

If administered intravenously, carriers include physiological saline and phosphate buffered saline (PBS).

In certain embodiments, the active compounds are prepared with carriers that will protect the compound against rapid elimination from the body, such as a controlled release formulation, including but not limited to implants and microencapsulated delivery systems. Biodegradable, biocompatible polymers can be used, such as ethylene vinyl acetate, polyanhydrides, polyglycolic acid, collagen, polyorthoesters and polylactic acid. For example, enterically coated compounds can be used to protect cleavage by stomach acid. Methods for preparation of such formulations will be apparent to those skilled in the art. Suitable materials can also be obtained commercially.

Liposomal suspensions (including but not limited to liposomes targeted to infected cells with monoclonal antibodies to viral antigens) are also preferred as pharmaceutically acceptable carriers. These can be prepared according to methods known to those skilled in the art, for example, as described in U.S. Pat. No. 4,522,811 (incorporated by reference). For example, liposome formulations can be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound is then introduced into the container. The container is then swirled by hand to free lipid material from the sides of the container and to disperse lipid aggregates, thereby forming the liposomal suspension.

## EXEMPLARY METHODS OF THE INVENTION

In certain embodiments, the invention relates to a compound described herein for use in a method of treating a disease or a condition selected from of idiopathic pulmonary fibrosis, diabetic nephropathy, focal segmental glomerulosclerosis, chronic kidney disease, nonalcoholic steatohepatitis, primary biliary cholangitis, primary sclerosing cholangitis, solid tumors, hematological tumors, organ transplant, Alport syndrome, interstitial lung disease, radiation-induced fibrosis, bleomycin-induced fibrosis, asbestos-induced fibrosis, flu-induced fibrosis, coagulation-induced fibrosis, vascular injury-induced fibrosis, aortic stenosis, and cardiac fibrosis comprising the step of: administering to a subject in need thereof a therapeutically effective amount of any one of the aforementioned compounds.

In certain embodiments, the invention relates to a compound described herein for use in any one of the aforementioned methods, wherein the disease or condition is a solid tumor (sarcomas, carcinomas, and lymphomas). Exemplary tumors that may be treated in accordance with the invention include e.g., Ewing's sarcoma, rhabdomyosarcoma, osteosarcoma, myelosarcoma, chondrosarcoma, liposarcoma, leiomyosarcoma, soft tissue sarcoma, non-small cell lung cancer, small cell lung cancer, bronchus cancer, prostate cancer, breast cancer, pancreatic cancer, gastrointestinal cancer, colon cancer, rectum cancer, colon carcinoma, colorectal adenoma, thyroid cancer, liver cancer, intrahepatic bile duct cancer, hepatocellular cancer, adrenal gland cancer, stomach cancer, gastric cancer, glioma (e.g., adult, childhood brain stem, childhood cerebral astrocytoma, childhood visual pathway and hypothalamic), glioblastoma, endometrial cancer, melanoma, kidney cancer, renal pelvis cancer, urinary bladder cancer, uterine corpus, uterine cervical cancer, vaginal cancer, ovarian cancer, multiple myeloma, esophageal cancer, brain cancer (e.g., brain stem glioma, cerebellar astrocytoma, cerebral astrocytoma/malignant glioma, ependymoma, meduloblastoma, supratentorial primitive neuroectodermal tumors, visual pathway and hypothalamic glioma), lip and oral cavity and pharynx, larynx, small intestine, melanoma, villous colon adenoma, a neoplasia, a neoplasia of epithelial character, lymphomas (e.g., AIDS-related, Burkitt's, cutaneous T-cell, Hodgkin, non-Hodgkin, and primary central nervous system), a mammary carcinoma, basal cell carcinoma, squamous cell carcinoma, actinic keratosis, tumor diseases, including solid tumors, a tumor of the neck or head, polycythemia vera, essential thrombocythemia, myelofibrosis with myeloid metaplasia, Waldenstrom's macroglobulinemia, adrenocortical carcinoma, AIDS-related cancers, childhood cerebellar astrocytoma, childhood cerebellar astrocytoma, basal cell carcinoma, extrahepatic bile duct cancer, malignant fibrous histiocytoma bone cancer, bronchial adenomas/carcinoids, carcinoid tumor, gastrointestinal carcinoid tumor, primary central nervous system, cerebellar astrocytoma, childhood cancers, ependymoma, extracranial germ cell tumor, extragonadal germ cell tumor, extrahepatic bile duct cancer, intraocular melanoma eye cancer, retinoblastoma eye cancer, gallbladder cancer, gastrointestinal carcinoid tumor, germ cell tumors (e.g., extracranial, extragonadal, and ovarian), gestational trophoblastic tumor, hepatocellular cancer, hypopharyngeal cancer, hypothalamic and visual pathway glioma, islet cell carcinoma (endocrine pancreas), laryngeal cancer, malignant fibroushistiocytoma of bone/osteosarcoma, meduloblastoma, mesothelioma, metastatic squamous neck cancer with occult primary, multiple endocrine neoplasia syndrome, multiple myeloma/plasma cell neoplasm, mycosis fungoides, nasal cavity and paranasal sinus cancer, nasopharyngeal cancer, neuroblastoma, oral cancer, oropharyngeal cancer, ovarian epithelial cancer, ovarian germ cell tumor, ovarian low malignant potential tumor, islet cell pancreatic cancer, parathyroid cancer, pheochromocytoma, pineoblastoma, pituitary tumor, pleuropulmonary blastoma, ureter transitional cell cancer, retinoblastoma, rhabdomyosarcoma, salivary gland cancer, Sezary syndrome, non-melanoma skin cancer, Merkel cell carcinoma, squamous cell carcinoma, testicular cancer, thymoma, gestational trophoblastic tumor, and Wilms' tumor.

In certain embodiments, the invention relates to a compound described herein for use in any one of the aforementioned methods, wherein the disease is disease or condition is a hematological tumor. Exemplary homatological tumors that may be treated in accordance with the invention include e.g., acute lymphocytic leukemia, acute myelogenous leukemia, chronic lymphocytic leukemia, chronic myelogenous leukemia, Hodgkin lymphoma, non-Hodgkin lymphoma, and multiple myeloma.

In certain embodiments, the invention relates to a compound described herein for use in any one of the aforementioned methods, wherein the disease or condition is selected from idiopathic pulmonary fibrosis, systemic sclerosis associated interstitial lung disease, myositis associated interstitial lung disease, systemic lupus erythematosus associated interstitial lung disease, rheumatoid arthritis, and associated interstitial lung disease.

In certain embodiments, the invention relates to a compound described herein for use in any one of the aforementioned methods, wherein the disease or condition is selected from the group consisting of diabetic nephropathy, focal segmental glomerulosclerosis, and chronic kidney disease.

In certain embodiments, the invention relates to a compound described herein for use in any one of the aforementioned methods, wherein the disease or condition is selected from the group consisting of nonalcoholic steatohepatitis, primary biliary cholangitis, and primary sclerosing cholangitis.

In certain embodiments, the invention relates to a compound described herein for use in any one of the aforementioned methods, wherein the subject is a mammal. In certain embodiments, the invention relates to a compound described herein for use in any one of the aforementioned methods, wherein the subject is human.

## EXEMPLIFICATION

The invention now being generally described, it will be more readily understood by reference to the following examples, which are included merely for purposes of illustration of certain aspects and embodiments of the present invention, and are not intended to limit the invention.

### General schemes and procedures for the preparation of compounds of the invention

The moiety $R_1$ and $R_2$ represents appropriate substituents; L represents an appropriate linker, and X represents an appropriate halogen, such as Br, Cl or I, or another leaving group such as mesylate or tosylate.

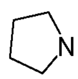

represents an appropriate optionally substituted pyrrolidine.

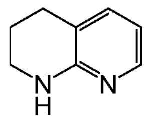

represents an appropriate optionally substituted tetrahydronaphthyridine.

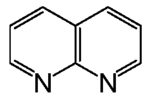

represents an appropriate optionally substituted naphthyridine.

## General Schemes for the synthesis of αvβ6 inhibitors

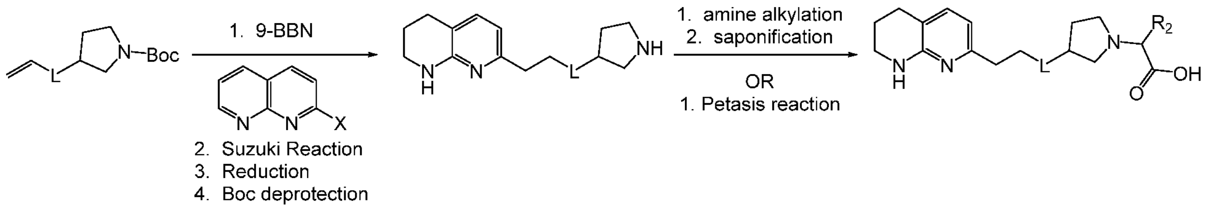


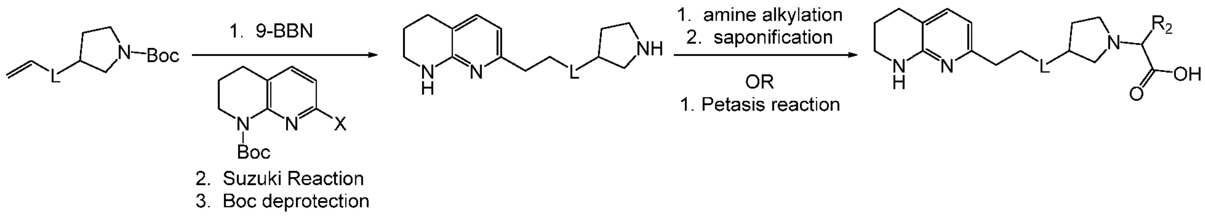


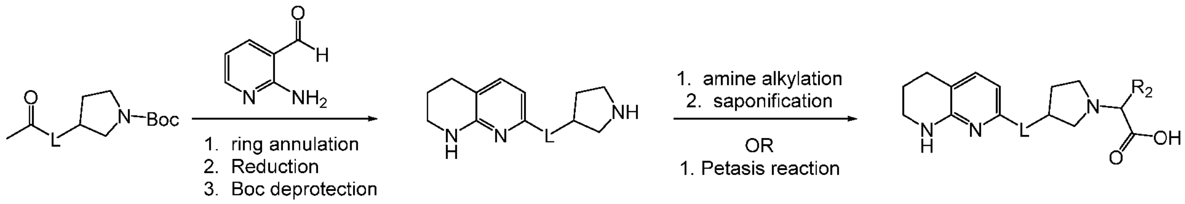


## General Procedures

### 9-BBN and Suzuki Reactions

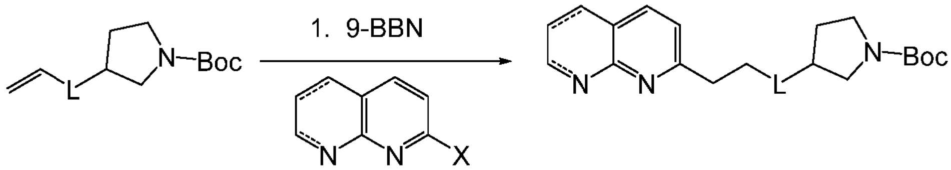


Alkene intermediates may be cross-coupled to 2-halo naphthyridines or tetrahydronaphthyridines by the following procedure. To a solution of alkene (1 equiv.) in dry THF (2-10 mL/mmol) under Ar was added 9-BBN (0.5M solution in THF, 1-2 equiv.). The reaction was stirred at 40-80 °C for 1-4 hours, then cooled to room temperature. This solution was added to a mixture of 2-halonaphthyridine or Boc-protected 2-halotetrahydronaphthyridine (1-1.5 equiv.), cesium carbonate (2-5 equiv.) and Pd(PPh3)4 or another appropriate Pd/ligand combination (0.05 to 0.1 equiv.) in 1,4-Dioxane (2-10 mL/mmol). The reaction was stirred at 80-100 °C for 12-24 hours. Solvent was removed in vacuo, and the residue was purified by silica gel column to give the alkyl linked naphthyridine product.

### Ring Annulations

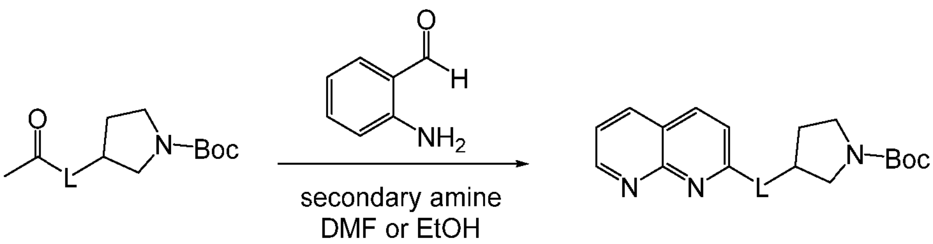


Naphthyridines may also be made from methyl ketones by the following procedure. A mixture of methyl ketone (1

equiv.), 2-aminonicotinaldehyde (1-2 equiv.) and secondary amine such as pyrrolidine or L-proline (1-2 equiv.) in DMF or EtOH (1-10 mL/mmol) was stirred at 70-100 °C for 2- 10 hours. Solvent was removed *in vacuo,* and the residue was purified by silica gel column to give the desired naphthyridine product.

**Naphthyridine Reduction**

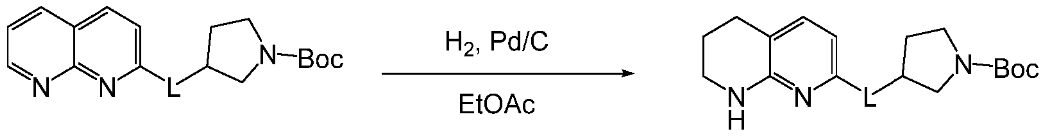


Naphthyridines may be reduced to tetrahydronaphthyridines by the following procedure. A mixture of an appropriate naphthyridine (1 equiv.) and Pd/C (5-20 weight percent Pd, 0.05 to 0.2 equiv.) in ethyl acetate or another appropriate solvent (2-10 mL/mmol) was stirred under $H_2$ balloon at room temperature to 50 °C for 2-20 hours. The reaction was filtered and concentrated in vacuo to give the desired tetrahydronaphthyridine product.

**Boc eprotection**

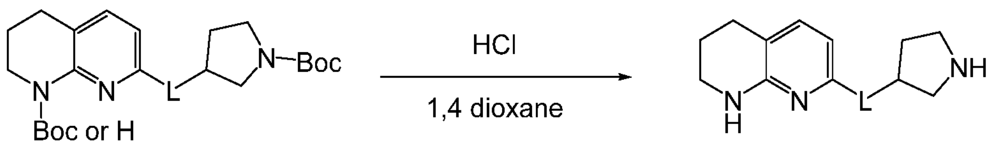


Boc-protected amine (1 equiv.) was treated with HCl (4-100 equiv.) in 1,4-dioxane (1-50 mL/mmol amine) at room temperature to 50 °C for 1-4 hours. The reaction was concentrated in vacuo, and the amine product was used crude or after purification by silica gel column. The amine could be used crude as a dihydrochloride salt or converted to the free base by dissolving in an appropriate solvent and washing with aqueous $NaHCO_3$.

**Amine alkylation:**

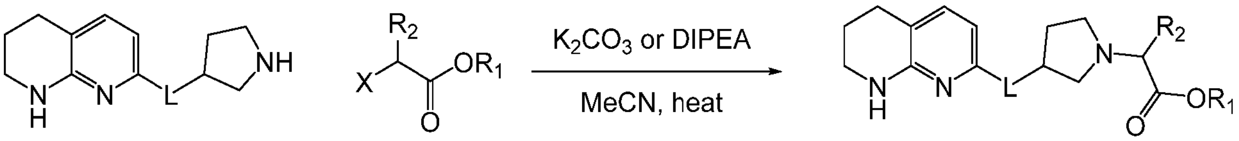


A mixture of amine (1 equiv.), alkylating agent (1-1.5 equiv.) and $K_2CO_3$ or N,N-diisopropylethylamine (2-10 equiv.) in MeCN or DMF (3-10 mL/mmole amine) was stirred at room temperature to 80 °C for 4-16 hours. The reaction was concentrated in vacuo, and the residue was purified by silica gel column to give the desired amino acetic acid ester. The amine used may be the free base or a salt such hydrochloride or dihydrochloride. If the reaction is done with a salt of the amine, additional equivalents of base may be needed.

**Saponification:**

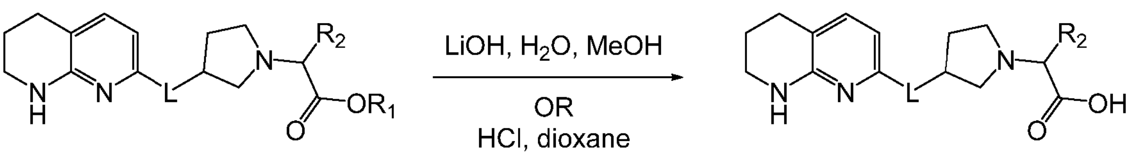

For certain esters such as $R_1$ = Me or ethyl, the ester may be saponified under basic conditions. The ester (1 equiv.) was treated with LiOH-$H_2O$ (3-5 equiv.) in MeOH (3-10 mL/mmol ester) and water (3-10 mL/mmol ester) at room temperature to 50 °C for 1-16 hours. The reaction was concentrated in vacuo, and the residue was purified by prep HPLC to give the desired carboxylic acid product.

For certain esters such as $R_1$ = tert-butyl, the ester may be saponified under acidic conditions. The ester (1 equiv.) was treated with 4 N HCl (4-100 equiv.) in 1,4-dioxane (1-25 mL/mmol ester) at room temperature to 50 °C for 1-16 hours. The reaction was concentrated in vacuo, and the residue was purified by prep HPLC to give the desired carboxylic acid product.

**Petasis reaction:**

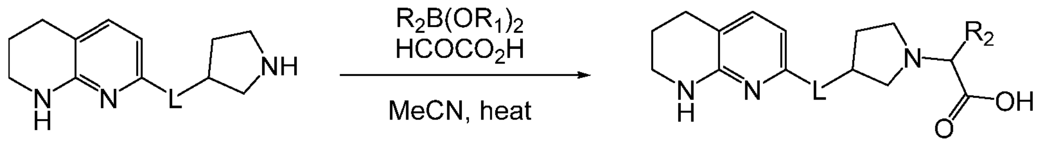


As an alternative to the amine alkylation/saponification sequence, a Petasis reaction can be used to prepare certain aryl analogs: A mixture of amine (1 equiv.) aryl boronic acid or aryl boronate ester (1-1.5 equiv.) and 2-oxoacetic acid (1.5-2 equiv.) in MeCN or DMF (2-10 mL/mmole amine) was stirred at 50-80 °C for 2-16 hours. The reaction was concentrated in vacuo, and the residue was purified by prep HPLC to give the desired amino acetic acid.

## Analytical Methods

### Prep-HPLC Methods

Crude samples were dissolved in MeOH and purified by prep HPLC using a Gilson 215 instrument, detection wavelength 214 nm:

Prep HPLC A: column: XBridge C18, 21.2 * 250 mm, 10 μm; mobile phase: A water (10 mM ammonium hydrogen carbonate), B $CH_3CN$; gradient elution as in text; flow rate: 20 mL/min.

Prep HPLC B: column: XBridge C18, 21.2 * 250 mm, 10 μm; mobile phase: A water (10 mM formic acid), B $CH_3CN$; gradient elution as in text; flow rate: 20 mL/min.

Prep HPLC C: column: XBridge OBD C18, 19 * 100 mm, 5 μm; mobile phase: A water, B $CH_3CN$; gradient elution as in text; flow rate: 20 mL/min.

### Prep Chiral SFC Methods

Racemic products were separated to individual enantiomers by chiral Prep SFC using an SFC-80 (Thar, Waters) instrument, detection wavelength 214 nm:

Prep chiral SFC A: column: (R,R)-Whelk-O1, 20*250mm, 5 μm (Decial), column temperature: 35 °C, mobile phase: $CO_2$/methanol (0.2% methanol ammonia)= 60/40, flow rate: 80 g/min, back pressure: 100 bar.

Prep chiral SFC B: column: AD 20*250mm, 10 μm (Daicel), column temperature: 35 °C,

mobile phase: $CO_2$/methanol (0.2% methanol ammonia)= 60/40, flow rate: 80 g/min, back pressure: 100 bar.

Prep chiral SFC C: column: AS 20*250mm, 10 μm (Daicel), column temperature: 35 °C,

mobile phase: $CO_2$/methanol (0.2% methanol ammonia)= 60/40, flow rate: 80 g/min, back pressure: 100 bar.

Prep chiral SFC D: column: OD 20*250mm, 10 μm (Daicel), column temperature: 35 °C,

mobile phase: $CO_2$/methanol (0.2% methanol ammonia)= 60/40, flow rate: 80 g/min, back pressure: 100 bar.

Prep chiral SFC E: column: Cellulose-SC 20*250mm, 10 μm (Daicel), column temperature: 35 °C, mobile phase: $CO_2$/methanol (0.2% methanol ammonia)= 60/40, flow rate: 80 g/min, back pressure: 100 bar.

Prep chiral SFC F: column: OZ 20*250mm, 10 μm (Daicel), column temperature: 35 °C, mobile phase: $CO_2$/methanol (0.2% methanol ammonia)= 60/40, flow rate: 80 g/min, back pressure: 100 bar.

Prep chiral SFC G: column: IC 20*250mm, 10 μm (Daicel), column temperature: 35 °C, mobile phase: $CO_2$/methanol (0.2% methanol ammonia)= 60/40, flow rate: 80 g/min, back pressure: 100 bar.

Prep chiral SFC H: column: (S,S)-Whelk-O1, 20*250mm, 5 μm (Decial), column temperature: 35 °C, mobile phase: $CO_2$/methanol (0.2% methanol ammonia)= 60/40, flow rate: 80 g/min, back pressure: 100 bar.

**Analytical Chiral SFC Methods**

Chiral products were analyzed by chiral SFC using an SFC-80 (Thar, Waters) instrument, detection wavelength 214 nm:

Chiral SFC A: column: (R,R)-Whelk-O1, 4.6*100mm, 5 μm (Decial), column temperature: 40 °C, mobile phase: $CO_2$/methanol (0.2% methanol ammonia), isocratic elution as in text, flow rate: 4 g/min, back pressure: 120 bar.

Chiral SFC B: column: AD 4.6*100mm, 5 μm (Daicel), column temperature: 40 °C, mobile phase: $CO_2$/methanol (0.2% methanol ammonia), isocratic elution as in text, flow rate: 4 g/min, back pressure: 120 bar.

Chiral SFC C: column: AS 4.6*100mm, 5 μm (Daicel), column temperature: 40 °C, mobile phase: $CO_2$/methanol (0.2% methanol ammonia), isocratic elution as in text, flow rate: 4 g/min, back pressure: 120 bar.

Chiral SFC D: column: OD 4.6*100mm, 5 μm (Daicel), column temperature: 40 °C, mobile phase: $CO_2$/methanol (0.2% methanol ammonia), isocratic elution as in text, flow rate: 4 g/min, back pressure: 120 bar.

Chiral SFC E: column: Cellulose-SC 4.6*100mm, 5 μm (Daicel), column temperature: 40 °C, mobile phase: CO2/methanol (0.2% methanol ammonia), isocratic elution as in text, flow rate: 4 g/min, back pressure: 120 bar.

Chiral SFC F: column: OZ 4.6*100mm, 5 μm (Daicel), column temperature: 40 °C, mobile phase: $CO_2$/methanol (0.2% methanol ammonia), isocratic elution as in text, flow rate: 4 g/min, back pressure: 120 bar.

Chiral SFC G: column: IC 4.6*100mm, 5 μm (Daicel), column temperature: 40 °C, mobile phase: $CO_2$/methanol (0.2% methanol ammonia), isocratic elution as in text, flow rate: 4 g/min, back pressure: 120 bar.

Chiral SFC H: column: (S,S)-Whelk-O1, 4.6*100mm, 5 μm (Decial), column temperature: 40 °C, mobile phase: $CO_2$/methanol (0.2% methanol ammonia), isocratic elution as in text, flow rate: 4 g/min, back pressure: 120 bar.

Chiral SFC I: column: IC 4.6*250mm, 5 μm (SHIMADZU), column temperature: 40 °C, mobile phase: n-Hexane(0.1% DEA):EtOH(0.1%DEA), isocratic elution as in text, flow rate: 1 mL/min.

Chiral SFC J: column: (S,S)-Whelk-O1 4.6*250mm, 5 μm (SHIMADZU), column temperature: 40 °C, mobile phase: n-Hexane(0.1%DEA):EtOH(0.1%DEA), isocratic elution as in text, flow rate: 1 mL/min.

Chiral SFC K: column: OZ-H 4.6*250mm, 5 μm (SHIMADZU), column temperature: 40 °C, mobile phase: n-Hexane(0.1%DEA):EtOH(0.1%DEA), isocratic elution as in text, flow rate: 1 mL/min.

Chiral SFC L: column: chiral PAK IG 4.6*250mm, 5 μm (SHIMADZU), column temperature: 35 °C, mobile phase: n-Hexane(0.1%DEA):EtOH(0.1%DEA), isocratic elution as in text, flow rate: 1 mL/min.

Chiral SFC M: column: EnantioPak OJ 4.6*250mm, 5 μm (Decial), column temperature: 40 °C, mobile phase: n-Hexane(0.1%DEA):EtOH(0.1%DEA), isocratic elution as in text, flow rate: 1 mL/min.

***Synthesis of Intermediates***

The following intermediates were prepared according to the procedures below for use in synthesizing compounds 1-3:

**Preparation of (R)-7-(4-(pyrrolidin-3-yloxy)butyl)-1,2,3,4-tetrahydro-1,8-naphthyridine dihydrochloride**

**Step 1: tert-butyl (R)-3-(4-(2-methyl-1,3-dioxolan-2-yl)butoxy)pyrrolidine-1-carboxylate**

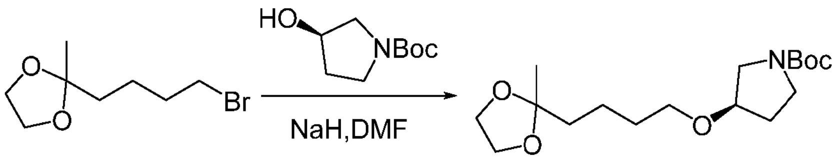


A mixture of (R)-tert-butyl 3-hydroxypyrrolidine-1-carboxylate (1.09 g, 5.41 mmol), 2-(4-bromobutyl)-2-methyl-1,3-dioxolane (1.2 g, 5.41 mmol) and sodium hydride (260 mg, 10.82 mmol) in DMF (5 mL) was stirred at 100 °C for 6h. Solvent was removed *in vacuo,* and the residue was purified by silica gel column (pet ether: EtOAc 10:1) to give the desired product (R)-tert-butyl 3-(4-(2-methyl-1,3-dioxolan-2-yl)butoxy)pyrrolidine-1-carboxylate as a colorless oil (380 mg). Yield 21% (ESI 330.2 (M+H) $^+$).

**Step 2: (R)-tert-butyl3-(5-oxohexyloxy)pyrrolidine-1-carboxylate**

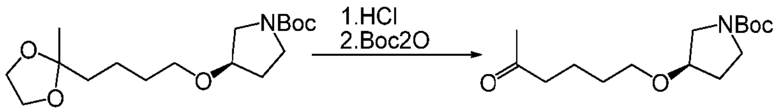


(R)-tert-butyl3-(4-(2-methyl-1,3-dioxolan-2-yl)butoxy)pyrrolidine-1-carboxylate (1.3 g, 3.95 mmol) was treated with a solution of HCl/dioxane (4.0 M, 10 mL) at room temperature for 2 hours. The solvent was removed in *vacuo,* and the residue was diluted with acetone (10 mL) and $H_2O$ (1 mL). Potassium carbonate was added to adjust the pH to 8~9, followed by $Boc_2O$ (1.24 g 5.69 mmol). The reaction was stirred at room temperature for 3, then filtered and concentrated under vacuum. The residue was purified by silica gel column (pet ether: EtOAc 15:1) to give the desired product (R)-tert-butyl3-(5-oxohexyloxy)pyrrolidine-1-carboxylate as a colorless oil (820 mg). Yield 73% (ESI 186 (M-100) $^+$, 230 (M-56) $^+$).

**Step 3: (R)-tert-butyl 3-(4-(1,8-naphthyridin-2-yl)butoxy)pyrrolidine-1-carboxylate**

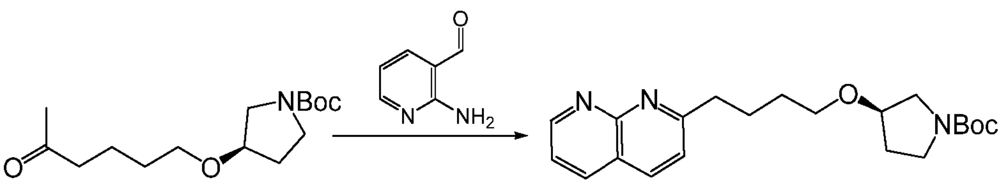

A mixture of (R)-tert-butyl3-(5-oxohexyloxy)pyrrolidine-1-carboxylate (820 mg, 2.88 mmol), 2-aminonicotinaldehyde (456 mg, 3.77 mmol) and pyrrolidine (265 mg, 3.77 mmol) in DMF (5 mL) was stirred at 85 °C for 4h . Solvent was removed *in vacuo,* and the residue was purified by silica gel column (DCM:MeOH 15:1) to give the desired product (R)-tert-butyl 3-(4-(1,8-naphthyridin-2-yl)butoxy)pyrrolidine-1-carboxylate as a colorless oil (750 mg). Yield 70% (ESI 372.2 (M+H) $^+$).

**Step 4: (R)-7-(4-(pyrrolidin-3-yloxy)butyl)-1,2,3,4-tetrahydro-1,8-naphthyridine dihydrochloride**

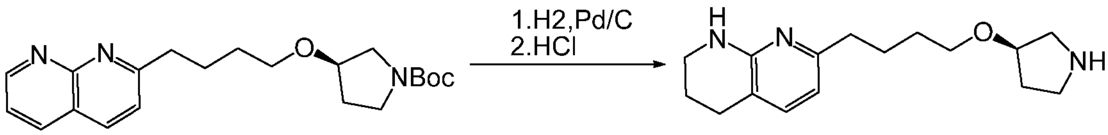


A mixture of (R)-tert-butyl 3-(4-(1,8-naphthyridin-2-yl)butoxy)pyrrolidine-1-carboxylate (750 mg, 2.02 mmol), Pd/C (10%, 500 mg) in EtOAc (10 mL) was stirred at 60 °C for 6 hours under hydrogen. The reaction was filtered and concentrated *in vacuo.* The residue was treated with a solution of HCl/dioxane (4.0 M, 4 mL) at room temperate for 2 hours, and the solvent was removed in *vacuo* to give the desired product (R)-7-(4-(pyrrolidin-3-yloxy)butyl)-1,2,3,4-tetrahydro-1,8-naphthyridine dihydrochloride as a white solid (600 mg). Yield 96% (ESI 276.2 (M+H) +).

**Preparation of (R)-5-methoxy-7-(4-(pyrrolidin-3-yloxy)butyl)-1,2,3,4-tetrahydro-1,8-naphthyridine dihydrochloride**

**Step 1: (R)-tert-butyl 3-(4-bromobutoxy)pyrrolidine-1-carboxylate**

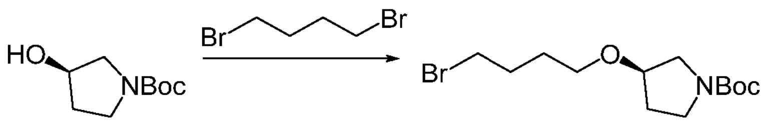

To a solution of tert-butyl (R)-3-hydroxypyrrolidine-1-carboxylate (500 mg, 2.67 mmol) in n-Heptane (10 mL) was added sodium hydroxide 50% solution in water (5 mL, 31.2 mmol), tetrabutylammonium bromide (43.0 mg, 0.13 mmol) and 1,4-dibromobutane (1.595 mL, 13.35 mmol). The mixture was stirred at 80 °C for 2 hours, then cooled to room temperature, diluted with water (10 mL) and extracted with diethyl ether (3x 10 mL). The combined organic layers were dried over Na2SO4, filtered and concentrated in vacuo. The residue was purified by silica gel column (pet ether: EtOAc=4:1) to give the desired product (R)-tert-butyl 3-(4-bromobutoxy)pyrrolidine-1-carboxylate as a colorless oil (686 mg). Yield 80% (ESI 314 (M+H-Boc) +).

**Step 2: (R)-tert-butyl 3-(but-3-enyloxy)pyrrolidine-1-carboxylate**

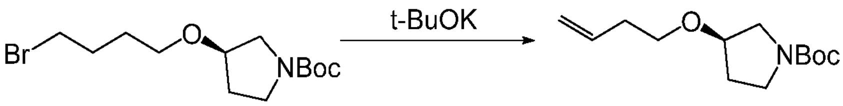


To a solution of tert-butyl (R)-3-(4-bromobutoxy)pyrrolidine-1-carboxylate (512 mg, 1.58 mmol) in THF (10 mL) at 0 °C was added t-BuOK (446 mg, 3.97 mmol). The reaction was stirred at room temperature for 1 hour, then diluted with water (20 mL) and extracted with diethyl ether (3x 20mL). The combined organic layers were dried over sodium sulfate, filtered and concentrated in vacuo to give the desired product (R)-tert-butyl 3-(but-3-enyloxy)pyrrolidine-1-carboxylate as a colorless oil (355 mg). Yield 90% (ESI 186 (M+H-Boc) +).

**Step 3: (R)-tert-butyl 3-(4-(4-chloro-1,8-naphthyridin-2-yl)butoxy)pyrrolidine-1-carboxylate**

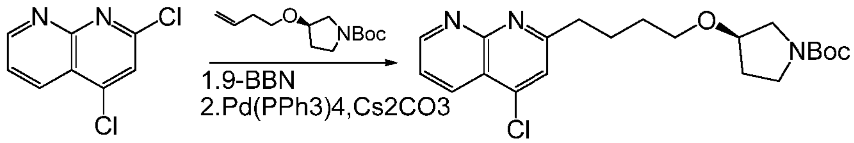


To a solution of tert-butyl (R)-tert-butyl 3-(but-3-enyloxy)pyrrolidine-1-carboxylate (486 mg, 1.8 mmol) in THF (dry, 2 mL) under Ar was added 9-BBN (0.5M solution in THF, 7.2 mL, 3.6 mmol). The reaction was stirred at 50 °C for 2 hours, then cooled to room temperature. This solution was added to a mixture of 2,4-dichloro-1,8-naphthyridine (360 mg, 1.8 mmol), cesium carbonate (1730 mg, 5.4 mmol) and Pd(PPh3)4 (208 mg, 0.18 mmol) in 1,4-dioxane (7 mL). The reaction was stirred at 90 °C for 1.5 hours. Solvent was removed in vacuo, and the residue was purified by silica gel column (pet ether: EtOAc 1:1 to 1:10) to give the desired product (R)-tert-butyl 3-(4-(4-chloro-1,8-naphthyridin-2-yl)butoxy) pyrrolidine-1-carboxylate as a yellow oil (300 mg). Yield 41% (ESI 406 (M+H) +).

**Step 4: (R)-tert-butyl 3-(4-(4-methoxy-1,8-naphthyridin-2-yl)butoxy)pyrrolidine-1-carboxylate**

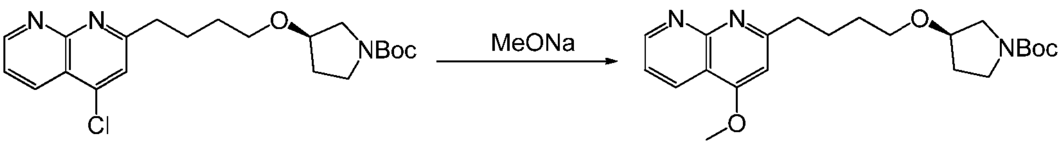


To a solution of (R)-tert-butyl 3-(4-(4-chloro-1,8-naphthyridin-2-yl)butoxy)pyrrolidine-1-carboxylate (76 mg, 0.13 mmol) in MeOH (5 mL) was added NaOMe (45 mg, 0.26 mmol). The reaction was stirred under reflux overnight, then concentrated in vacuo, diluted with ethyl acetate (30 mL), washed with water (2 x 20 mL), dried over MgSO4, filtered and concentrated in vacuo to give the desired product (R)-tert-butyl 3-(4-(4-methoxy-1,8-naphthyridin-2-yl)butoxy)pyrrolidine-1-carboxylate as a colorless oil (60 mg). Yield 80% (ESI 402 (M+H) +).

**Step 5: (R)-5-methoxy-7-(4-(pyrrolidin-3-yloxy)butyl)-1,2,3,4-tetrahydro-1,8-naphthyridine dihydrochloride**

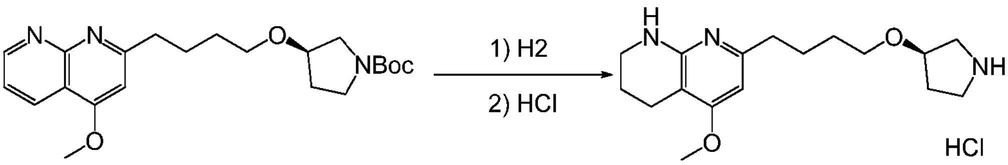


A mixture of (R)-tert-butyl 3-(4-(4-methoxy-1,8-naphthyridin-2-yl)butoxy)pyrrolidine-1-carboxylate (60 mg, 0.15 mmol) and Pd/C (10%, 30 mg) in EtOAc (10 mL) was stirred under balloon hydrogen at 30 °C for 17 hours. The mixture was filtered and concentrated *in vacuo.* The residue was treated with 4M HCl in dioxane (3 mL, 12 mmol) at room temperature for 2 hours. Solvent was removed *in vacuo* to give the desired product (R)-5-methoxy-7-(4-(pyrrolidin-3-yloxy)butyl)-1,2,3,4-tetrahydro-1,8-naphthyridine dihydrochloride as a colorless oil (45 mg). Yield 88% (ESI 306 (M+H) $^{+}$).

**Preparation of (R)-7-(5,5-difluoro-5-(pyrrolidin-3-yl)pentyl)-1,2,3,4-tetrahydro-1,8-naphthyridine dihydrochloride**

**Step 1: (R)-tert-butyl 3-(methoxy(methyl)carbamoyl)pyrrolidine-1-carboxylate**

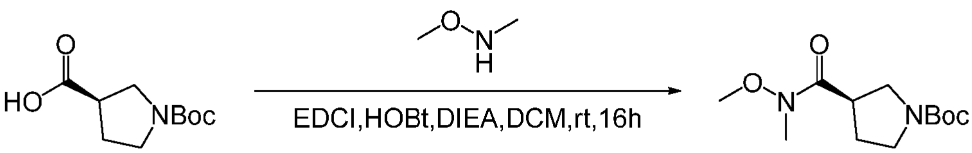

A mixture of (R)-1-(tert-butoxycarbonyl)pyrrolidine-3-carboxylic acid (10.0 g, 46.5 mmol), N,O-dimethylhydroxylamine hydrochloride (6.8 g, 69.8 mmol), EDCI (17.8g, 93.0 mmol), HOBt (12.6g, 93.0mmol) and diisopropylethylamine (18.1g, 139.5 mmol) in dichloromethane (120 mL) was stirred at room temperature overnight. Solvent was removed in vacuo, and the residue was purified by silica gel column (pet ether: EtOAc 2:1) to give the desired product (R)-tert-butyl 3-(methoxy(methyl)carbamoyl) pyrrolidine-1-carboxylate as a colorless oil (7.5 g). Yield 63%. (ESI 259.0 (M+H) +).

**Step 2: (R)-tert-butyl 3-pent-4-enoylpyrrolidine-1-carboxylate**

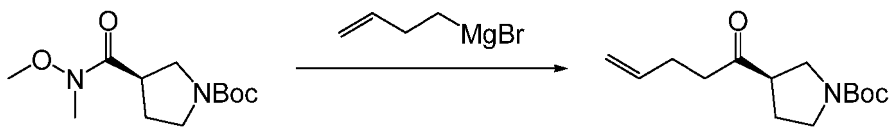

To a solution of (R)-tert-butyl 3-(methoxy(methyl)carbamoyl) pyrrolidine-1-carboxylate (7g, 27.1 mmol) in THF (40 mL) at 0 °C, was added 3-butenylmagnesium bromide solution 0.5 M in THF (108 mL, 54 mmol) dropwise. The reaction was stirred at 0° C for 1 hour, then stirred at room temperature overnight, quenched with sat. NH4Cl (10 mL) and extracted with EtOAc (3x 40mL). The organic combined phases were washed with brine, dried over Na2SO4, filtered and concentrated in vacuo. The residue was purified by silica gel column (pet ether: EtOAc 5:1) to give the desired product (R)-tert-butyl 3-pent-4-enoylpyrrolidine-1-carboxylate as a yellow oil ( 4.4g). Yield 64%.(ESI 254.0 (M+H) +).

**Step 3: (R)-tert-butyl 3-(1,1-difluoropent-4-enyl)pyrrolidine-1-carboxylate**

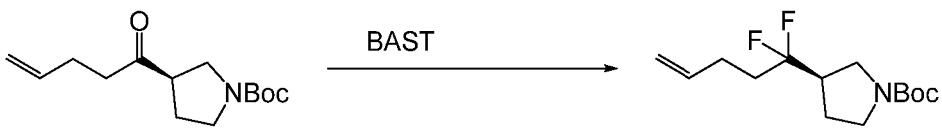


A mixture of (R)-tert-butyl 3-pent-4-enoylpyrrolidine-1-carboxylate (4.4g, 17.4 mmol) and BAST (19.2 g, 87mmol) was stirred at room temperature for 40 hours. The reaction was quenched with MeOH (2 mL), diluted with H2O (10 mL) and extracted with EtOAc (50 mL). The organic layer was washed with brine, dried over Na2SO4, filtered and concentrated in vacuo. The residue was purified by silica gel column (pet ether: EtOAc 10:1) to give the desired product (R)-tert-butyl 3-(1,1-difluoropent-4-enyl) pyrrolidine -1-carboxylate as a yellow oil (1.7g). Yield 36% (ESI 276 (M+H) +).

**Step 4: (R)-tert-butyl 3-(1,1-difluoropent-4-enyl)pyrrolidine-1-carboxylate**

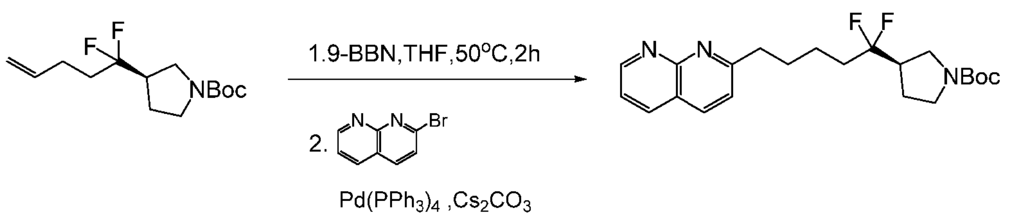


To a solution of (S)-tert-butyl 3-(1,1-difluoropent-4-enyl)pyrrolidine-1-carboxylate (1.4g, 5.1 mmol) in THF (dry, 5 mL) under Ar, was added 9-BBN (0.5M solution in THF, 20.4 mL, 10.2 mmol). The reaction was stirred at 50 °C for 2 hours, then cooled to room temperature. This solution was added to a mixture of 2-bromo-1,8-naphthyridine (1.07g, 5.1 mmol), cesium carbonate (4.98g, 15.3 mmol) and Pd(PPh3)4 (295mg, 0.255 mmol) in 1,4-dioxane (10 mL). The reaction was stirred at 90 °C for 1.5 hours, then concentrated *in vacuo,* and the residue was purified by silica gel column (DCM:MeOH 30:1) to give the desired product (R)-tert-butyl 3-(1,1-difluoro-5-(1,8-naphthyridin-2-yl)pentyl)pyrrolidine-1-carboxylate as a yellow oil (1g). Yield 48% (ESI 406 (M+H) +).

**Step 5: (R)-tert-butyl 3-(1,1-difluoro-5-(5,6,7,8-tetrahydro-1,8-naphthyridin-2-yl)pentyl)pyrrolidine-1-**carboxylate

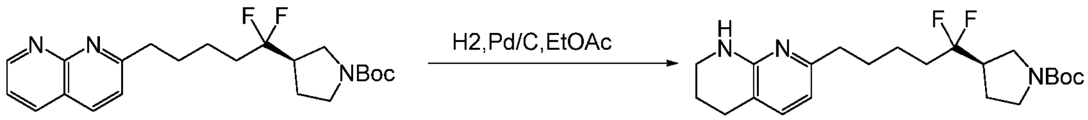


A mixture of (R)-tert-butyl 3-(1,1-difluoro-5-(1,8-naphthyridin-2-yl)pentyl)pyrrolidine-1-carboxylate (1g, 2.47 mmol), Pd/C (200 mg, 20Wt%) in ethyl acetate (20 mL) was stirred under H2 balloon at 40 °C for 16 hours. The reaction was filtered and concentrated in vacuo to give the desired product (R)-tert-butyl 3-(1,1-difluoro-5-(5,6,7,8-tetrahydro-1,8-naphthyridin-2-yl)pentyl)pyrrolidine-1-carboxylate as a yellow oil (1g). Yield 98% (ESI 410 (M+H) +).

**Step 6: (R)-7-(5,5-difluoro-5-(pyrrolidin-3-yl)pentyl)-1,2,3,4-tetrahydro-1,8-naphthyridine**

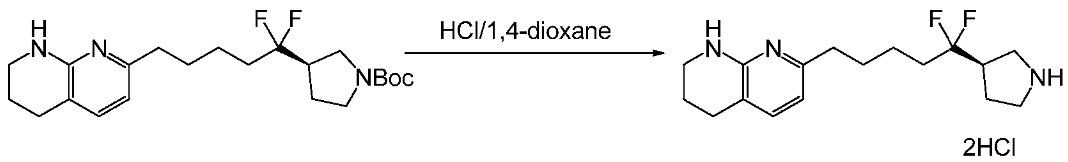


(R)-tert-butyl 3-(1,1-difluoro-5-(5,6,7,8-tetrahydro-1,8-naphthyridin-2-yl)pentyl)pyrrolidine-1-carboxylate (1g, 2.44 mmol) was treated with HCl in 1,4-dioxane(4M, 10 mL) at room temperature for 2 hours. Solvent was removed in vacuo to give the desired product (R)-7-(5,5-difluoro-5-(pyrrolidin-3-yl)pentyl)-1,2,3,4-tetrahydro-1,8-naphthyridine as a HCl salt (930 mg). Yield 99% (ESI 310 (M+H) +).

**Preparation of (R)-7-(5,5-difluoro-5-(pyrrolidin-3-yl)pentyl)-5-methoxy-1,2,3,4-tetrahydro-1,8-naphthyridine dihydrochloride**

**Step 1: (R)-tert-butyl 3-(1,1-difluoropent-4-enyl)pyrrolidine-1-carboxylate**

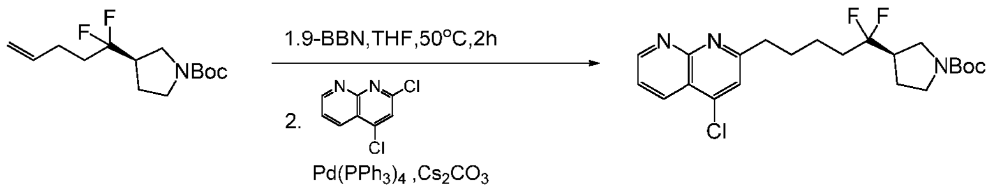


To a solution of (S)-tert-butyl 3-(1,1-difluoropent-4-enyl)pyrrolidine-1-carboxylate (1.4g, 5.1 mmol) in THF (dry, 5 mL) under Ar, was added 9-BBN (0.5M solution in THF, 20.4 mL, 10.2 mmol). The reaction was stirred at 50 °C for 2 hours, then cooled to room temperature and added to a mixture of 2,4-dichloro-1,8-naphthyridine (1.01g, 5.1 mmol), cesium carbonate (4.98g, 15.3 mmol) and Pd(PPh3)4 (295mg, 0.255 mmol) in 1,4-Dioxane (10 mL). The reaction was stirred at 90 °C for 2 hours. Solvent was removed in vacuo, and the residue was purified by silica gel column (DCM:MeOH 30:1) to give the desired product as a yellow oil (0.95 g). Yield 42% (ESI 440 (M+H) +).

**Step 2: (R)-tert-butyl 3-(1,1-difluoro-5-(4-methoxy-1,8-naphthyridin-2-yl)pentyl)pyrrolidine-1-carboxylate**

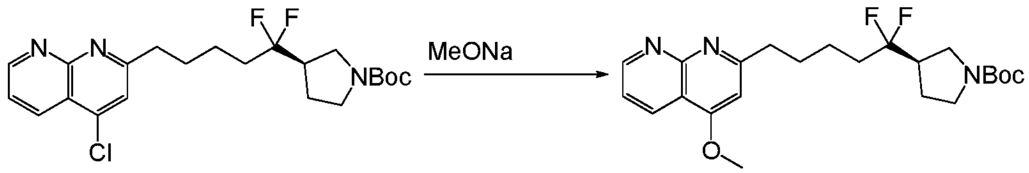


To a solution of (R)-tert-butyl 3-(5-(4-chloro-1,8-naphthyridin-2-yl)-1,1-difluoropentyl)pyrrolidine-1-carboxylate (0.95 g, 2.16 mmol) in MeOH (30 mL)was added sodium methoxide (233 mg, 4.32 mmol). The reaction was refluxed for 15 hours, then concentrated in vacuo. The residue was dissolved in ethyl acetate (20 mL), washed with water (2 × 20 mL), dried over MgSO4, filtered and concentrated in vacuo to give the desired product as a colorless oil (750 mg). Yield 80% (ESI 436 (M+H) +).

**Step 3: (R)-7-(5,5-difluoro-5-(pyrrolidin-3-yl)pentyl)-5-methoxy-1,2,3,4-tetrahydro-1,8-naphthyridine dihydrochloride**

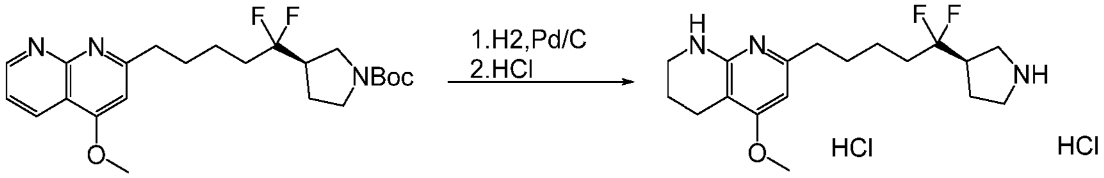


A mixture of (R)-tert-butyl 3-(1,1-difluoro-5-(4-methoxy-1,8-naphthyridin-2-yl)pentyl)pyrrolidine-1-carboxylate (750 mg, 1.72 mmol) and Pd/C (10%, 75 mg) in EtOAc (50 mL) was stirred under balloon hydrogen at room temperature for 16 hours. The mixture was filtered and concentrated in vacuo. The residue was treated with HCl in 1,4-dioxane (4M, 10 mL) at 25 °C for 2 hours. Solvent was removed in vacuo to give (R)-7-(5,5-difluoro-5-(pyrrolidin-3-yl)pentyl)-5-methoxy-1,2,3,4-tetrahydro-1,8-naphthyridine dihydrochloride as a colorless oil (620 mg). Yield 88% (ESI 340 (M+H) +).

**Preparation of 7-(5-fluoro-5-((R)-pyrrolidin-3-yl)pentyl)-1,2,3,4-tetrahydro-1,8-naphthyridine dihydrochloride**

**Step 1: (3R)-tert-butyl 3-(1-hydroxypent-4-enyl)pyrrolidine-1-carboxylate**

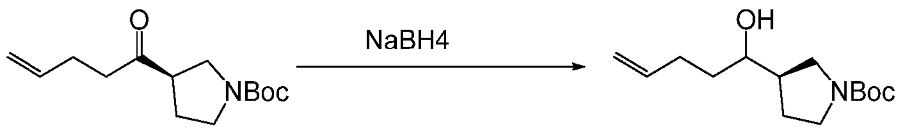


To a solution of (R)-tert-butyl 3-pent-4-enoylpyrrolidine-1-carboxylate (253 mg, 1 mmol) in MeOH (10 mL) at 0 °C, was added NaBH4 (46 mg, 1.2 mmol). The reaction was stirred at 0 °C for 1 hour. Solvent was removed in vacuo, and the residue was purified by silica gel column (pet ether: EtOAc 2:1) to give the desired product (3R)-tert-butyl 3-(1-hydroxypent-4-enyl)pyrrolidine-1-carboxylate as a colorless oil (210 mg). Yield 82% (ESI 256 (M+H) +).

**Step 2: (3R)-tert-butyl 3-(1-fluoropent-4-enyl)pyrrolidine-1-carboxylate**

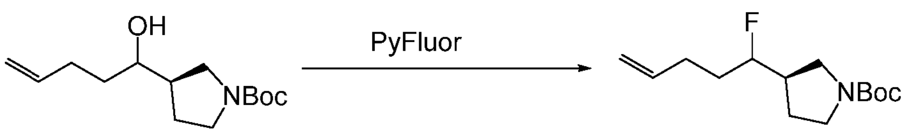


A mixture of (3R)-tert-butyl 3-(1-hydroxypent-4-enyl)pyrrolidine-1-carboxylate (210 mg, 0.82mmol), PyFluor (199mg, 1.23mmol) and DBU (187mg, 1.23mmol) in toluene (2 mL) in a sealed tube was stirred at 50 °C overnight. The

reaction mixture was partitioned between 0.5 M aq. HCl (10 mL) and EtOAc (10 mL). The layers were separated and the aqueous layer was extracted with EtOAc (2x 10 mL). The combined organic layer was concentrated in vacuo, and the residue was purified by silica gel column (pet ether: EtOAc=2:1) to give the desired product (3R)-tert-butyl 3-(1-fluoropent-4-enyl)pyrrolidine-1-carboxylate as a colorless oil (85 mg). Yield 40 % (ESI 258 (M+H) +). The racemic product was separated by Prep chiral SFC (IG column) to give (3R)-tert-butyl 3-(1-fluoropent-4-enyl)pyrrolidine-1-carboxylate stereoisomer A (Rt = 1.88 min, 30 mg) and (3R)-tert-butyl 3-(1-fluoropent-4-enyl)pyrrolidine-1-carboxylate stereoisomer B (Rt = 2.96 min, 25 mg) as colorless oil.

**Step 3: tert-butyl 7-(5-((R)-1-(tert-butoxycarbonyl)pyrrolidin-3-yl)-5-fluoropentyl)-3,4-dihydro-1,8-naphthyridine-1(2H)-carboxylate stereoisomer A**

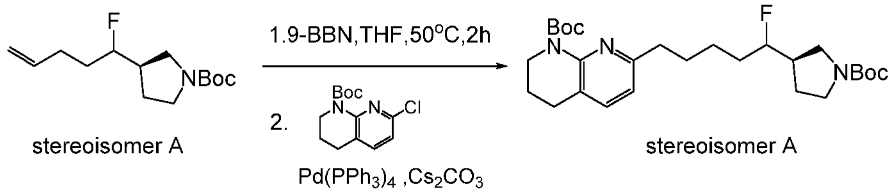


To a solution of (3R)-tert-butyl 3-(1-fluoropent-4-enyl)pyrrolidine-1-carboxylate stereoisomer A (1 g, 3.89 mmol) in THF (dry, 10 mL) under Ar, was added 9-BBN (0.5M solution in THF, 15.6 mL, 7.78 mmol). The reaction was stirred at 50 °C for 2 hours, then cooled to room temperature. This solution was added to a mixture of tert-butyl 7-chloro-3,4-dihydro-1,8-naphthyridine-1(2H)-carboxylate (1.05 g, 3.89 mmol), cesium carbonate (3.81 g, 11.7 mmol) and Pd(PPh3)4 (225 mg, 0.19 mmol) in 1,4-Dioxane (15 mL). The reaction was stirred at 90 °C for 17 hours. Solvent was removed in vacuo, and the residue was purified by silica gel column (pet ether: EtOAc=1:1 to 1:10) to give the desired product tert-butyl 7-(5-((R)-1-(tert-butoxycarbonyl)pyrrolidin-3-yl)-5-fluoropentyl)-3,4-dihydro-1,8-naphthyridine-1(2H)-carboxylate stereoisomer A as a yellow oil (1.1 g). Yield 57% (ESI 492 (M+H) +).

**Step 4: 7-(5-fluoro-5-((R)-pyrrolidin-3-yl)pentyl)-1,2,3,4-tetrahydro-1,8-naphthyridine stereoisomer A dihydrochloride**

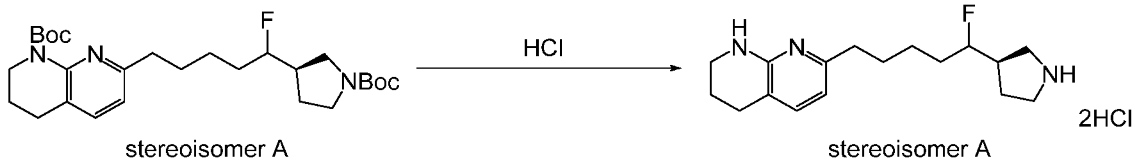


tert-butyl 7-(5-((R)-1-(tert-butoxycarbonyl)pyrrolidin-3-yl)-5-fluoropentyl)-3,4-dihydro-1,8-naphthyridine-1(2H)-carboxylate stereoisomer A (1.1 g, 2.24 mmol) was treated with HCl in 1,4-dioxane (4M, 20 mL) at 25 °C for 2 hours. Solvent was removed in vacuo to give the desired product 7-(5-fluoro-5-((R)-pyrrolidin-3-yl)pentyl)-1,2,3,4-tetrahydro-1,8-naphthyridine stereoisomer A dihydrochloride as a colorless oil (783 mg). Yield 96% (ESI 292 (M+H) +).

**7-(5-fluoro-5-((R)-pyrrolidin-3-yl)pentyl)-1,2,3,4-tetrahydro-1,8-naphthyridine dihydrochloride stereoisomer B** was prepared by the same method starting from (3R)-tert-butyl 3-(1-fluoropent-4-enyl)pyrrolidine-1-carboxylate stereoisomer B.

**7-(5-fluoro-5-((R)-pyrrolidin-3-yl)pentyl)-1,2,3,4-tetrahydro-1,8-naphthyridine dihydrochloride** was also prepared as a mixture of stereoisomer A and stereoisomer B by the same method starting from (3R)-tert-butyl 3-(1-fluoropent-4-enyl)pyrrolidine-1-carboxylate mixture of stereoisomer A and stereoisomer B.

**Preparation of 7-(5-fluoro-5-((R)-pyrrolidin-3-yl)pentyl)-5-methoxy-1,2,3,4-tetrahydro-1,8-naphthyridine dihydrochloride**

**Step 1: tert-butyl (3R)-3-(5-(4-chloro-1,8-naphthyridin-2-yl)-1-fluoropentyl)pyrrolidine-1-carboxylate stereoisomer A**

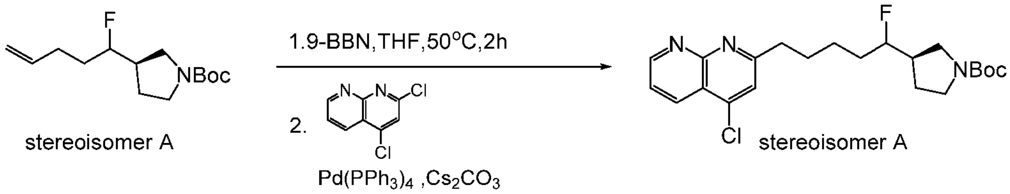


To a solution of (3R)-tert-butyl 3-(1-fluoropent-4-enyl)pyrrolidine-1-carboxylate stereoisomer A (1.3 g, 5.0 mmol) in THF (dry, 5 mL) under Ar, was added 9-BBN (0.5M solution in THF, 20 mL, 10.0 mmol). The reaction was stirred at 50 °C for 2 hours, then cooled to room temperature. This solution was added to a mixture of 2,4-dichloro-1,8-naphthyridine (1.01 g, 5.1 mmol), cesium carbonate (4.98 g, 15.3 mmol) and Pd(PPh3)4 (295 mg, 0.255 mmol) in 1,4-dioxane (10 mL). The reaction was stirred at 90 °C for 2 hours. Solvent was removed in vacuo, and the residue was purified by silica gel column (DCM:MeOH 30:1) to give the desired product tert-butyl (3R)-3-(5-(4-chloro-1,8-naphthyridin-2-yl)-1-fluoropentyl)pyrrolidine-1-carboxylate stereoisomer A as a yellow oil (0.95 g). Yield 45% (ESI 422 (M+H) +).

**Step 2: tert-butyl (3R)-3-(1-fluoro-5-(4-methoxy-1,8-naphthyridin-2-yl)pentyl)pyrrolidine-1-carboxylate stereoisomer A**

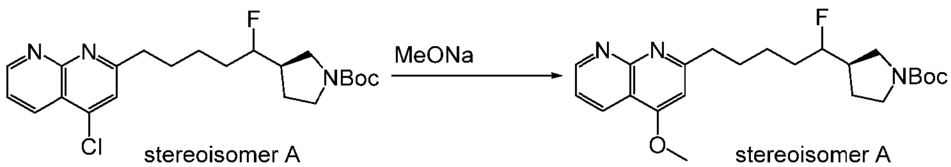


To a solution of (3R)-3-(5-(4-chloro-1,8-naphthyridin-2-yl)-1-fluoropentyl)pyrrolidine-1-carboxylate stereoisomer A (0.95 g, 2.25 mmol) in MeOH (30 mL) was added NaOMe (243 mg, 4.50 mmol). The reaction was refluxed for 15 hours, then concentrated in vacuo. The residue was dissolved in ethyl acetate (20 mL), washed with water (2 x 20 mL), dried over MgSO4, filtered, and concentrated in vacuo to give the desired product tert-butyl (3R)-3-(1-fluoro-5-(4-methoxy-1,8-naphthyridin-2-yl)pentyl)pyrrolidine-1-carboxylate stereoisomer A as a colorless oil (780 mg). Yield 83% (ESI 418 (M+H) +).

**Step 3: 7-(5-fluoro-5-((R)-pyrrolidin-3-yl)pentyl)-5-methoxy-1,2,3,4-tetrahydro-1,8-naphthyridine stereoisomer A dihydrochloride**

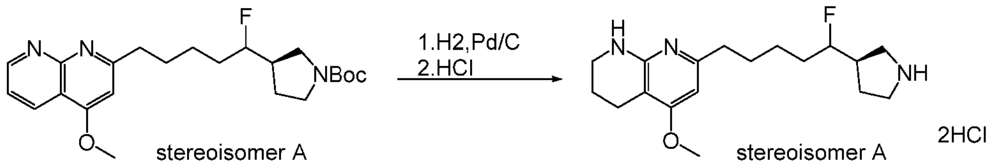


A mixture of tert-butyl (3R)-3-(1-fluoro-5-(4-methoxy-1,8-naphthyridin-2-yl)pentyl)pyrrolidine-1-carboxylate stereoisomer A (780 mg, 1.87 mmol) and Pd/C (10%, 78 mg) in EtOAc (50 mL) was stirred under balloon hydrogen at room temperature for 16 hours. The mixture was filtered and concentrated in vacuo. The residue was treated with HCl in 1,4-dioxane(4M, 10 mL) at 25 °C for 2 hours. Solvent was removed in vacuo to give 7-(5-fluoro-5-((R)-pyrrolidin-3-yl) pentyl)-5-methoxy-1,2,3,4-tetrahydro-1,8-naphthyridine stereoisomer A dihydrochloride as a colorless oil (690 mg). Yield 94% (ESI 322 (M+H)+).

**7-(5-fluoro-5-((R)-pyrrolidin-3-yl)pentyl)-5-methoxy-1,2,3,4-tetrahydro-1,8-naphthyridine stereoisomer B dihydrochloride** was prepared by the same method starting from (3R)-tert-butyl 3-(1-fluoropent-4-enyl)pyrrolidine-1-carboxylate stereoisomer B.

**7-(5-fluoro-5-((R)-pyrrolidin-3-yl)pentyl)-5-methoxy-1,2,3,4-tetrahydro-1,8-naphthyridine dihydrochloride** was also prepared as a mixture of stereoisomer A and stereoisomer B by the same method starting from (3R)-tert-butyl 3-(1-fluoropent-4-enyl)pyrrolidine-1-carboxylate mixture of stereoisomer A and stereoisomer B.

**Preparation of (S)-7-(4,4-difluoro-5-(pyrrolidin-3-yl)pentyl)-1,2,3,4-tetrahydro-1,8-naphthyridine dihydrochloride**

**Step 1: tert-butyl (S)-3-(2-(methoxy(methyl)amino)-2-oxoethyl)pyrrolidine-1-carboxylate**

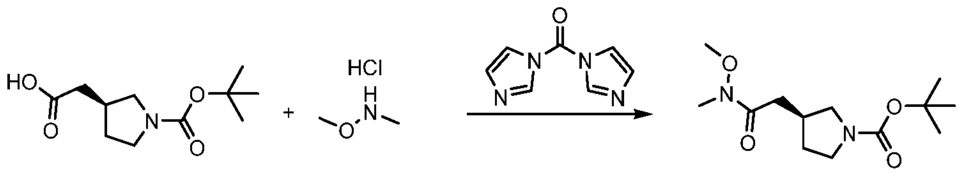

To a solution of (S)-2-(1-(tert-butoxycarbonyl)pyrrolidin-3-yl)acetic acid (3 g, 13.08 mmol) in dichloromethane (75 mL) was added CDI (2.122 g, 13.08 mmol), and the mixture was stirred for 1.5 hours at room temperature. Next, N,O-dimethylhydroxylamine hydrochloride (1.276 g, 13.08 mmol) was added, and after stirring for an additional 3 hours, the mixture was quenched with hydrochloric acid (1 M solution in water). The organic phase was separated, and the aqueous phase was extracted twice with dichloromethane. The combined organic layers were washed with saturated aqueous sodium hydrogencarbonate, dried over sodium sulfate and concentrated in vacuo. The residue was purified by flash column chromatography (80 g silica; heptane, 10%-80% ethyl actate) to give the desired product tert-butyl (S)-3-(2-(methoxy(methyl)amino)-2-oxoethyl)pyrrolidine-1-carboxylate (2.95 g). Yield 83% (ESI 217 (M-tBu+H) $^+$).

**Step 2: tert-butyl (S)-3-(2-oxopent-4-en-1-yl)pyrrolidine-1-carboxylate**

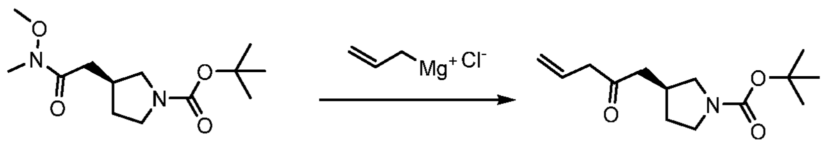

To an ice cooled solution of tert-butyl (S)-3-(2-(methoxy(methyl)amino)-2-oxoethyl)pyrrolidine-1-carboxylate (4.77 g, 17.51 mmol) in THF (90 mL) was added allylmagnesium chloride (12.36 mL, 21.02 mmol, 1.7 M solution in THF) dropwise over a period of 10 minutes. After 10 minutes cooling was stopped, and the mixture was stirred at room temperature for 90 minutes, then poured into saturated aqueous ammonium chloride, diluted with water and extracted three times with ethyl acetate. The combined organic layers were washed with brine, dried over sodium sulfate, and concentrated. The residue was purified by flash column chromatography (80 g silica; heptane, 10%-40% ethyl actate) to give the desired product tert-butyl (S)-3-(2-oxopent-4-en-1-yl)pyrrolidine-1-carboxylate (4.05 g). Yield 91% (ESI 198 (M-tBu+H) $^+$).

**Step 3: tert-butyl (S)-3-(2,2-difluoropent-4-en-1-yl)pyrrolidine-1-carboxylate**

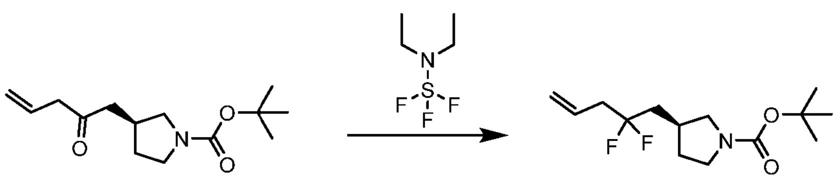

In a 24 mL vial, DAST (21.79 mL, 178 mmol) was added to tert-butyl (S)-3-(2-oxopent-4-en-1-yl)pyrrolidine-1-carboxylate (3.69 g, 14.57 mmol). The vial was sealed and stirred for 20 hours at room temperature. The mixture was diluted with dichloromethane (500 mL) and dropwise added to saturated aqueous sodium hydrogencarbonate. The layers were separated, and the water layer was extracted twice with dichloromethane. The combined organic layers were washed with brine, dried over sodium sulfate, concentrated and the residue was purified by flash column chromatography (220 g silica; heptane, 2%-20% ethyl actate) to give the desired product tert-butyl (S)-3-(2,2-difluoropent-4-en-1-yl) pyrrolidine-1-carboxylate (1.01 g). Yield 25% (ESI 220 (M-tBu+H) $^+$).

**Step 4: tert-butyl 7-chloro-3,4-dihydro-1,8-naphthyridine-1(2H)-carboxylate**

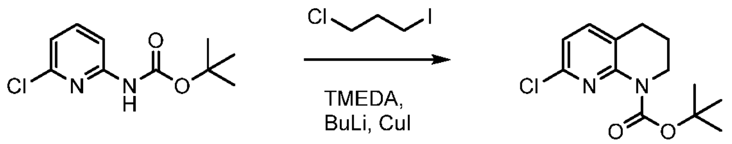


To a solution of TMEDA (4.36 mL, 28.9 mmol) in THF (30 mL) at -20 °C was added n-butyllithium (12.03 mL, 28.9 mmol, 2.4 M solution in hexanes) over ten minutes. The mixture was stirred at 10 °C for 30 minutes, then cooled to -78 °C, and a solution of tert-butyl (6-chloropyridin-2-yl)carbamate (3 g, 13.12 mmol) in THF (15 mL) was added over a period of 5 minutes. After 40 minutes, copper(I) iodide (2.499 g, 13.12 mmol) was added, and the mixture was stirred at -10 °C for 1 hour. 1-Chloro-3-iodopropane (2.113 mL, 19.68 mmol) was added over a period of 1 minute, and the mixture was allowed to reach room temperature. After 2.5 hours the mixture was added to saturated aqueous sodium hydrogencarbonate and extracted three times with ethyl acetate. The combined organic layers were dried over sodium sulfate, filtered and concentrated. The residue was stirred in dichloromethane (30 mL), filtered through a glass-sintered funnel and concentrated to half volume and then applied onto a flash column (120 g silica), eluting with 2-20% ethyl acetate in heptane. The eluent coming off the column was passed through a column of potassium carbonate. This gave the desired product tert-butyl 7-chloro-3,4-dihydro-1,8-naphthyridine-1(2H)-carboxylate (2.637 g). Yield 74% $^1$H NMR (400 MHz, Chloroform-d) δ 7.32 (d, $J$ = 7.9 Hz, 1H), 6.94 (d, $J$ = 7.8 Hz, 1H), 3.81 - 3.69 (m, 2H), 2.73 (t, $J$ = 6.6 Hz, 2H), 1.93 (p, $J$ = 6.5 Hz, 2H), 1.54 (s, 9H).

**Step 5: Tert-butyl (S)-7-(5-(1-(tert-butoxycarbonyl)pyrrolidin-3-yl)-4,4-difluoropentyl)-3,4-dihydro-1,8-naphthyridine-1(2H)-carboxylate**

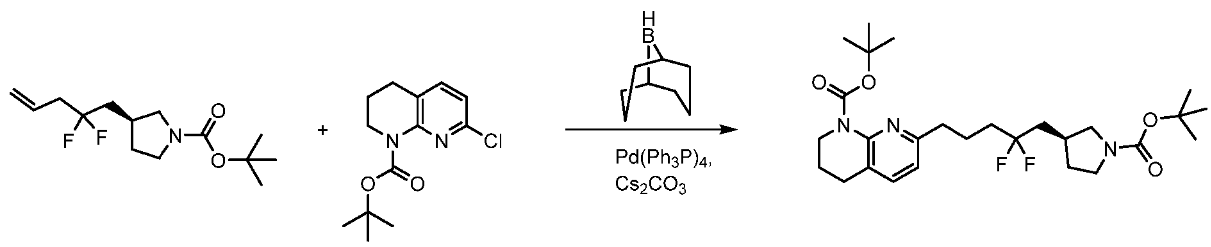


A solution of tert-butyl (S)-3-(2,2-difluoropent-4-en-1-yl)pyrrolidine-1-carboxylate (205 mg, 0.745 mmol) in THF (2 mL) was purged with argon for 10 minutes. Then, 9-BBN (1.638 mL, 0.819 mmol, 0.5 M solution in THF) was added. After 1 hour, additional 9-BBN, 0.5 M solution in THF (0.893 mL, 0.447 mmol) was added. After 30 minutes, this solution was added to a mixture of tert-butyl 7-chloro-3,4-dihydro-1,8-naphthyridine-1(2H)-carboxylate (200 mg, 0.745 mmol), cesium carbonate (364 mg, 1.117 mmol) and tetrakis(triphenylphosphine)palladium(0) (43.0 mg, 0.037 mmol) in 1,4-dioxane (3 mL), which had been purged with argon for 10 minutes. The resulting mixture was heated to 70 °C for 16 hours, then cooled to room temperature, diluted with water and extracted with ethyl acetate. The organic layer was washed brine, dried over sodium sulfate and concentrated. The residue was purified by flash column chromatography (12 g; heptane, 10%-33% ethyl actate) to give the desired product tert-butyl (S)-7-(5-(1-(tert-butoxycarbonyl)pyrrolidin-3-yl)-4,4-difluoropentyl)-3,4-dihydro-1,8-naphthyridine-1(2H)-carboxylate (216 mg). Yield 51% (ESI 510 (M+H) $^+$).

**Step 6: (S)-7-(4,4-difluoro-5-(pyrrolidin-3-yl)pentyl)-1,2,3,4-tetrahydro-1,8-naphthyridine dihydrochloride**

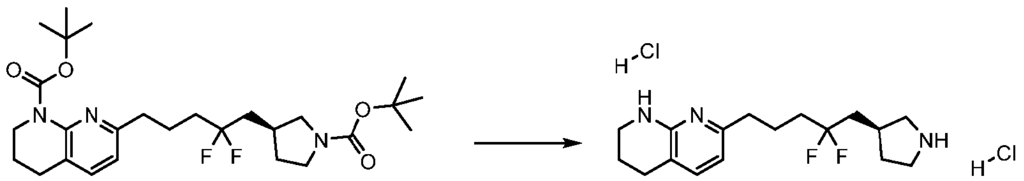

To a solution of tert-butyl (S)-7-(5-(1-(tert-butoxycarbonyl)pyrrolidin-3-yl)-4,4-difluoropentyl)-3,4-dihydro-1,8-naphthyridine-1(2H)-carboxylate (827 mg, 1.623 mmol) in methanol (16 mL) was added hydrochloric acid (16.23 mL, 64.9 mmol, 4N solution in dioxane). After 22 hours, additional hydrochloric acid (8.11 mL, 32.5 mmol, 4N solution in dioxane) was added. The mixture was stirred for 23 hours, then concentrated, and the residue was co-evaporated twice with acetonitrile to give the desired product (S)-7-(4,4-difluoro-5-(pyrrolidin-3-yl)pentyl)-1,2,3,4-tetrahydro-1,8-naphthyridine dihydrochloride (674 mg). Yield 100% (ESI 310 (M-2HCl+H)$^+$). $^1$H NMR (400 MHz, DMSO-$d_6$) δ 14.40 (s, 1H), 9.40 (d, *J* = 26.3 Hz, 2H), 8.13 (s, 1H), 7.61 (d, *J* = 7.3 Hz, 1H), 6.63 (d, *J* = 7.3 Hz, 1H), 3.53 - 3.26 (m, 4H), 3.26 - 3.14 (m, 1H), 3.14 - 2.98 (m, 1H), 2.82 - 2.65 (m, 4H), 2.47 - 2.29 (m, 1H), 2.21 - 2.02 (m, 4H), 2.02 - 1.72 (m, 5H), 1.63 - 1.48 (m, 1H).

**Preparation of 2-(4-(((R)-pyrrolidin-3-yl)oxy)butyl)-1,2,3,4-tetrahydro-1,8-naphthyridine dihydrochloride**

**Step 1: Tert-butyl (R)-3-(hex-5-en-1-yloxy)pyrrolidine-1-carboxylate**

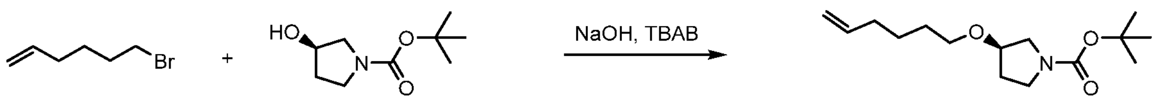


To a suspension of tert-butyl (R)-3-hydroxypyrrolidine-1-carboxylate (12.8 g, 68.4 mmol), tetrabutylammonium bromide (1.102 g, 3.42 mmol) and 6-bromo-1-hexene (13.71 mL, 103 mmol) in heptane (256 mL) was added sodium hydroxide (128 mL, 68.4 mmol, 50 wt% solution in water). The mixture was vigourously stirred at 80 °C for 2 hours, then cooled to room temperature, diluted with water and extracted with heptane and twice with diethyl ether/heptane. The combined organic layers were washed with brine, dried over sodium sulfate and concentrated in vacuo. Purification by column chromatography (600 g silica, 5 -> 14% ethyl acetate in heptane) afforded the desired product tert-butyl (R)-3-(hex-5-en-1-yloxy)pyrrolidine-1-carboxylate (14.93 g). Yield 81%. $^1$H NMR (400 MHz, Chloroform-*d*) δ 5.87 - 5.74 (m, 1H), 5.05 - 4.91 (m, 2H), 4.04 - 3.95 (m, 1H), 3.48 - 3.27 (m, 6H), 2.07 (q, J = 7.2 Hz, 2H), 2.02 - 1.84 (m, 2H), 1.60 - 1.51 (m, 2H), 1.51 - 1.38 (m, 11H).

**Step 2: tert-butyl (R)-3-((5-oxopentyl)oxy)pyrrolidine-1-carboxylate**

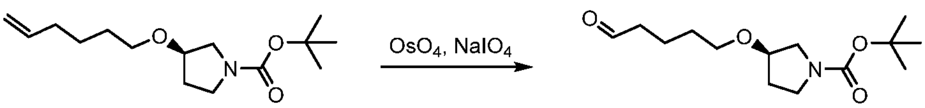


To a solution of tert-butyl (R)-3-(hex-5-en-1-yloxy)pyrrolidine-1-carboxylate (14.93 g, 55.4 mmol) in THF (420 mL) and water (140 mL) was added sodium periodate (26.1 g, 122 mmol) and osmium tetroxide (1.5 mL, 0.232 mmol, 4% solution in water). After 1 hour, additional sodium periodate (5 g, 23.38 mmol) was added. After 30 minutes, the mixture was diluted with water and extracted three times with ethyl acetate. The combined organic layers were washed with brine, dried over sodium sulfate and concentrated in vacuo. The residue was purified by flash column chromatography (~600 g silica, 20 ->50% ethyl acetate in heptane). This afforded the desired product tert-butyl (R)-3-((5-oxopentyl)oxy)pyrrolidine-1-carboxylate (10.97 g). Yield 72%. $^1$H NMR (400 MHz, Chloroform-d) δ 9.77 (s, 1H), 4.03 - 3.94 (m, 1H), 3.50 - 3.27 (m, 6H), 2.47 (t, J = 7.2 Hz, 2H), 2.02 - 1.84 (m, 2H), 1.77 - 1.65 (m, 2H), 1.65 - 1.54 (m, 2H), 1.46 (s, 9H).

**Step 3: tert-butyl (3R)-3-((5-hydroxyhept-6-en-1-yl)oxy)pyrrolidine-1-carboxylate**

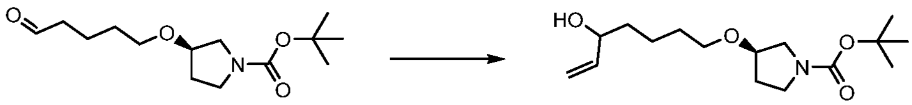

To a solution of tert-butyl (R)-3-((5-oxopentyl)oxy)pyrrolidine-1-carboxylate (10.97 g, 40.4 mmol) in THF (70 mL) at 0 °C was added dropwise vinylmagnesium bromide (66.4 mL, 46.5 mmol, 0.7 M solution in THF). After 16 hours, the mixture was quenched with aqueous saturated ammonium chloride and extracted with ethyl acetate. The organic layer was washed with brine, dried over sodium sulfate and concentrated, and the residue was purified by flash column chromatography (20->50% EtOAc in heptane) to give the desired product tert-butyl (3R)-3-((5-hydroxyhept-6-en-1-yl) oxy)pyrrolidine-1-carboxylate (7.68 g). Yield 63%. $^1$H NMR (400 MHz, Chloroform-*d*) δ 5.93 - 5.80 (m, 1H), 5.28 - 5.17 (m, 1H), 5.15 - 5.06 (m, 1H), 4.17 - 4.05 (m, 1H), 3.99 (s, 1H), 3.52 - 3.23 (m, 6H), 2.03 - 1.83 (m, 2H), 1.65 - 1.50 (m, 5H), 1.50 - 1.33 (m, 11H).

**Step 4: tert-butyl (R)-3-((7-(2-chloropyridin-3-yl)-5-oxoheptyl)oxy)pyrrolidine-1-carboxylate**

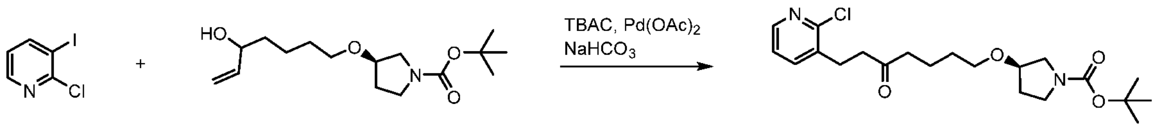


Tert-butyl (3R)-3-((5-hydroxyhept-6-en-1-yl)oxy)pyrrolidine-1-carboxylate (10.48 g, 35.0 mmol), 2-chloro-3-iodopyridine (4.19 g, 17.50 mmol), tetrabutylammonium chloride (0.486 g, 1.750 mmol) and sodium hydrogencarbonate (3.68 g, 43.8 mmol) were dissolved/suspended under argon in DMF (35 mL), and argon was bubbled through this mixture for 15 minutes. Palladium(II) acetate (0.393 g, 1.750 mmol) was added, and the mixture was heated to 50 °C for 24 hours, then cooled to room temperature, diluted with water and extracted three times with ethyl acetate. The combined organic layers were washed three times with brine, dried over sodium sulfate and concentrated, and the residue was purified by column chromatography (silica, 20 -> 55% EtOAc in heptane) to afford the desired product tert-butyl (R)-3-((7-(2-chloropyridin-3-yl)-5-oxoheptyl)oxy)pyrrolidine-1-carboxylate (3.05 g). Yield 42%. $^1$H NMR (400 MHz, Chloroform-d) δ 8.25 (dd, J = 4.7, 1.9 Hz, 1H), 7.62 (dd, J = 7.5, 1.9 Hz, 1H), 7.17 (dd, J = 7.5, 4.7 Hz, 1H), 4.04 - 3.93 (m, 1H), 3.51 - 3.25 (m, 6H), 2.99 (t, J = 7.3 Hz, 2H), 2.79 (t, J = 7.3 Hz, 2H), 2.43 (t, J = 7.2 Hz, 2H), 2.01 - 1.83 (m, 2H), 1.72 - 1.48 (m, 4H), 1.30 (s, 9H).

**Step 5: tert-butyl (R)-3-((5-(((R)-tert-butylsulfinyl)imino)-7-(2-chloropyridin-3-yl)heptyl)oxy)pyrrolidine-1-carboxylate**

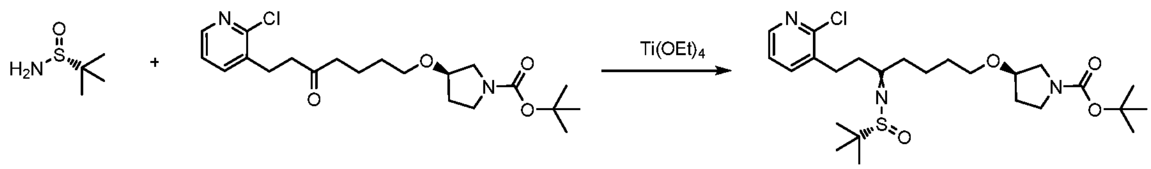


To a solution of (R)-(+)-2-methyl-2-propanesulfinamide (1.799 g, 14.84 mmol) and tert-butyl (R)-3-((7-(2-chloropyridin-3-yl)-5-oxoheptyl)oxy)pyrrolidine-1-carboxylate (3.05 g, 7.42 mmol) in THF (30 mL) was added titanium(IV) ethoxide (7.24 mL, 22.27 mmol). The resulting mixture was heated to 50 °C for 20 hours, then poured out on half-saturated aqueous sodium hydrogencarbonate, stirred for 10 minutes, transferred to 2 centrifuge vials and centrifuged for 5 min at 7800 rpm. The liquids were decanted into a separation funnel. The vials were then filled with ethyl acetate, shaken vigorously and centrifuged again for 5 minutes at 7800 rpm. The liquids were combined in the separation funnel. The layers were separated, and the aqueous phase was extracted once more with ethyl acetate. The combined organic layers were washed with brine, dried over sodium sulfate and concentrated, and the residue was purified by flash column chromatography (300 g silica, 35 - 65% EtOAc in heptane) to afford the desired product tert-butyl (R)-3-((5-(((R)-tert-butylsulfinyl) imino)-7-(2-chloropyridin-3-yl)heptyl)oxy)pyrrolidine-1-carboxylate (3.15 g). Yield 78% (ESI 514/516 (M+H) +). $^1$H NMR (400 MHz, Chloroform-*d*) δ 8.27 (dd, J = 4.7, 1.9 Hz, 1H), 7.64 (dd, J = 62.5, 7.4 Hz, 1H), 7.19 (dd, J = 7.5, 4.8 Hz, 1H), 3.99 (s, 1H), 3.54 - 3.24 (m, 6H), 3.16 - 2.87 (m, 2H), 2.87 - 2.60 (m, 2H), 2.60 - 2.35 (m, 1H), 1.94 (s, 2H), 1.77 - 1.53 (m, 5H), 1.45

(s, 9H), 1.35 - 1.11 (m, 9H).

**Step 6: tert-butyl (3R)-3-((5-(((R)-tert-butylsulfinyl)amino)-7-(2-chloropyridin-3-yl)heptyl)oxy)pyrrolidine-1-carboxylate**

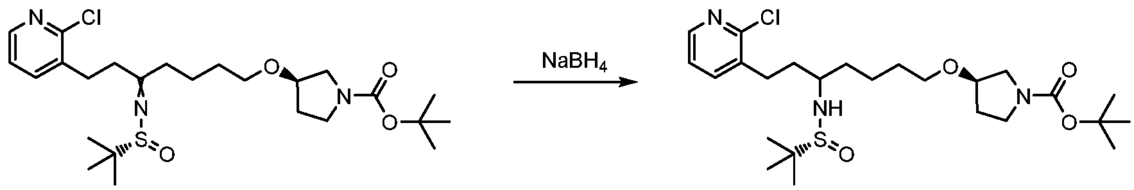


To a solution of tert-butyl (R)-3-((5-(((R)-tert-butylsulfinyl)imino)-7-(2-chloropyridin-3-yl)heptyl)oxy)pyrrolidine-1-carboxylate (3.15 g, 6.13 mmol) in methanol (20 mL) was added sodium borohydride (0.278 g, 7.35 mmol). After 2 hours, the mixture was quenched with saturated aqueous ammonium chloride and extracted three times with ethyl acetate. The combined organic layers were washed with brine, dried over sodium sulfate and concentrated, and the residue was purified by flash column chromatography (50 -> 100% EtOAc in heptane) to afford the desired product tert-butyl (3R)-3-((5-(((R)-tert-butylsulfinyl)amino)-7-(2-chloropyridin-3-yl)heptyl)oxy)pyrrolidine-1-carboxylate (3.05 g). Yield 85%. $^1$H NMR (400 MHz, Chloroform-*d*) δ 8.28 - 8.22 (m, 1H), 7.72 - 7.51 (m, 1H), 7.22 - 7.15 (m, 1H), 4.03 - 3.95 (m, 1H), 3.49 - 3.22 (m, 7H), 3.16 - 3.06 (m, 1H), 2.96 - 2.64 (m, 2H), 2.00 - 1.83 (m, 4H), 1.80 - 1.70 (m, 1H), 1.65 - 1.51 (m, 3H), 1.51 - 1.37 (m, 11H), 1.25 (s, 9H).

**Step 7: tert-butyl (3R)-3-(4-(1-((R)-tert-butylsulfinyl)-1,2,3,4-tetrahydro-1,8-naphthyridin-2-yl)butoxy)pyrrolidine-1-carboxylate**

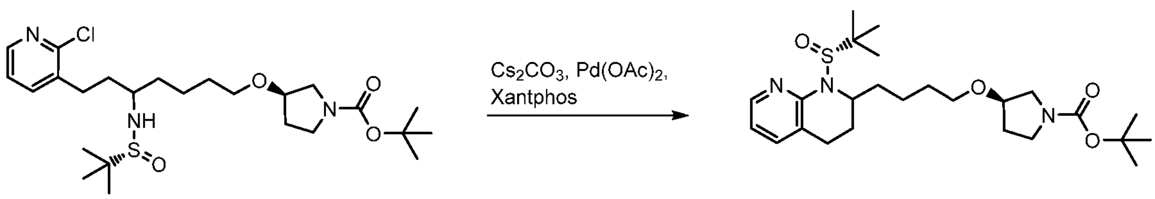


To a solution of tert-butyl (3R)-3-((5-(((R)-tert-butylsulfinyl)amino)-7-(2-chloropyridin-3-yl)heptyl)oxy)pyrrolidine-1-carboxylate (3.05 g, 5.20 mmol) in 1,4-dioxane (25 mL) was added Xantphos (0.602 g, 1.040 mmol) and cesium carbonate (3.39 g, 10.40 mmol). The mixture was bubbled through with argon for 15 minutes. Palladium(II) acetate (0.117 g, 0.520 mmol) was added, and the reaction was bubbled through with argon for 1 minute and stirred at 100 °C for16 hours, then cooled to room temperature, quenched with water and extracted three times with ethyl acetate. The combined organic layers were washed with brine, dried over sodium sulfate and concentrated, and the residue was purified by flash column chromatography (40 -> 100% EtOAc in heptane) to afford the desired product tert-butyl (3R)-3-(4-(1-((R)-tert-butylsulfinyl)-1,2,3,4-tetrahydro-1,8-naphthyridin-2-yl)butoxy)pyrrolidine-1-carboxylate (928 mg). Yield 36% (ESI 480 (M+H) +). The compound was separated by chiral SFC to give stereoisomer A and stereoisomer B. Apparatus: Waters Prep 100 SFC UV directed system; Waters 2998 Photodiode Array (PDA) Detector; Waters 2767 Sample Manager; Masslynx™ Software; FractionLynx™ Application Manager, Acq. Method: Cell-2_f70_10_50_8mn_SW_120bar, Loading: 50 mg, Column: Phenomenex Lux Cellulose-2 (250x21.2 mm, 5 μm), Flow: 70 mL/min, Column temp: 35°C; ABPR: 120 bar; Eluent A: $CO_2$, Eluent B: 20 mM ammonia in methanol, Linear gradient: t=0 min 10% B, t=5 min 50% B, t=7.5 min 50% B, t=8 min 10% B. Injection: Sandwich 100 μl methanol, Detection PDA: 210-320 nm, Collection: Based on PDA TIC.

tert-butyl (3R)-3-(4-(1-((R)-tert-butylsulfinyl)-1,2,3,4-tetrahydro-1,8-naphthyridin-2-yl)butoxy)pyrrolidine-1-carboxylate stereoisomer A: 0.58 grams, LC/MS ESI 480 (M+H) +. $^1$H NMR (400 MHz, Chloroform-*d*) δ 8.06 - 7.98 (m, 1H), 7.37 - 7.29 (m, 1H), 6.73 - 6.63 (m, 1H), 4.20 - 4.09 (m, 1H), 4.03 - 3.93 (m, 1H), 3.50 - 3.25 (m, 6H), 2.94 - 2.79 (m, 1H), 2.77 - 2.65 (m, 1H), 2.20 - 2.08 (m, 1H), 2.00 - 1.49 (m, 7H), 1.46 (s, 9H), 1.43 - 1.27 (m, 2H), 1.21 (s, 9H).

tert-butyl (3R)-3-(4-(1-((R)-tert-butylsulfinyl)-1,2,3,4-tetrahydro-1,8-naphthyridin-2-yl)butoxy)pyrrolidine-1-carboxylate stereoisomer B: 0.45 grams, LC/MS ESI 480 (M+H) +. $^1$H NMR (400 MHz, Chloroform-*d*) δ 8.20 - 8.09 (m, 1H), 7.39 - 7.31 (m, 1H), 6.90 - 6.80 (m, 1H), 4.25 - 4.12 (m, 1H), 4.04 - 3.92 (m, 1H), 3.51 - 3.24 (m, 6H), 2.87 - 2.62 (m, 2H), 2.11 - 1.83 (m, 3H), 1.76 - 1.49 (m, 6H), 1.46 (s, 9H), 1.40 - 1.27 (m, 10H).

**Step 8: 2-(4-(((R)-pyrrolidin-3-yl)oxy)butyl)-1,2,3,4-tetrahydro-1,8-naphthyridine stereoisomer A dihydrochloride**

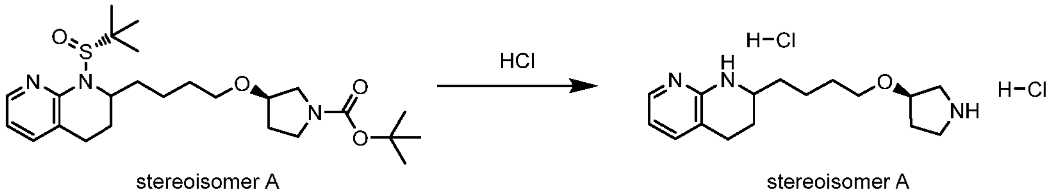


To a solution of tert-butyl (3R)-3-(4-(1-((R)-tert-butylsulfinyl)-1,2,3,4-tetrahydro-1,8-naphthyridin-2-yl)butoxy) pyrrolidine-1-carboxylate stereoisomer A (0.58 g, 1.209 mmol) in methanol (5 mL) was added hydrochloric acid (5 mL, 20.00 mmol, 4N solution in dioxane). The mixture was stirred at room temperature for 3 hours, then concentrated in vacuo and coevaporated with methanol. Diethyl ether was added which started a slow crystallisation of the product. After standing overnight the crystallised material was scratched loose, and the material was triturated with diethyl ether. After a few hours the solids were collected by filtration, rinsed with fresh diethyl ether and dried under vacuum to afford the desired product 2-(4-(((R)-pyrrolidin-3-yl)oxy)butyl)-1,2,3,4-tetrahydro-1,8-naphthyridine stereoisomer A dihydrochloride (438 mg) as a beige solid. Yield 100%. LC/MS ESI 276 (M-2HCl+H) +. $^1$H NMR (400 MHz, Methanol-d4) δ 7.75 - 7.66 (m, 2H), 6.80 (t, J = 6.8 Hz, 1H), 4.30 - 4.23 (m, 1H), 3.67 - 3.56 (m, 1H), 3.56 - 3.44 (m, 2H), 3.44 - 3.33 (m, 3H), 3.25 (dd, J = 12.5, 4.2 Hz, 1H), 2.96 - 2.78 (m, 2H), 2.26 - 2.16 (m, 1H), 2.13 - 1.99 (m, 2H), 1.77 - 1.46 (m, 7H), 1.23 - 1.12 (m, 1H). Specific Optical Rotation: 33.9° c=0.5, MeOH, 22.7°C, 589 nm.

**Step 9: 2-(4-(((R)-pyrrolidin-3-yl)oxy)butyl)-1,2,3,4-tetrahydro-1,8-naphthyridine stereoisomer B dihydrochloride**

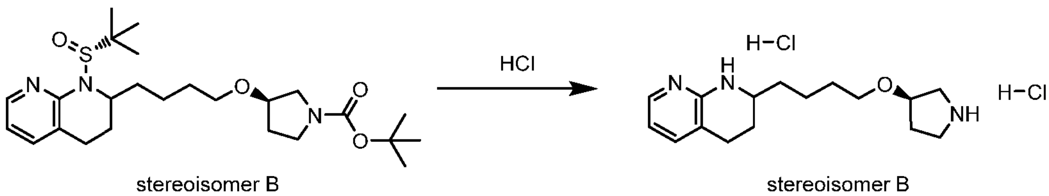


To a solution of tert-butyl (3R)-3-(4-(1-((R)-tert-butylsulfinyl)-1,2,3,4-tetrahydro-1,8-naphthyridin-2-yl)butoxy) pyrrolidine-1-carboxylate stereoisomer B (0.45 g, 0.938 mmol) in methanol (5 mL) was added hydrochloric acid (5 mL, 20.00 mmol, 4N solution in dioxane). The mixture was stirred at room temperature for 3 hours, then concentrated in vacuo and coevaporated with methanol. Diethyl ether was added which started a slow crystallisation of the product. After standing overnight the crystallised material was scratched loose, and the material was triturated with diethyl ether. After a few hours, the solids were collected by filtration, rinsed with fresh diethyl ether and dried under vacuum to afford the desired product 2-(4-(((R)-pyrrolidin-3-yl)oxy)butyl)-1,2,3,4-tetrahydro-1,8-naphthyridine stereoisomer B dihydrochloride (289 mg) as a beige solid. Yield 85%. LC/MS ESI 276 (M-2HCl+H) +. $^1$H NMR (400 MHz, Methanol-d4) δ 7.74 - 7.66 (m, 2H), 6.80 (t, J = 6.8 Hz, 1H), 4.30 - 4.23 (m, 1H), 3.66 - 3.57 (m, 1H), 3.57 - 3.45 (m, 2H), 3.45 - 3.33 (m, 3H), 3.29 - 3.21 (m, 1H), 2.96 - 2.78 (m, 2H), 2.26 - 2.16 (m, 1H), 2.13 - 1.97 (m, 2H), 1.76 - 1.44 (m, 7H). Specific Optical Rotation: -48.3° c=0.5, MeOH, 22.7°C, 589 nm.

The following methods were used to prepare compounds 1-3:

**Example 1: Preparation of 2-(3-isopropylisochroman-5-yl)-2-((R)-3-(4-(5,6,7,8-tetrahydro-1,8-naphthyridin-2-yl) butoxy)pyrrolidin-1-yl)acetic acid (compounds 1-E1 and 1-E2)**

**Step 1: 2-(2-bromophenyl)acetaldehyde**

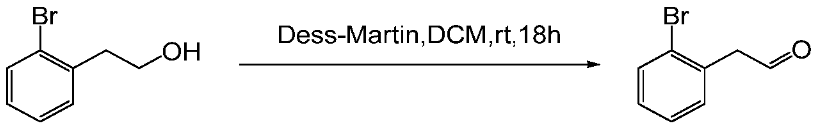


A mixture of 2-(2-bromophenyl)ethanol (6.0 g, 29.8 mmol) and Dess-Martin periodinane ( 15.2g, 35.8 mmol) in DCM (60 mL) was stirred at room temperature overnight. Solvent was removed in vacuo, and the residue was purified by silica gel column (pet ether: EtOAc 10:1) to give the desired product 2-(2-bromophenyl)acetaldehyde as a colorless oil (4.5g). Yield 76%. 1H NMR (400 MHz, CDCl3) δ 9.77 (s, 1H), 7.69-7.24(m, 4H), 3.87 (d, J = 5.0 Hz, 2H).

**Step 2: 1-(2-bromophenyl)-3-methylbutan-2-ol**

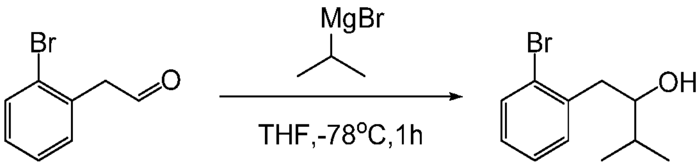


To a solution of 2-(2-bromophenyl)acetaldehyde (4.5g, 22.6 mmol) in THF (16 mL) at - 78 °C was added isopropylmagnesium bromide solution (3M, 11.3 mL, 33.9 mmol) dropwise. The reaction was stirred at -78 °C for 1hour. Solvent was removed in vacuo, and the residue was purified by silica gel column (pet ether: EtOAc 10:1) to give the desired product 1-(2-bromophenyl)-3-methylbutan-2-ol as a yellow oil (2.8g). Yield 51%. 1H NMR (400 MHz, CDCl3) δ 7.58 (d, J = 5.0 Hz,1H), 7.29-7.27(m, 2H), 7.11 (d, J = 5.0 Hz,1H), 3.71-3.69 (m, 1H), 3.11-3.08 (m, 1H), 2.71-2.66 (m, 1H), 2.02-1.81 (m, 1H), 1.06-1.04(m, 6H).

**Step 3: 1-bromo-2-(2-(methoxymethoxy)-3-methylbutyl)benzene**

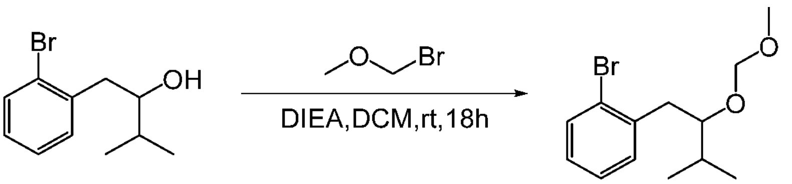


A mixture of 1-(2-bromophenyl)-3-methylbutan-2-ol (2.8g, 11.5 mmol), bromo(methoxy)methane (2.16g, 17.3 mmol) and diisopropylethylamine (3.26g, 25.3 mmol) in DCM (16 mL) was stirred at room temperature for 18 hours. Solvent was removed in vacuo, and the residue was purified by silica gel column (pet ether: EtOAc 5:1) to give the desired product 1-bromo-2-(2-(methoxymethoxy)-3-methylbutyl)benzene as a yellow oil (1g). Yield 30%. 1H NMR (400 MHz, CDCl3) δ 7.54-7.52 (m,2H), 7.29-7.27(m, 2H), 4.49-4.47 (m, 1H), 4.33-4.31 (m, 1H), 3.77-3.75 (m, 1H), 3.26(s, 1H), 3.11-3.01 (m, 1H), 2.82-2.77 (m, 2H), 1.96-1.91(m, 1H), 1.06-0.93(m,6H).

**Step 4: 5-bromo-3-isopropylisochroman**

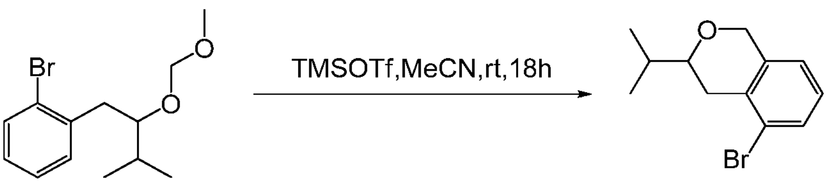


A mixture of 1-bromo-2-(2-(methoxymethoxy)-3-methylbutyl)benzene (1g, 3.5 mmol) and TMSOTf (777mg, 3.5 mmol) in MeCN (12 mL) was stirred at room temperature for 18 hours. Solvent was removed in vacuo, and the residue was

purified by silica gel column (pet ether: EtOAc 5:1) to give the desired product 5-bromo-3-isopropylisochroman as a yellow oil (610mg). Yield 68%. 1H NMR (400 MHz, CDCl3) δ 7.44-7.42 (m,2H), 7.06-6.95(m, 2H), 4.85-4.71(m, 2H), 3.37-3.31 (m, 1H), 2.82-2.77 (m, 1H), 2.61-2.56 (m, 1H), 1.90-1.85 (m, 1H), 1.08-1.03(m, 6H).

**Step 5: 3-isopropylisochroman-5-ylboronic acid**

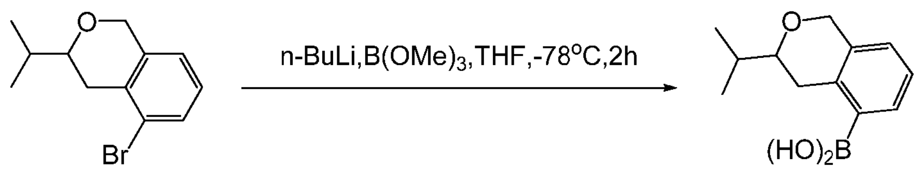


To a solution of 5-bromo-3-isopropylisochroman (400mg, 1.57 mmol) in THF (10 mL) under Ar was added n-BuLi (2.5M, 1mL, 2.5 mmol) dropwise. The reaction was stirred for 1 h at -78 °C, and a solution of trimethyl borate (442mg, 2.35 mmol) in THF (5 mL) was added dropwise. The reaction was stirred for another 1 hour at -78 °C, then slowly warmed to room temperature and stirred overnight. Aqueous HCl (1N, 20 mL) was added to the reaction, and it was stirred at room temperature for 30 min, then extracted with DCM (3 × 20 mL). The combined organic layers were dried over Na2SO4, filtered and concentrated in vacuo. The residue was purified by silica gel column (pet ether: EtOAc =1:1) to give the desired product as a white solid (250 mg). Yield 72% (ESI: 219[M-H]-).

**Step 6: 2-(3-isopropylisochroman-5-yl)-2-((R)-3-(4-(5,6,7,8-tetrahydro-1,8-naphthyridin-2-yl)butoxy)pyrrolidin-1-yl)acetic acid (diastereomeric compounds 1-E1 and 1-E2)**

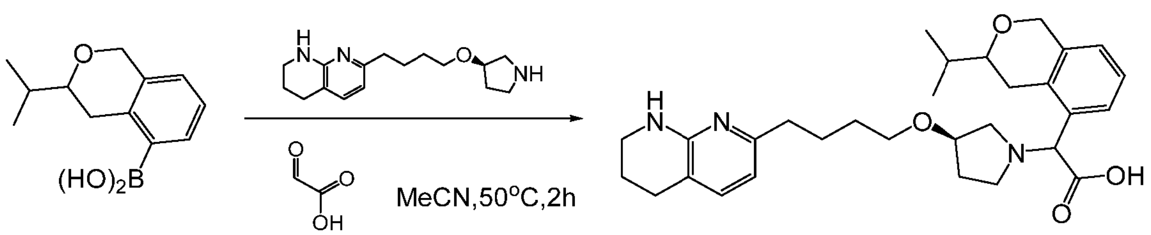


A mixture of 3-isopropylisochroman-5-ylboronic acid (250 mg, 1.14mmol), (R)-7-(4-(pyrrolidin-3-yloxy)butyl)-1,2,3,4-tetrahydro-1,8-naphthyridine (314mg, 1.14mmol) and 2-oxoacetic acid (50% in water) (169mg, 1.14 mmol) in acetonitrile (10 mL) was stirred at 50 °C for 2 hours. Solvent was removed in vacuo, and the residue was purified by Prep-HPLC A (33-65% MeCN) to give the product 2-(3-isopropylisochroman-5-yl)-2-((R)-3-(4-(5,6,7,8-tetrahydro-1,8-naphthyridin-2-yl)butoxy)pyrrolidin-1-yl)acetic acid (**compound 1**) as a white solid (255mg, 50% yield). The racemic product was separated by Prep chiral SFC F to give diastereomeric products **compound 1-E1** (33 mg) and **compound 1-E2** (87 mg) as white solids, each as a mixture of two stereoisomers.

**Compound 1-E1 (mixture of 2 stereoisomers)** LC/MS ESI 508.7 (M+H) +. 1H NMR (400 MHz, MeOD) δ 7.51 (d, J = 6.0 Hz, 1H), 7.28 - 7.18 (m, 3H), 6.37 (d, J = 7.3Hz, 1H), 5.50 (s, 1H), 4.89 - 4.69 (m, 3H), 4.17 (s, 1H), 3.69 - 3.34 (m, 6H), 3.20-3.13 (m, 2H), 3.01 - 2.48 (m, 6H), 2.55-2.03 (m, 2H), 1.95 -1.58 (m, 7H), 1.14 - 0.96 (m, 6H). Chiral SFC F (45% MeOH): ee 100%, Rt = 2.92min

**Compound 1-E2 (mixture of 2 stereoisomers)** LC/MS ESI 508.7 (M+H) +. 1H NMR (400 MHz, MeOD) δ 7.51 (d, J = 6.0 Hz, 1H), 7.28 - 7.18 (m, 3H), 6.37 (d, J = 7.3Hz, 1H), 5.50-4.95 (m, 1H), 4.89 - 4.69 (m, 3H), 4.17 (s, 1H), 3.69 - 3.34 (m, 6H), 3.20-3.13 (m, 2H), 3.01 - 2.48 (m, 6H), 2.55-2.03 (m, 2H), 1.95 -1.58 (m, 7H), 1.14 - 0.96 (m, 6H). Chiral SFC F (45% MeOH): ee 100%, Rt = 8.03min

**Example 2: Preparation of 2-(3-isopropyl-3,4-dihydro-1H-pyrano[3,4-c]pyridin-5-yl)-2-((R)-3-(4-(5,6,7,8-tetrahydro-1,8-naphthyridin-2-yl)butoxy)pyrrolidin-1-yl)acetic acid (compounds 2-E1 and 2-E2)**

**Step 1: 1-(3,5-dibromopyridin-4-yl)-3-methylbutan-2-one**

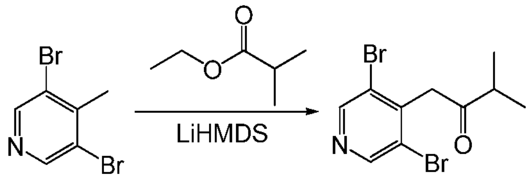


To a solution of 3,5-dibromo-4-methylpyridine (2.0 g, 7.97 mmol) in THF (20 mL) at -78 °C under Ar, was added LiHMDS (1 M in THF, 12 mL, 12 mmol) dropwise. After 10 min the solution was allowed to warm to 0 °C and stirred for 1 hour. A solution of ethyl isobutyrate (1.39 g, 12 mmol) in THF (5 mL) was added dropwise, and the reaction was stirred at room temperature for 2 hours, then quenched with aq. NH4Cl, diluted with water (10 mL) and extracted with EtOAc (3x 20 mL). The combined organic layers were concentrated in vacuo, and the residue was purified by silica gel column (pet ether: EtOAc 5:1) to give the desired product 1-(3,5-dibromopyridin-4-yl)-3-methylbutan-2-one as a colorless oil (1.6 g). Yield 63% (ESI 320 (M+H) +).

**Step 2: 1-(3,5-dibromopyridin-4-yl)-3-methylbutan-2-ol**

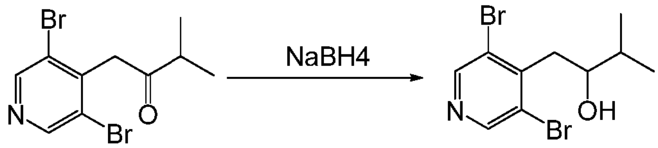


To a solution of 1-(3,5-dibromopyridin-4-yl)-3-methylbutan-2-one (1.6 g, 4.98 mmol) in MeOH (30 mL) at 0 °C, was added NaBH4 (284 mg, 7.47 mmol). The reaction was stirred at 0 °C for 2 hours, then concentrated in vacuo. The residue was adjusted to pH= 6 with aq. NH4Cl, diluted with water (10 mL) and extracted with EtOAc (3x 20 mL). The combined organic layers were dried over Na2SO4, filtered, and concentrated in vacuo to give the desired product 1-(3,5-dibromopyridin-4-yl)-3-methylbutan-2-ol as a colorless oil (1.5 g). Yield 93% (ESI 322 (M+H) +).

**Step 3: 5-bromo-3-isopropyl-3,4-dihydro-1H-pyrano[3,4-c]pyridin-1-ol**

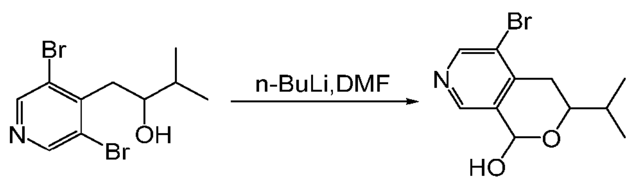


To a solution of 1-(3,5-dibromopyridin-4-yl)-3-methylbutan-2-ol (4.0 g, 12.4 mmol) in THF (40 mL) at -78 °C was added BuLi (2.5M, 7.4 mL, 18.5 mmol) dropwise. The reaction was stirred for 20 min at -78°C. Then DMF (1.35 g, 18.5mmol) was added, and the reaction was stirred for 10 min at -78°C, then poured into sat NH4Cl solution (20mL) and extracted with EtOAc (30mL). The aqueous phase was back extracted with EtOAc (2x 30mL), and the combined organic phase was washed with brine, dried over Na2SO4, filtered and concentrated in vacuo. The residue was purified by silica gel column (pet ether: EtOAc =2:1) to give the desired product 5-bromo-3-isopropyl-3,4-dihydro-1H-pyrano[3,4-c] pyridin-1-ol as a colorless oil (850 mg). Yield 24% (ESI 286 (M+H) +). 1H NMR (500 MHz, CDCL3) $\delta$8.61 (s, 1H) , 8.47 (s,1H), 6.08 (s,1H), 3.95 (m,1H), 3.19 (s,1H), 2.77 (m, 1H), 2.50 (m, 1H), 1.90 (m, 1H), 1.10-0.95 (m, 6H).

**Step 4: 5-bromo-3-isopropyl-3,4-dihydro-1H-pyrano[3,4-c]pyridine**

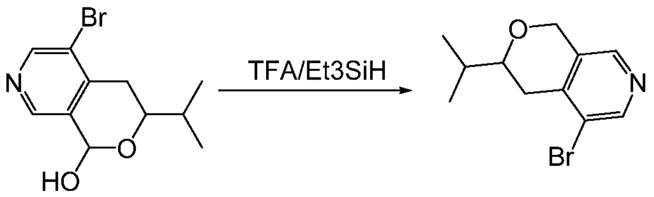


To a solution of 5-bromo-3-isopropyl-3,4-dihydro-1H-pyrano[3,4-c]pyridin-1-ol (200 mg, 0.7 mmol) in DCM (15mL) at 0 °C was added TFA (0.35 mL). The mixture was stirred for 10 min. Then triethylsilane (0.7mL) was added, and the reaction was stirred at room temperature for 30 min, then adjusted to pH=7~8 with sat. NaHCO3 solution and extracted with DCM (2x 20mL). The combined organic phase was washed with brine, dried over Na2SO4, filtered and concentrated to give the desired product 5-bromo-3-isopropyl-3,4-dihydro-1H-pyrano[3,4-c]pyridine as a colorless oil (160 mg). Yield 89% (ESI 256 (M+H) +).

**Step 5: tert-butyl 2-(3-isopropyl-3,4-dihydro-1H-pyrano[3,4-c]pyridin-5-yl)acetate**

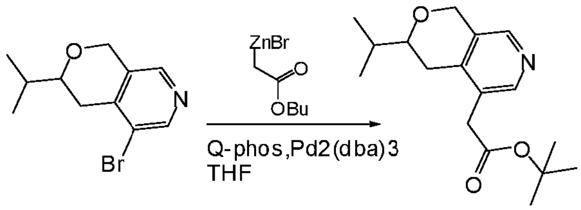


To a mixture of 3-bromo-2,6-dicyclopropylpyridine (100 mg, 0.4 mmol), Pd2(dba)3 (30 mg, 0.02 mmol) and Qphos (15 mg, 0.02 mmol) in THF (10 mL) was added (2-tert-butoxy-2-oxoethyl)zinc(II) bromide solution (1.0 M in THF, 3.85 mL, 3.85 mmol). The reaction was stirred at 50 °C for 2 hours, then adjusted to pH=7~8 with sat.NaHCO3 solution and extracted with EtOAc (2x 20mL). The combined organic phase was washed with brine, dried over Na2SO4, filtered and concentrated in vacuo. The residue was purified by silica gel column (pet ether: EtOAc =2:1) to give the desired product tert-butyl 2-(3-isopropyl-3,4-dihydro-1H-pyrano[3,4-c]pyridin-5-yl)acetate as a yellow oil (38 mg). Yield 33% (ESI 292 (M+H) +).

**Step 6: tert-butyl 2-bromo-2-(3-isopropyl-3,4-dihydro-1H-pyrano[3,4-c]pyridin-5-yl)acetate**

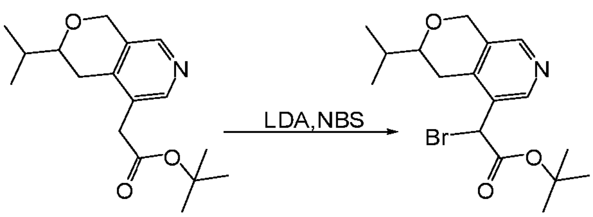


To a solution of tert-butyl 2-(3-isopropyl-3,4-dihydro-1H-pyrano[3,4-c]pyridin-5-yl)acetate (60 mg, 0.21 mmol) in THF (5 mL) at -78 °C was added lithium diisopropylamide solution 2.0 M in THF/hexanes (0.21mL, 0.41 mmol) dropwise. The reaction was stirred at -78°C for 30 min, and a solution of chlorotrimethylsilane (45 mg, 0.41 mmol) in THF (1mL) was added. The reaction was stirred at -78°C for another 30 min, and a solution of NBS (75mg, 0.41 mmol) in THF (2 mL) was added. The reaction was stirred at -78°C for 1 hour, then quenched with MeOH (2 mL) and concentrated in vacuo. The residue was purified by silica gel column (pet ether: EtOAc 2:1) to give the desired product tert-butyl 2-bromo-2-(3-isopropyl-3,4-dihydro-1H-pyrano[3,4-c]pyridin-5-yl)acetate as a yellow oil (65 mg). Yield 85 % (ESI 370,372(M+H)+.

**Step 7: tert-butyl 2-(3-isopropyl-3,4-dihydro-1H-pyrano[3,4-c]pyridin-5-yl)-2-((R)-3-(4-(5,6,7,8-tetrahydro-1,8-naphthyridin-2-yl)butoxy)pyrrolidin-1-yl)acetate**

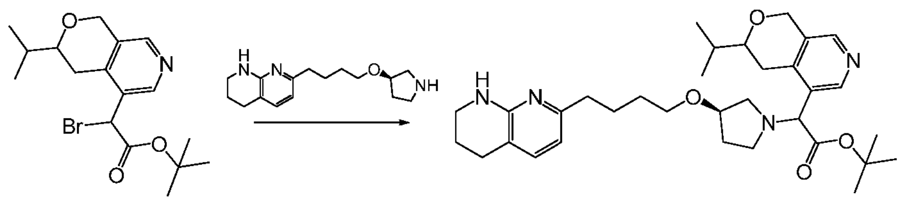

A mixture of tert-butyl 2-bromo-2-(3-isopropyl-3,4-dihydro-1H-pyrano[3,4-c]pyridin-5-yl)acetate (85 mg, 0.23 mmol), (R)-7-(4-(pyrrolidin-3-yloxy)butyl)-1,2,3,4-tetrahydro-1,8-naphthyridine (63 mg, 0.23 mmol) and diisopropylethylamine (90 mg, 0.69 mmol) in acetonitrile (5 mL) was stirred at room temperature for 2 hours. The mixture was diluted with water (8mL) and extracted with EtOAc (25mL). The organic phase was washed with brine, dried over Na2SO4, filtered and concentrated in vacuo. The residue was purified by silica gel column (DCM:MeOH 20:1) to give the desired product tert-butyl 2-(3-isopropyl-3,4-dihydro-1H-pyrano[3,4-c]pyridin-5-yl)-2-((R)-3-(4-(5,6,7,8-tetrahydro-1,8-naphthyridin-2-yl)butoxy)pyrrolidin-1-yl)acetate as a colorless oil (60 mg). Yield 46% (ESI 565 (M+H)+.

**Step 8: 2-(3-isopropyl-3,4-dihydro-1H-pyrano[3,4-c]pyridin-5-yl)-2-((R)-3-(4-(5,6,7,8-tetrahydro-1,8-naphthyridin-2-yl)butoxy)pyrrolidin-1-yl)acetic acid (compounds 2-E1 and 2-E2)**

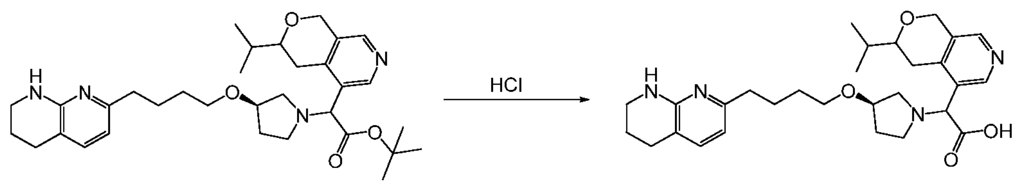


A solution of tert-butyl 2-(3-isopropyl-3,4-dihydro-1H-pyrano[3,4-c]pyridin-5-yl)-2-((R)-3-(4-(5,6,7,8-tetrahydro-1,8-naphthyridin-2-yl)butoxy)pyrrolidin-1-yl)acetate (60 mg, 0.11 mmol) in 4N HCl/dioxane (3 mL, 12 mmol) was stirred at room temperature for 12 hours. Solvent was removed in vacuo, and the residue was adjusted to pH=7 with NH3/MeOH (7N) and purified by prep-HPLC A (40-70% MeCN) to give diastereomeric products **compound 2-E1** (20 mg) and **compound 2-E2** (10 mg) as white solids, each as a mixture of two stereoisomers.

**Compound 2-E1 (mixture of 2 stereoisomers)** LC/MS ESI 509 (M+H) +. 1H NMR (400 MHz, MeOD) δ 8.65-8.63 (m, 1H), 8.24 - 8.22 (m, 1H), 7.28- 7.26 (m, 1H), 6.45 - 6.42 (m, 1H), 4.95 (s, 1H), 4.78-4.58 (m, 2H), 4.15-4.13 (m, 1H), 3.50 - 3.40 (m, 5H), 3.30-2.50 (m, 10H), 2.20 - 1.50 (m, 9H), 1.02-1.00 (m, 6H).

**Compound 2-E2 (mixture of 2 stereoisomers)** LC/MS ESI 509 (M+H) +. 1H NMR (400 MHz, MeOD) δ 8.65-8.63 (m, 1H), 8.23 - 8.21 (m, 1H), 7.28- 7.26 (m, 1H), 6.44 - 6.41 (m, 1H), 4.95 (s, 1H), 4.78-4.64 (m, 2H), 4.15-4.13 (m, 1H), 3.52 - 3.40 (m, 5H), 3.30-2.50 (m, 10H), 2.15 - 1.50 (m, 9H), 1.05-1.00 (m, 6H).

**Example: Preparation of 2-(3-(2-methoxypropan-2-yl)isochroman-5-yl)-2-((R)-3-(4-(5,6,7,8-tetrahydro-1,8-naphthyridin-2-yl)butoxy)pyrrolidin-1-yl)acetic acid (compounds 3-E1 and 3-E2)**

**Step 1: 1,3-dibromo-2-(bromomethyl)benzene**

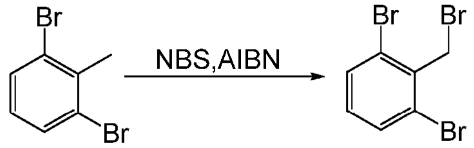


A mixture of 1,3-dibromo-2-methylbenzene (7.5 g, 30 mmol) and NBS (8.01 g, 45 mmol) in CHCl3 (120 mL) was stirred under reflux overnight, then diluted with water (20 mL), washed with sat. NaHCO3 solution and brine, dried over Na2SO4, filtered and concentrated in vacuo. The residue was purified by silica gel column (pet ether: EtOAc 40:1~10:1) to give the desired product 1,3-dibromo-2-(bromomethyl)benzene as a white solid (8.7g, 88% yield). 1H NMR (500 MHz, CDCl3) δ 7.58 (d, J=8.0Hz, 2H), 7.04 (t, J=8.0Hz, 1H), 4.85(s, 2H).

**Step 2: 1,3-dibromo-2-(3-methylbut-2-enyl)benzene**

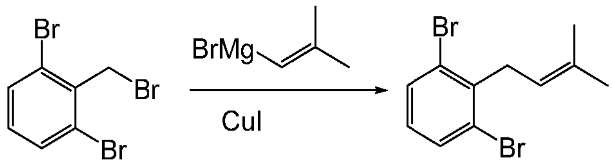


A mixture of 1,3-dibromo-2-(bromomethyl)benzene (10 mmol, 3.28 g), CuI (2.0 mol, 381 mg) and 2,2'-bipyridyl (2.0 mol, 312 mg) in THF (80 mL) under nitrogen atmosphere was stirred at room temperature for 1 hour. Then 2-methyl-1-propenyl magnesium bromide (20 mmol, 1.0 M in THF, 20 mL) was added at 0 °C, and the reaction mixture was stirred at room temperature for 6 hours. Additional 2-methyl-1-propenyl magnesium bromide (20 mmol, 1.0 M in THF, 20 mL) was added at 0°C, and the reaction mixture was stirred at room temperature overnight. Saturated aq NH4Cl (45 mL) and 25 percent aq NH3 (25 mL) were added, and the mixture was stirred for 10 min, filtered to remove solid and extracted with EtOAc (2x 40mL). The combined organic layer was washed with brine, dried over Na2SO4, filtered and concentrated in vacuo. The residue was purified by silica gel column (pet ether: EtOAc 20:1) to give the desired product 1,3-dibromo-2-(3-methylbut-2-enyl)benzene as a colorless oil (730mg). Yield 24%. 1H NMR (500 MHz, CDCl3) δ 7.52 (d, J=7.5Hz, 2H), 6.92 (t, J=7.5Hz, 1H), 5.09 (m,1H), 3.73 (d, J=6.5Hz, 2H), 1.84 (s, 3H), 1.74(s, 3H).

**Step 3: 3-(2,6-dibromobenzyl)-2,2-dimethyloxirane**

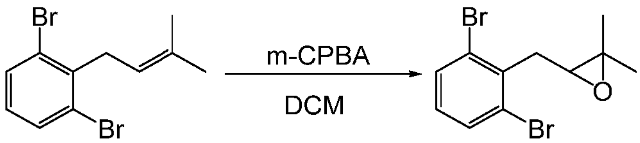


To a solution of 1,3-dibromo-2-(3-methylbut-2-enyl)benzene (450 mg, 2mmol) in DCM (20 mL) at room temperature was added m-CPBA (2.4mmol, 1.2 equiv.). The reaction was stirred at room temperature overnight, then adjusted to pH=7~8 with sat.NaHCO3 solution and extracted with DCM (2x 20mL). The combined organic phase was washed with brine, dried over Na2SO4, filtered and concentrated in vacuo. The residue was purified by silica gel column (pet ether: EtOAc = 40:1 ~ 20:1) to give the desired product 3-(2,6-dibromobenzyl)-2,2-dimethyloxirane as a colorless oil (200 mg). Yield 41%. (ESI 320.9 (M+H) +).

**Step 4: 1-(2,6-dibromophenyl)-3-methoxy-3-methylbutan-2-ol**

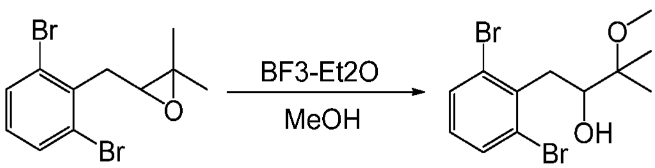


To a solution of 3-(2,6-dibromobenzyl)-2,2-dimethyloxirane (1.02g, 3.2mmol) in MeOH (30mL) was slowly added BF3.Et2O (0.64 mL) at 0 °C. The reaction was stirred for 10 min at 0 °C, then adjusted to pH=7~8 with Sat.NaHCO3 solution, concentrated to remove MeOH and extracted with EtOAc (2x 20mL). The combined organic phase was washed with brine, dried over Na2SO4, filtered and concentrated in vacuo. The residue was purified by silica gel column (pet ether: EtOAc = 10:1) to give the desired product 1-(2,6-dibromophenyl)-3-methoxy-3-methylbutan-2-ol as a colorless oil (980 mg). Yield 87% (ESI 375 (M+Na)+).

**Step 5: 5-bromo-3-(2-methoxypropan-2-yl)isochroman-1-ol**

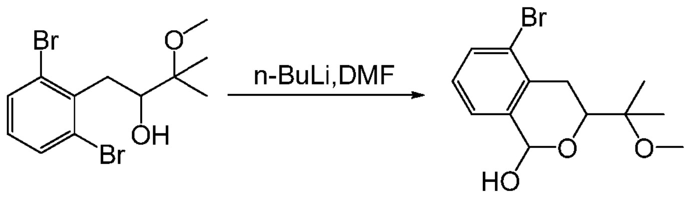


To a solution of 1-(2,6-dibromophenyl)-3-methoxy-3-methylbutan-2-ol (880mg, 2.5mmol) in THF (8mL) at -78 °C was added BuLi (2.5M, 1.5mL, 3.75mmol) dropwise. The reaction was stirred for 20 min at -78 °C, and DMF (548mg, 7.5mmol) was added. The mixture was stirred for 10 min at -78°C, then poured into sat. NH4Cl solution (12mL) and extracted with EtOAc (3x 20mL). The combined organic phase was washed with brine, dried over Na2SO4, filtered and concentrated in vacuo. The residue was purified by silica gel column (pet ether: EtOAc =10:1) to give the crude product, which was purified by Prep-HPLC (TFA/H2O, MeCN) to give the desired product 5-bromo-3-(2-methoxypropan-2-yl) isochroman-1-ol as a white solid (212mg). Yield 28% (ESI 323 (M+Na)+).

**Step 6: 5-bromo-3-(2-methoxypropan-2-yl)isochroman**

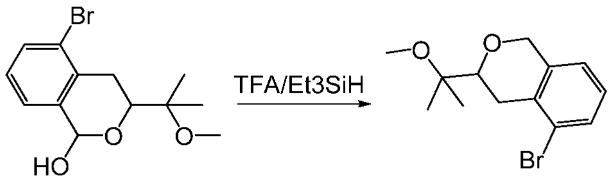


To a solution of 5-bromo-3-(2-methoxypropan-2-yl)isochroman-1-ol (211mg, 0.7 mmol) in DCM (15mL) at 0 °C was added TFA (0.35 mL). The reaction was stirred for 10min, and triethylsilane (0.7mL) was added. The mixture was stirred at room temperature for 30min, then adjusted to pH=7~8 with sat. NaHCO3 solution and extracted with DCM (2x 20mL). The combined organic phase was washed with brine, dried over Na2SO4, filtered and concentrated to give the desired product 5-bromo-3-(2-methoxypropan-2-yl)isochroman as a colorless oil. Yield 94% (ESI 307 (M+Na) +).

**Step 7: tert-butyl 2-(3-(2-methoxypropan-2-yl)isochroman-5-yl)acetate**

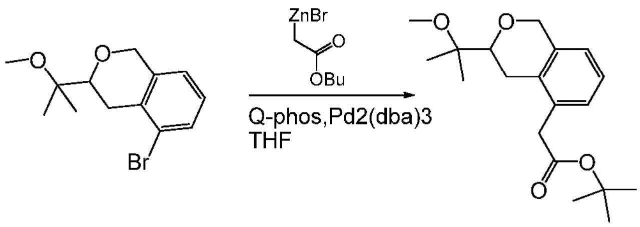


To a mixture of 5-bromo-3-(2-methoxypropan-2-yl)isochroman (220mg, 0.77mmol), $Pd_2(dba)_3$ (28mg, 0.031mmol) and Q-phos (22mg, 0.031mmol) in THF (16 mL) was added (2-tert-butoxy-2-oxoethyl)zinc(II) bromide solution (1.0 M in THF, 3.85 mL, 3.85 mmol). The reaction was stirred at 50 °C for 2 hours, then adjusted to pH=7~8 with sat. NaHCO3 solution and extracted with EtOAc (2x 20mL). The combined organic phase was washed with brine, dried over Na2SO4, filtered and concentrated in vacuo. The residue was purified by silica gel column (pet ether: EtOAc =10:1) to give the desired product tert-butyl 2-(3-(2-methoxypropan-2-yl)isochroman-5-yl)acetate as a pink oil (410 mg). Yield 82% (ESI 343.1 (M+23) +).

**Step 8: tert-butyl 2-bromo-2-(3-(2-methoxypropan-2-yl)isochroman-5-yl)acetate**

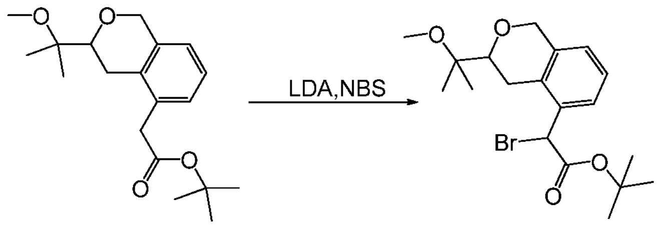


To a solution of tert-butyl 2-(3-(2-methoxypropan-2-yl)isochroman-5-yl)acetate (203mg, 0.63mmol) in THF (8 mL) at -78 °C was added lithium diisopropylamide solution 2.0 M in THF/hexanes (0.63mL, 1.26 mmol) dropwise. The reaction was stirred at -78 °C for 20 min. Then a solution of chlorotrimethylsilane (137mg, 1.26 mmol) in THF (0.5mL) was added, and the reaction was stirred at -78 °C for another 10 min. Then a solution of NBS (224mg, 1.26 mmol) in THF (2 mL) was added, and the reaction was stirred at -78°C for 10 min, then poured into water (10mL) and extracted with EtOAc (20mL). The organic phase was washed with sat.NaHCO3 soltion and water and concentrated in vacuo to give the crude product as a yellow oil (250 mg), which was used into the next step directly without further purification. Yield 75% (ESI 421.0 (M+23) +).

**Step 9: tert-butyl 2-(3-(2-methoxypropan-2-yl)isochroman-5-yl)-2-((R)-3-(4-(5,6,7,8-tetrahydro-1,8-naphthyridin-2-yl)butoxy)pyrrolidin-1-yl)acetate**

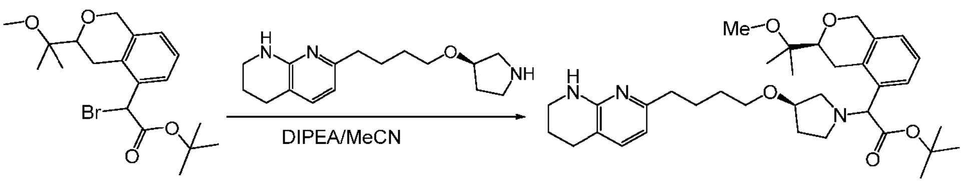


A mixture of tert-butyl 2-bromo-2-(3-(2-methoxypropan-2-yl)isochroman-5-yl)acetate (250mg, 0.62mmol), (R)-7-(4-(pyrrolidin-3-yloxy)butyl)-1,2,3,4-tetrahydro-1,8-naphthyridine (172mg, 0.62mmol) and diisopropylethylamine ( 242mg, 1.86 mmol) in acetonitrile (10 mL) was stirred at room temperature for 1 hour. The mixture was diluted with water (8mL) and extracted with EtOAc (25mL). The organic phase was washed with brine, dried over Na2SO4, filtered and concentrated in vacuo. The residue was purified by prep-HPLC A (40-70% MeCN) to give the desired tert-butyl 2-(3-(2-methoxypropan-2-yl)isochroman-5-yl)-2-((R)-3-(4-(5,6,7,8-tetrahydro-1,8-naphthyridin-2-yl)butoxy)pyrrolidin-1-yl)acetate as a white solid (181 mg). Yield 47% (ESI 594.3 (M+H)+).

**Step 10: 2-(3-(2-methoxypropan-2-yl)isochroman-5-yl)-2-((R)-3-(4-(5,6,7,8-tetrahydro-1,8-naphthyridin-2-yl)butoxy)pyrrolidin-1-yl)acetic acid (compounds** 3-**E1 and 3-E2)**

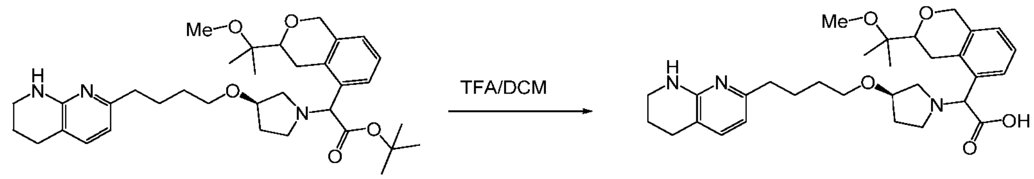


A solution of tert-butyl 2-(3-(2-methoxypropan-2-yl)isochroman-5-yl)-2-((R)-3-(4-(5,6,7,8-tetrahydro-1,8-naphthyridin-2-yl)butoxy)pyrrolidin-1-yl)acetate (181mg, 0.30 mmol) and TFA (1mL) in DCM (4mL) was stirred at room temperature overnight. Solvent was removed in vacuo, and the residue was purified by Prep-HPLC A (30-65% MeCN) to give the desired racemic product (130mg, 83% yield). The racemic product was separated by Prep chiral SFC F to give diastereomeric products **compound 3-E1** (49 mg) and **compound 3-E2** (52mg) as white solids, each as a mixture of two stereoisomers.

**Compound 3-E1 (mixture of 2 stereoisomers)** LC/MS ESI 538.3 (M+H) +. 1H NMR (500 MHz, MeOD) δ7.54(d, J=7.5Hz, 1H) , 7.27(m,1H), 7.15(m,1H), 7.07(m,1H), 6.38(m,1H),4.85 (m, 1H), 4.73 (m, 2H), 4.16 (m, 1H), 3.64(m, 1H), 3.44-3.49(m, 5H), 3.33 (s, 3H), 3.15-3.21 (m, 4H), 2.96 - 2.99 (m, 2H), 2.53 - 2.72 (m, 4H), 2.14(m, 2H), 1.87-1.89(m,2H),

1.61 - 1.72(m,4H), 1.27-1.31(m,7H). Chiral SFC F (45% MeOH): ee 100%, Rt = 4.15min.

**Compound 3-E2 (mixture of 2 stereoisomers)** LC/MS ESI 538.3 (M+H) +. 1H NMR (500 MHz, MeOD) δ7.52(d, J=7.5Hz, 1H) , 7.24(m,1H), 7.15(m,1H), 7.08(m,1H), 6.38(m,1H),4.86 (m, 1H), 4.78 (m, 2H), 4.20 (m, 1H), 3.50(m, 1H), 3.34-3.49(m, 5H), 3.30 (s, 3H), 3.05-3.21 (m, 4H), 2.91(m,1H), 2.53 - 2.72 (m, 4H), 2.11(m, 2H), 1.87-1.90(m,2H), 1.61 - 1.72(m,4H), 1.25-1.30(m,7H). Chiral SFC F (45% MeOH): ee 100%, Rt = 8.79min.

**Example 4: Preparation of 2-(3-(2-fluoropropan-2-yl)isochroman-5-yl)-2-((R)-3-(4-(5,6,7,8-tetrahydro-1,8-naphthyridin-2-yl)butoxy)pyrrolidin-1-yl)acetic acid (compounds 4-E1 and 4-E2)**

**Step 1: 1-(2,6-dibromophenyl)-3-fluoro-3-methylbutan-2-ol**

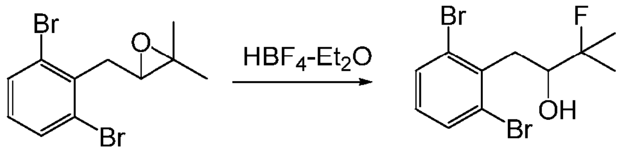


To a cooled solution of 3-(2,6-dibromobenzyl)-2,2-dimethyloxirane (4.8 g, 15 mmol) in DCM (120 mL) at -78 °C, was added $HBF_4$-$Et_2O$ (2.43 g, 15 mmol) dropwise. The mixtture was stirred at -78°C for 10 min and then quenched with sat. aq. NaHCO3 solution (20 mL) and extracted with DCM (2 × 30 mL). The combined organic extracts were washed with bine, dried over anhydrous sodium sulfate, filtered and concentrated in vacuo. The residue was purified by silica gel column (pet ether: EtOAc 100:1 → 12:1) to give the desired product 1-(2,6-dibromophenyl)-3-fluoro-3-methylbutan-2-ol as a colorless oil (2.74 g). Yield 54% (ESI 320.9 (M-HF), 362.8(M+23) +). $^1$H NMR (400 MHz, CDCl3) δ7.55 (m, 2H), 6.97 (m, 1H), 4.01 (m, 1H), 3.34 (m, 1H), 3.21(m, 1H), 1.91 (m, 1H), 1.55 (s, 3H), 1.49 (s, 3H).

**Step 2: 5-bromo-3-(2-fluoropropan-2-yl)isochroman-1-ol**

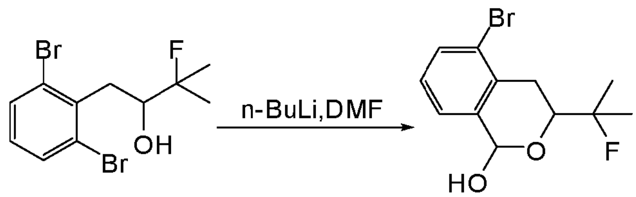


To a cooled solution of 1-(2,6-dibromophenyl)-3-fluoro-3-methylbutan-2-ol (1.36 g, 4.0 mmol) in THF (dry, 20 mL) at -78 °C under Ar atmophere was added a solution of nBuLi (2.5M in heptane, 2.4 mL, 6.0 mmol) dropwise. The mixture was stirred at -78 °C for 20 min. DMF (877 mg, 12 mmol) was added dropwise, and the reaction was stirred at -78 °C for 20 min. The reaction was poured into sat. NH4Cl soltion (20 mL), and the crude product extracted with EtOAc (20 mL). The organic phase was washed with bine, dried over anhydrous sodium sulfate, filtered, and concentrated in vacuo. The residue was purified by silica gel column (pet ether: EtOAc 100:1 → 20:1) to give the product 5-bromo-3-(2-fluoropropan-2-yl)isochroman-1-ol as a colorless oil (281 mg). Yield 18% (ESI 311 (M+23) +).

**Step 3: 5-bromo-3-(2-fluoropropan-2-yl)isochroman**

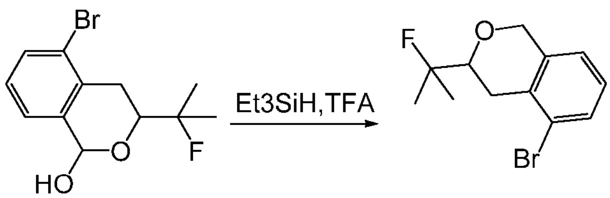


To a cooled solution of 5-bromo-3-(2-fluoropropan-2-yl)isochroman-1-ol (570 mg) in DCM (10 mL) at 0 °C, was

added TFA (0.6 mL). The mixture was stirred at 0 °C for 10 min, then triethylsilane (1.2 mL) was added, and the reaction mixture was stirred at 0 °C for 30 min. The mixture was quenched with sat. aq. NaHCO3 solution (10 mL), then extracted with DCM (2 × 10mL). The combined organic extracts were washed with brine, dried over anhydrous sodium sulfate, filtered and concentrated in vacuo. The residue was purified by silica gel column (pet ether: EtOAc 30:1 → 20:1) to give the desired product 5-bromo-3-(2-fluoropropan-2-yl)isochroman as a colorless oil (436 mg). Yield 81%. ESI 253 (M-HF). $^1$H NMR (400 MHz, CDCl3) δ 7.45 (m, 1H), 7.07 (m, 2H), 6.98 (m, 1H), 4.91-4.77 (m, 4H), 3.67 (m, 1H), 2.86-2.66 (m,2H), 1.52 (d, J=6.0Hz, 3H), 1.46 (d, J=6.0Hz, 3H).

**Step 4: tert-butyl 2-(3-(2-fluoropropan-2-yl)isochroman-5-yl)acetate**

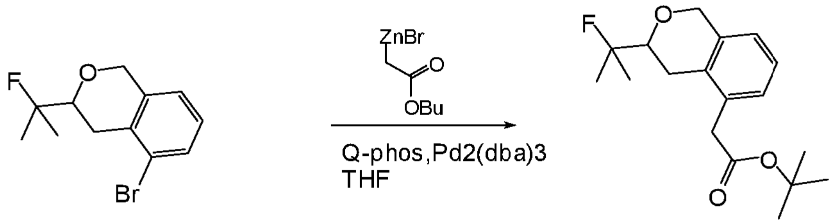


To a mixture of 5-bromo-3-(2-fluoropropan-2-yl)isochroman (436 mg, 1.6 mmol), $Pd_2(dba)_3$ (73 mg, 0.08mmol) and Q-phos (57 mg, 0.08 mmol) in THF (3 mL) under Ar was added a solution of (2-tert-butoxy-2-oxoethyl)zinc(II) bromide (1 M in THF, 8 mL, 8.0 mmol) at room temperature. The reaction was warmed to 50 °C and stirred for 2 hours. The mixture was quenched with sat. aq. NaHCO3 solution, filtered and concentrated in vacuo, and the residue was purified by silica gel column (pet ether: EtOAc 100:1 → 20:1) to give the desired product tert-butyl 2-(3-(2-fluoropropan-2-yl)isochroman-5-yl) acetate as a pale red oil (153 mg). Yield 31% (ESI 331.0 (M+23) +).$^1$H NMR (500 MHz, CDCl3) δ 7.15 (m, 2H), 6.94 (m, 1H), 4.93-4.82 (m, 2H), 3.67 (m, 1H), 3.53 (s, 2H), 2.74 (m, 2H), 1.46 (s, 9H), 1.29 (s, 3H), 1.27 (s, 3H).

**Step 5: tert-butyl 2-bromo-2-(3-(2-fluoropropan-2-yl)isochroman-5-yl)acetate**

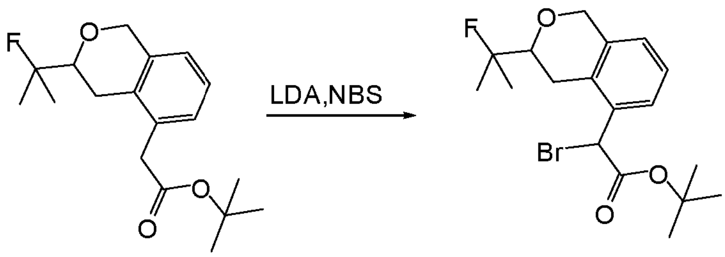


To a cooled solution of tert-butyl 2-(3-(2-fluoropropan-2-yl)isochroman-5-yl)acetate (153 mg, 0.5 mmol) in THF (5mL) at -78°C was added a solution of lithium diisopropylamide (2.0 M in THF/hexanes, 0.5 mL, 1.0 mmol) dropwise. The reaction was stirred at -78 °C for 30 min, then chlorotrimethylsilane (109 mg, 1.0 mmol) was added, and the reaction was stirred at -78 °C for another 30 min. Then a solution of N-bromosuccinimide (178 mg, 1.0 mmol) in THF (2 mL) was added, and the reaction was stirred at -78 °C for 30 min. The reaction was quenched with water (10 mL), then extracted with EtOAc (2 × 20mL). The combined organic phases were washed with sat. aq. NaHCO3 solution and brine, dried over anhydrous sodium sulfate, filtered and concentrated in vacuo to give the crude product tert-butyl 2-bromo-2-(3-(2-fluoropropan-2-yl) isochroman-5-yl)acetate as an orange oil (193mg, 61% yield), which was used to the next step directly without further purification.

**Step 6: tert-butyl 2-(3-(2-fluoropropan-2-yl)isochroman-5-yl)-2-((R)-3-(4-(5,6,7,8-tetrahydro-1,8-naphthyridin-2-yl)butoxy)pyrrolidin-1-yl)acetate**

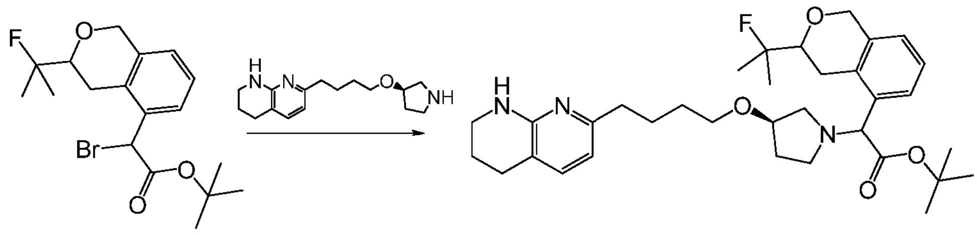

A mixture of tert-butyl 2-bromo-2-(3-(2-fluoropropan-2-yl)isochroman-5-yl)acetate (193 mg, 0.5 mmol), (R)-7-(4-(pyrrolidin-3-yloxy)butyl)-1,2,3,4-tetrahydro-1,8-naphthyridine (138 mg, 0.5 mmol), and DIPEA (194 mg, 1.5 mmol) in acetonitrile (8 mL) was stirred at room temperature for 2 hours. Solvent was removed in vacuo, and the residue was purified by Prep-HPLC A (30 → 65% MeCN) to give the desired product tert-butyl 2-(3-(2-fluoropropan-2-yl) isochroman-5-yl)-2-((R)-3-(4-(5,6,7,8-tetrahydro-1,8-naphthyridin-2-yl)butoxy)pyrrolidin-1-yl)acetate as a white solid (58 mg). Yield 20% (ESI 582 (M+H) +).

**Step 7: 2-(3-(2-fluoropropan-2-yl)isochroman-5-yl)-2-((R)-3-(4-(5,6,7,8-tetrahydro-1,8-naphthyridin-2-yl)butoxy) pyrrolidin-1-yl)acetic acid (compounds 4-E1 and 4-E2)**

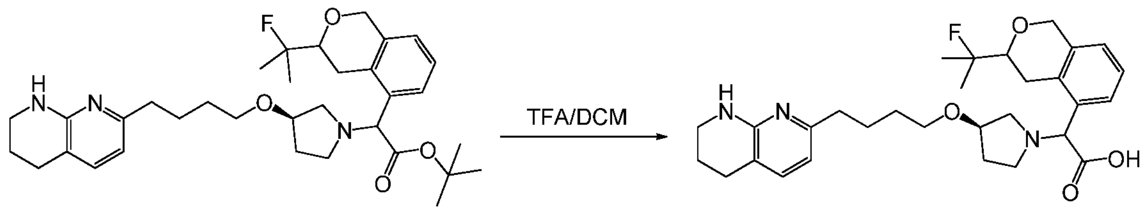


To a solution of tert-butyl 2-(3-(2-fluoropropan-2-yl)isochroman-5-yl)-2-((R)-3-(4-(5,6,7,8-tetrahydro-1,8-naphthyridin-2-yl)butoxy)pyrrolidin-1-yl)acetate (58 mg, 0.1mmol) in DCM (1.0 mL) was added TFA (1.0 mL). The mixture was stirred at room temperature for 18 hours. Solvent was removed in vacuo, and the residue was purified by Prep-HPLC A (30 → 65% MeCN) to give **compound 4** as a white solid (31 mg, 59% yield), which was separated by Prep chiral SFC F to give products **compound 4-E1** (11 mg) and **compound 4-E2** (11 mg) as white solids, each as a mixture of 2 stereoisomers.

**Compound 4-E1 (mixture of 2 stereoisomers)** LC/MS ESI 526.2 (M+H) +. 1H NMR (400 MHz, MeOD) δ 7.54-7.52 (m, 1H), 7.24-7.21 (m, 1H), 7.13-7.07(m, 2H), 6.37 (d, J=7.2Hz, 1H), 4.85-4.74 (m, 3H), 4.18 (s, 1H), 3.69-3.68 (m, 1H), 3.48-3.36 (m, 5H), 3.28-2.99(m, 5H), 2.72-2.69 (m, 2H), 2.56-2.52 (m, 2H), 2.12-2.07 (m, 2H), 1.91-1.84 (m, 2H), 1.72-1.60 (m, 4H), 1.48-1.40 (m, 6H). Chiral SFC F (45% MeOH): ee 100%, Rt = 3.27 min.

**Compound 4-E2 (mixture of 2 stereoisomers)** LC/MS ESI 526.2 (M+H) +. 1H NMR (400 MHz, MeOD) δ 7.56-7.54 (m,1H), 7.24-7.21 (m, 1H), 7.14-7.06 (m, 2H), 6.36 (d, J=7.2Hz, 1H), 4.85-4.64 (m, 3H), 4.13 (s, 1H), 3.69-3.66 (m, 1H), 3.48-3.36 (m, 5H), 3.28-2.98(m, 5H), 2.71-2.68 (m, 2H), 2.55-2.52 (m, 2H), 2.13-2.07 (m, 2H), 1.89-1.86 (m, 2H), 1.70-1.60 (m, 4H), 1.49-1.40 (m, 6H). Chiral SFC F (45% MeOH): ee 100%, Rt = 7.70 min.

**Example 5: Preparation of 2-((R)-3-(1,1-difluoro-5-(5,6,7,8-tetrahydro-1,8-naphthyridin-2-yl)pentyl)pyrrolidin-1-yl)-2-(3-(2-methoxypropan-2-yl)isochroman-5-yl)acetic acid (compounds 5-A-E1, 5-A-E2, 5-B-E1 and 5-B-E2)**

**Step 1: tert-butyl 2-(3-(2-methoxypropan-2-yl)isochroman-5-yl)acetate stereoisomers A and B**

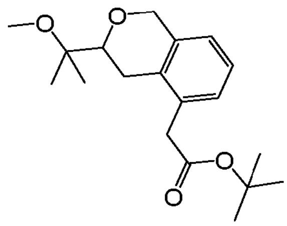

Racemic tert-butyl 2-(3-(2-methoxypropan-2-yl)isochroman-5-yl)acetate (1.22 g) was separated by Prep chiral SFC D to give the two isomers stereoisomer A (540 mg) and **stereoisomer B** (560 mg) as yellow oils.

**Step 2: tert-butyl 2-bromo-2-(3-(2-methoxypropan-2-yl)isochroman-5-yl)acetate stereoisomer A**

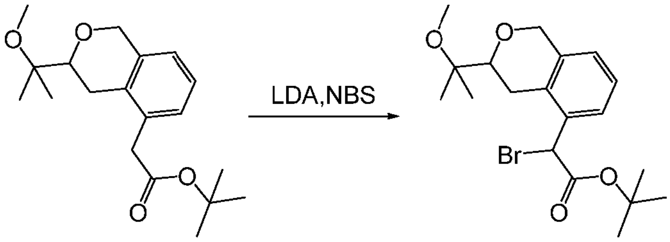


stereoisomer A stereoisomer A

To a solution of tert-butyl 2-(3-(2-methoxypropan-2-yl)isochroman-5-yl)acetate stereoisomer A (240 mg, 1.0 mmol) in THF (10 mL) at -78°C was added a solution of lithium diisopropylamide (2.0 M in THF/hexanes, 1.0 mL, 2.0 mmol) dropwise. The reaction was stirred at -78 °C for 30 min, then chlorotrimethylsilane (217 mg, 2.0 mmol) was added, and the reaction was stirred at -78 °C for another 30 min. Then a solution of N-bromosuccinimide (356 mg, 2.0 mmol) in THF (4 mL) was added, and the reaction was stirred at -78 °C for 30 min. The reaction was quenched with H2O (10 mL), then extracted with EtOAc (2 × 20mL). The combined oragnic phase was washed with Sat. NaHCO3 aq. solution, brine, dried over Na2SO4, filtered and concentrated in vacuo. The residue was purified by silica gel column (pet ether: EtOAc 8:1) to give the desired product tert-butyl 2-bromo-2-(3-(2-methoxypropan-2-yl)isochroman-5-yl)acetate stereoisomer A as a yellow oil (287 mg). Yield 72% (ESI 421.0 (M+H) +).

**Step 3: tert-butyl 2-((R)-3-(1,1-difluoro-5-(5,6,7,8-tetrahydro-1,8-naphthyridin-2-yl)pentyl)pyrrolidin-1-yl)-2-(3-(2-methoxypropan-2-yl)isochroman-5-yl)acetate iPr-stereoisomer A**

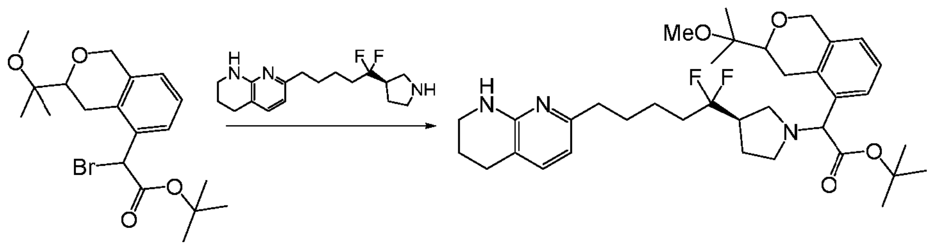

stereoisomer A iPr-stereoisomer A

A mixture of tert-butyl 2-bromo-2-(3-(2-methoxypropan-2-yl)isochroman-5-yl)acetate stereoisomer A (287 mg, 0.72 mmol), (R)-7-(5,5-difluoro-5-(pyrrolidin-3-yl)pentyl)-1,2,3,4-tetrahydro-1,8-naphthyridine (220 mg, 0.72 mmol) and DIPEA (279 mg, 2.16 mmol) in acetonitrile (8 mL) was stirred at room temperature for 2 hours. Solvent was removed in vacuo, and the residue was purified by silica gel column (DCM: MeOH 10:1) to give the desired product tert-butyl 2-((R)-3-(1,1-difluoro-5-(5,6,7,8-tetrahydro-1,8-naphthyridin-2-yl)pentyl)pyrrolidin-1-yl)-2-(3-(2-methoxypropan-2-yl) isochroman-5-yl)acetate iPr-stereoisomer A as a yellow oil (384 mg). Yield 85% (ESI 628.0 (M+H) +).

**Step 4: 2-((R)-3-(1,1-difluoro-5-(5,6,7,8-tetrahydro-1,8-naphthyridin-2-yl)pentyl)pyrrolidin-1-yl)-2-(3-(2-methoxypropan-2-yl)isochroman-5-yl)acetic acid iPr-stereoisomer A (compounds 5-A-E1 and 5-A-E2)**

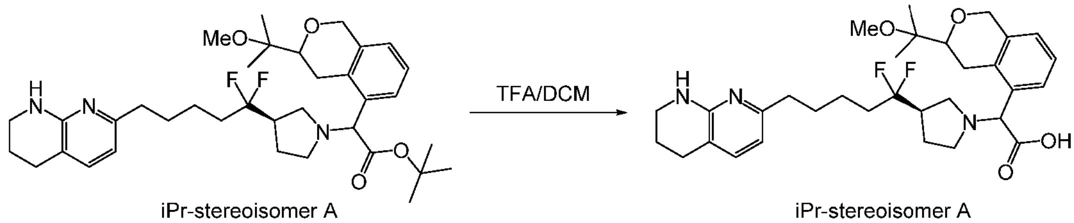


iPr-stereoisomer A

iPr-stereoisomer A

To a solution of tert-butyl 2-((R)-3-(1,1-difluoro-5-(5,6,7,8-tetrahydro-1,8-naphthyridin-2-yl)pentyl)pyrrolidin-1-yl)-2-(3-(2-methoxypropan-2-yl)isochroman-5-yl)acetate stereoisomer A (384 mg, 0.611 mmol) in DCM ( 6.0 mL) was added TFA (3.0 mL), then the mixture was stirred at room temperature for 18 hours. Solvent was removed in vacuo, and the residue was purified by Prep-HPLC A (30-65% MeCN) to give **compound 5- A** (290 mg, 83% yield) as a white solid (290 mg, 83% yield), which was separated by Prep chiral SFC F to give diastereomeric products **compound 5-A-E1** (104 mg) and **compound 5-A-E2** (97 mg) as white solids.

**Compound 5-A-E1** LC/MS ESI 572.3 $(M+H)^+$. 1H NMR (500 MHz, MeOD) δ 7.54 (d, J=8.0Hz,1H), 7.25 (m, 1H), 7.17 (d,J=8.0Hz,1H), 7.11 (d,J=7.5Hz,1H), 6.39 (d, J=7.5Hz,1H), 4.87 (s, 1H), 4.82-4.79 (m, 2H), 3.68 (m, 1H), 3.41-3.33 (m, 3H), 3.32-2.84 (m, 5H), 2.72 (m, 1H), 2.57-2.54 (m, 2H), 2.18-2.08 (m, 2H), 1.92-1.87 (m, 4H), 1.70-1.66 (m, 2H), 1.56 - 1.51 (m, 2H), 1.33 (s, 3H),1.32 (s, 3H). Chiral SFC F (45% EtOH): ee 100%, Rt = 2.62 min.

**Compound 5-A-E2** LC/MS ESI 572.3 $(M+H)^+$. 1H NMR (500 MHz, MeOD) δ 7.53 (d, J=8.0Hz,1H), 7.25 (m, 1H), 7.19 (d,J=8.0Hz,1H), 7.10 (d,J=7.5Hz,1H), 6.40 (d, J=7.5Hz,1H), 4.87 (s, 1H), 4.82-4.78 (m, 2H), 3.66-3.62 (m, 2H), 3.41-3.39 (m, 2H), 3.20-2.95 (m, 6H), 2.74-2.71 (m, 2H), 2.58-2.56 (m, 2H), 2.18 - 2.09 (m, 2H), 1.92-1.87 (m, 4H), 1.71-1.68 (m, 2H), 1.55 - 1.52 (m, 2H), 1.33 (s, 3H),1.30 (s, 3H). Chiral SFC F (45% EtOH): ee 100%, Rt = 5.41 min.

**Step 5: Preparation of 2-((R)-3-(1,1-difluoro-5-(5,6,7,8-tetrahydro-1,8-naphthyridin-2-yl)pentyl)pyrrolidin-1-yl)-2-(3-(2-methoxypropan-2-yl)isochroman-5-yl)acetic acid iPr-stereoisomer B (compounds 5-B-E1 and 5-B-E2)**

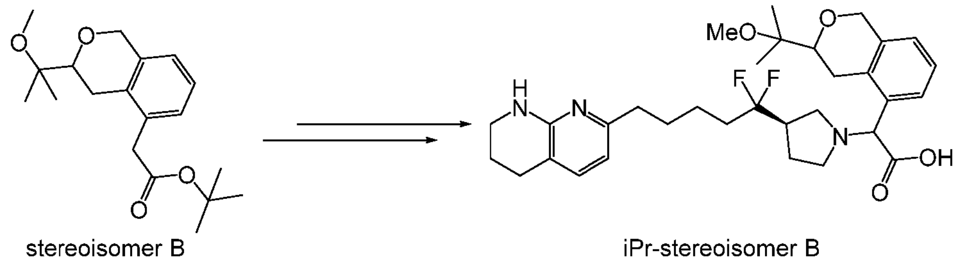

stereoisomer B

iPr-stereoisomer B

2-((R)-3-(1,1-difluoro-5-(5,6,7,8-tetrahydro-1,8-naphthyridin-2-yl)pentyl)pyrrolidin-1-yl)-2-(3-(2-methoxypropan-2-yl)isochroman-5-yl)acetic acid iPr-stereoisomer B (**compounds 5-B-E1 and 5-B-E2**) was synthesized from tert-butyl 2-(3-(2-methoxypropan-2-yl)isochroman-5-yl)acetate stereoisomer B by the same procedures as for stereoisomer A.

**Compound 5-B-E1** LC/MS ESI 572.3 $(M+H)^+$. 1H NMR (400 MHz, MeOD) δ 7.53 (d, J=7.6Hz, 1H), 7.25-7.24 (m, 1H), 7.17 (d, J=7.6Hz, 1H), 7.09 (d, J=7.2Hz, 1H), 6.38 (d, J=7.2Hz, 1H), 4.86-4.79 (m, 3H), 3.62-3.60 (m, 1H), 3.40-3.37 (m, 2H), 3.33-2.93 (m, 10H), 2.72-2.70 (m, 2H), 2.57-2.53 (m, 2H), 2.16-2.02 (m, 2H), 1.91-1.85 (m, 4H), 1.70 -1.66 (m, 2H), 1.53 -1.49 (m, 2H), 1.32 (s, 3H), 1.29 (s, 3H). Chiral SFC B (30% IPA): ee 100%, Rt = 1.07 min.

**Compound 5-B-E2** LC/MS ESI 572.3 $(M+H)^+$. 1H NMR (400 MHz, MeOD) δ 7.52 (d, J=7.6Hz, 1H), 7.24-7.08 (m, 3H), 6.39 (d, J=7.2Hz, 1H), 4.85-4.76 (m, 3H), 3.66-3.62 (m, 2H), 3.40-3.37 (m, 2H), 3.33-2.84 (m, 9H), 2.73-2.70 (m, 2H), 2.58-2.54 (m, 2H), 2.12-2.02 (m, 2H), 1.93-1.85 (m, 4H), 1.70 -1.67 (m, 2H), 1.54 -1.50 (m, 2H), 1.31 (s, 3H),1.25 (s, 3H). Chiral SFC B (30% IPA): ee 100%, Rt = 3.01 min.

**Example 6: Preparation of 2-((R)-7-fluoro-3-isopropyl-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetic acid (compounds 6-E1 and 6-E2)**

**Step 1: (S)-methyl 2-hydroxy-3-methylbutanoate**

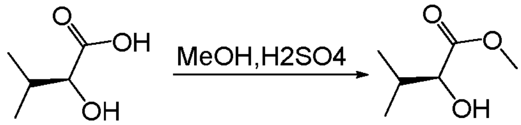


To a solution of (S)-2-hydroxy-3-methylbutanoic acid (4.14g, 35 mmol) in MeOH (50 mL) was added H2SO4 (2.0 mL) at room temperature in portions. The reaction was refluxed for 4 hours. The reaction solution was cooled to room temperature and concentrated in vacuo. The residue was diluted with DCM (50 mL), washed with sat. aq. Na2CO3 and brine, dried over anhydrous sodium sulfate, filtered, and concentrated in vacuo to give the desired product 1,3-dibromo-2-(bromomethyl)benzene as a pale orange oil (4.21 g). Yield 91%. 1H NMR (400 MHz, CDCl3) δ 4.06 (m, 1H), 3.81 (s, 3H), 2.68 (m, 1H), 2.08 (m,1H), 1.03(d, J=6.8Hz, 3H), 0.87(d, J=6.8Hz, 3H).

**Step 2: (R)-methyl 2-(2,6-dibromo-4-fluorophenoxy)-3-methylbutanoate**

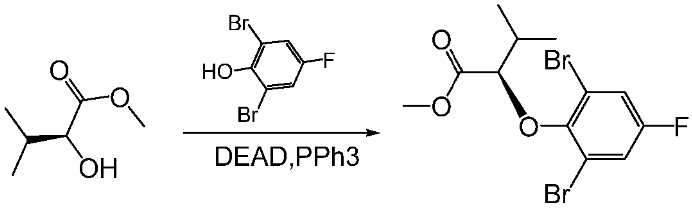


To a cooled solution of (S)-methyl 2-hydroxy-3-methylbutanoate (1.39 g, 10.5 mmol) and 2,6-dibromo-4-fluorophenol (1.90 g, 7 mmol) in THF (20 mL) at 0 °C under Ar atmophere, was added PPh3 (5.51 g, 21 mmol). The mixture was stirred at 0 °C for 10 minutes. Diethyl azodicarboxylate (3.66 g, 21 mmol) was added dropwise, and the reaction mixture was stirred at room temperature overnight. The reaction mixture was diluted with water (10 mL) and EtOAc (60 mL). The organic phase was washed with brine, dried over anhydrous sodium sulfate, filtered, and concentrated in vacuo. The residue was purified by silica gel column (pet ether: EtOAc 100:1 → 20:1) to give the desired product (R)-methyl 2-(2,6-dibromo-4-fluorophenoxy)-3-methylbutanoate as a pale yellow oil (2.45 g). Yield 91% (ESI 385 (M+H) +). $^1$H NMR (400 MHz, CDCl3) δ 7.28 (m, 2H), 4.86 (m, 1H), 3.73 (s, 3H), 2.44 (m,1H), 1.17 -1.34 (m, 6H).

**Step 3: (R)-2-(2,6-dibromo-4-fluorophenoxy)-3-methylbutan-1-ol**

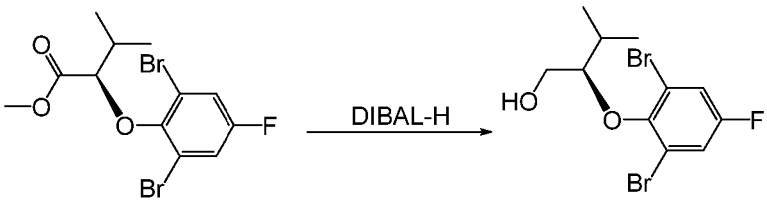


To a cooled solution of (R)-methyl 2-(2,6-dibromo-4-fluorophenoxy)-3-methylbutanoate (2.45 g, 6.38 mmol) in THF (60 mL) at -60 °C, was added a solution of DIBAL-H (1 M in toluene, 19 mL, 19 mmol) dropwise. The reaction mixture was stirred at -60 °C for 1 hour. The reaction was quenched with $Na_2SO_4$.10H2O, stirred at room temperature for 30 minutes, filtered and concentrated in vacuo. The residue was purified by silica gel column (pet ether: EtOAc 10:1 → 3:1) to give the desired product (R)-2-(2,6-dibromo-4-fluorophenoxy)-3-methylbutan-1-ol as a colorless oil (1.91 g). Yield 84% (ESI 378.9 (M+23) +).

**Step 4: (R)-8-bromo-6-fluoro-2-isopropyl-2,3-dihydrobenzo[b][1,4]dioxine**

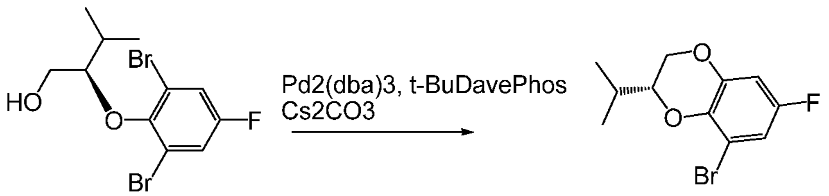


A mixture of (R)-2-(2,6-dibromo-4-fluorophenoxy)-3-methylbutan-1-ol (1.07 g, 3.0 mmol), Pd(OAc)2 (27 mg), t-BuDavephos (51 mg) and Cs2CO3 (1.47 g, 4.5 mmol) in toluene (20 mL) was warmed to 100 °C and stirred under Ar for 3 days. The mixture was diluted with water (10 mL) and extracted with EtOAc (40 mL). The organic phase was washed with brine, dried over anhydrous sodium sulfate, filtered, and concentrated in vacuo. The residue was purified by silica gel column (pet ether: EtOAc 10:1) to give the desired product (R)-8-bromo-6-fluoro-2-isopropyl-2,3-dihydrobenzo[b][1,4] dioxine as a colorless oil (361 mg). Yield 35% (ESI 275 (M+H) +). $^{1}$H NMR (400 MHz, CDCl3) 6.82 (m, 1H), 6.58 (m, 1H), 4.30 (m, 1H), 3.96 (m, 1H), 3.81 (m, 1H), 1.91 (m, 7H), 1.15 - 1.03 (m,6H).

**Step 5: tert-butyl 2-(3-(2-methoxypropan-2-yl)isochroman-5-yl)acetate**

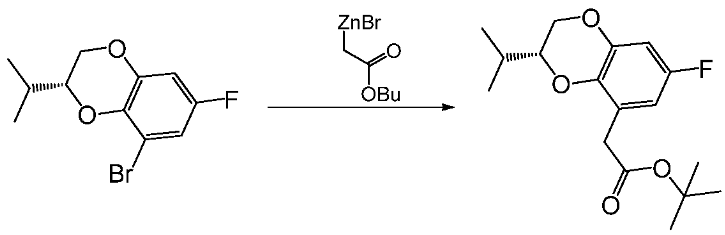

To a mixture of (R)-8-bromo-6-fluoro-2-isopropyl-2,3-dihydrobenzo[b][1,4]dioxine (495 mg, 1.8 mmol), $Pd_2(dba)_3$ (66 mg, 0.072 mmol) and Q-phos (51mg, 0.072 mmol) in THF (4 mL) at room temperature under Ar was added a solution of (2-tert-butoxy-2-oxoethyl)zinc(II) bromide (1 M in THF, 9 mL, 9 mmol). The reaction was warmed to 50 °C and stirred for 2 hours. The reaction was quenched with sat. aq. NaHCO3 solution, filtered and concentrated in vacuo, and the residue was purified by silica gel column (pet ether: EtOAc 10:1 → 5:1) to give the desired product tert-butyl 2-(3-(2-methoxypropan-2-yl)isochroman-5-yl)acetate as a pale orange oil (318 mg). Yield 57% (ESI 333.10 (M+23) +).$^{1}$H NMR (400 MHz, CDCl3) 6.53 (m, 2H), 4.27 (m, 1H), 3.92 (m, 1H), 3.76 (m, 1H), 3.50 (s, 2H), 1.91 (m, 1H), 1.46 (s, 9H), 1.10 (d, J=6.8Hz, 3H), 1.03 (d, J=6.8Hz, 3H).

**Step 6: tert-butyl 2-bromo-2-((R)-7-fluoro-3-isopropyl-2,3-dihydrobenzo [b] [1,4] dioxin-5-yl)acetate**

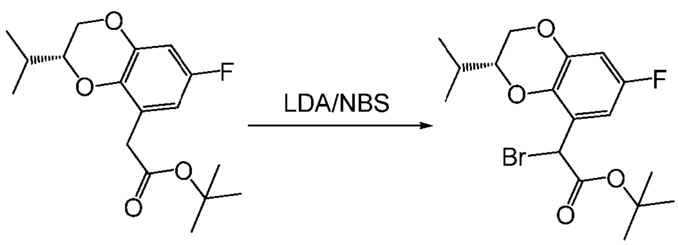


To a cooled solution of tert-butyl 2-(3-(2-methoxypropan-2-yl)isochroman-5-yl)acetate (310 mg, 1.0 mmol) in THF (10 mL) at -78 °C was added a solution of lithium diisopropylamide (2.0 M in THF/hexanes, 1.0 mL, 2.0 mmol) dropwise. The reaction was stirred at -78 °C for 30 min. Chlorotrimethylsilane (217 mg, 2.0 mmol) was added, and the reaction was stirred at -78 °C for another 30 min. Then a solution of N-bromosuccinimide (356 mg, 2.0 mmol) in THF (4 mL) was added, and the reaction was stirred at -78 °C for 30 min. The reaction was quenched with water (10 mL), extracted with EtOAc (3 × 20mL). The combined organic phase was washed with sat. NaHCO3 aq. solution and brine, dried over anhydrous sodium sulfate, filtered, and concentrated to give the desired product tert-butyl 2-bromo-2-((R)-7-fluoro-3-isopropyl-2,3-dihydrobenzo[b][1,4]dioxin-5-yl)acetate as an orange oil (390 mg), which was used in the next step directly

without further purification. Yield 82% (ESI 412.9 (M+23) +).

**Step 7: tert-butyl 2-((R)-7-fluoro-3-isopropyl-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetate**

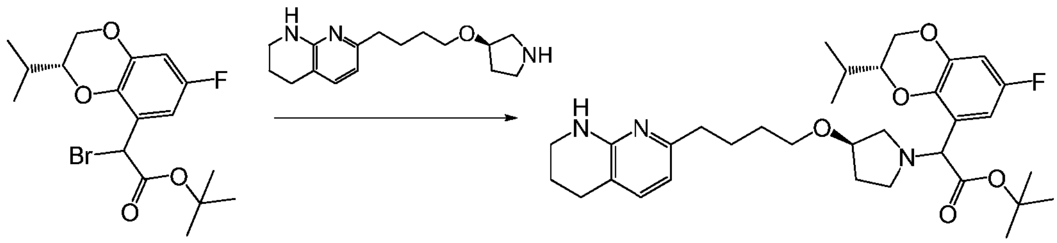

A mixture of tert-butyl 2-bromo-2-((R)-7-fluoro-3-isopropyl-2,3-dihydrobenzo[b][1,4]dioxin-5-yl)acetate (389 mg, 1.0 mmol), (R)-7-(4-(pyrrolidin-3-yloxy)butyl)-1,2,3,4-tetrahydro-1,8-naphthyridine (275 mg, 1.0 mmol) and DIPEA (387 mg, 3.0 mmol) in acetonitrile (10 mL) was stirred at room temperature for 4 hours. Solvent was removed in vacuo, and the residue was purified by Prep-HPLC A (30 → 65% MeCN) to give the desired product tert-butyl 2-((R)-7-fluoro-3-isopropyl-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetate as a white solid (241 mg). Yield 41% (ESI 584.3 (M+H) +).

**Step 8: 2-((R)-7-fluoro-3-isopropyl-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetic acid (compounds 6-E1 and 6-E2)**

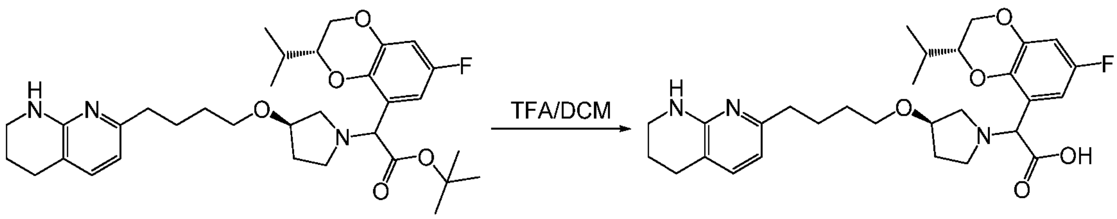


To a solution of tert-butyl 2-((R)-7-fluoro-3-isopropyl-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetate (240 mg, 0.410 mmol) in DCM (2.0 mL) was added TFA (2.0 mL). The mixture was stirred at room temperature for 18 hours. Solvent was removed in vacuo, and the residue was purified by Prep-HPLC A (30 → 65% MeCN) to give 2-((R)-7-fluoro-3-isopropyl-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetic acid as a white solid (162 mg, 75% yield), which was separated by Prep chiral SFC B to give diastereomeric products **compound 6-E1** (41 mg) and **compound 6-E2** (97 mg) as white solids.

**Compound 6-E1** LC/MS ESI 528.2 (M+H) +. 1H NMR (400 MHz, MeOD) δ 7.15 (d, J=7.2Hz,1H), 6.93-6.90 (m,1H), 6.70-6.67 (m,1H), 6.37 (d, J=7.2Hz,1H), 4.97 (s, 1H), 4.41-4.38 (m, 1H), 4.16 (s, 1H), 4.01-3.96 (m, 1H), 3.83-3.80 (m, 1H), 3.56-3.3.54 (m, 1H), 3.47-3.43 (m, 2H), 3.39-3.36 (m, 2H), 3.33-3.20 (m, 2H), 3.05 (m, 1H), 2.72-2.69 (m, 2H), 2.56-2.52 (m, 2H), 2.07-1.95 (m, 3H), 1.89-1.86 (m, 2H), 1.72-1.68 (m, 2H), 1.61-1.57 (m, 2H), 1.11 (d, J=6.8Hz,3H),1.04 (d, J=6.8Hz,3H). Chiral SFC B (30% EtOH): ee 100%, Rt = 1.01min.

**Compound 6-E2** LC/MS ESI 528.2 (M+H) +. 1H NMR (400 MHz, MeOD) δ 7.15 (d, J=7.2Hz,1H), 6.93-6.90 (m,1H), 6.70-6.67 (m,1H), 6.37 (d, J=7.2Hz,1H), 4.97 (s, 1H), 4.39-4.37(m, 1H), 4.16 (s, 1H), 4.00-3.93 (m, 2H), 3.62-3.36 (m, 6H), 3.33-3.20 (m, 2H), 2.72-2.69 (m, 2H), 2.56-2.52 (m, 2H), 2.13-2.10 (m, 2H), 1.98-1.96 (m, 1H), 1.89-1.86 (m, 2H), 1.71-1.67 (m, 2H), 1.61-1.55 (m, 2H), 1.11 (d, J=6.8Hz,3H),1.05 (d, J=6.8Hz,3H). Chiral SFC B (30% EtOH): ee 100%, Rt = 3.43min.

**Example 7: Preparation of 2-((R)-6-fluoro-2-isopropyl-4-methyl-3,4-dihydro-2H-benzo[b][1,4]oxazin-8-yl)-2-((R)-3-(4-(5,6,7,8-tetrahydro-1,8-naphthyridin-2-yl)butoxy)pyrrolidin-1-yl)acetic acid (compounds 7-E1 and 7-E2 )**

**Step1: (R)-2-(2,6-dibromo-4-fluorophenoxy)-3-methylbutyl methanesulfonate**

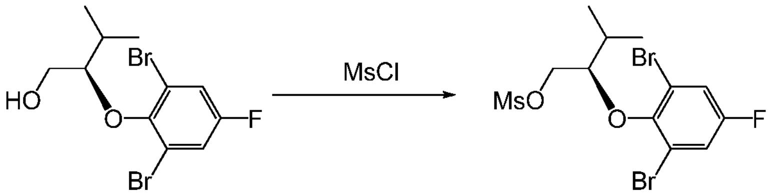


To a cooled mixture of (R)-2-(2,6-dibromo-4-fluorophenoxy)-3-methylbutan-1-ol (854 mg, 2.40 mmol) and TEA (728 mg, 7.2 mmol) in DCM (20 mL) at 0 °C was added a solution of MsCl (412 mg, 3.6 mmol) in DCM (2 mL) dropwise. The reaction mixture was stirred at 0 °C for 1 hour, then diluted with water (10 mL) and extracted with DCM (20 mL). The organic phase was dried over anhydrous sodium sulfate, filtered, and concentrated in vacuo to give the desired product (R)-2-(2,6-dibromo-4-fluorophenoxy)-3-methylbutyl methanesulfonate as a colorless oil (1.01 g). Yield 97% (ESI 456.8 (M+23) $^{+}$).

**Step 2: (R)-8-bromo-6-fluoro-2-isopropyl-4-methyl-3,4-dihydro-2H-benzo[b][1,4]oxazine**

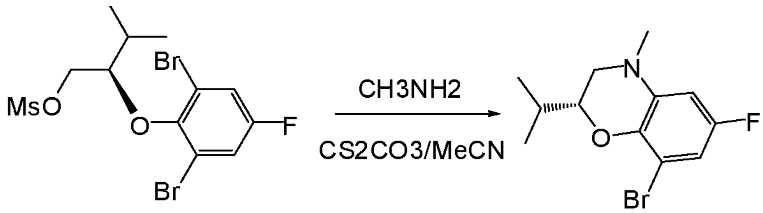


A mixture of (R)-2-(2,6-dibromo-4-fluorophenoxy)-3-methylbutyl methanesulfonate (434 mg, 1.0 mmol), NaI (150 mg, 1.0 mmol), Cs2CO3(652 mg, 2.0 mmol) and MeNH2 (8M in MeOH, 2.5 mL) in MeCN (10 mL) was warmed to 80 °C and stirred for 2 days. The reaction mixture was diluted with water (10 mL) and extracted with EtOAc (40 mL). The organic phase was washed with brine, dried over anhydrous sodium sulfate, filtered, and concentrated in vacuo. The resulting residue was purified by silica gel column (pet ether: EtOAc 5:1 → 2:1) to give the desired product (R)-8-bromo-6-fluoro-2-isopropyl-4-methyl-3,4-dihydro-2H-benzo[b][1,4]oxazine as a colorless oil (248 mg). Yield 86% (ESI 288.0 (M+H) +). 1H NMR (400 MHz, CDCl3) δ 6.60 (m, 1H), 6.30 (m, 1H), 3.81 (m, 1H), 3.28 (m, 1H), 3.14 (m, 1H), 2.88 (s, 3H), 1.92 (m, 1H), 1.15 (d, J=6.8Hz, 3H), 1.02 (d, J=6.8Hz, 3H).

**Step 3: (R)-tert-butyl 2-(6-fluoro-2-isopropyl-4-methyl-3,4-dihydro-2H-benzo[b][1,4]oxazin-8-yl)acetate**

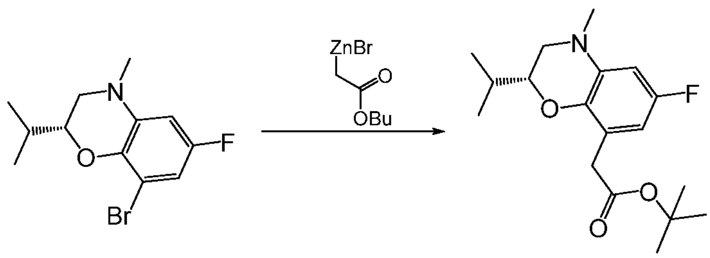

To a mixture of (R)-8-bromo-6-fluoro-2-isopropyl-4-methyl-3,4-dihydro-2H-benzo[b][1,4]oxazine (288 mg, 1.0 mmol), Pd2(dba)3 (37 mg, 0.04 mmol), and Q-phos (28 mg, 0.04 mmol) in THF (2 mL) was added a solution of (2-tert-butoxy-2-oxoethyl)zinc(II) bromide (1 M in THF, 5 mL, 5 mmol) at room temperature under Ar. The reaction mixture was warmed to 50 °C and stirred for 2 hours. The reaction was quenched with sat. aq. NaHCO3 solution, filtered and concentrated in vacuo, and the resulting residue was purified by silica gel column (pet ether: EtOAc 5:1 → 2:1) to give the desired product (R)-tert-butyl 2-(6-fluoro-2-isopropyl-4-methyl-3,4-dihydro-2H-benzo[b][1,4]oxazin-8-yl)acetate as a pale red oil (210 mg). Yield 65% (ESI 324.10(M+H) +). 1H NMR (400 MHz, CDCl3) δ 6.29 (m, 2H), 3.76 (m, 1H), 3.46

(s, 2H), 3.20 (m, 1H), 3.08(m, 1H), 2.86 (s, 3H), 1.86 (s, 3H), 1.46 (s, 9H), 1.10 (d, J=6.8Hz, 3H), 1.01 (d, J=6.8Hz, 3H).

**Step 4: tert-butyl 2-bromo-2-((R)-6-fluoro-2-isopropyl-4-methyl-3,4-dihydro-2H-benzo[b][1,4]oxazin-8-yl)acetate**

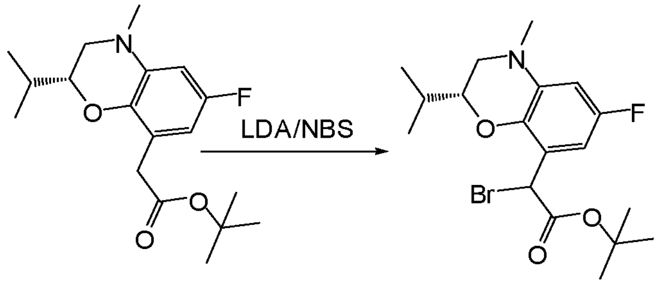


To cooled a solution of (R)-tert-butyl 2-(6-fluoro-2-isopropyl-4-methyl-3,4-dihydro-2H-benzo[b][1,4]oxazin-8-yl) acetate (323 mg, 1.0 mmol) in THF (10 mL) at -78 °C was added a solution of lithium diisopropylamide (2.0 M in THF/hexanes, 1.0 mL, 2.0 mmol) dropwise. The reaction was stirred at -78 °C for 30 min, then chlorotrimethylsilane (217 mg, 2.0 mmol) was added, and the reaction was stirred at -78 °C for another 30 min. A solution of N-bromosuccinimide (356 mg, 2.0 mmol) in THF (4 mL) was added, and the reaction was stirred at -78 °C for 30min. The reaction was quenched with H2O (10 mL) and extracted with ethyl acetate (3 × 20 mL). The combined organic layer was washed with brine, dried over anhydrous sodium sulfate, filtered, and concentrated in vacuo to give the desired product tert-butyl 2-bromo-2-((R)-6-fluoro-2-isopropyl-4-methyl-3,4-dihydro-2H-benzo[b][1,4]oxazin-8-yl)acetate as a pale yellow oil (401 mg), which was used in the next step directly without further purification. Yield 91% (ESI 402.0 (M+H) $^+$).

**Step 5: tert-butyl 2-((R)-6-fluoro-2-isopropyl-4-methyl-3,4-dihydro-2H-benzo[b][1,4]oxazin-8-yl)-2-((R)-3-(4-(5,6,7,8-tetrahydro-1,8-naphthyridin-2-yl)butoxy)pyrrolidin-1-yl)acetate**

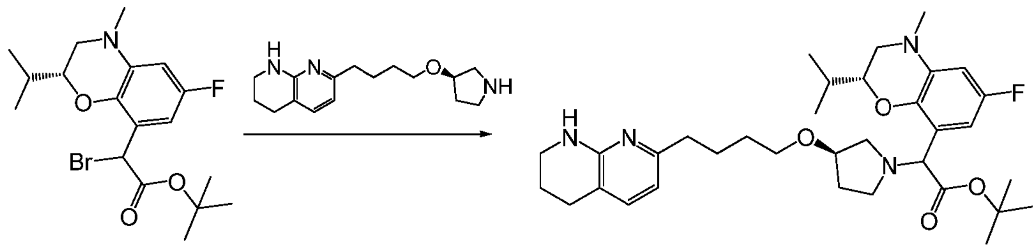

A mixture of tert-butyl 2-bromo-2-((R)-6-fluoro-2-isopropyl-4-methyl-3,4-dihydro-2H-benzo[b][1,4]oxazin-8-yl) acetate (401 mg, 1.0 mmol), (R)-7-(4-(pyrrolidin-3-yloxy)butyl)-1,2,3,4-tetrahydro-1,8-naphthyridine (275 mg, 1.0 mmol) and DIPEA (387 mg, 3.0 mmol) in acetonitrile (10 mL) was stirred at room temperature for 4 hours. Solvent was removed in vacuo, and the residue was purified by Prep-HPLC A (30 → 65% MeCN) to give the desired product tert-butyl 2-((R)-6-fluoro-2-isopropyl-4-methyl-3,4-dihydro-2H-benzo[b][1,4]oxazin-8-yl)-2-((R)-3-(4-(5,6,7,8-tetrahydro-1,8-naphthyridin-2-yl)butoxy)pyrrolidin-1-yl)acetate as a white solid (352 mg). Yield 59% (ESI 597.3 (M+H) +).

**Step 6: 2-((R)-7-fluoro-3-isopropyl-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetic acid (compounds 7-E1 and 7-E2)**

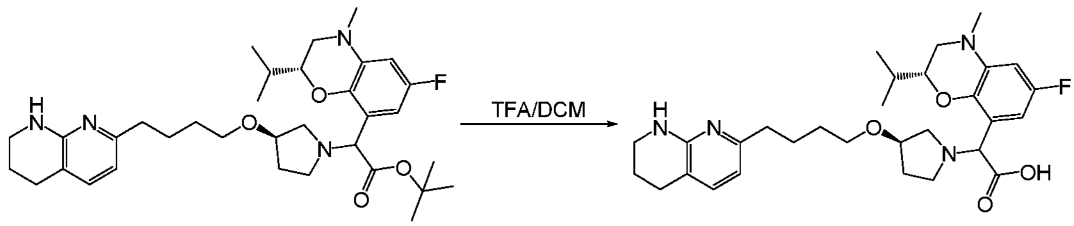


To a solution of tert-butyl 2-((R)-6-fluoro-2-isopropyl-4-methyl-3,4-dihydro-2H-benzo[b][1,4]oxazin-8-yl)-2-((R)-3-(4-(5,6,7,8-tetrahydro-1,8-naphthyridin-2-yl)butoxy)pyrrolidin-1-yl)acetate (352 mg, 0.59 mmol) in DCM (2.0 mL) was added TFA (2.0 mL). The mixture was stirred at room temperature for 18 hours. Solvent was removed in vacuo, and the resulting residue was purified by Prep-HPLC A (30 → 65% MeCN) to give the desired product 2-((R)-7-fluoro-3-isopropyl-2,3-dihydrobenzo[b][1,4]dioxin-5-yl)-2-((R)-3-(4-(5,6,7,8-tetrahydro-1,8-naphthyridin-2-yl)butoxy) pyrrolidin-1-yl)acetic acid as a white solid (230 mg, 72% yield). ESI 541.2 (M+H) +. The racemic product was separated by Prep chiral SFC A to give **compound 7-E1** (81 mg) and **compound 7-E2** (87 mg) as white solids.

**Compound 7-E1** LC/MS ESI 541.2 (M+H) $^{+}$. 1H NMR (400 MHz, MeOD) δ 7.13 (d, J=7.2Hz,1H), 6.58-6.55(m, 1H), 6.48-6.45 (m,1H), 6.37 (d, J=7.2Hz,1H), 5.02 (s, 1H), 4.17-4.15 (m, 1H), 3.76 (m, 1H), 3.60 (m, 1H), 3.47-3.44 (m, 2H), 3.43-3.36 (m, 4H), 3.23-3.08 (m, 3H), 2.91 (s, 3H), 2.72-2.69 (m, 2H), 2.55-2.51 (m, 2H), 2.12-2.02 (m, 2H), 1.92-1.86 (m, 3H), 1.72-1.57 (m, 4H), 1.11 (d, J=6.8Hz,3H), 1.02 (d, J=6.8Hz,3H). Chiral SFC F (45% MeOH): ee 100%, Rt = 2.15 min.

**Compound 7-E2** LC/MS ESI 541.2 (M+H) $^{+}$. 1H NMR (400 MHz, MeOD) δ 7.13 (d, J=7.2Hz,1H), 6.58-6.55(m, 1H), 6.47-6.44 (m,1H), 6.36 (d, J=7.2Hz,1H), 4.91 (s, 1H), 4.17-4.15 (m, 1H), 3.91 (m, 1H), 3.56 (m, 1H), 3.46-3.35 (m, 6H), 3.35-3.26 (m, 3H), 2.90 (s, 3H), 2.72-2.69 (m, 2H), 2.54-2.50 (m, 2H), 2.14-2.12 (m, 2H), 1.93-1.86 (m, 3H), 1.70-1.55 (m, 4H), 1.11 (d, J=6.8Hz,3H), 1.03 (d, J=6.8Hz,3H). Chiral SFC F (45% MeOH): ee 99%, Rt = 4.15 min.

**Example 8: Preparation of 2-(7-fluoro-3-(2-fluoropropan-2-yl)isochroman-5-yl)-2-((R)-3-(4-(5,6,7,8-tetrahydro-1,8-naphthyridin-2-yl)butoxy)pyrrolidin-1-yl)acetic acid (compounds 8-E1 and 8-E2)**

**Step 1: 1,3-dibromo-5-fluoro-2-(3-methylbut-2-enyl)benzene**

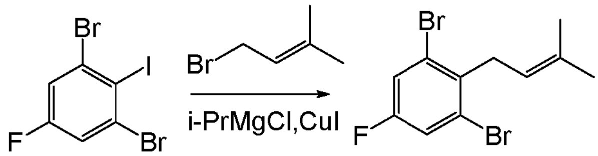


To a cooled solution of 1,3-dibromo-5-fluoro-2-iodobenzene (10 g, 26.5 mmol) in THF (100 mL) at -20 °C under Ar atmosphere was added a solution of i-PrMgCl (1 M in THF, 31.7 mL, 31.7 mmol) slowly dropwise. The mixture was stirred for 10 min at -20 °C and then warmed to 0 °C and stirred for an additional 50 min. CuI (1.26 g, 6.62 mmol) was added, and the reaction was stirred at 0 °C for 10 min. A solution of 1-bromo-3-methylbut-2-ene (5.87 g, 39.69 mmol) in THF (10 mL) was added. The reaction mixture was allowed to warm to room temperature and stirred overnight, then quenched with saturated aq. $NH_4Cl$ (20 mL) and extracted with EtOAc (3×50 mL). The combined organic layers were washed with brine, dried over anhydrous sodium sulfate, filtered and concentrated in vacuo. The residue was purified by silica gel column (100% pet ether) to afford the desired product 1,3-dibromo-5-fluoro-2-(3-methylbut-2-enyl)benzene as a colorless oil (7.2 g). Yield 85%. 1H NMR (400 MHz, CDCl3) δ 7.35-7.25 (m, 2H), 5.05-4.98 (m, 1H), 3.75-3.60 (m, 2H), 1.81 (s, 3H), 1.71 (s, 3H).

**Step 2: 3-bromo-5-fluoro-2-(3-methylbut-2-enyl)benzaldehyde**

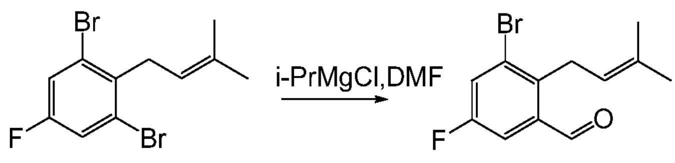

To a cooled solution of 1,3-dibromo-5-fluoro-2-(3-methylbut-2-enyl)benzene (7.2 g, 22.5 mmol) in THF (100 mL) at 0 °C under Ar atmosphere was added a solution of i-PrMgCl (1 M in THF, 33.8 mL, 33.8 mmol) dropwise slowly. The reaction was warmed to room temperature and stirred for 1 hour. DMF (4.93 g, 67.5 mmol) was added, and the reaction was stirred at room temperature for 1 hour. The reaction was quenched with saturated aq. NH4Cl (20 mL) and extracted with EtOAc (3 × 50 mL). The combined organic layers were washed with brine, dried over anhydrous sodium sulfate, filtered, and concentrated in vacuo. The residue was purified by silica gel column (pet ether: EtOAc 0% → 10%) to afford the desired product 3-bromo-5-fluoro-2-(3-methylbut-2-enyl)benzaldehyde as a colorless oil (5.2 g). Yield 85%. 1H NMR (400 MHz, CDCl3) δ 10.22 (s, 1H), 7.60-7.52 (m, 2H), 5.05-4.95 (m, 1H), 3.95-3.85 (m, 2H), 1.80 (s, 3H), 1.70 (s, 3H).

**Step 3: (3-bromo-5-fluoro-2-(3-methylbut-2-enyl)phenyl)methanol**

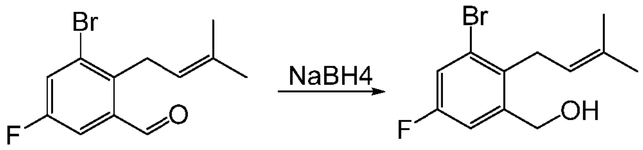


To a solution of 3-bromo-5-fluoro-2-(3-methylbut-2-enyl)benzaldehyde (5.2 g, 19.26 mmol) in MeOH (50 mL) at 0 °C, was added NaBH4 (1.46 g, 38.52 mmol) in portions. The mixture was stirred at room temperature for 1 hour. Solvent was removed in vacuo, and the residue was purified by silica gel column (pet ether: EtOAc 0% → 20%) to give the desired product (3-bromo-5-fluoro-2-(3-methylbut-2-enyl)phenyl)methanol as a colorless oil (4.4 g). Yield 84% (ESI 255 (M-$H_2O$+H) +).

**Step 4: 3-bromo-5-fluoro-2-(3-methylbut-2-enyl)benzyl acetate**

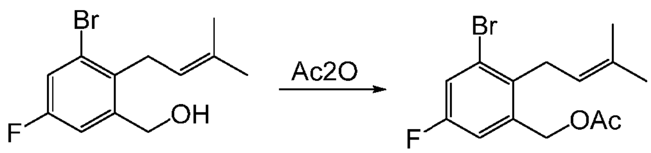


A solution of (3-bromo-5-fluoro-2-(3-methylbut-2-enyl)phenyl)methanol (4.4 g, 16.18 mmol) and $Ac_2O$ (1.82 g, 17.80 mmol) in pyridine (18 mL) was stirred at room temperature overnight. The mixture was quenched with aqueous HCl (1M, 100 mL) and extracted with EtOAc (3×100 mL). The combined oragnic phase was washed with brine, dried over anhydrous sodium sulfate, filtered and concentrated in vacuo. The residue was purified by silica gel column (pet ether: EtOAc 0% → 20%) to afford the desired product 3-bromo-5-fluoro-2-(3-methylbut-2-enyl)benzyl acetate as a colorless oil (4.4 g). Yield 86%. 1H NMR (400 MHz, CDCl3) δ 7.30-7.26 (m, 1H), 7.10-7.04 (m, 1H), 5.09 (s, 2H), 4.97-4.93 (m, 1H), 3.55-3.45 (m, 2H), 2.12 (s, 3H), 1.77 (s, 3H), 1.70 (s, 3H).

**Step 5: 3-bromo-2-((3,3-dimethyloxiran-2-yl)methyl)-5-fluorobenzyl acetate**

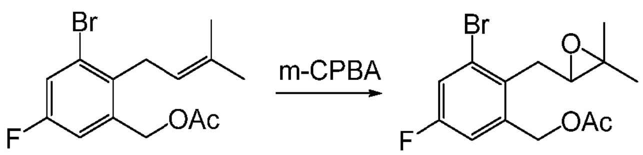


A mixture of 3-bromo-5-fluoro-2-(3-methylbut-2-enyl)benzyl acetate (4.4 g, 14.01 mmol) and m-CPBA (2.42 g, 14.01 mmol) in DCM (60 mL) was stirred at room temperature overnight. The mixture was quenched with saturated aq. NaHCO3 (50 mL) and extracted with DCM (3 × 50 mL). The combined organic phase was washed with brine, dried over anhydrous sodium sulfate, filtered, and concentrated in vacuo. The residue was purified by silica gel column (pet ether: EtOAc 0% → 20%) to afford the desired product 3-bromo-2-((3,3-dimethyloxiran-2-yl)methyl)-5-fluorobenzyl acetate as a

colorless oil (3.8 g). Yield 82% (ESI 271 (M-59) +).

**Step 6: 2-(5-bromo-7-fluoroisochroman-3-yl)propan-2-ol**

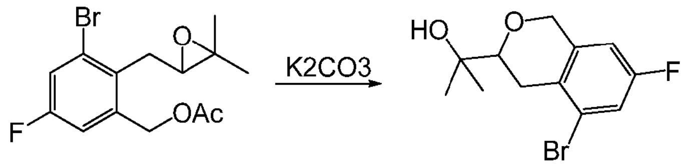


A mixture of 3-bromo-2-((3,3-dimethyloxiran-2-yl)methyl)-5-fluorobenzyl acetate (3.8 g, 11.52 mmol) and $K_2CO_3$ (4.8 g, 34.56 mmol) in MeOH (50 mL) was stirred at room temperature overnight. Solvent was removed in vacuo, and the residue was purified by silica gel column (pet ether: EtOAc 10:1 → 3:1) to afford the desired product 2-(5-bromo-7-fluoroisochroman-3-yl)propan-2-ol as a colorless oil (2.7 g). Yield 81% (ESI 271 (M-$H_2$O+H) +).

**Step 7: 5-bromo-7-fluoro-3-(2-fluoropropan-2-yl)isochroman**

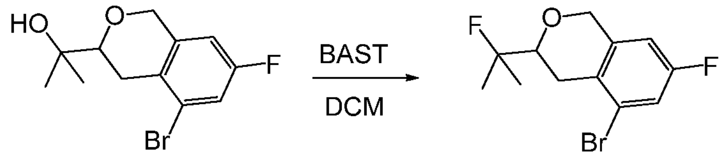


To a cooled solution of 2-(5-bromo-7-fluoroisochroman-3-yl)propan-2-ol (2.7 g, 9.38 mmol) in DCM (100 mL) at 0 °C was added bis(2-methoxyethyl)aminosulfur trifluoride (10.4 g, 46.9 mmol) dropwise. The reaction mixture was stirred at room temperature overnight. The reaction was quenched with H2O (20 mL) and extracted with DCM (3 × 20 mL). The combined organic phases were washed with sat. aq. NaHCO3 solution, dried over anhydrous sodium sulfate, filtered and concentrated in vacuo. The residue was purified by silica gel column (pet ether: EtOAc 0% → 10%) to afford the desired product 5-bromo-7-fluoro-3-(2-fluoropropan-2-yl)isochroman as a colorless oil (1.4 g). Yield 51% (ESI 271).

**Step 8: tert-butyl 2-(7-fluoro-3-(2-fluoropropan-2-yl)isochroman-5-yl)acetate**

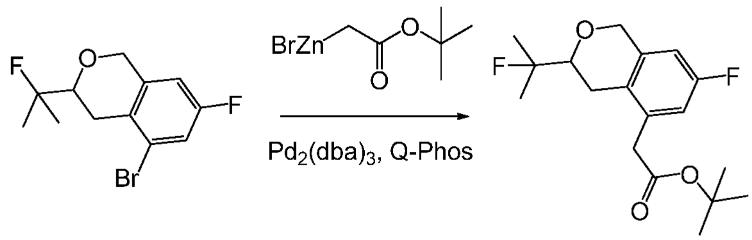


To a mixture of 5-bromo-7-fluoro-3-(2-fluoropropan-2-yl)isochroman (1.4 g, 4.83 mmol), $Pd_2(dba)_3$ (252 mg, 0.24 mmol), and Q-Phos (170 mg, 0.24 mmol) was added as solution of (2-tert-butoxy-2-oxoethyl)zinc(II) bromide (1 M in THF, 24 mL, 24 mmol). The reaction mixture was stirred at 60 °C overnight. The mixture was quenched with NaHCO3 aq. solution, filtered and concentrated in vacuo, and the resulting residue was purified by silica gel column (pet ether: EtOAc 0% → 20%) to afford the desired product tert-butyl 2-(7-fluoro-3-(2-fluoropropan-2-yl)isochroman-5-yl)acetate as a red oil (850 mg). Yield 54% (ESI 349 (M+Na) +).

**Step 9: tert-butyl 2-bromo-2-(7-fluoro-3-(2-fluoropropan-2-yl)isochroman-5-yl)acetate**

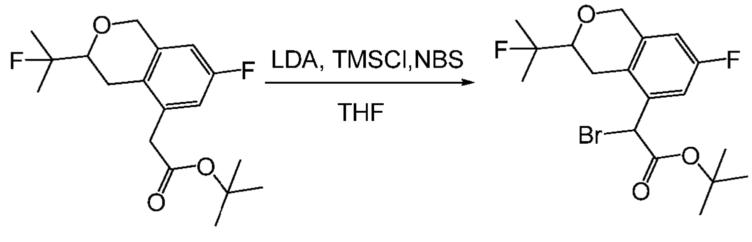


To a cooled solution of tert-butyl 2-(7-fluoro-3-(2-fluoropropan-2-yl)isochroman-5-yl)acetate (850 mg, 2.6 mmol) in THF (50 mL) at -78 °C was added a solution of lithium diisopropylamide (2.0 M in toluene, 3.3 mL, 6.5 mmol) dropwise. The reaction was stirred at -78 °C for 30 min. Then a solution of chlorotrimethylsilane (702 mg, 6.5 mmol) in THF (5 mL) was added, and the reaction was stirred at -78 °C for another 30 min. A solution of N-bromosuccinimide (1.16 g, 6.5 mmol) in THF (10 mL) was added, and the reaction was stirred at -78 °C for 30 min. The reaction was quenched with MeOH (2 mL) and concentrated in vacuo, and the residue was purified by silica gel column (pet ether: EtOAc 10:1) to give the desired product tert-butyl 2-bromo-2-(7-fluoro-3-(2-fluoropropan-2-yl)isochroman-5-yl)acetate as a pale yellow oil (950 mg). Yield 90% (ESI 427 (M+Na) +).

**Step 10: tert-butyl 2-(7-fluoro-3-(2-fluoropropan-2-yl)isochroman-5-yl)-2-((R)-3-(4-(5,6,7,8-tetrahydro-1,8-naphthyridin-2-yl)butoxy)pyrrolidin-1-yl)acetate**

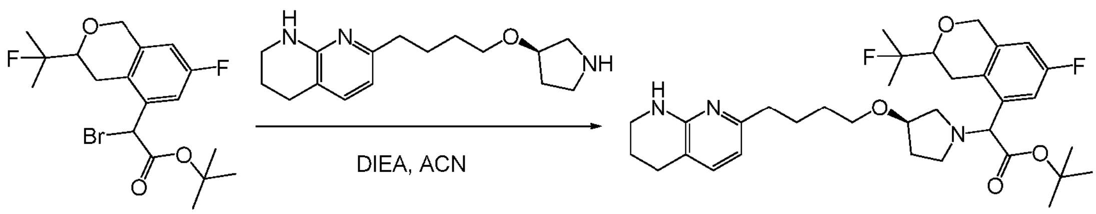


A mixture of (R)-7-(4-(pyrrolidin-3-yloxy)butyl)-1,2,3,4-tetrahydro-1,8-naphthyridine (250 mg, 0.72 mmol), tert-butyl 2-bromo-2-(7-fluoro-3-(2-fluoropropan-2-yl)isochroman-5-yl)acetate (291 mg, 0.72 mmol), and DIEA (279 mg, 2.16 mmol) in acetonitrile (10 mL) was stirred at room temperature overnight. Solvent was removed in vacuo, and the residue was purified by silica gel column (DCM/MeOH 0% → 10%) to give the desired product tert-butyl 2-(7-fluoro-3-(2-fluoropropan-2-yl)isochroman-5-yl)-2-((R)-3-(4-(5,6,7,8-tetrahydro-1,8-naphthyridin-2-yl)butoxy)pyrrolidin-1-yl)acetate as a pale yellow oil (380 mg). Yield 88% (ESI 600 (M+H) +).

Step 11: **2-(7-fluoro-3-(2-fluoropropan-2-yl)isochroman-5-yl)-2-((R)-3-(4-(5,6,7,8-tetrahydro-1,8-naphthyridin-2-yl)butoxy)pyrrolidin-1-yl)acetic acid (compounds 8-E1 and 8-E2)**

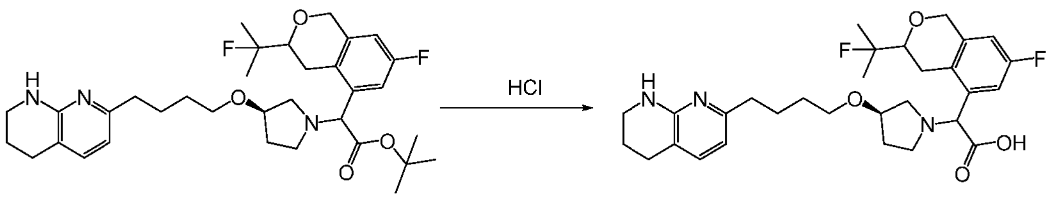


Tert-butyl 2-(7-fluoro-3-(2-fluoropropan-2-yl)isochroman-5-yl)-2-((R)-3-(4-(5,6,7,8-tetrahydro-1,8-naphthyridin-2-yl)butoxy)pyrrolidin-1-yl)acetate (380 mg, 0.63 mmol) was treated with HCl (4M in 1,4-dioxane, 20 mL) at room temperature overnight. Solvent was removed in vacuo, and the residue was purified by Prep-HPLC A (40 → 70% MeCN) to give the desired product (110 mg, 32% yield). The racemic product was separated by Prep chiral SFC E to give products **compound 8-E1** (43 mg as a mixture of 3 stereoisomers) and **compound 8-E2** (18 mg as a single stereoisomer) as white solids.

**Compound 8-E1 (mixture of 3 stereoisomers)** LC/MS ESI 544.2 (M+H) +. 1H NMR (400 MHz, MeOD) δ 7.40-7.35 (m, 1H), 7.22-7.18 (m, 1H), 6.90-6.85 (m, 1H), 6.45-6.40 (m, 1H), 4.90-4.75 (m, 3H), 4.20-4.15 (m, 1H),

3.75-3.70 (m, 1H), 3.55-3.35 (m, 4H), 3.30-3.00 (m, 4H), 2.75-2.70 (m, 2H), 2.65-2.58 (m, 2H), 2.20-2.10 (m, 2H), 1.98-1.85 (m, 2H), 1.80-1.55 (m, 4H), 1.50-1.20 (m, 8H).

**Compound 8-E2** LC/MS ESI 544.2 (M+H) +. 1H NMR (400 MHz, MeOD) δ 7.40-7.35 (m, 1H), 7.22-7.18 (m, 1H), 6.90-6.85 (m, 1H), 6.45-6.40 (m, 1H), 4.90-4.75 (m, 3H), 4.20-4.15 (m, 1H), 3.75-3.70 (m, 1H), 3.55-3.35 (m, 4H), 3.30-3.00 (m, 4H), 2.75-2.70 (m, 2H), 2.65-2.58 (m, 2H), 2.15-2.05 (m, 2H), 1.95-1.85 (m, 2H), 1.80-1.60 (m, 4H), 1.48-1.25 (m, 8H).

**Example 9: Preparation of 2-(7-fluoro-3-methylchroman-5-yl)-2-((R)-3-(4-(5,6,7,8-tetrahydro-1,8-naphthyridin-2-yl)butoxy)pyrrolidin-1-yl)acetic acid (compounds 9-E1, 9-E2 and 9-E3)**

**Step 1: 1-bromo-3,5-difluoro-2-(2-methylallyl)benzene**

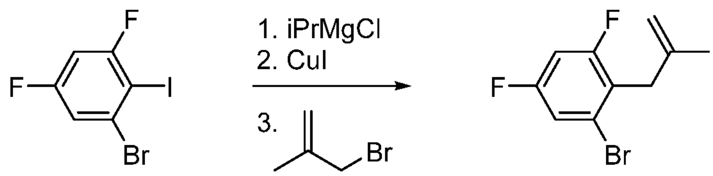


To a cooled solution of 1-bromo-3,5-difluoro-2-iodobenzene (3.00 g, 9.41 mmol) in THF (15 mL, 0.63 M) at -20 °C was added a solution of iPrMgCl (2M in THF, 6.12 mL, 12.23 mmol) dropwise. The mixture was stirred for 10 min and then warmed to 0 °C and stirred an additional 50 min. Copper iodide (0.448 g, 2.35 mmol) was added, and the reaction was stirred for 10 min. 3-bromo-2-methylprop-1-ene (1.04 mL, 10.4 mmol) was added, and the mixture was warmed to room temperature and stirred overnight. The reaction was quenched with sat. aq. NH4Cl (20 mL), diluted with EtOAc (20 mL) and extracted with EtOAc (3 x 10 mL). The combined organics were washed with brine (2 x 20 mL), dried over anhydrous sodium sulfate, filtered, and concentrated in vacuo. The resulting residue was purified by silica gel column (100% Hex) to give the desired product 1-bromo-3,5-difluoro-2-(2-methylallyl)benzene as a colorless and clear oil (1.60 g). Yield 69%.

**Step 2: 3-(2-bromo-4,6-difluorophenyl)-2-methylpropan-1-ol**

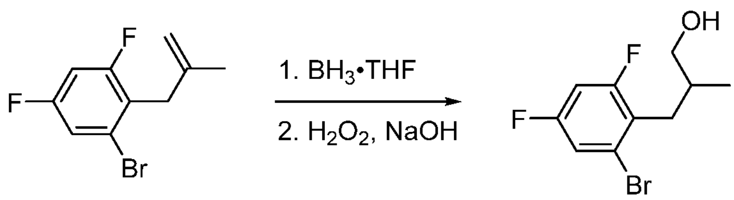


To a cooled solution of 1-bromo-3,5-difluoro-2-(2-methylallyl)benzene (1.5 g, 6.07 mmol) in THF (10 mL, 0.61 M) at 0 °C was added a solution of BH3 THF (1 M in THF, 8.50 mL, 8.50 mmol) dropwise. The mixture was stirred for 30 min and then warmed to room temperature and stirred for 2 hours. The reaction was cooled to 0 °C, and an aqueous 3M solution of NaOH (10.1 mL, 30.4 mmol) was added followed by H2O2 (3.10 mL, 30.4 mmol). The mixture was stirred for 30 min and then warmed to 60 °C and stirred an additional 1.5 hours. The reaction was cooled to room temperature, quenched with water (10 mL) and extracted with EtOAc (3 x 10 mL). The combined organics were washed with brine (3 x 20 mL), dried over anhydrous sodium sulfate, filtered, and concentrated in vacuo. The resulting residue was purified by silica gel column (EtOAc/Hex 0% -10%) to give the desired product 3-(2-bromo-4,6-difluorophenyl)-2-methylpropan-1-ol as a clear and colorless oil (1.34 g). Yield 83%.

**Step 3: 5-bromo-7-fluoro-3-methylchromane**

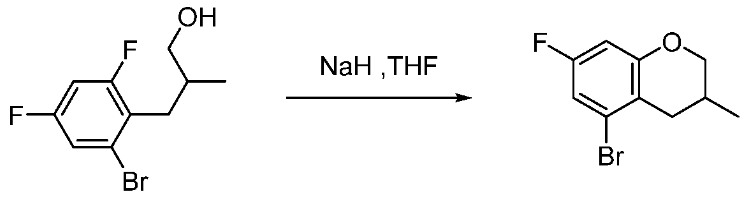


To a cooled solution of 3-(2-bromo-4,6-difluorophenyl)-2-methylpropan-1ol (980 mg, 3.70 mmol) in a 4:1 mixture of toluene (16 mL) and DMF (4 mL) at 0 °C, was added sodium hydride (325 mg, 8.13 mmol). The mixture was stirred for 2 hours and then warmed to room temperature. The reaction was quenched with sat. aq. NH4Cl (5 mL), diluted with water (15 mL) and DCM (5 mL) and extracted with DCM (3 x 5 mL). The combined organics were washed with brine, dried over anhydrous sodium sulfate, filtered, and concentrated in vacuo. The resulting residue was purified by silica gel column (100% Hexanes) to give the desired product 5-bromo-7-fluoro-3-methylchromane (667 mg). Yield 73%.

**Step 4: tert-butyl 2-(7-fluoro-3-methylchroman-5-yl)acetate**

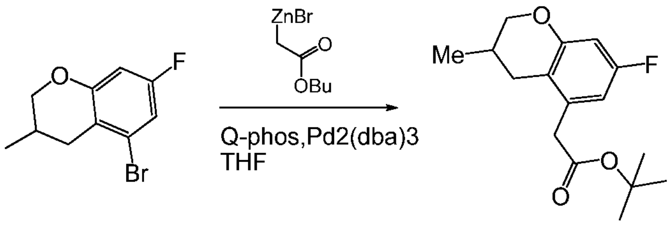


A mixture of 5-bromo-7-fluoro-3-methylchroman (100 mg, 0.41 mmol), (2-tert-butoxy-2-oxoethyl)zinc(II) bromide solution (0.5 M in THF, 4.1 mL, 2.05 mmol), Pd2(dba)3 (22 mg, 0.020 mmol), and Q-phos ( 15 mg, 0.020 mmol) in THF (2 mL) was warmed to 60 °C and stirred for 2 hours. The mixture was quenched with NaHCO3 aq. solution, filtered and concentrated in vacuo, and the residue was purified by silica gel column (pet ether: EtOAc 10:1) to give the desired product tert-butyl 2-(7-fluoro-3-methylchroman-5-yl)acetate as a pale red oil (110 mg). Yield 96% (ESI 303/305 [M+Na]+).

**Step 5: tert-butyl 2-bromo-2-(7-fluoro-3-methylchroman-5-yl)acetate**

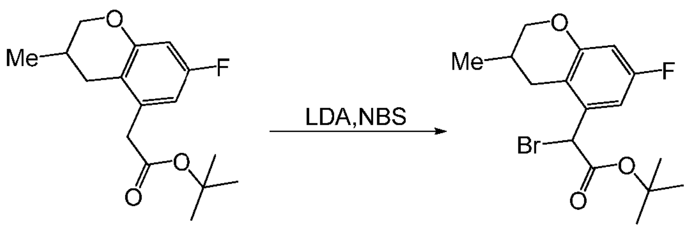


To a cooled solution of tert-butyl 2-(7-fluoro-3-methylchroman-5-yl)acetate (110 mg, 0.40 mmol) in THF (10 mL) at -78 °C was added a solution of lithium diisopropylamide (2.0 M in THF/hexanes, 0.53 mL, 1.07 mmol) dropwise. The reaction was stirred at -78 °C for 30 min, then a solution of chlorotrimethylsilane (116 mg, 1.07 mmol) in THF (1mL) was added, and the reaction was stirred at -78 °C for another 30 min. A solution of N-bromosuccinimide (190 mg, 1.07 mmol) in THF (10 mL) was added, and the reaction was stirred at -78 °C for 1 hour. The reaction was quenched with MeOH (2 mL) and concentrated in vacuo, and the residue was purified by silica gel column (pet ether: EtOAc 10:1) to give the desired product tert-butyl 2-bromo-2-(7-fluoro-3-methylchroman-5-yl)acetate as a colorless oil (134 mg). Yield 95% (ESI 381/383 [M+Na]+).

**Step 6: tert-butyl 2-(7-fluoro-3-methylchroman-5-yl)-2-((R)-3-(4-(5,6,7,8-tetrahydro-1,8-naphthyridin-2-yl)butoxy)pyrrolidin-1-yl)acetate**

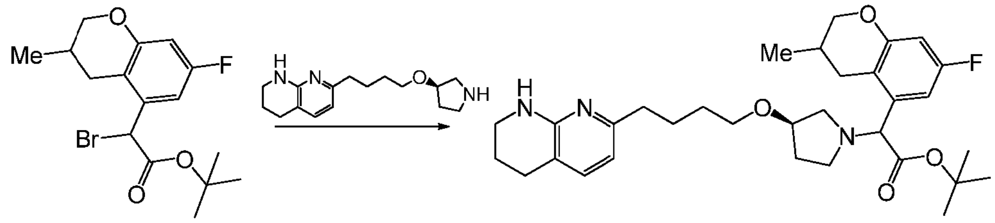

A mixture of tert-butyl 2-bromo-2-(7-fluoro-3-methylchroman-5-yl)acetate (134 mg, 0.44 mmol), (R)-5-methoxy-7-(4-(pyrrolidin-3-yloxy)butyl)-1,2,3,4-tetrahydro-1,8-naphthyridine (135 mg, 0.44 mmol), DIPEA (166 mg, 1.32 mmol), and NaI (20 mg) in acetonitrile (20 mL) was warmed to 40 °C and stirred for 17 hours. The mixture was diluted with water (8 mL) and extracted with EtOAc (25 mL). The organic phase was washed with brine, dried over anhydrous sodium sulfate, filtered, and concentrated in vacuo. The residue was purified by silica gel column (DCM: MeOH 20:1) to give the desired product tert-butyl 2-(7-fluoro-3-methylchroman-5-yl)-2-((R)-3-(4-(5,6,7,8-tetrahydro-1,8-naphthyridin-2-yl)butoxy)pyrrolidin-1-yl)acetate as a colorless oil (151 mg). Yield 62% (ESI 554 [M+H]+).

**Step 7: 2-(7-fluoro-3-methylchroman-5-yl)-2-((R)-3-(4-(5,6,7,8-tetrahydro-1,8-naphthyridin-2-yl)butoxy)pyrrolidin-1-yl)acetic acid (compounds 9-E1, 9-E2 and 9-E3)**

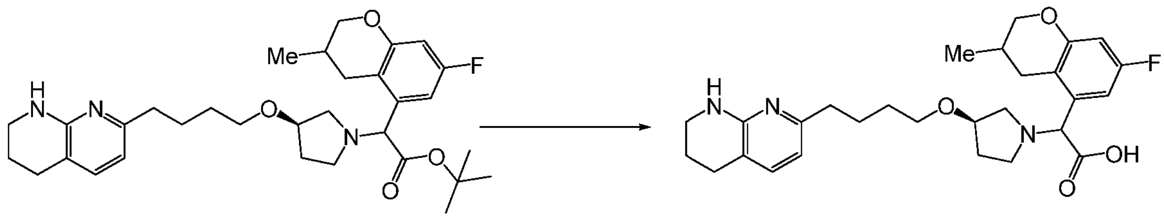

To a solution of tert-butyl 2-(7-fluoro-3-methylchroman-5-yl)-2-((R)-3-(4-(5,6,7,8-tetrahydro-1,8-naphthyridin-2-yl)butoxy)pyrrolidin-1-yl)acetate (151 mg, 0.27 mmol) in DCM (10 mL) was added TFA (10 mL). The mixture was stirred at room temperature for 17 hours. Solvent was removed in vacuo, and the residue was purified by Prep-HPLC A (40 → 70% MeCN) to give the desired product (120 mg). Yield 88%. The racemic product was separated by Prep chiral SFC E to give products **compound 9-E1** (50 mg as a mixture of 2 stereoisomers), **compound 9-E2** (10 mg as a single stereoisomer) and **compound 9-E3** (9 mg as a single stereoisomer) as white solids.

**Compound 9-E1 (mixture of 2 stereoisomers)** LC/MS ESI 498 (M+H) +. 1H NMR (400 MHz, MeOD) δ 7.18 (d, J = 7.6 Hz, 1H), 7.01-6.99 (m, 1H), 6.58-6.56 (m, 1H), 6.39 (d, J = 7.6 Hz, 1H), 4.73-4.72 (m, 1H), 4.19-4.17 (m, 1H), 3.62-3.31 (m, 6H), 3.30 -2.95 (m, 4H), 2.75 -2.01 (m, 8H), 1.85-1.60 (m, 6H), 1.05-1.01 (m, 3H). Chiral SFC E (45% MeOH/MeCN 1:1): ee 100%, Rt = 2.92min.

**Compound 9-E2** LC/MS ESI 498 (M+H) +. 1H NMR (400 MHz, MeOD) δ 7.18 (d, J = 7.2 Hz, 1H), 6.98-6.96 (m, 1H), 6.58-6.56 (m, 1H), 6.40 (d, J = 7.2 Hz, 1H), 4.79 (s, 1H), 4.19-4.17 (m, 1H), 3.62-3.31 (m, 6H), 3.30 -2.85 (m, 4H), 2.75 -2.01 (m, 8H), 1.85-1.60 (m, 6H), 1.05-1.01 (m, 3H). Chiral SFC E (45% MeOH/MeCN 1:1): ee 100%, Rt = 3.96min.

**Compound 9-E3** LC/MS ESI 498 (M+H) +. 1H NMR (400 MHz, MeOD) δ 7.18 (d, J = 7.2 Hz, 1H), 6.98-6.96 (m, 1H), 6.58-6.56 (m, 1H), 6.40 (d, J = 7.2 Hz, 1H), 4.80 (s, 1H), 4.19-4.17 (m, 1H), 3.62-3.31 (m, 6H), 3.30 -3.01 (m, 4H), 2.75 -2.01 (m, 8H), 1.85-1.60 (m, 6H), 1.05-1.01 (m, 3H). Chiral SFC E (45% MeOH/MeCN 1:1): ee 98%, Rt = 5.05min.

**Example 10: Preparation of 2-(3-(2-fluoropropan-2-yl)chroman-5-yl)-2-((R)-3-(4-(5,6,7,8-tetrahydro-1,8-naphthyridin-2-yl)butoxy)pyrrolidin-1-yl)acetic acid (compounds 10-A-E1, 10-A-E2, 10-B-E1 and 10-B-E2)**

**Step 1: ethyl 2-(2-bromo-6-fluorobenzyl)-3-methylbut-3-enoate**

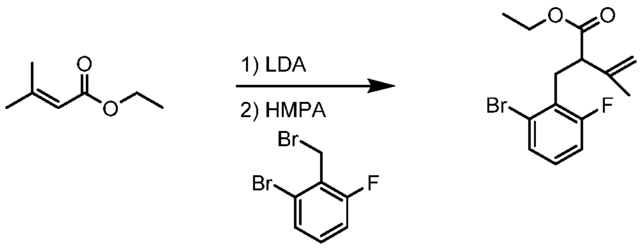


To a solution of diisopropylamine (2.36 mL, 16.81 mmol) in dry tetrahydrofuran (20 mL) at -78 °C was added n-butyllithium in hexanes (2.5 M, 6.47 mL, 16.18 mmol). The mixture was stirred at -78 °C for 45 minutes. Then ethyl 3,3-dimethylacrylate (2.08 mL, 14.94 mmol) was added dropwise, and the mixture was stirred at -78 °C for 30 minutes. Next, a mixture of 1-bromo-2-(bromomethyl)-3-fluorobenzene (3.71 g, 12.45 mmol) and HMPA (2.17 mL, 12.45 mmol) in dry tetrahydrofuran (5 mL) were added at -78 °C. The mixture was stirred at -78 °C for a few minutes, at 0 °C for 20 minutes and allowed to come to room temperature overnight. The reaction mixture was quenched with saturated aqueous ammonium chloride and extracted three times with diethyl ether. The combined organic layers were washed with brine, dried over sodium sulfate and concentrated in vacuo. Purification by column chromatography (silica, 0% to 10% diisopropyl ether in heptane) afforded the desired product ethyl 2-(2-bromo-6-fluorobenzyl)-3-methylbut-3-enoate (3.04 g). Yield 77%. $^1$H NMR (400 MHz, Chloroform-d) δ 7.34 (d, J = 8.1, 1.3 Hz, 1H), 7.10 - 7.02 (m, 1H), 7.02 - 6.93 (m, 1H), 4.87 - 4.80 (m, 1H), 4.72 (s, 1H), 4.14 (q, J = 7.1 Hz, 2H), 3.53 (dd, J = 8.5, 6.9 Hz, 1H), 3.28 (ddd, J = 13.8, 6.9, 1.8 Hz, 1H), 3.15 (ddd, J = 13.8, 8.5, 1.7 Hz, 1H), 1.80 (d, J = 0.7 Hz, 3H), 1.22 (t, J = 7.1 Hz, 3H).

**Step 2: 2-(2-bromo-6-fluorobenzyl)-3-methylbut-3-en-1-ol**

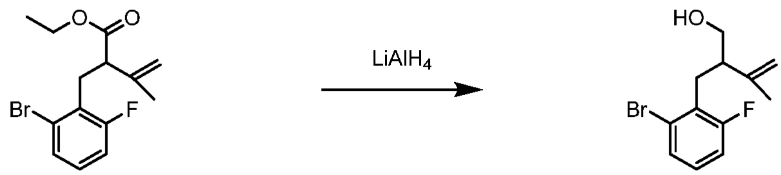


To a solution of ethyl 2-(2-bromo-6-fluorobenzyl)-3-methylbut-3-enoate (3.03 g, 9.61 mmol) in dry tetrahydrofuran (20 mL) at 0 °C was added lithium aluminium hydride in tetrahydrofuran (2.4 M, 4.0 mL, 9.6 mmol). The mixture was allowed to warm to room temperature. After 2 hours, ethyl acetate (10 mL) was added dropwise, and the mixture was stirred for 15 minutes. Then, 1M HCl was added untill all solids dissolved, and the mixture was stirred for an additional 15 minutes. The layers were separated, and the water layer was extracted twice with ethyl acetate. The combined organic layers were washed with 1M HCl, brine, dried over sodium sulfate and concentrated in vacuo. Purification by column chromatography (silica, 3% to 20% ethyl acetate in heptane) afforded the desired product 2-(2-bromo-6-fluorobenzyl)-3-methylbut-3-en-1-ol (2.19 g). Yield 83%. $^1$H NMR (400 MHz, Chloroform-d) δ 7.38 - 7.32 (m, 1H), 7.10 - 7.02 (m, 1H), 7.02 - 6.95 (m, 1H), 4.96 - 4.89 (m, 1H), 4.81 (s, 1H), 3.69 - 3.52 (m, 2H), 2.94 - 2.81 (m, 2H), 2.75 - 2.64 (m, 1H), 1.81 (s, 3H), 1.46 - 1.38 (m, 1H).

**Step 3: 5-bromo-3-(prop-1-en-2-yl)chromane**

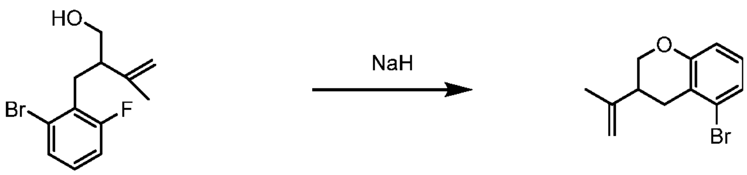


To a solution of 2-(2-bromo-6-fluorobenzyl)-3-methylbut-3-en-1-ol (2.19 g, 8.0 mmol) in dry N-Methyl-2-pyrrolidinone (40 mL) was added sodium hydride (60% dispersion in mineral oil, 0.704 g, 17.6 mmol). The resulting mixture was heated to 100 °C for 75 minutes, then allowed to cool to room temperature overnight, quenched with saturated aqueous ammonium chloride and diluted with ethyl acetate and brine. The layers were separated, and the water layer was extracted

3 times with ethyl acetate. The combined organic layers were washed with brine (four times), dried over sodium sulfate and concentrated in vacuo. Purification by column chromatography (silica, heptane) afforded the desired product 5-bromo-3-(prop-1-en-2-yl)chromane (0.76 g) Yield 30%. $^1$H NMR (400 MHz, Chloroform-d) δ 7.13 (dd, J = 7.9, 1.2 Hz, 1H), 6.97 (t, J = 8.0 Hz, 1H), 6.79 (dd, J = 8.1, 1.1 Hz, 1H), 4.93 (q, J = 1.5 Hz, 1H), 4.80 (s, 1H), 4.28 (dt, J = 10.5, 2.8 Hz, 1H), 3.89 - 3.77 (m, 1H), 3.02 - 2.89 (m, 1H), 2.70 - 2.54 (m, 2H), 1.85 (s, 3H).

**Step 4: 5-bromo-3-(2-fluoropropan-2-yl)chromane**

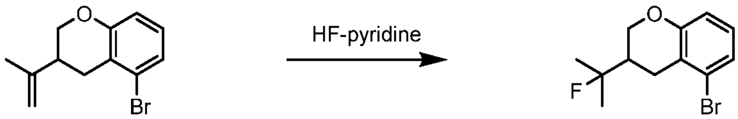


Hydrogen fluoride-pyridine (2.2 mL, 24.4 mmol) was cooled to 0 °C, and 5-bromo-3-(prop-1-en-2-yl)chromane (548 mg, 2.17 mmol) was added by syringe. After stirring at 0 °C for a few minutes, the mixture was allowed to warm to room temperature and stirred for 1 hour. The reaction mixture was quenched on ice and diluted with diethyl ether and heptane. Solid sodium bicarbonate was added until effervescence ceased. The mixture was extracted with a mixture of heptane and diethyl ether, and the organic layer was washed with 1M HCl, dried over sodium sulfate and concentrated in vacuo. Purification by column chromatography (silica, heptane) afforded the desired product 5-bromo-3-(2-fluoropropan-2-yl) chromane (0.53 g). Yield 90%. $^1$H NMR (400 MHz, Chloroform-d) δ 7.15 (d, J = 7.7 Hz, 1H), 6.98 (t, J = 8.1 Hz, 1H), 6.80 (d, J = 8.2 Hz, 1H), 4.46 - 4.38 (m, 1H), 3.80 (t, J = 10.5 Hz, 1H), 2.94 - 2.83 (m, 1H), 2.63 - 2.51 (m, 1H), 2.32 - 2.18 (m, 1H), 1.45 (dd, J = 21.9, 12.9 Hz, 6H).

A racemic mixture of 5-bromo-3-(2-fluoropropan-2-yl)chromane (0.53 g) was separated by chiral preparative SFC. Apparatus: Waters Prep 100 SFC UV/MS directed system; Waters 2998 Photodiode Array (PDA) Detector; Waters Acquity QDa MS detector; Waters 2767 Sample Manager; Column: Phenomenex Lux Amylose-1 (250x21mm, 5μm); Column temp: 35 °C; Flow: 100 mL/min; ABPR: 120 bar; Eluent A: $CO_2$, Eluent B: 0.1% TFA in Methanol; Isocratic method: 5% B for 3 min; Loading: 15 mg; Detection: PDA (210-400 nm); Fraction collection based on PDA TIC. The first eluting fraction **(stereoisomer A,** 0.21 g) was isolated as a clear liquid, yield 39%. $t_R$: 0.95 min., 100% ee. Apparatus: Waters Acquity UPC$^2$ System; Column: Phenomenex Amylose-1 (100x4.6mm, 5μm), column temp: 35 °C; flow: 2.5 mL/min; BPR: 170 bar; Eluent A: $CO_2$, Eluent B: 20 mM Ammonia in Methanol; Gradient method: t=0 min 5% B, t=5 min 50% B, t=6 min 50% B. Detection: PDA (210-320 nm). The second eluting fraction **(stereoisomer B,** 0.175 g) was isolated as a clear liquid, yield 33%. $t_R$: 1.13 min., 97% ee. Apparatus: Waters Acquity UPC$^2$ System; Column: Phenomenex Amylose-1 (100x4.6mm, 5μm), column temp: 35 °C; flow: 2.5 mL/min; BPR: 170 bar; Eluent A: $CO_2$, Eluent B: 20 mM Ammonia in Methanol; Gradient method: t=0 min 5% B, t=5 min 50% B, t=6 min 50% B. Detection: PDA (210-320 nm).

**Step 5: (+)-tert-butyl 2-(3-(2-fluoropropan-2-yl)chroman-5-yl)acetate stereoisomer A**

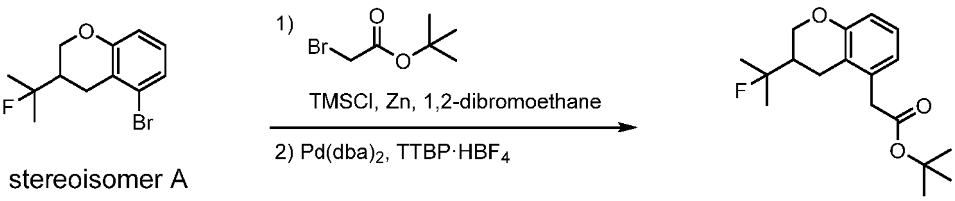


stereoisomer A

stereoisomer A

An oven dried flask was charged with zinc (dust, 0.2 g, 3.1 mmol) and heated with a heat gun under an argon flow. After cooling to room temperature, dry tetrahydrofuran (4 mL) was added followed by 1,2-dibromoethane (7 μL, 0.07 mmol). The mixture was heated to reflux and cooled to room temperature 3 times. Then trimethylsilyl chloride (10 μL, 0.08 mmol) was added, causing the mixture to reflux spontaneously and the zinc to change morphology. After stirring for 20 minutes, tert-butyl bromoacetate (0.22 mL, 1.53 mmol) was added dropwise, resulting in an exotherm. The mixture was kept at an elevated temperature (45 °C) for 30 minutes and then allowed to cool to room temperature. A separate flask was charged with 5-bromo-3-(2-fluoropropan-2-yl)chromane stereoisomer A (0.21 g, 0.76 mmol), tri-tert-butylphosphine tetrafluoroborate (0.024 g, 0.08 mmol) and bis-(dibenzylideneacetone)palladium (0.044 g, 0.08 mmol). The reaction vessel was flushed with argon, dry tetrahydrofuran (4 mL) was added and argon was bubbled through for five minutes. The

zincate solution was added by syringe, and the reaction mixture was heated to reflux for 1 hour. The mixture was cooled to room temperature overnight, quenched with saturated aqueous ammonium chloride and extracted with ethyl acetate three times. The combined organic layers were dried over sodium sulfate and concentrated in vacuo. Purification by column chromatography (silica, 0% to 12% ethyl acetate in heptane) afforded the desired product (+)-tert-butyl 2-(3-(2-fluoropropan-2-yl)chroman-5-yl)acetate stereoisomer A (218 mg). Yield: 92%. $^1$H NMR (400 MHz, Chloroform-d) δ 7.06 (t, J = 7.8 Hz, 1H), 6.83 - 6.73 (m, 2H), 4.44 - 4.35 (m, 1H), 3.82 (t, J = 10.4 Hz, 1H), 3.51 (s, 2H), 2.84 - 2.74 (m, 1H), 2.65 - 2.54 (m, 1H), 2.33 - 2.19 (m, 1H), 1.50 - 1.35 (m, 15H). Specific Optical Rotation: +61.3°, c=0.19, $CHCl_3$, 21.0 °C, 589 nm.

**Step 6: (-)-tert-butyl 2-(3-(2-fluoropropan-2-yl)chroman-5-yl)acetate stereoisomer B**

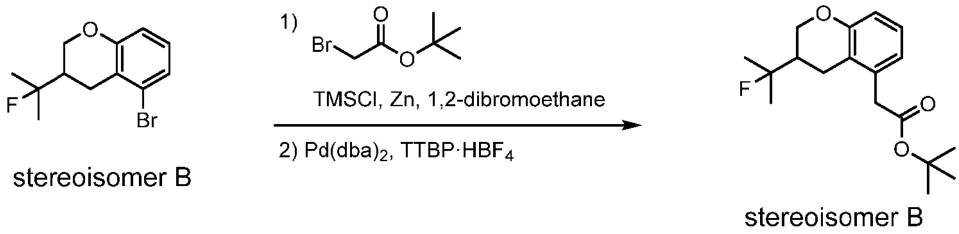


An oven dried flask was charged with zinc (dust, 0.168 g, 2.56 mmol) and heated with a heat gun under an argon flow. After cooling to room temperature, dry tetrahydrofuran (4 mL) was added followed by 1,2-dibromoethane (6 μL, 0.06 mmol). The mixture was heated to reflux and cooled to room temperature 3 times. Then trimethylsilyl chloride (8 μL, 0.06 mmol) was added, causing the mixture to reflux spontaneously and the zinc to change morphology. After stirring for 20 minutes, tert-butyl bromoacetate (0.19 mL, 1.28 mmol) was added dropwise, resulting in an exotherm. The mixture was kept at an elevated temperature (45 °C) for 30 minutes and then allowed to cool to room temperature. A separate flask was charged with 5-bromo-3-(2-fluoropropan-2-yl)chromane stereoisomer B (0.175 g, 0.64 mmol), tri-tert-butylphosphine tetrafluoroborate (0.020 g, 0.07 mmol) and bis-(dibenzylideneacetone)palladium (0.037 g, 0.06 mmol). The reaction vessel was flushed with argon, dry tetrahydrofuran (4 mL) was added and argon was bubbled through for five minutes. The zincate solution was added by syringe, and the reaction mixture was heated to reflux for 2 hours. The mixture was cooled to room temperature overnight, quenched with saturated aqueous ammonium chloride and extracted with ethyl acetate three times. The combined organic layers were dried over sodium sulfate and concentrated in vacuo. Purification by column chromatography (silica, 0% to 12% ethyl acetate in heptane) afforded the desired product (-)-tert-butyl 2-(3-(2-fluoropropan-2-yl)chroman-5-yl)acetate stereoisomer B (162 mg). Yield: 82%. $^1$H NMR (400 MHz, Chloroform-d) δ 7.06 (t, J = 7.8 Hz, 1H), 6.83 - 6.73 (m, 2H), 4.43 - 4.35 (m, 1H), 3.82 (t, J = 10.4 Hz, 1H), 3.51 (s, 2H), 2.84 - 2.74 (m, 1H), 2.65 - 2.55 (m, 1H), 2.33 - 2.19 (m, 1H), 1.48 - 1.36 (m, 15H). Specific Optical Rotation: -52.2°, c=0.2, $CHCl_3$, 21.2 °C, 589 nm.

**Step 7: tert-butyl 2-bromo-2-(3-(2-fluoropropan-2-yl)chroman-5-yl)acetate stereoisomer B**

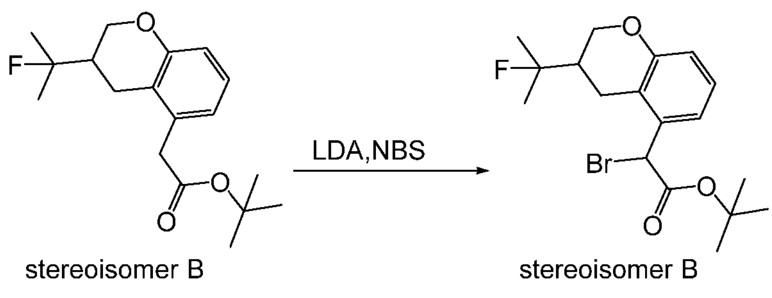


To a cooled solution of tert-butyl 2-(3-(2-fluoropropan-2-yl)chroman-5-yl)acetate stereoisomer B (308 mg, 1 mmol) in THF (4 mL) at -78°C was added a solution of lithium diisopropylamide (2.0 M in THF/hexanes, 1.25 mL, 2.5 mmol) dropwise. The reaction was stirred at -78 °C for 30 min, then chlorotrimethylsilane (270 mg, 2.5 mmol) was added, and the reaction was stirred at -78 °C for another 30 min. Then a solution of N-bromosuccinimide (445 mg, 2.5 mmol) in THF (6 mL) was added, and the reaction was stirred at -78 °C for 30 min. The reaction was quenched with MeOH (2 mL), solvent was removed in vacuo, and the residue was purified by silica gel column (petroleum ether: ethyl acetate = 10:1) to give the desired product tert-butyl 2-bromo-2-(3-(2-fluoropropan-2-yl)chroman-5-yl)acetate stereoisomer B as a yellow oil (305

mg). Yield 80%. ESI: 330 ($M-C_4H_9 + H$)+.

**Step 8: tert-butyl 2-(3-(2-fluoropropan-2-yl)chroman-5-yl)-2-((R)-3-(4-(5,6,7,8-tetrahydro-1,8-naphthyridin-2-yl) butoxy)pyrrolidin-1-yl)acetate iPr-stereoisomer B**

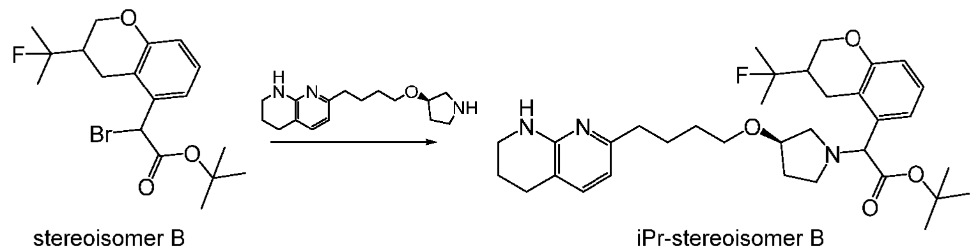


A mixture of (R)-7-(4-(pyrrolidin-3-yloxy)butyl)-1,2,3,4-tetrahydro-1,8-naphthyridine (180 mg, 0.52 mmol), tert-butyl 2-bromo-2-(3-(2-fluoropropan-2-yl)chroman-5-yl)acetate stereoisomer B (200 mg, 0.52 mmol), DIPEA (200 mg, 1.56 mmol), and NaI (20 mg, 0.13 mmol) in acetonitrile (10 mL) was warmed to 50 °C and stirred for 6 hours. Solvent was removed in vacuo, and the residue was purified by silica gel column (DCM: MeOH 20:1) to give the desired product tert-butyl 2-((S)-3-(2-fluoropropan-2-yl)chroman-5-yl)-2-((R)-3-(4-(5,6,7,8-tetrahydro-1,8-naphthyridin-2-yl)butoxy) pyrrolidin-1-yl)acetate iPr-stereoisomer B as a yellow oil (145 mg). Yield 48%. ESI: 582 (M + H)+.

**Step 9: 2-(3-(2-fluoropropan-2-yl)chroman-5-yl)-2-((R)-3-(4-(5,6,7,8-tetrahydro-1,8-naphthyridin-2-yl)butoxy) pyrrolidin-1-yl)acetic acid iPr-stereoisomer B (compounds 10-B-E1 and 10-B-E2)**

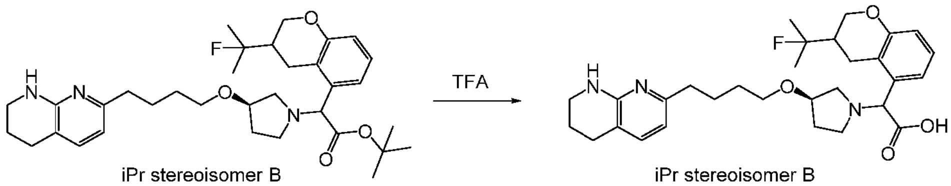


Tert-butyl 2-(3-(2-fluoropropan-2-yl)chroman-5-yl)-2-((R)-3-(4-(5,6,7,8-tetrahydro-1,8-naphthyridin-2-yl)butoxy)pyrrolidin-1-yl)acetate iPr-stereoisomer B (120 mg, 0.21 mmol) was treated with a mixture of DCM (3 mL) and TFA (3 mL) at 25 °C overnight. Solvent was removed in vacuo, and the residue was purified by Prep-HPLC A (40 → 70% MeCN) to give the desired product as a white solid (81 mg). Yield 75%. The racemic product was separated by Prep chiral SFC B to give diastereomeric products **compound 10-B-E1** (29 mg) and **compound 10-B-E2** (10 mg) as white solids.

**Compound 10-B-E1** LC/MS ESI 526.2 (M+H)+. $^1$H NMR (400 MHz, MeOD) δ 7.18-7.13 (m, 3H), 6.82 (d, J = 7.6 Hz, 1H), 6.38 (d, J = 7.2 Hz, 1H), 4.81 (s, 1H), 4.43 (d, J = 10.4 Hz, 1H), 4.21 (s, 1H), 3.85 (t, J = 10.4 Hz, 1H), 3.51-3.48 (m, 2H), 3.40-3.37 (m, 2H), 3.32-3.21 (m, 4H), 2.73-2.70 (m, 3H), 2.55 (t, J = 7.2 Hz, 2H), 2.31-2.02 (m, 3H), 1.90-1.88 (m, 2H), 1.73-1.62 (m, 4H), 1.48-1.40 (m, 6H). Chiral SFC B (25% EtOH): ee 100%, Rt = 1.35min.

**Compound 10-B-E2** LC/MS ESI 526.2 (M+H)+. $^1$H NMR (400 MHz, MeOD) δ 7.24-7.10 (m, 3H), 6.80 (d, J = 8.0 Hz, 1H), 6.37 (d, J = 7.6 Hz, 1H), 4.58 (s, 1H), 4.38 (d, J = 10.0 Hz, 1H), 4.13 (s, 1H), 3.80 (t, J = 10.4 Hz, 1H), 3.50-3.37 (m, 5H), 3.32-2.95 (m, 4H), 2.73-2.71 (m, 3H), 2.52 (t, J = 7.6 Hz, 2H), 2.23-2.05 (m, 4H), 1.99-1.82 (m, 2H), 1.75-1.48 (m, 5H), 1.37-1.25 (m, 6H). Chiral SFC B (25% EtOH): ee 71%, Rt = 1.89min.

**Step 10: 2-(3-(2-fluoropropan-2-yl)chroman-5-yl)-2-((R)-3-(4-(5,6,7,8-tetrahydro-1,8-naphthyridin-2-yl)butoxy) pyrrolidin-1-yl)acetic acid iPr-stereoisomer A (diastereomeric compounds 10-A-E1 and 10-A-E2)**

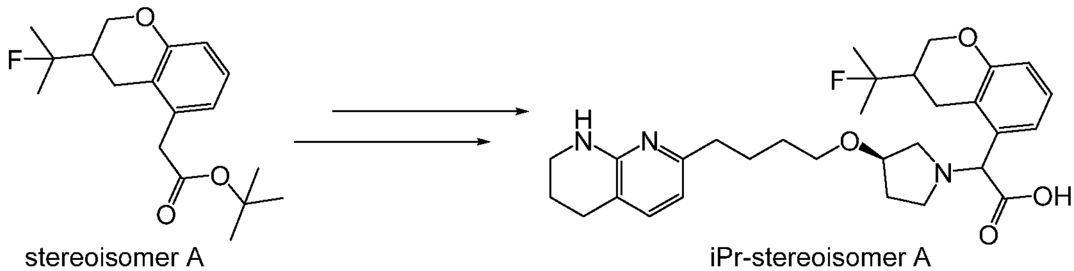

stereoisomer A

iPr-stereoisomer A

2-(3-(2-fluoropropan-2-yl)chroman-5-yl)-2-((R)-3-(4-(5,6,7,8-tetrahydro-1,8-naphthyridin-2-yl)butoxy)pyrrolidin-1-yl)acetic acid iPr-stereoisomer A **(compounds 10-A-E1 and 10-A-E2)** was synthesized from (+)-tert-butyl 2-(3-(2-fluoropropan-2-yl)chroman-5-yl)acetate stereoisomer A by the same procedures as for stereoisomer B.

**Compound 10-A-E1** LC/MS ESI 526.3 (M+H)+, 1H NMR (400 MHz, MeOD) δ 7.33 - 7.00 (m, 3H), 6.81 (d, J = 8.3 Hz, 1H), 6.37 (d, J = 7.4 Hz, 1H), 4.69 (s, 1H), 4.44 - 4.42 (m, 1H), 4.15 (s, 1H), 3.84 -3.82 (m, 1H), 3.65 - 3.34 (m, 6H), 3.18 - 3.15 (m, 2H), 2.97 - 2.40 (m, 6H), 2.23 - 2.15 (m, 3H), 1.99 - 1.78 (m, 2H), 1.78 - 1.53 (m, 4H), 1.48 - 1.42 (m, 6H). Chiral SFC F (45% EtOH): ee 100 %, Rt = 2.44 min.

**Compound 10-A-E2** LC/MS ESI 526.3 (M+H)+, 1H NMR (400 MHz, MeOD) δ 7.22 - 7.17 (m, 3H), 6.87-6.84 (m, 1H), 6.41 (d, J = 7.6 Hz, 1H), 4.40 - 4.37 (m, 1H), 4.25 (s, 1H), 3.84 - 3.34 (m, 7H), 3.18 - 3.15 (m, 2H), 2.97 - 2.40 (m, 6H), 2.23 - 2.15 (m, 3H), 1.99 - 1.78 (m, 2H), 1.78 - 1.53 (m, 4H), 1.48 - 1.42 (m, 6H). Chiral SFC F (45% EtOH): ee 100 %, Rt = 5.02 min.

**Example 11: Preparation of 2-(7-fluoro-3-(2-fluoropropan-2-yl)chroman-5-yl)-2-((R)-3-(4-(4-methoxy-5,6,7,8-tetrahydro-1,8-naphthyridin-2-yl)butoxy)pyrrolidin-1-yl)acetic acid (compounds 11-E1 and 11-E2)**

**Step 1: ethyl 2-(2-bromo-4,6-difluorobenzyl)acrylate**

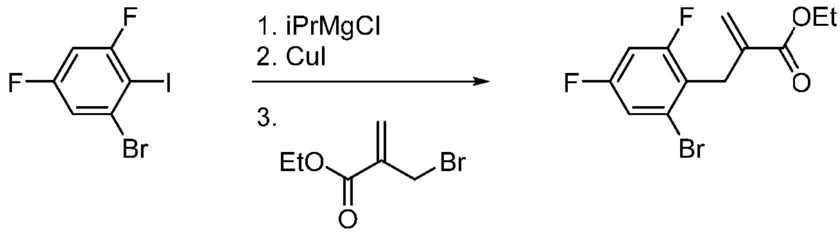


To a cooled solution of 1-bromo-3,5-difluoro-2-iodobenzene (10.0 g, 31.4 mmol) in THF (25 mL, 1.3 M) at -20 °C was added a solution of iPrMgCl (2M in THF, 20.4 mL, 40.8 mmol) dropwise. The mixture was stirred for 10 min and then warmed to 0 °C and stirred an additional 50 min. Copper iodide (1.49 g, 7.84 mmol) was added, and the mixture was stirred for 10 min. Ethyl 2-(bromomethyl)acrylate (6.36 g, 4.55 mL, 32.9 mmol) was added, and the mixture was warmed to room temperature and stirred overnight. The reaction was quenched with sat. aq. NH4Cl (25 mL), diluted with EtOAc (25 mL) and extracted with EtOAc (3 × 30 mL). The combined organics were washed with brine (4 × 50 mL), dried over anhydrous sodium sulfate, filtered, and concentrated in vacuo. The resulting residue was purified by silica gel column to give the desired product 1-bromo-3,5-difluoro-2-(2-methylallyl)benzene as a colorless and clear oil (10 g). Yield >95%.

**Step 2: 3-(2-bromo-4,6-difluorobenzyl)-2-methylbut-3-en-2-ol**

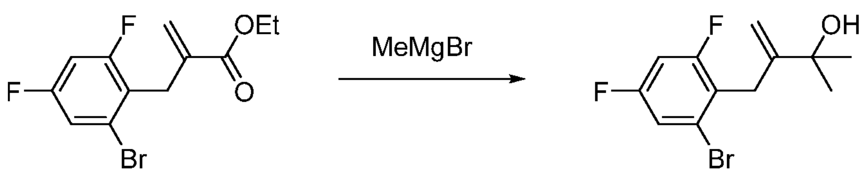


To a cooled solution of ethyl 2-(2-bromo-4,6-difluorobenzyl)acrylate (9.35 g, 30.7 mmol) in THF at 0 °C, was

added a solution of methylmagnesium bromide (3M in Et2O, 22.5 mL, 67.4 mmol) dropwise. The mixture was stirred for 30 min and warmed to room temperature and stirred overnight. The reaction was quenched with sat. aq. NH4Cl (50 mL) and extracted with EtOAc (3x 30 mL). The combined organics were washed with brine (3 × 100 mL), dried over anhydrous sodium sulfate, filtered, and concentrated in vacuo. The resulting residue was purified by silica gel column (EtOAc/Hex 0% → 10%) to give the desired product 3-(2-bromo-4,6-difluorobenzyl)-2-methylbut-3-en-2-ol as a clear yellow oil (6.75 g). Yield 76%.

**Step 3: 2-(2-bromo-4,6-difluorobenzyl)-3-methylbutane-1,3-diol**

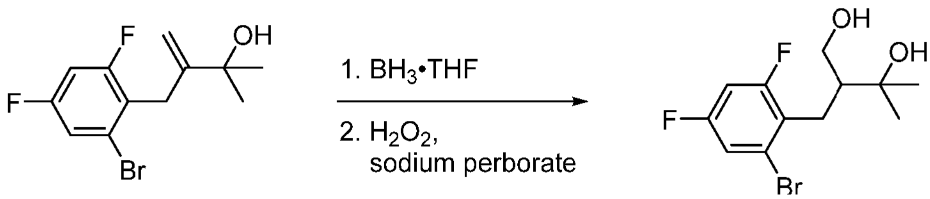


To a cooled solution of 3-(2-bromo-4,6-difluorobenzyl)-2-methylbut-3-en-2-ol (3.00 g, 10.3 mmol) in THF (30 mL, 0.34 M) at 0 °C, was added a solution of BH3 THF (1 M in THF, 15.5 mL, 15.5 mmol) dropwise. The mixture was stirred for 30 min and then warmed to room temperature and stirred for 2 hours. The reaction was cooled to 0 °C, and water (10 mL) was added followed by an aqueous 3M solution of NaOH (17.2 mL, 51.5 mmol) and sodium perborate monohydrate (5.14 g, 51.5 mmol). The mixture was warmed to 50 °C, stirred an additional 2 hour, then cooled to room temperature, quenched with water and extracted with EtOAc. The combined organics were washed with brine, dried over anhydrous sodium sulfate, filtered, and concentrated in vacuo. The resulting residue was purified by silica gel column (EtOAc/Hex 0% -20%) to give the desired product 2-(2-bromo-4,6-difluorobenzyl)-3-methylbutane-1,3-diol (6.93 g). Yield 75%.

**Step 4: 2-(5-bromo-7-fluorochroman-3-yl)propan-2-ol:**

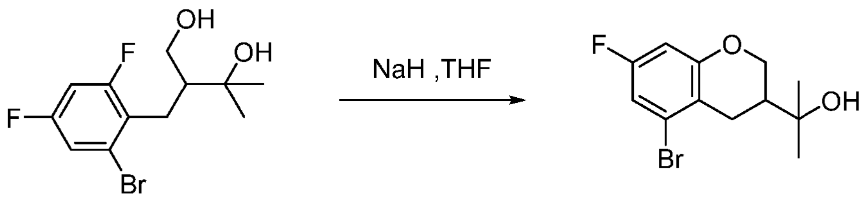


To a cooled solution of 2-(2-bromo-4,6-difluorobenzyl)-3-methylbutane-1,3-diol (3.20 g, 10.4 mmol) in a 4:1 mixture of toluene (48 mL) and DMF (12 mL) at 0 °C was added sodium hydride (911 mg, 22.8 mmol) in three portions. The mixture was warmed to 100 °C and stirred for 1 hour, then cooled to room temperature, quenched with sat. aq. NH4Cl, diluted with water and extracted with DCM. The combined organics were washed with brine, dried over anhydrous sodium sulfate, filtered, and concentrated in vacuo. The resulting residue was purified by silica gel column (EtOAc/Hex 0% -10%) to give the desired product 5-bromo-7-fluoro-3-methylchromane as a clear and colorless oil (2.61 g). Yield 87%.

**Step 5: 5-bromo-7-fluoro-3-(2-fluoropropan-2-yl)chromane**

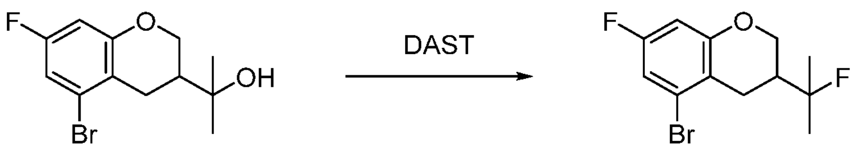


To a cooled solution of 2-(5-bromo-7-fluorochroman-3-yl)propan-2-ol (2.61 g, 9.03 mmol) in DCM (30 mL, 0.3 M) at 0 °C, was added diethylamino sulfur trifluoride (1.55 mL, 11.7 mmol) dropwise. The mixture was allowed to stir for 30 min, then quenched with sat. aq. solution of sodium bicarbonate and extracted with DCM. The combined organics were washed

with brine, dried over anhydrous sodium sulfate, and concentrated. The resulting residue was purified by silica gel column (100% Hexanes) to give the desired product 5-bromo-7-fluoro-3-(2-fluoropropan-2-yl)chromane (1.96 g). Yield 75%.

**Step 6: tert-butyl 2-(7-fluoro-3-(2-fluoropropan-2-yl)chroman-5-yl)acetate**

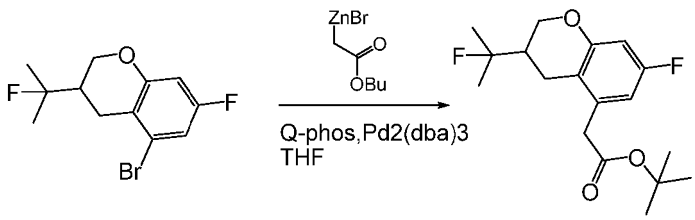


A mixture of 5-bromo-7-fluoro-3-(2-fluoropropan-2-yl)chroman (400 mg, 1.38 mmol), (2-tert-butoxy-2-oxoethyl) zinc(II) bromide solution (0.5 M in THF, 13.8 mL, 6.89 mmol), Pd2(dba)3 (72 mg, 0.069 mmol) and Q-phos (49 mg, 0.069 mmol) in THF (2 mL) was stirred at 60 °C for 2 hours. The mixture was quenched with NaHCO3 aq. solution, filtered and concentrated in vacuo, and the resulting residue was purified by silica gel column (pet ether: EtOAc 10:1) to give the desired product tert-butyl 2-(7-fluoro-3-(2-fluoropropan-2-yl)chroman-5-yl)acetate as a pale red oil (342 mg). Yield 76% (ESI 349/351 [M+Na]+).

**Step 7: tert-butyl 2-bromo-2-(7-fluoro-3-(2-fluoropropan-2-yl)chroman-5-yl)acetate**

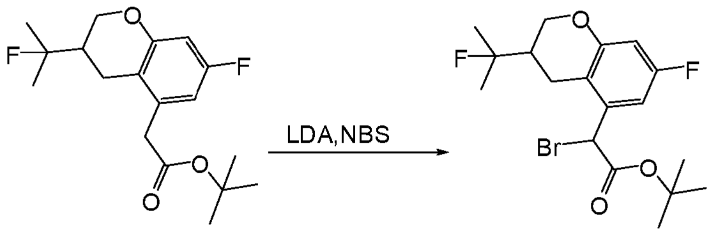


To a solution of tert-butyl 2-(7-fluoro-3-(2-fluoropropan-2-yl)chroman-5-yl)acetate (342 mg, 1.05 mmol) in THF (10 mL) at -78 °C, was added a solution of lithium diisopropylamide (2.0 M in THF/hexanes, 1.31 mL, 2.62 mmol) dropwise. The reaction was stirred at -78 °C for 30 min, then a solution of chlorotrimethylsilane (283 mg, 2.62 mmol) in THF (1 mL) was added, and the reaction was stirred at -78 °C for another 30 min. A solution of N-bromosuccinimide (467 mg, 2.62 mmol) in THF (10 mL) was added, and the reaction was stirred at -78 °C for 1 hour. The reaction was quenched with MeOH (2 mL) and concentrated in vacuo, and the residue was purified by silica gel column (pet ether: EtOAc 10:1) to give the desired product tert-butyl 2-bromo-2-(7-fluoro-3-(2-fluoropropan-2-yl)chroman-5-yl)acetate as a colorless oil (401 mg). Yield 94% (ESI 427/429 [M+Na]+).

**Step 8: tert-butyl 2-(7-fluoro-3-(2-fluoropropan-2-yl)chroman-5-yl)-2-((R)-3-(4-(4-methoxy-5,6,7,8-tetrahydro-1,8-naphthyridin-2-yl)butoxy)pyrrolidin-1-yl)acetate**

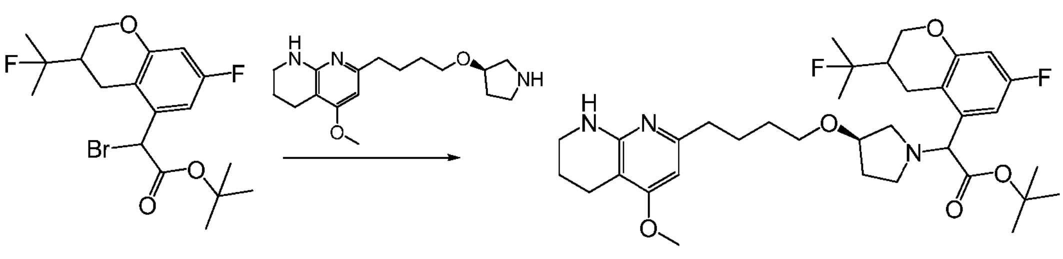

A mixture of tert-butyl 2-bromo-2-(7-fluoro-3-(2-fluoropropan-2-yl)chroman-5-yl)acetate (215 mg, 0.53 mmol),

(R)-5-methoxy-7-(4-(pyrrolidin-3-yloxy)butyl)-1,2,3,4-tetrahydro-1,8-naphthyridine (162 mg, 0.53 mmol), DIPEA ( 206 mg, 1.59 mmol), and NaI (20 mg) in acetonitrile (20 mL) was warmed to 40 °C and stirred for 17 hours. The mixture was diluted with water (8 mL) and extracted with EtOAc (25mL). The organic phase was washed with brine, dried over anhydrous sodium sulfate, filtered and concentrated in vacuo. The residue was purified by silica gel column (DCM:MeOH 20:1) to give the desired product tert-butyl 2-(7-fluoro-3-(2-fluoropropan-2-yl)chroman-5-yl)-2-((R)-3-(4-(4-methoxy-5,6,7,8-tetrahydro-1,8-naphthyridin-2-yl)butoxy)pyrrolidin-1-yl)acetate as a colorless oil (130 mg). Yield 38 % (ESI 630 [M+H]+).

**Step 9: 2-(7-fluoro-3-(2-fluoropropan-2-yl)chroman-5-yl)-2-((R)-3-(4-(4-methoxy-5,6,7,8-tetrahydro-1,8-naphthyridin-2-yl)butoxy)pyrrolidin-1-yl)acetic acid (compounds 11-E1 and 11-E2)**

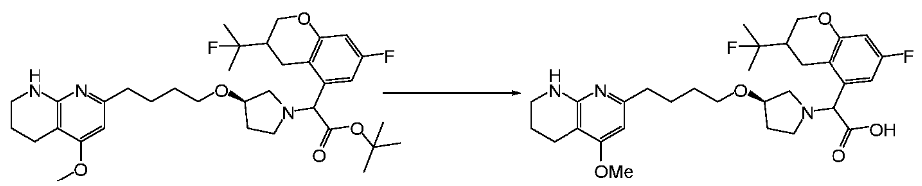

To a solution of tert-butyl 2-(7-fluoro-3-(2-fluoropropan-2-yl)chroman-5-yl)-2-((R)-3-(4-(4-methoxy-5,6,7,8-tetrahydro-1,8-naphthyridin-2-yl)butoxy)pyrrolidin-1-yl)acetate (130 mg, 0.21 mmol) in DCM (10 mL) was added TFA (10 mL). The mixture was stirred at room temperature for 17 hours. Solvent was removed in vacuo, and the residue was purified by Prep-HPLC A (40 → 70% MeCN) to give the desired product (60 mg). Yield 49%. The racemic product was separated by Prep chiral SFC B to give diastereomeric products **11-E1** (28 mg) and **11-E2** (26 mg) as white solids, each as a mixture of 2 stereoisomers.

**Compound 11-E1 (mixture of 2 stereoisomers)** LC/MS ESI 574 (M+H) +. 1H NMR (400 MHz, MeOD) δ 7.05-7.03 (m, 1H), 6.54-6.52 (m, 1H), 6.33-6.32 (m, 1H), 4.62-4.60 (m, 1H), 4.43-4.41 (m, 1H), 4.20-4.18 (m, 1H), 3.85-3.80 (m, 4H), 3.50-3.31 (m, 4H), 3.30 -2.95 (m, 5H), 2.75 -2.57 (m, 5H), 2.23-1.60 (m, 9H), 1.45-1.41 (m, 6H). Chiral SFC B (20% EtOH): ee 98%, Rt = 1.51 min.

**Compound 11-E2 (mixture of 2 stereoisomers)** LC/MS ESI 574 (M+H) +. 1H NMR (400 MHz, MeOD) δ 7.06-7.04 (m, 1H), 6.54-6.52 (m, 1H), 6.35-6.34 (m, 1H), 4.54-4.52 (m, 1H), 4.43-4.41 (m, 1H), 4.20-4.18 (m, 1H), 3.82-3.80 (m, 4H), 3.50-3.31 (m, 4H), 3.30 -2.95 (m, 5H), 2.75 -2.57 (m, 5H), 2.23-1.60 (m, 9H), 1.45-1.41 (m, 6H). Chiral SFC B (20% EtOH): ee 96%, Rt = 2.36 min.

**Additional Examples**

Compounds 12-30 were prepared using general procedures based on the methods used to prepare compounds 1-11.

**2-(3-ethylisochroman-5-yl)-2-((R)-3-(4-(5,6,7,8-tetrahydro-1,8-naphthyridin-2-yl)butoxy)pyrrolidin-1-yl)acetic acid (diastereomeric compounds 12-E1 and 12-E2)**

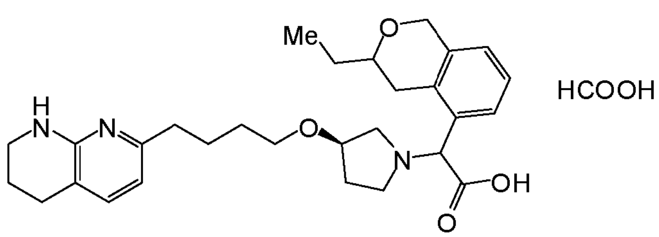

**Compound 12-E1 (mixture of 2 stereoisomers)** LC/MS ESI 494 (M+H) +. 1H NMR (400 MHz, MeOD) δ 8.53 (s, 1H), 7.54-7.52 (m, 1H), 7.44-7.42 (m, 1H), 7.26-7.24 (m, 1H), 7.14-7.12 (m, 1H), 6.54-6.52 (m, 1H), 5.01-4.76 (m, 3H), 4.23-4.20 (m, 1H), 3.60-3.10 (m, 10H), 2.79-2.50 (m, 5H), 2.20-1.50 (m, 10H), 1.02-1.00 (m, 3H).

**Compound 12-E2 (mixture of 2 stereoisomers)** LC/MS ESI 494 (M+H) +. 1H NMR (400 MHz, MeOD) δ 8.44 (s, 1H), 7.52-7.47 (m, 2H), 7.28-7.26 (m, 1H), 7.14-7.12 (m, 1H), 6.58-6.53 (m, 1H), 5.01 (s, 1H) 5.00-4.76 (m, 2H), 4.26-4.25

(m, 1H), 3.60-3.32 (m, 7H), 3.31-2.60 (m, 8H), 2.20-1.50 (m, 10H), 1.02-1.00 (m, 3H).

**2-(3-cyclopropylisochroman-5-yl)-2-((R)-3-(4-(5,6,7,8-tetrahydro-1,8-naphthyridin-2-yl)butoxy)pyrrolidin-1-yl) acetic acid (diastereomeric compounds 13-E1 and 13-E2)**

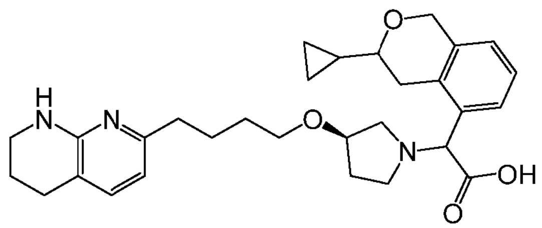

**Compound 13-E1 (mixture of 2 stereoisomers)** LC/MS ESI 506 (M+H) +. 1H NMR (400 MHz, MeOD) δ 7.53-7.50 (m, 1H), 7.28-7.05 (m, 3H), 6.34-6.32 (m, 1H), 4.90-4.76 (m, 3H), 4.20 (s, 1H), 3.55-3.05 (m, 13H), 2.70-2.50 (m, 4H), 2.20-2.05 (m, 2H), 1.90-1.55 (m, 6H), 1.02-0.95 (m, 1H), 0.55-0.25 (m, 4H). Chiral SFC F (45% EtOH): ee 100%, Rt = 3.83min.

**Compound 13-E2 (mixture of 2 stereoisomers)** LC/MS ESI 506 (M+H) +. 1H NMR (400 MHz, MeOD) δ 7.51-7.48 (m, 1H), 7.28-7.05 (m, 3H), 6.34-6.32 (m, 1H), 4.90-4.76 (m, 3H), 4.21 (s, 1H), 3.55-2.95 (m, 12H), 2.70-2.50 (m, 5H), 2.18-2.01 (m, 2H), 1.90-1.55 (m, 6H), 1.02-0.95 (m, 1H), 0.55-0.25 (m, 4H). Chiral SFC F (45% EtOH): ee 100%, Rt = 9.50min.

**2-(3-(2-fluoropropan-2-yl)isochroman-5-yl)-2-((R)-3-(4-(5,6,7,8-tetrahydro-1,8-naphthyridin-2-yl)butoxy)pyrrolidin-1-yl)acetic acid (diastereomeric compounds** 14-**A-E1, 14-A-E2, 14-B-E1 and 14-B-E2)**

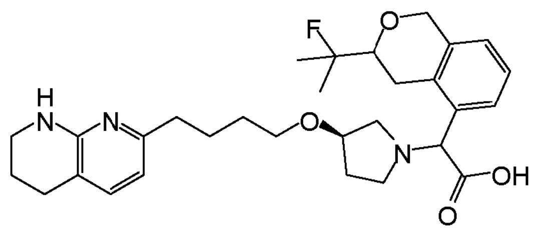

**Compound 14-A-E1** LC/MS ESI 526 (M+H) +. 1H NMR (400 MHz, MeOD) δ 7.55-7.53 (m, 1H), 7.28-7.11 (m, 3H), 6.38 (d, J=7.2Hz, 1H), 4.92-4.80 (m, 3H), 4.18-4.17 (m, 1H), 3.72-3.70 (m, 1H), 3.51-3.32 (m, 5H), 3.30-2.85 (m, 5H), 2.72-2.70 (m, 2H), 2.55-2.50 (m, 2H), 2.10-1.55 (m, 8H), 1.48-1.41 (m, 6H).

**Compound 14-A-E2** LC/MS ESI 526 (M+H) +. 1H NMR (400 MHz, MeOD) δ 7.55-7.53 (m, 1H), 7.29-7.11 (m, 3H), 6.38 (d, J=7.2Hz, 1H), 4.93-4.84 (m, 3H), 4.18-4.17 (m, 1H), 3.72-3.32 (m, 6H), 3.30-3.05 (m, 4H), 2.88-2.85 (m, 1H), 2.72-2.70 (m, 2H), 2.55-2.50 (m, 2H), 2.10-1.55 (m, 8H), 1.48-1.41 (m, 6H).

**Compound 14-B-E1** LC/MS ESI 526 (M+H) +. 1H NMR (400 MHz, MeOD) δ 7.55-7.53 (m, 1H), 7.26-7.22 (m, 1H), 7.15-7.13 (m, 1H), 7.10-7.08 (m, 1H), 6.38 (d, J=7.2Hz, 1H), 4.92-4.78 (m, 3H), 4.19-4.18 (m, 1H), 3.72-3.70 (m, 1H), 3.49-3.31 (m, 5H), 3.30-2.80 (m, 5H), 2.72-2.70 (m, 2H), 2.58-2.56 (m, 2H), 2.10-1.60 (m, 8H), 1.46-1.41 (m, 6H). Chiral SFC H (35% MeOH): ee 100%, Rt = 3.46min.

**Compound 14-B-E2** LC/MS ESI 526 (M+H) +. 1H NMR (400 MHz, MeOD) δ 7.58-7.56 (m, 1H), 7.25-7.21 (m, 1H), 7.15-7.13 (m, 1H), 7.10-7.08 (m, 1H), 6.38 (d, J=7.2Hz, 1H), 4.92-4.72 (m, 3H), 4.18-4.17 (m, 1H), 3.72-3.70 (m, 1H), 3.49-3.31 (m, 5H), 3.30-2.80 (m, 5H), 2.72-2.70 (m, 2H), 2.58-2.56 (m, 2H), 2.10-1.60 (m, 8H), 1.46-1.41 (m, 6H). Chiral SFC H (35% MeOH): ee 100%, Rt = 4.34min.

**2-(7-fluoro-3-(2-methoxypropan-2-yl)isochroman-5-yl)-2-((R)-3-(4-(5,6,7,8-tetrahydro-1,8-naphthyridin-2-yl)butoxy)pyrrolidin-1-yl)acetic acid (diastereomeric compounds 15-E1, 15-E2 and 15-E3)**

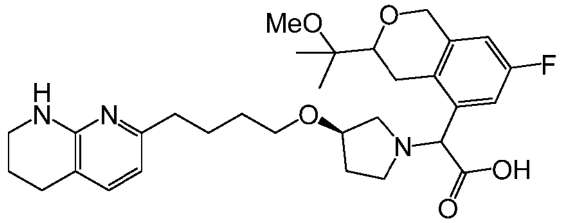

**Compound 15-E1 (mixture of 2 stereoisomers)** LC/MS ESI 556 (M+H) +. 1H NMR (500 MHz, MeOD) δ 7.30-7.27 (m, 1H), 7.21-7.19 (m, 1H), 6.88-6.87 (m, 1H), 6.43-6.40 (m, 1H), 4.92-4.74 (m, 3H), 4.18-4.17 (m, 1H), 3.55-3.31 (m, 6H), 3.30-2.80 (m, 8H), 2.70-2.52 (m, 4H), 2.20-2.08 (m, 2H), 1.85-1.60 (m, 6H), 1.30-1.24 (m, 6H). Chiral SFC B (25% IPA): Rt = 2.06min, 2.25min.

**Compound 15-E2** LC/MS ESI 556 (M+H) +. 1H NMR (500 MHz, MeOD) δ 7.33 (d, J=2.0Hz, 1H), 7.19 (d, J=7.5Hz, 1H), 6.88-6.87 (m, 1H), 6.41 (d, J=7.0Hz, 1H), 4.90-4.76 (m, 3H), 4.18-4.17 (m, 1H), 3.64-3.31 (m, 6H), 3.30-3.10 (m, 7H), 2.80-2.52 (m, 5H), 2.20-2.08 (m, 2H), 1.90-1.64 (m, 6H), 1.30 (s, 6H). Chiral SFC B (25% IPA): Rt = ee 75%, Rt = 2.75min.

**Compound 15-E3** LC/MS ESI 556 (M+H) +. 1H NMR (500 MHz, MeOD) δ 7.32-7.31 (m, 1H), 7.19 (d, J=7.0Hz, 1H), 6.89-6.88 (m, 1H), 6.41 (d, J=7.5Hz, 1H), 4.90-4.76 (m, 3H), 4.18 (s, 1H), 3.64-3.31 (m, 6H), 3.30-3.05 (m, 7H), 2.80-2.52 (m, 5H), 2.20-2.02 (m, 2H), 1.90-1.64 (m, 6H), 1.29 (s, 6H). Chiral SFC B (25% IPA): Rt = ee 90%, Rt = 5.00min.

**2-((R)-3-(1-fluoro-5-(5,6,7,8-tetrahydro-1,8-naphthyridin-2-yl)pentyl)pyrrolidin-1-yl)-2-(3-(2-methoxypropan-2-yl)isochroman-5-yl)acetic acid F-stereoisomer A (diastereomeric compounds 16-A-E1, 16-A-E2, 16-B-E1 and 16-B-E2)**

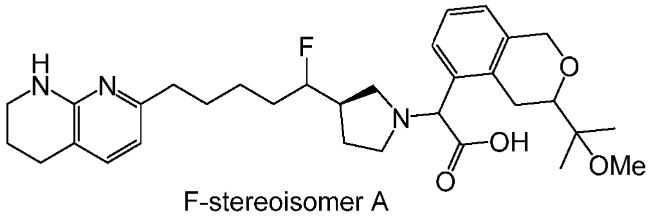

F-stereoisomer A

**Compound 16-A-E1** LC/MS ESI 554 (M+H) +. 1H NMR (400 MHz, MeOD) δ 7.65-7.63 (m, 1H), 7.13-7.09 (m,2H), 6.88 (d, J = 7.2Hz, 1H), 6.34 (d, J = 7.2Hz, 1H), 4.85-4.77(m, 2H), 4.34-4.21 (m, 1H), 3.99 (s, 1H), 3.63-3.60(m, 1H), 3.38-3.33 (m, 2H), 2.97 (m, 1H),2.78-2.67 (m, 3H), 2.58-2.56(m, 3H), 2.50-2.29 (m, 2H), 2.09-2.07 (m, 1H), 1.94(s, 3H), 1.90-1.84(m, 3H), 1.76-1.39 (m, 7H), 1.38(s, 3H), 1.29(s, 3H). Chiral SFC F (45% MeOH:MeCN=3:2): Rt = ee 100%, Rt = 3.01min.

**Compound 16-A-E2** LC/MS ESI 554 (M+H) +. 1H NMR (400 MHz, MeOD) δ 7.64-7.62 (m, 1H), 7.12-7.08 (m,2H), 6.88 (d, J = 7.2Hz, 1H), 6.35 (d, J = 7.2Hz, 1H), 4.84-4.79 (m, 2H), 4.38-4.28 (m, 1H), 4.02 (s, 1H), 3.61-3.57(m, 1H), 3.39-3.36 (m, 2H), 3.00-2.97 (m, 3H),2.71-2.68 (m, 2H), 2.60-2.51(m, 3H), 2.48-2.38 (m, 2H), 2.21-2.17 (m, 1H), 1.94(s, 3H), 1.91-1.85 (m, 3H), 1.74-1.49 (m, 7H), 1.32(s, 3H), 1.28(s, 3H). Chiral SFC F (45% MeOH:MeCN=3:2): Rt = ee 100%, Rt = 6.33min.

**Compound 16-B-E1** LC/MS ESI 554 (M+H)+. $^1$H NMR (500 MHz, MeOD) δ 7.41 (d, *J* = 7.7 Hz, 1H), 7.13 (t, 1H), 7.02 (d, *J* = 7.3 Hz, 1H), 6.98 (d, *J* = 7.6 Hz, 1H), 6.25 (d, *J* = 7.3 Hz, 1H), 4.76 - 4.65 (m, 3H), 4.42 (d, *J* = 48.9 Hz, 1H), 3.67 - 3.51 (m, 2H), 3.28 - 3.25 (m, 2H), 3.20 - 3.18 (m, 3H), 3.12 - 3.09 (m, 1H), 2.97 - 2.72 (m, 3H), 2.59 (t, *J* = 6.3 Hz, 3H), 2.42 (t, *J* = 7.6 Hz, 2H), 2.02 - 1.90 (m, 2H), 1.79 - 1.75 (m, 2H), 1.60 - 1.30 (m, 6H), 1.20 (s, 3H), Chiral SFC H (35% EtOH): ee 100 %, Rt = 3.97 min.

**Compound 16-B-E2** LC/MS ESI 554 (M+H) +. $^1$H NMR (500 MHz, MeOD) δ 7.43 (d, *J* = 7.7 Hz, 1H), 7.12 (t, *J* = 7.7 Hz, 1H), 7.02 (d, *J* = 7.3 Hz, 1H), 6.96 (d, *J* = 7.6 Hz, 1H), 6.24 (d, *J* = 7.3 Hz, 1H), 4.77 - 4.65 (m, 3H), 4.40 - 4.30 (m, 1H), 3.51 (dd, *J*= 11.3, 3.4 Hz, 1H), 3.33 - 3.23 (m, 5H), 3.13 - 2.81 (m, 5H), 2.59 - 2.39(m, 5H), 2.05 - 1.89 (m, 2H), 1.78 - 1.73 (m, 2H), 1.57 - 1.26 (m, 6H), 1.21 - 1.16 (m, 6H). Chiral SFC H (35% EtOH): ee 100 %, Rt = 6.05 min.

**2-((R)-7-fluoro-2-isopropyl-2,3-dihydro-5H-benzo[e][1,4]dioxepin-9-yl)-2-((R)-3-(4-(5,6,7,8-tetrahydro-1,8-naphthyridin-2-yl)butoxy)pyrrolidin-1-yl)acetic acid (diastereomeric compounds 17-E1 and 17-E2)**

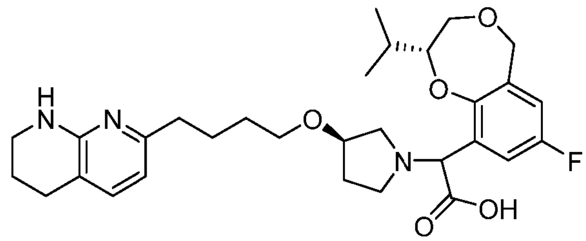

**Compound 17-E1** LC/MS ESI 542 (M+H)+, $^1$H NMR (500 MHz, MeOD) δ 7.31 (dd, *J* = 9.0, 3.1 Hz, 1H), 7.17 (d, *J* = 7.4 Hz, 1H), 7.08 (dd, *J* = 8.1, 3.1 Hz, 1H), 6.39 (d, *J* = 7.3 Hz, 1H), 5.18 (s, 1H), 4.69 (d, *J* = 13.7 Hz, 1H), 4.61 (d, *J* = 13.6 Hz, 1H), 4.16 (d, *J* = 11.4 Hz, 2H), 3.93 (dd, *J* = 9.0, 4.1 Hz, 1H), 3.84 - 3.77 (m, 1H), 3.65 - 3.39 (m, 6H), 3.25 - 3.17 (m, 2H), 2.73 (t, *J* = 6.2 Hz, 2H), 2.55 (t, *J* = 7.6 Hz, 2H), 2.21 - 2.08 (m, 3H), 1.93 - 1.86 (m, 2H), 1.75 - 1.57 (m, 4H), 1.13 (dd, *J* = 6.9, 3.1 Hz, 6H).

**Compound 17-E2** LC/MS ESI 542 (M+H)+, $^1$H NMR (500 MHz, MeOD) δ 7.31 (dd, *J*= 8.8, 3.0 Hz, 1H), 7.19 (d, *J* = 7.3 Hz, 1H), 7.05 (dd, *J* = 8.2, 3.1 Hz, 1H), 6.40 (d, *J* = 7.3 Hz, 1H), 5.07 (s, 1H), 4.69 (d, *J* = 13.7 Hz, 1H), 4.60 (d, *J* = 13.6 Hz, 1H), 4.21 - 4.11 (m, 2H), 3.85 - 3.76 (m, 2H), 3.56 - 3.38 (m, 5H), 3.25 - 3.05 (m, 2H), 2.73 (t, *J* = 6.2 Hz, 2H), 2.57 (t, *J* = 7.4 Hz, 2H), 2.27 - 2.03 (m, 3H), 1.93 - 1.87 (m, 2H), 1.75 - 1.60 (m, 4H), 1.13 - 1.01 (m, 6H).

**2-(7-fluoro-3-(tetrahydro-2H-pyran-4-yl)isochroman-5-yl)-2-((R)-3-(4-(5,6,7,8-tetrahydro-1,8-naphthyridin-2-yl) butoxy)pyrrolidin-1-yl)acetic acid (diastereomeric compounds 18-E1 and 18-E2)**

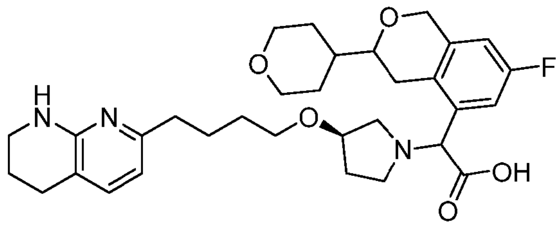

**Compound 18-E1 (mixture** of **2 stereoisomers)** LC/MS ESI 568 (M+H)+. $^1$H NMR (400 MHz, MeOD) δ 7.33 (d, *J* = 7.5 Hz, 1H), 7.25 - 7.14 (m, 1H), 6.87 (d, *J* = 6.5 Hz, 1H), 6.56 - 6.36 (m, 1H), 4.84 - 4.74 (m, 2H), 4.17 (s, 1H), 4.02 - 3.93 (m, 2H), 3.55 - 3.37 (m, 8H), 3.21 - 3.17 (m, 2H), 2.94 - 2.55 (m, 6H), 2.22 - 2.07 (m, 2H), 1.93 - 1.88 (m, 3H), 1.80 - 1.43 (m, 9H). Chiral SFC F (45% EtOH): ee 100 %, Rt = 2.17 min.

**Compound 18-E2 (mixture of 2 stereoisomers)** LC/MS ESI 568 (M+H)+. $^1$H NMR (400 MHz, MeOD) δ 7.18 (d, *J*= 9.8 Hz, 1H), 7.10 (d, *J = 7.4* Hz, 1H), 6.77 (d, *J*= 7.1 Hz, 1H), 6.31 (d, *J = 7.3* Hz, 1H), 4.76 - 4.70 (m, 3H), 4.65 (s, 1H), 4.08 (s, 1H), 3.85 - 3.82 (m, 2H), 3.40 - 3.26 (m, 8H), 3.06 - 2.96 (m, 3H), 2.61 (t, *J* = 6.2 Hz, 2H), 2.51 - 2.44 (m, 3H), 2.02 - 1.75 (m, 6H), 1.65 - 1.41 (m, 8H). Chiral SFC F (45% EtOH): ee 85 %, Rt = 3.58 min.

**2-((S)-7-fluoro-3-isopropyl-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetic acid (diastereomeric compounds 19-E1 and 19-E2)**

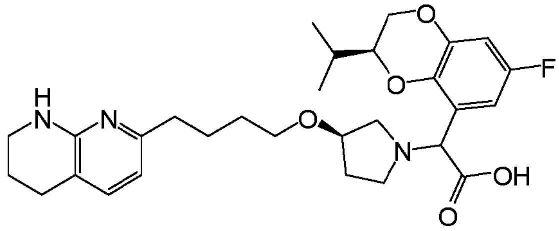

**Compound 19-E1** LC/MS ESI 528 (M+H) +. 1H NMR (400 MHz, MeOD) δ 7.16 (d, J = 7.2Hz, 1H), 6.89-6.86 (m,1H), 6.72-6.70 (m, 1H), 6.38 (d, J = 7.2Hz, 1H), 5.01 (s, 1H), 4.37 (m, 1H), 4.18 (m, 1H), 4.00-3.92(m, 2H), 3.61-3.57 (m, 1H), 3.49 (m, 2H), 3.48-3.46 (m, 4H), 3.12 (m, 1H),2.73-2.69 (m, 2H), 2.57-2.53(m, 2H), 2.14-1.87 (m, 5H), 1.73-1.70(m, 2H), 1.64-1.60 (m, 2H), 1.08(d, 6.8Hz, 3H), 1.03(d, 6.8Hz, 3H). Chiral SFC B (25% EtOH): ee 100 %, Rt = 1.52 min.

**Compound 19-E2** LC/MS ESI 528 (M+H) +. 1H NMR (400 MHz, MeOD) δ 7.34 (d, J = 7.2Hz, 1H), 6.92-6.89 (m,1H), 6.76-6.73 (m, 1H), 6.48 (d, J = 7.2Hz, 1H), 4.98 (s, 1H), 4.40 (m, 1H), 4.22 (m, 1H), 4.05-4.00(m, 1H), 3.91-3.90(m, 1H), 3.88 (m, 1H), 3.76-3.40 (m, 6H), 3.28 (m, 1H),2.77-2.77 (m, 2H), 2.63-2.59(m, 2H), 2.22-2.12(m, 2H), 1.99-1.89(m, 3H), 1.81-1.63(m, 4H), 1.10(d, 6.8Hz, 3H), 1.04(d, 6.8Hz, 3H). Chiral SFC B (25% EtOH): ee 100 %, Rt = 4.19 min.

**2-(3-(2-fluoropropan-2-yl)isochroman-5-yl)-2-((R)-3-(4-(4-methoxy-5,6,7,8-tetrahydro-1,8-naphthyridin-2-yl)butoxy)pyrrolidin-1-yl)acetic acid (diastereomeric compounds 20-E1 and 20-E2)**

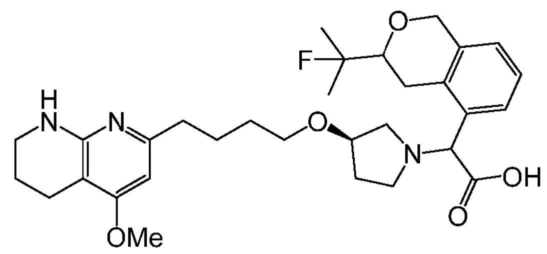

**Compound 20-E1 (mixture of 2 stereoisomers)** LC/MS ESI 556.3 (M+H) +. 1H NMR (400 MHz, MeOD) δ 7.56-7.52 (m, 1H), 7.28-7.24 (m, 1H), 7.18-7.15 (m, 1H), 6.28 (s, 1H), 4.95-4.75 (m, 3H), 4.20-4.18 (m, 1H), 3.88-3.84 (m, 3H), 3.68-3.62 (m, 1H), 3.60-3.42 (m, 3H), 3.20-3.00 (m, 3H), 2.98-2.88 (m, 2H), 2.70-2.55 (m, 4H), 2.21-2.00 (m, 2H), 1.90-1.58 (m, 7H), 1.50-1.30 (m, 7H). Chiral SFC A (45% MeOH): ee 100 %, Rt = 2.07 min.

**Compound 20-E2 (mixture of 2 stereoisomers)** LC/MS ESI 556.3 (M+H) +. 1H NMR (400 MHz, MeOD) δ 7.54 (d, J=7.6Hz, 1H), 7.25-7.20 (m, 1H), 7.08 (d, J=7.2Hz, 1H), 6.28 (s, 1H), 4.95-4.75 (m, 3H), 4.20-4.17 (m, 1H), 3.86 (s, 3H), 3.75-3.68 (m, 1H), 3.60-3.42 (m, 3H), 3.30-2.95 (m, 5H), 2.75-2.55 (m, 4H), 2.20-2.10 (m, 2H), 1.85-1.58 (m, 7H), 1.50-1.30 (m, 7H). Chiral SFC A (45% MeOH): ee 96 %, Rt = 2.85 min.

**2-(7-fluoro-3-(2-methoxypropan-2-yl)isochroman-5-yl)-2-((R)-3-(4-(4-methoxy-5,6,7,8-tetrahydro-1,8-naphthyridin-2-yl)butoxy)pyrrolidin-1-yl)acetic acid (diastereomeric compounds 21-E1 and 21-E2)**

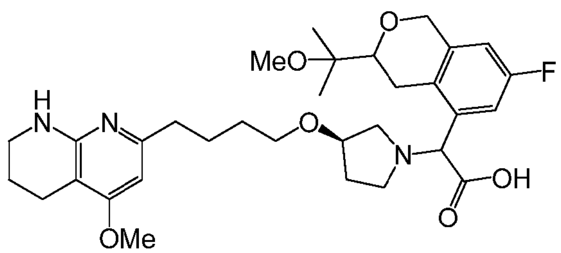

**Compound 21-E1 (mixture of 3 stereoisomers)** LC/MS ESI 586.2 (M+H) +. 1H NMR (400 MHz, MeOD) δ 7.34-7.32 (m, 1H), 6.81-6.79 (m, 1H), 6.35-6.33 (m, 1H), 4.88-4.55 (m, 3H), 4.14-4.12 (m, 1H), 3.89-3.87 (m, 3H), 3.59-3.32 (m, 3H), 3.20-2.57 (m, 15H), 2.21-1.98 (m, 2H), 1.86-1.64 (m, 6H), 1.25-1.18 (m, 6H). Chiral SFC B (25% IPA): Rt = 2.00 min, 2.21 min, 2.72 min.

**Compound 21-E2** LC/MS ESI 556.3 (M+H) +. 1H NMR (400 MHz, MeOD) δ 7.33-7.30 (m, 1H), 6.84-6.81 (m, 1H), 6.31 (s, 1H), 4.87-4.68 (m, 3H), 4.18-4.16 (m, 1H), 3.84 (s, 3H), 3.62-3.32 (m, 3H), 3.20-2.57 (m, 15H), 2.21-1.98 (m, 2H), 1.87-1.64 (m, 6H), 1.24 (s, 6H). Chiral SFC B (25% IPA): ee 100%, Rt = 4.43 min.

**2-(3-isopropylchroman-5-yl)-2-((R)-3-(4-(5,6,7,8-tetrahydro-1,8-naphthyridin-2-yl)butoxy)pyrrolidin-1-yl)acetic acid (compound 22)**

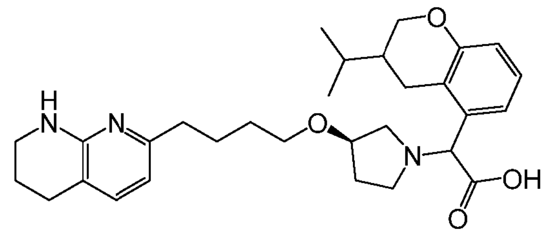

**Compound 22 (mixture of 4 stereoisomers)** LC/MS ESI 508.2 (M+H)+, 1H NMR (400 MHz, MeOD) δ 7.20-7.15 (m, 3H), 6.82-6.78 (m, 1H), 6.40-6.38 (m, 1H), 4.80 (s, 1H), 4.35-4.15 (m, 2H), 3.80-3.70 (m, 1H), 3.55-3.35 (m, 4H), 3.30-2.80 (m, 5H), 2.75-2.65 (m, 2H), 2.60-2.45 (m, 3H), 2.30-2.00 (m, 2H), 1.95-1.85 (m, 2H), 1.80-1.50 (m, 6H), 1.10-0.95 (m, 6H).

**2-(7-fluoro-3-(2-fluoropropan-2-yl)isochroman-5-yl)-2-((R)-3-(4-(4-methoxy-5,6,7,8-tetrahydro-1,8-naphthyridin-2-yl)butoxy)pyrrolidin-1-yl)acetic acid (diastereomeric compounds 23-E1 and 23-E2)**

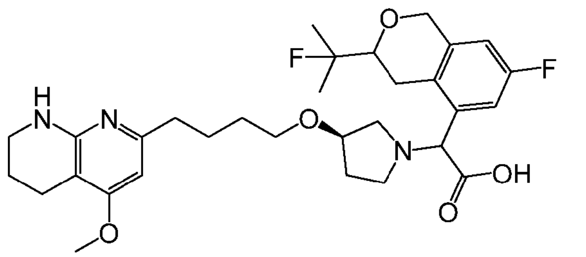

**Compound 23-E1 (mixture of 3 stereoisomers)** LC/MS ESI 574.2 (M+H)+, 1H NMR (400 MHz, MeOD) δ 7.25-7.20 (m, 1H), 6.80-6.70 (m, 1H), 6.22 (s, 1H), 4.85-4.62 (m, 3H), 4.05-4.00 (m, 1H), 3.82-3.78 (m, 3H), 3.62-3.30 (m, 3H), 3.15-2.95 (m, 4H), 2.90-2.78 (m, 2H), 2.75-2.40 (m, 5H), 2.15-1.90 (m, 2H), 1.80-1.45 (m, 6H), 1.40-1.20 (m, 7H). Chiral SFC E (45% MeOH): Rt = 3.56 min, 4.21min, 4.50 min.

**Compound 23-E2** LC/MS ESI 574.2 (M+H)+, 1H NMR (400 MHz, MeOD) δ 7.38-7.35 (m, 1H), 6.90-6.85 (m, 1H), 6.38 (s, 1H), 4.85-4.78 (m, 3H), 4.20-4.18 (m, 1H), 3.90 (s, 3H), 3.70-3.62 (m, 1H), 3.58-3.45 (m, 2H), 3.30-3.10 (m, 4H), 3.05-2.90 (m, 1H), 2.65-2.55 (m, 4H), 2.15-2.00 (m, 2H), 1.90-1.60 (m, 6H), 1.45-1.35 (m, 8H). Chiral SFC E (45% MeOH): ee 100 %, Rt = 6.11 min.

**2-((R)-7-fluoro-3-(2-fluoropropan-2-yl)-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetic acid (compound 24)**

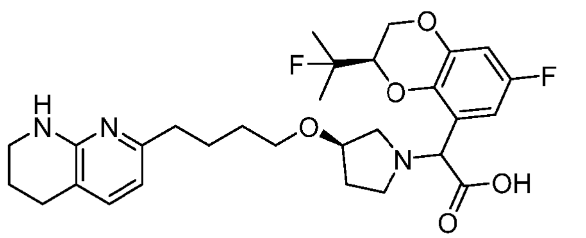

**Compound 24 (mixture of 2 stereoisomers)** LC/MS ESI 546 (M+H)+, $^{1}$H NMR (400 MHz, MeOD) δ 7.40 (t, *J* = 6.6 Hz, 1H), 6.83-6.82 (m, 1H), 6.72-6.71 (m, 1H), 6.45 (t, *J* = 7.4 Hz, 1H), 5.06 - 4.91 (m, 1H), 4.46 - 4.41 (m, 1H), 4.20 - 4.02 (m, 2H), 3.97 (t, *J* = 10.1 Hz, 1H), 3.76 - 3.73 (m, 1H), 3.51 - 2.95 (m, 6H), 3.08 - 3.04 (m, 1H), 2.70 - 2.52 (m, 4H), 2.18 - 2.04 (m, 2H), 2.04 - 1.76 (m, 6H), 1.42 - 1.36 (m, 6H).

**2-((R)-7-fluoro-3-(2-fluoropropan-2-yl)-2,3-dihydrobenzo[b] [1,4]dioxin-5-yl)-2-((R)-3-(4-(4-methoxy-5,6,7,8-tetrahydro-1,8-naphthyridin-2-yl)butoxy)pyrrolidin-1-yl)acetic acid (compound 25)**

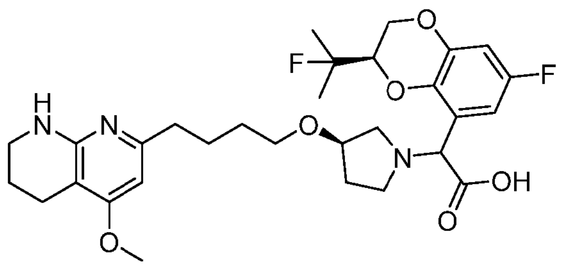

**Compound 25 (mixture of 2 stereoisomers)** LC/MS ESI 576 (M+H)+, $^1$H NMR (400 MHz, MeOD) δ 6.94 (dd, *J* = 8.8, 2.9 Hz, 1H), 6.87 - 6.79 (m, 1H), 6.58 (d, *J* = 5.8 Hz, 1H), 5.19 - 5.02 (m, 1H), 4.55 (td, *J* = 11.3, 1.9 Hz, 1H), 4.33 - 4.16 (m, 2H), 4.13 - 4.05 (m, 1H), 4.03 (s, 3H), 3.92 - 3.89 (m, 1H), 3.60 - 3.37 (m, 5H), 3.28 - 3.14 (m, 1H), 2.87 - 2.62 (m, 4H), 2.37 - 2.09 (m, 2H), 1.89 - 1.66 (m, 6H), 1.58 - 1.41 (m, 6H).

**2-(7-fluoro-3-(2-methoxypropan-2-yl)chroman-5-yl)-2-((R)-3-(4-(5,6,7,8-tetrahydro-1,8-naphthyridin-2-yl)butoxy)pyrrolidin-1-yl)acetic acid (diastereomeric compounds 26-E1 and 26-E2)**

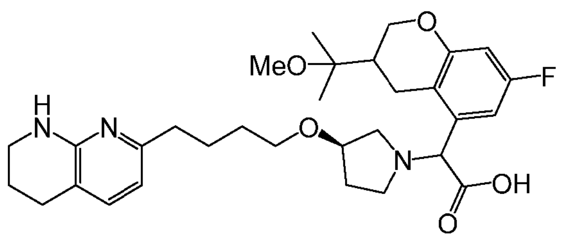

**Compound 26-E1 (mixture of 3 stereoisomers)** LC/MS ESI 556.2 (M+H)+, 1H NMR (400 MHz, MeOD) δ 7.20-7.15 (m, 1H), 7.00-6.95 (m, 1H), 6.58-6.54 (m, 1H), 6.41-6.38 (m, 1H), 4.78-4.70 (m, 1H), 4.41-4.37 (m, 1H), 4.20-4.16 (m, 1H), 3.80-3.76 (m, 1H), 3.55-3.28 (m, 8H), 3.23-3.10 (m, 3H), 2.88-2.82 (m, 1H), 2.75-2.52 (m, 4H), 2.20-2.06 (m, 3H), 1.92-1.80 (m, 2H), 1.78-1.56 (m, 4H), 1.38-1.15 (m, 7H). Chiral SFC E (45% EtOH): ee 100 %, Rt = 2.97 min.

**Compound 26-E2** LC/MS ESI 556.2 (M+H)+, 1H NMR (400 MHz, MeOD) δ 7.18-7.15 (m, 1H), 6.98-6.95 (m, 1H), 6.58-6.54 (m, 1H), 6.37 (d, J=7.2Hz, 1H), 4.80 (s, 1H), 4.41-4.38 (m, 1H), 4.20-4.16 (m, 1H), 3.80-3.76 (m, 1H), 3.55-3.28 (m, 8H), 3.22-3.05 (m, 3H), 2.70-2.52 (m, 4H), 2.20-2.00 (m, 3H), 1.90-1.82 (m, 2H), 1.78-1.58 (m, 4H), 1.32-1.18 (m, 7H). Chiral SFC E (45% EtOH): ee 100 %, Rt = 4.21 min.

**2-(7-fluoro-3-(2-methoxypropan-2-yl)chroman-5-yl)-2-((R)-3-(4-(4-methoxy-5,6,7,8-tetrahydro-1,8-naphthyridin-2-yl)butoxy)pyrrolidin-1-yl)acetic acid (diastereomeric compounds 27-E1 and 27-E2)**

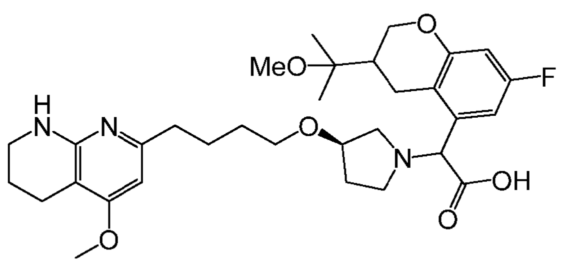

**Compound 27-E1 (mixture of 2 stereoisomers)** LC/MS ESI 586.2 (M+H)+, 1H NMR (400 MHz, MeOD) δ

6.91-6.87 (m, 1H), 6.44-6.39 (m, 1H), 6.21-6.19 (m, 1H), 4.60-4.45 (m, 1H), 4.25-4.22 (m, 1H), 4.05-4.00 (m, 1H), 3.78-3.75 (m, 3H), 3.65-3.62 (m, 1H), 3.45-3.32 (m, 2H), 3.18-3.00 (m, 3H), 2.95-2.74 (m, 3H), 2.55-2.42 (m, 4H), 2.06-1.90 (m, 3H), 1.78-1.48 (m, 6H), 1.25-1.08 (m, 11H).

**Compound 27-E2 (mixture of 2 stereoisomers)** LC/MS ESI 586.2 (M+H)+, 1H NMR (400 MHz, MeOD) δ 7.00-6.97 (m, 1H), 6.56-6.53 (m, 1H), 6.31 (s, 1H), 4.72 (s, 1H), 4.41-4.39 (m, 1H), 4.20-4.18 (m, 1H), 3.85 (s, 3H), 3.82-3.78 (m, 1H), 3.52-3.45 (m, 2H), 3.30-2.95 (m, 6H), 2.62-2.56 (m, 4H), 2.20-2.08 (m, 3H), 1.85-1.60 (m, 6H), 1.40-1.20 (m, 11H).

**2-(7-fluoro-3-(2-fluoropropan-2-yl)chroman-5-yl)-2-((R)-3-(4-(5,6,7,8-tetrahydro-1,8-naphthyridin-2-yl)butoxy) pyrrolidin-1-yl)acetic acid (diastereomeric compounds 28-E1 and 28-E2)**

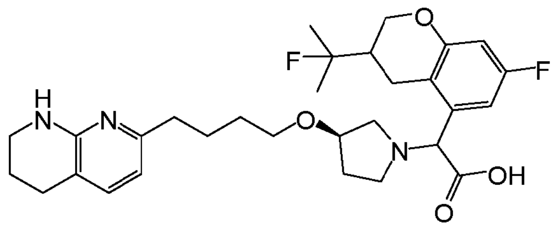

**Compound 28-E1 (mixture of 2 stereoisomers)** LC/MS ESI 544 (M+H) +. 1H NMR (400 MHz, MeOD) δ 7.20-7.18 (m, 1H), 7.04-7.01 (m, 1H), 6.58-6.55 (m, 1H), 6.38 (d, J=7.2Hz, 1H), 4.71 (s, 1H), 4.43-4.41 (m, 1H), 4.20-4.18 (m, 1H), 3.82-3.80 (m, 1H), 3.50-3.31 (m, 4H), 3.30 -2.95 (m, 5H), 2.75 -2.57 (m, 5H), 2.23-1.60 (m, 9H), 1.45-1.41 (m, 6H). Chiral SFC B (20% EtOH): ee 91%, Rt = 1.84min.

**Compound 28-E2 (mixture of 2 stereoisomers)** LC/MS ESI 544 (M+H) +. 1H NMR (400 MHz, MeOD) δ 7.20-7.18 (m, 1H), 7.06-7.03 (m, 1H), 6.58-6.55 (m, 1H), 6.39 (d, J=7.2Hz, 1H), 4.68 (s, 1H), 4.43-4.41 (m, 1H), 4.20-4.18 (m, 1H), 3.82-3.80 (m, 1H), 3.50-3.31 (m, 4H), 3.30 -2.95 (m, 5H), 2.75 -2.57 (m, 5H), 2.23-1.60 (m, 9H), 1.45-1.41 (m, 6H). Chiral SFC B (20% EtOH): ee 92%, Rt = 2.90min.

**2-(7-fluoro-3-methylisochroman-5-yl)-2-((R)-3-(4-(5,6,7,8-tetrahydro-1,8-naphthyridin-2-yl)butoxy)pyrrolidin-1-yl)acetic acid (diastereomeric compounds 29-E1 and 29-E2)**

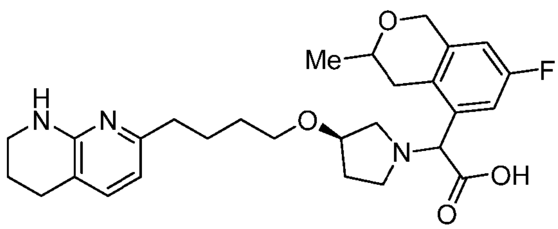

**Compound 29-E1 (mixture of 2 stereoisomers)** LC/MS ESI 498 (M+H)+, $^1$H NMR (400 MHz, MeOD) δ 7.30 (dd, *J* = 16.7, 7.2 Hz, 1H), 7.19 (d, *J* = 7.3 Hz, 1H), 6.89 (d, *J* = 9.0 Hz, 1H), 6.41 (d, *J* = 7.3 Hz, 1H), 4.83 - 4.76 (m, 3H), 4.21 - 4.17 (m, 1H), 3.87 - 3.77 (m, 1H), 3.59 - 3.37 (m, 5H), 3.17 - 3.07 (m, 4H), 2.72 (t, *J* = 6.3 Hz, 2H), 2.65 - 2.46 (m, 3H), 2.22 - 2.02 (m, 2H), 1.92 - 1.58 (m, 6H), 1.33 *(t, J*= 6.5 Hz, 3H).

**Compound 29-E2 (mixture of 2 stereoisomers)** LC/MS ESI 498 (M+H)+,$^1$H NMR (400 MHz, MeOD) δ 7.36 - 7.26 (m, 1H), 7.21 (d, *J* = 7.3 Hz, 1H), 6.88 (d, *J* = 8.8 Hz, 1H), 6.42 (dd, *J*= 7.3, 3.0 Hz, 1H), 4.84 - 4.77 (m, 3H), 4.19 (d, *J* = 10.9 Hz, 1H), 3.87 - 3.73 (m, 1H), 3.58 - 3.36 (m, 5H), 3.27 - 3.11 (m, 3H), 2.98 - 2.53 (m, 6H), 2.27 - 2.06 (m, 2H), 1.95 - 1.59 (m, 6H), 1.36 - 1.27 (m, 3H).

**2-((S)-7-fluoro-3-methyl-2,3-dihydrobenzo[b][1,4]dioxin-5-yl)-2-((R)-3-(4-(5,6,7,8-tetrahydro-1,8-naphthyridin-2-yl)butoxy)pyrrolidin-1-yl)acetic acid (compound 30)**

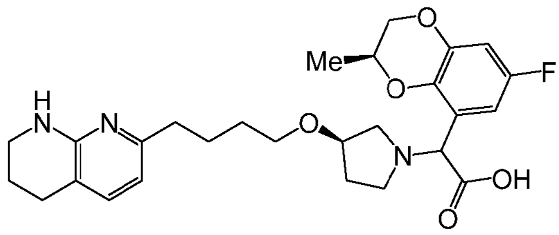

**Compound 30 (mixture of 2 stereoisomers)** LC/MS ESI 500.1 (M+H)+, 1H NMR (400 MHz, MeOD) δ 7.48 - 7.45 (m, 1H), 6.95-6.75 (m, 2H), 6.55-6.51 (m, 1H), 5.18-5.05 (m, 1H), 4.35 - 4.23 (m, 3H), 3.93 -3.86 (m, 1H), 3.65 - 3.34 (m, 6H), 3.18 - 3.15 (m, 2H), 2.80 - 2.62 (m, 4H), 2.30 - 1.55 (m, 8H), 1.48 - 1.42 (m, 3H).

**Example 12: Fluorescence polarization assays of compounds for αvβ6 binding**

Fluorescence Polarization (FP) assays were used to measure compound activity through binding competition with the fluorescein-labeled peptide GRGDLGRL. In the assay, 10 nM of integrin αvβ6 was incubated with the test compound in 2 mM manganese chloride, 0.1 mM calcium chloride, 20 mM HEPES buffer at pH 7.3, 150 mM sodium chloride, 0.01% Triton X-100, 2% DMSO, and 3 nM of the fluorescein-labeled peptide. The assays were run in 384-well plates. For both assay versions, the integrin protein was preincubated with the test compounds for 15 minutes at 22 °C before the fluorescein-labeled peptide was added. After the fluorescein-labeled peptide was added, the assay was incubated at 22 °C for 1 hour and fluorescence polarization was measured. $IC_{50}$ values were determined by nonlinear regression, 4-parameter curve fitting (Figure 1).

**Example 13: MDCK in vitro permeablity assays of compounds**

Compounds were tested for permeability in an MDCK permeability assay. This assay measures the ability of compounds to cross a layer of Madin-Darby Canine Kidney (MDCK) cells from the apical to basolateral side (A->B). This measurement is predictive of the ability of compounds to be absorbed in the gut following oral dosing, an essential characteristic of an orally administered small molecule integrin inhibitor drug.

The assay is run in two formats. One uses wild type MDCK cells with no inhibitor. This method works well in determining the passive permeability of compounds with low efflux by P-glyocprotein (Pgp), and was used to assess permeability of a Reference Compound having the chemical formula shown below. The MDCK value of less than 1 (i.e., less than about 0.23) was obtained for the Reference Compound using this method; an IC50 value of about 96.5 nM was obtained for the Reference Compound using the fluorescence polarization assay of Example 12.

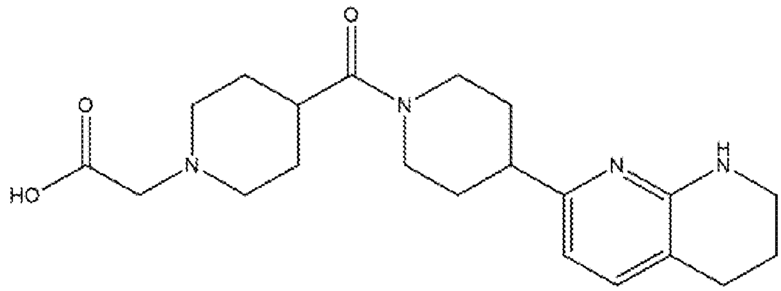

Reference Compound

| .avb6. (IC50) [nM] | MDCK (A->B) |
|---|---|
| 96.5 | <0.23 |

However, for compounds with Pgp efflux, it is necessary to include a Pgp inhibitor order to determine passive permeability for A->B transmission. In this case, a MDCK-MDR1 cell line overexpressing Pgp is used, and PGP inhibitor GF120918 is included at sufficient concentration (10 μM) to block activity of Pgp. This procedure (MDCK-MDR1 with PGP

inh (A->B) [10^6 cm/s]) was used to obtain the data presented in Tables shown in Figure 2. MDCK permeability values of less than 5 10^6 cm/s predict low absorption in the gut, while permeability values greater than 5 10^6 cm/s predict sufficient absorption in the gut for oral dosing of a small molecule drug.

The detailed experimental procedure is as follows:

**EQUIPMENT**

- 24-well Cell Culture Plate (PET membrane): Millipore # PSHT 010 R5
- 24-well feeder tray: Millipore #PSMW 010 R5
- Millicell ERS System - Millipore # MERS 000 01
- 96-well U-shape plates (BH Bio# BH-04ML-96U)
- 96-well microplates (Greiner#655209)
- 37□ $CO_2$ Incubator
- Infinite F2000pro (TECAN)

**REAGENTS**

- GF120918 (Pgp inhibitor)
- Lucifer Yellow
- Reference compounds: Quinidine, Metoprolol, Atenolol
- Cell line: MDCK (ATCC) or MDCK MDR1 (NKI)
- Cell culture growth medium (MEM + 10% FBS + 1% NEAA):
- Trypsin-EDTA (Invitrogen, Cat# 25200-072)
- Assay and dosing solution buffer: Hanks Balanced Salt Solution (HBSS, Invitrogen, Cat# 14025-092) with 25 mM HEPES, pH 7.4
- Test article and reference compound stock solutions were prepared in DMSO, Lucifer Yellow (LY) stock solution was prepared in the assay buffer.

**Cell culture and maintenance:**

- Cell stock cultures (MDCK or MDCK MDR1) are maintained in MEM + 10% FBS + 1% NEAA, grown in 75 $cm^2$ tissue culture treated flasks and split (passed) 2 times weekly to maintain desired confluence.
- For maintenance passage: trypsinized cells are routinely distributed into new flasks at a standard passage ratio of 1:20.

**Seeding assay plates:** MDCK assay plates are seeded with MDCK or MDCK MCR1 cells 3-4 days prior to running the assay. 24-well plates are seeded at a cell density of $0.88\times10^5$/well in a 400 μL apical chamber volume ($2.2\times10^5$/mL) with a 25 mL volume of growth medium to the 24-well basal chamber. Assay plates are generally provided with a growth medium change 24 hours prior to the assay.

**Preparation of the assay plates and Trans-epithelial Electrical Resistance (TEER)**

**measurement:** MDCK assay plates are rinsed with HBSS+ prior to running the assay. After rinsing, fresh HBSS+ is added to the assay plate in a 400 μL apical chamber volume and a 0.8 mL HBSS+ basal chamber volume. Measure the electrical resistance across the monolayer using the Millicell ERS system ohm meter. (The cells will be used if TEER is higher than 100 ohm*$cm^2$).

**Preparation of dosing solution.** Donor solutions are prepared in HBSS+ with 0.4% DMSO and 5 μM test compound. The donor solution contains 5 μM lucifer yellow for apical dosing, but no lucifer yellow for basolateral dosing. The donor solution may also contain 10 μM GF 120918 for Pgp inhibition. Receiver solutions are prepared with HBSS+ with 0.4% DMSO. Donor and receiver solutions were centrifuged at 4000 rpm, 5 min, and supernatants were used for compound dosing.

**Preparation of the cell plates:**

- Remove the buffer from the apical side and basolateral side. Add 600 μL of donor solution (for A-to-B) or 500 μL of receiver solution (for B-to-A) to the apical wells based on plate map.
- A fresh basolateral plate is prepared by adding 800 μL of receiver solution (for A-to-B) or 900 μL of donor solution (B-to-A) to the well of a new 24-well plate.

- Put the apical plate and basolateral plate into a 37□ $CO_2$ incubator.

**Preparation of analytical plate:**

- After 5 min, transfer 100 μL of samples from all donors (for both A-to-B and B-to-A) into appropriate wells of a sample plate for D0. And transfer 100 μL of samples from all apical chambers (the donor of A-to-B and receiver of B-to-A) into appropriate wells of a microplate for Lucifer Yellow D0 (D0 LY)

- Lay the apical plate to the basolateral plate to start transport process.

- At 90 min, separate the apical and basolateral plates and transfer 100 μL of samples from all donors (for both A-to-B and B-to-A) into appropriate wells of a new sample plate for D90, and transfer 200 μL of samples from all receivers into appropriate wells of a sample plate for R90. Transfer 100 μL of samples from all basolateral chambers (receiver of A-to-B and donor of B-to-A) into appropriate wells of a new microplate for Lucifer Yellow R90 (R90 LY).

- Determine LY permeability by reading D0 LY and R90 LY at an excitation wavelength of 485 nm and an emission wavelength of 535 nm using a fluorescent plate reader.

- LC/MS/MS Sample preparation:

    ➢ For receiver solution: 60 μL of sample + 60 μL ACN with IS (200 ng/mL Osalmid)

    ➢ For donor solution: 6 μL of sample + 54 μL 0.4% DMSO/HBSS + 60 μL ACN with IS (200 ng/mL Osalmid)

    ➢ The compound standard curve 20X solutions (0.1 - 60 μM range) are prepared in MeOH:$H_2O$ (1:1). 1X concentrated solutions (0.005 - 3 μM range) are prepared by mixing 3 μL of 20X solution with 57 μL 0.4% DMSO HBSS and 60 μL ACN with IS (200 ng/mL Osalmid).

**Calculations**

Transepithelial electrical resistance (TEER) = (Resistance $_{sample}$ -Resistance $_{blank}$) × Effective Membrane Area

Lucifer Yellow permeability:

$P_{app}$ = (VA/ (Area × time)) × ($[RFU]_{accepter}$- $[RFU]_{blank}$)/((( $[RFU]_{initial, donor}$ - $[RFU]_{blank}$) ×Dilution Factor) ×100

Plate drug transport assays using the following equation:

Transepithelial electrical resistance (TEER) = (Resistance $_{sample}$ -Resistance $_{blank}$) × Effective Membrane Area

Drug permeability:

$$P_{app} = (V_R / (\text{Area} \times \text{time})) \times ([\text{drug}]_{receiver} / (([\text{drug}]_{initial,\ donor}) \times \text{Dilution Factor})$$

Where $V_R$ is the volume in the receiver well (0.8 mL for A-to-B and 0.4 mL for B-to-A), area is the surface area of the membrane (0.7 $cm^2$ for Millipore-24 Cell Culture Plates), and time is the total transport time in seconds.

Percentage Recovery = 100 × (Total compound in donor at 90 min × Dilution Factor + Total compound in receiver at 90 min) / (Total compound in donor at 0 min × Dilution Factor)

## Claims

**1.** A compound selected from the group consisting of:

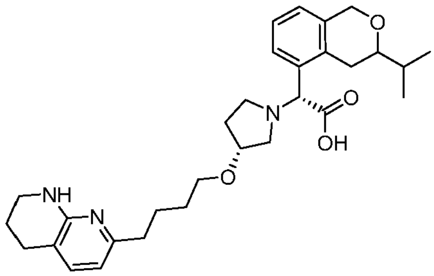, 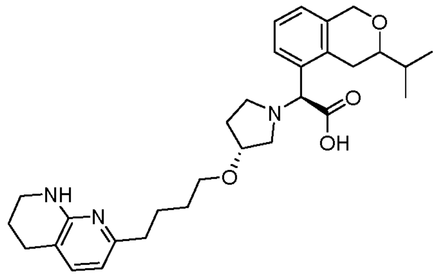,

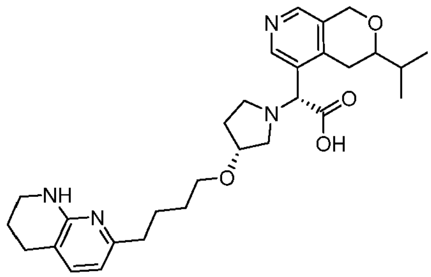, 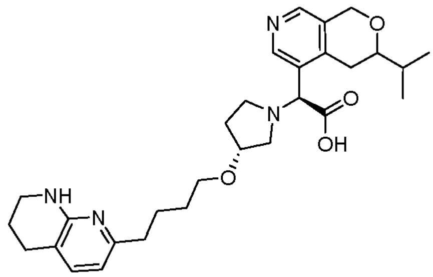,

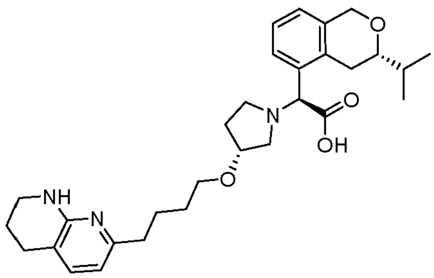, 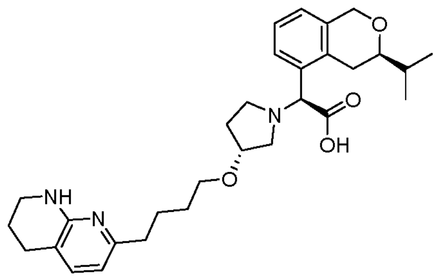,

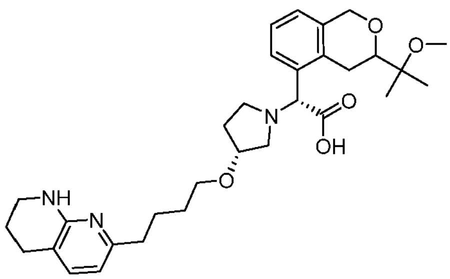, and 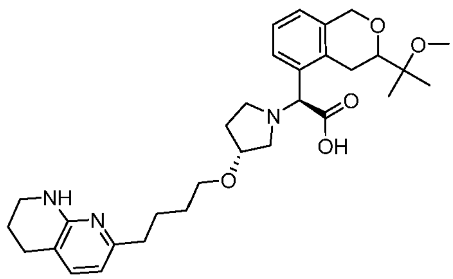;

or a

pharmaceutically acceptable salt thereof; or
selected from the group consisting of:

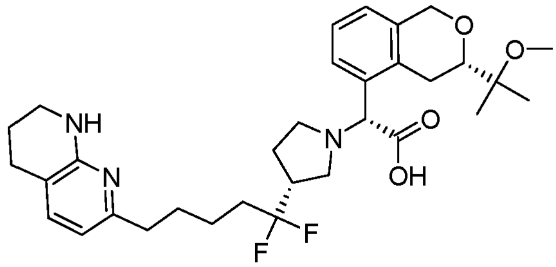

,

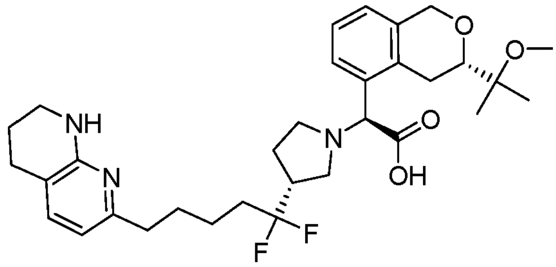

,

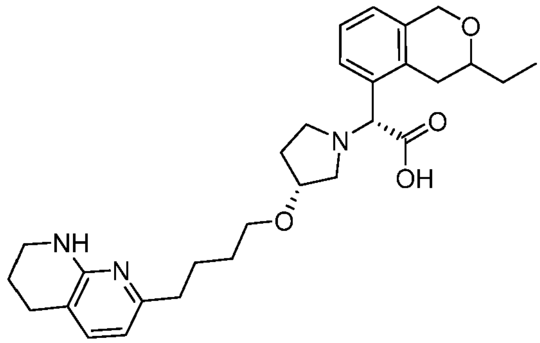

,

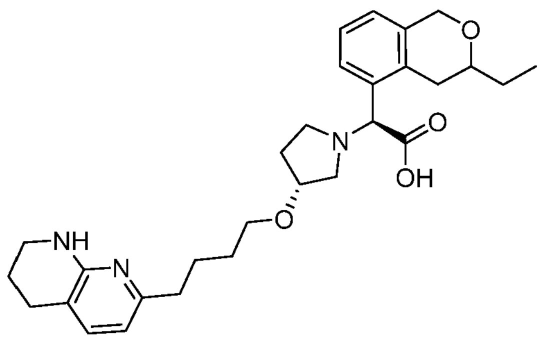

,

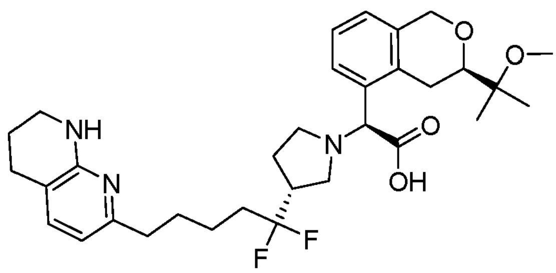

,

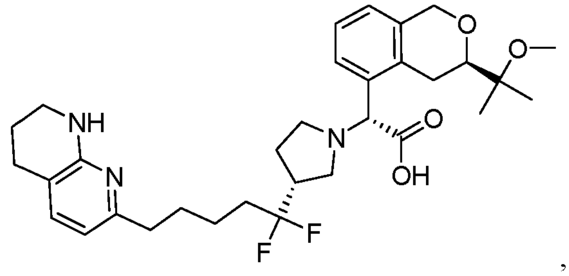

,

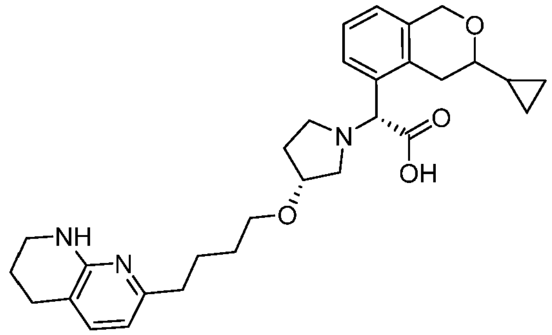

,

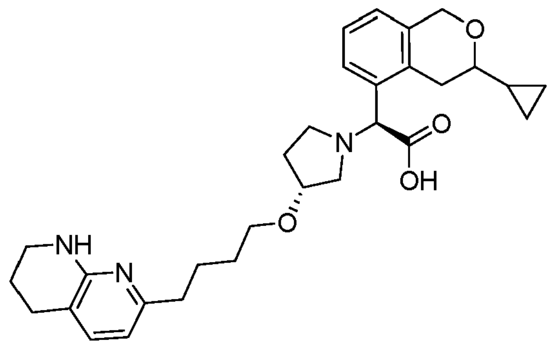

,

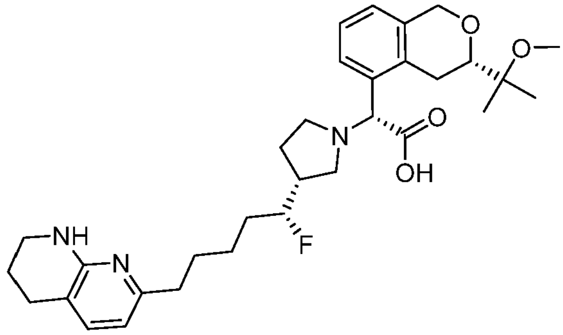

,

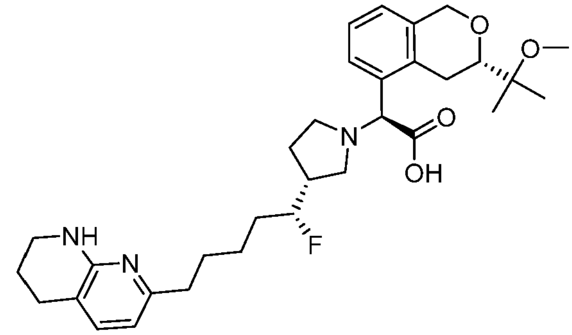

,

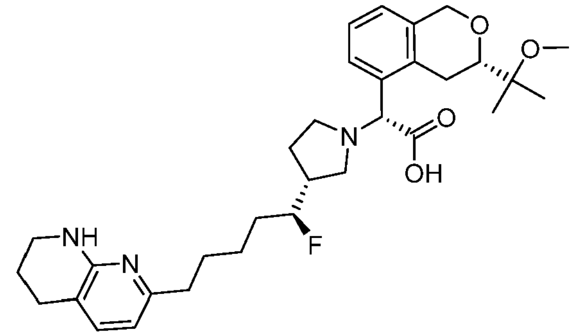

,

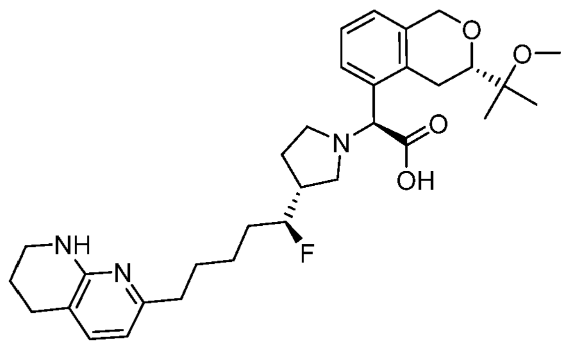

,

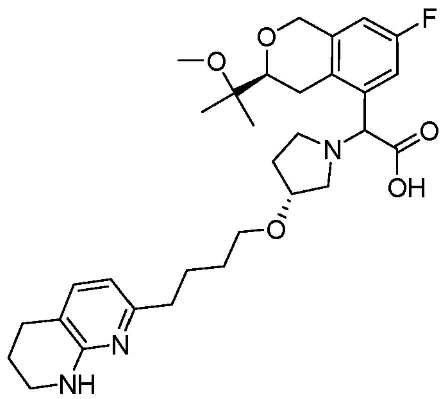

,

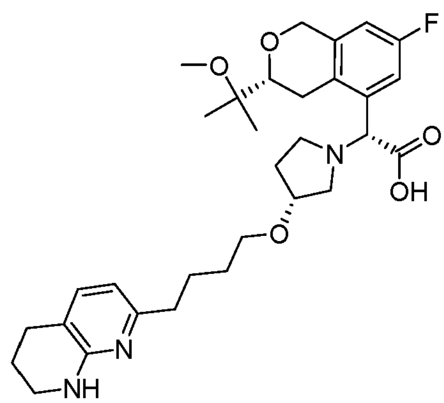

,

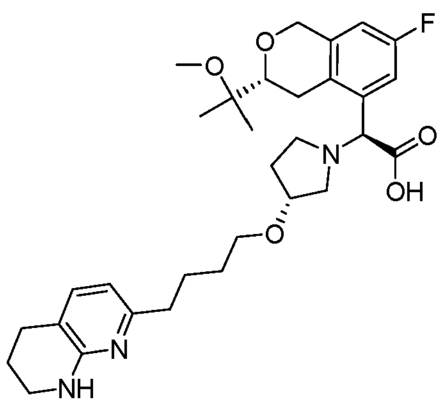

,

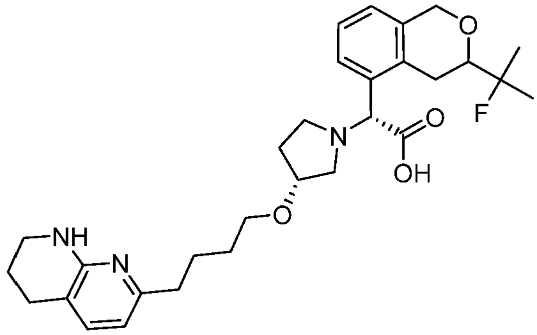

,

and

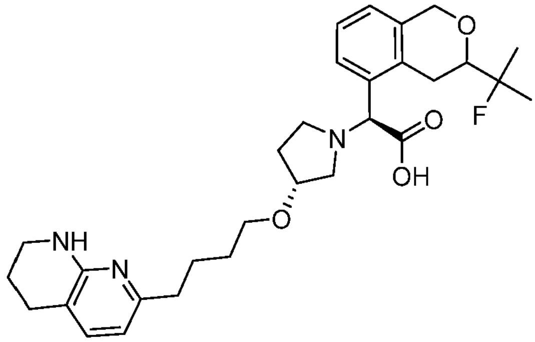

;

or a pharmaceutically acceptable salt thereof; or selected from the group consisting of:

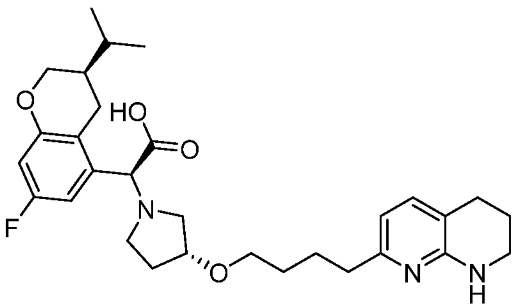

,

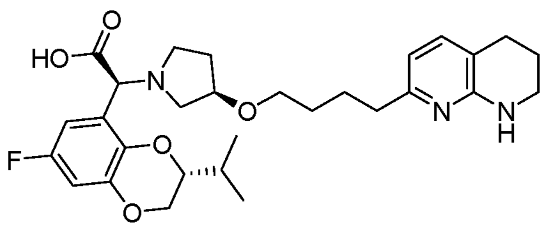

,

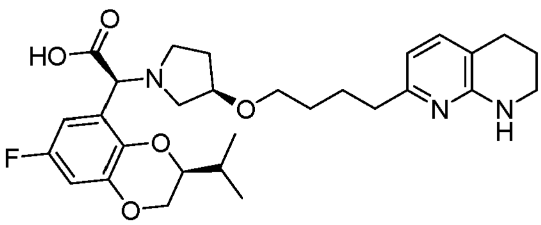

,

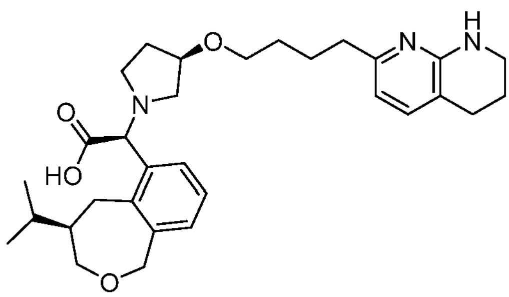

,

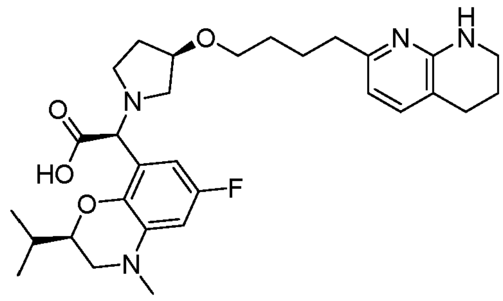
,
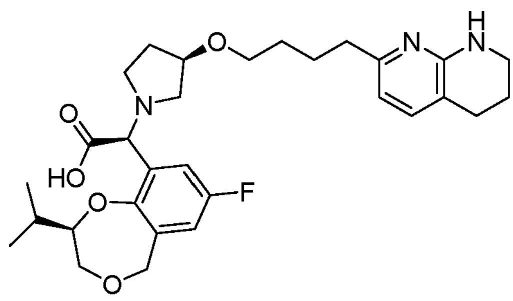
,

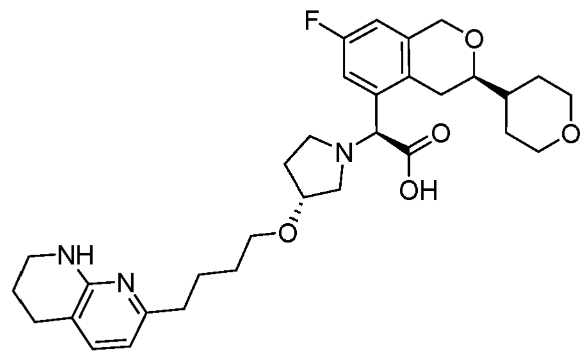
, and
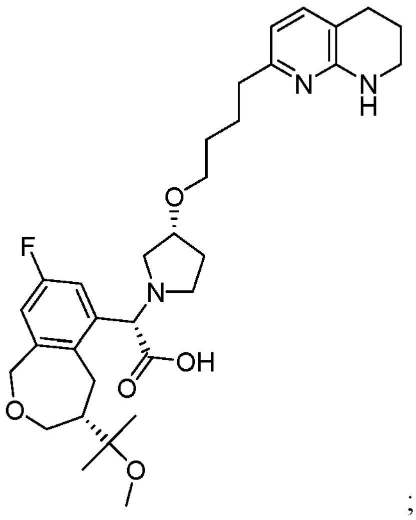
;

or a pharmaceutically acceptable salt thereof; or
selected from the group consisting of:

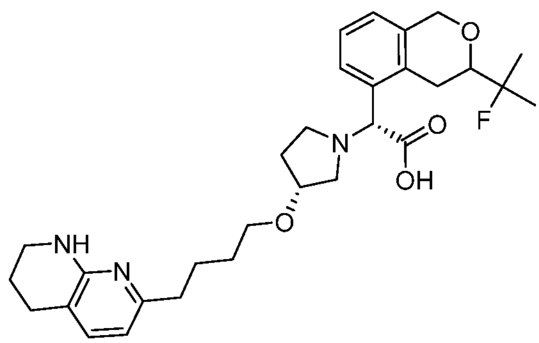
,
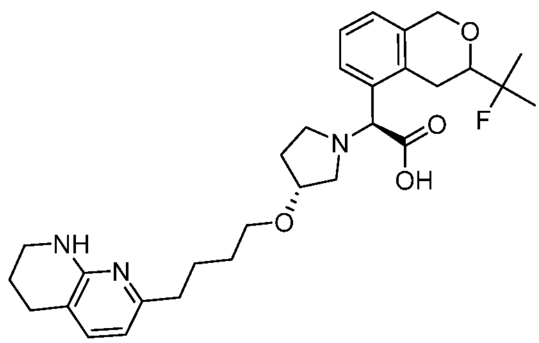
,

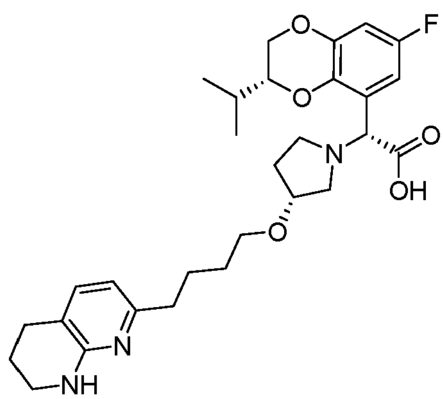
,
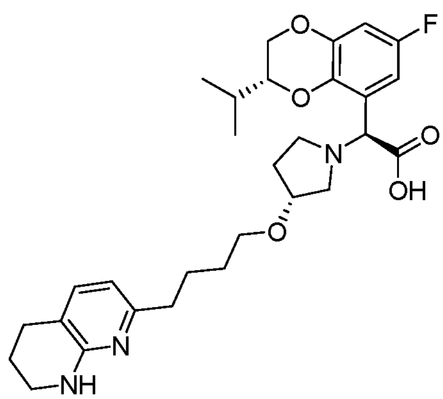
,

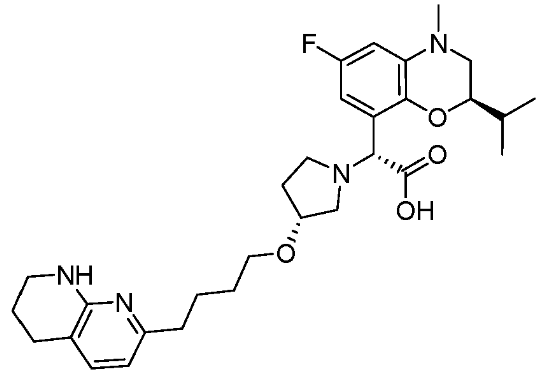

,

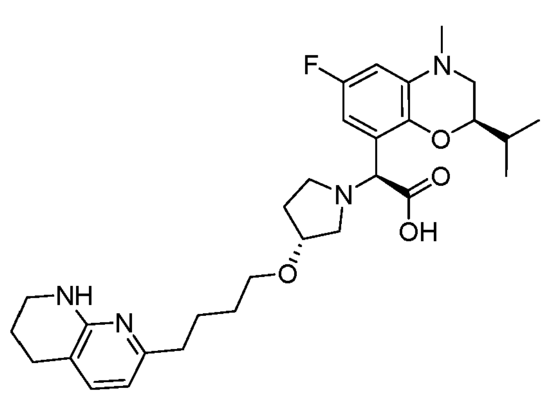

,

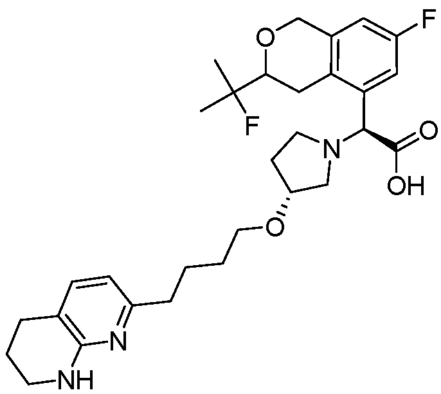

,

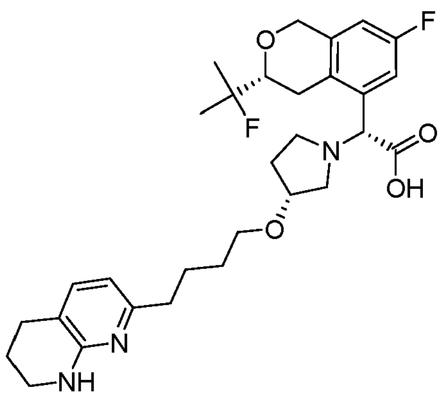

,

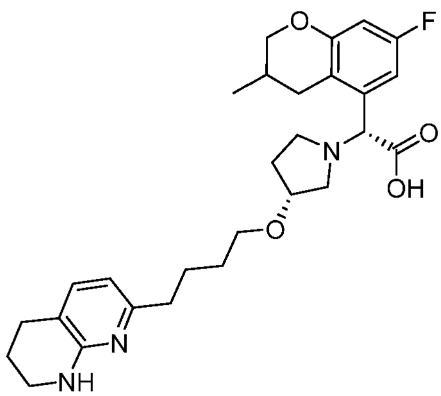

,

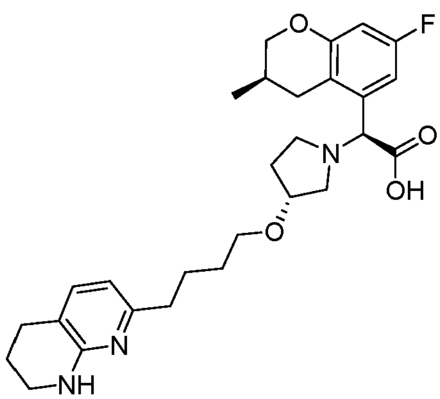

,

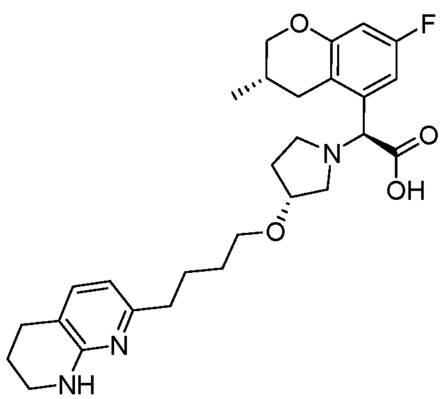

,

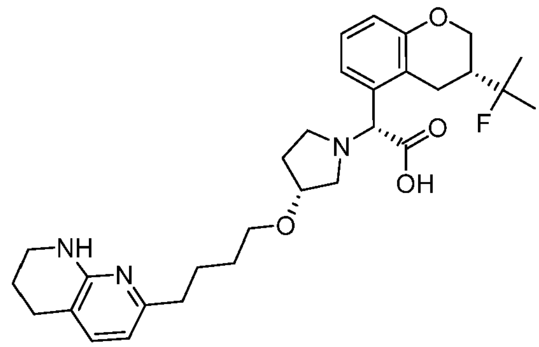

,

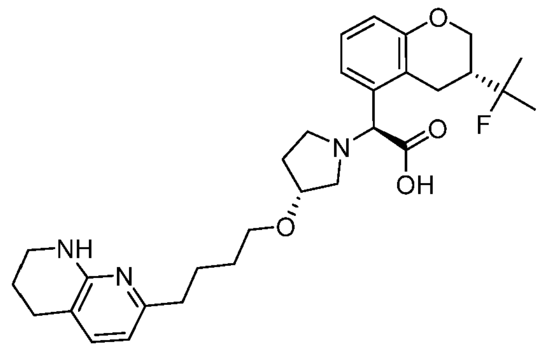

,

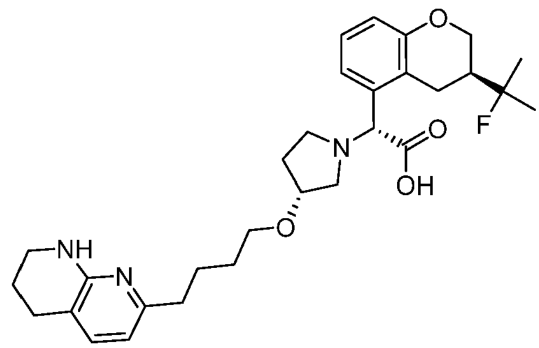

,

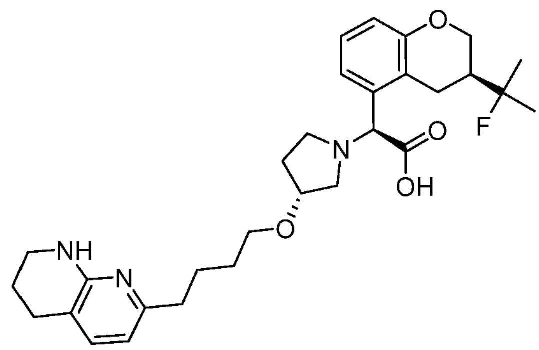

,

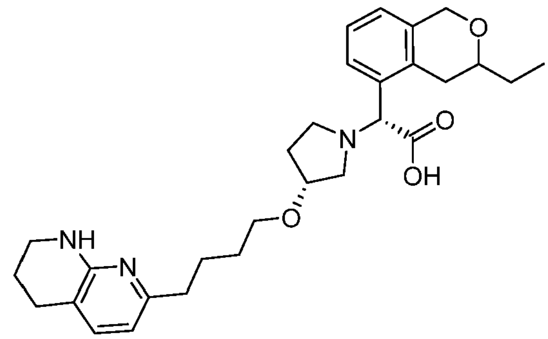

,

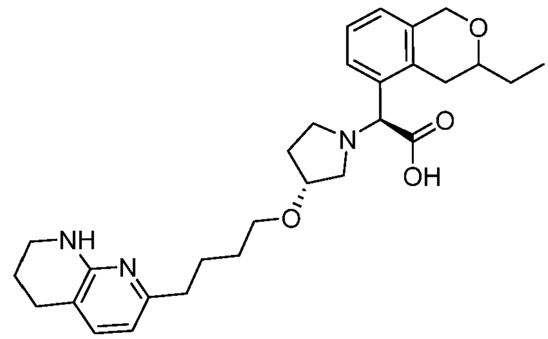

,

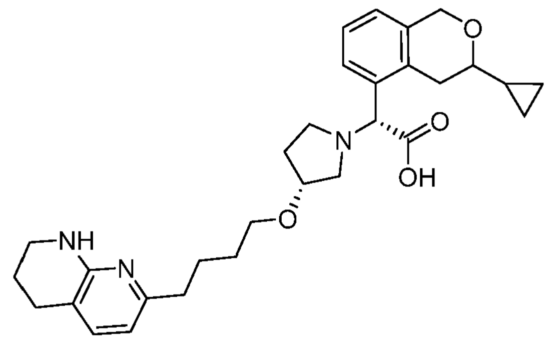

,

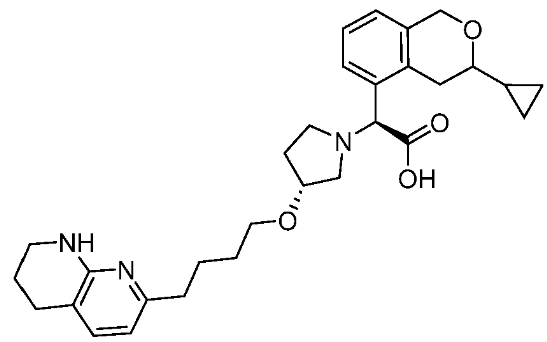

,

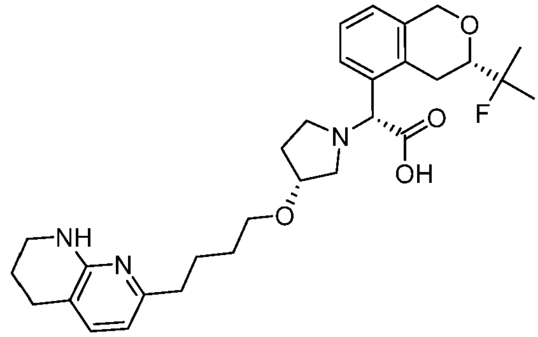

,

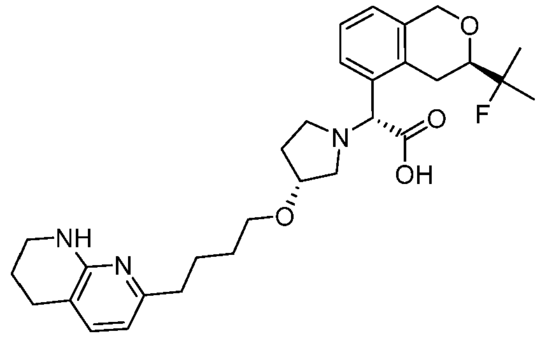

,

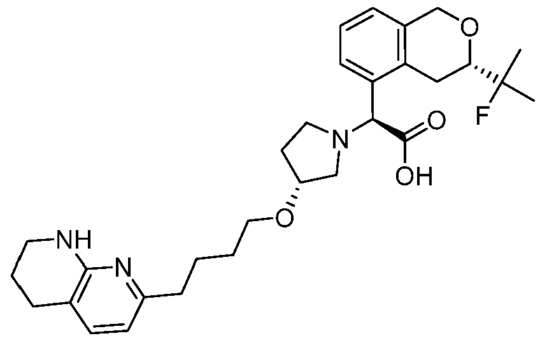

,

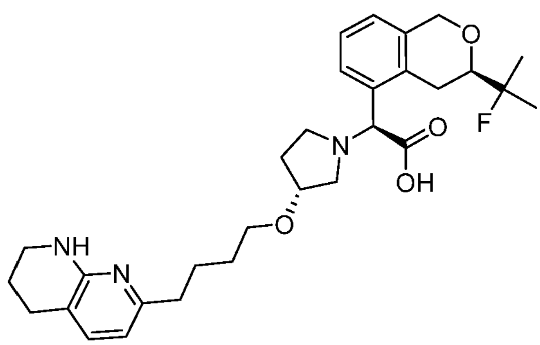

,

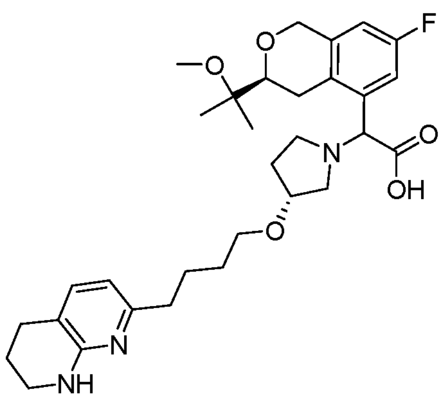

,

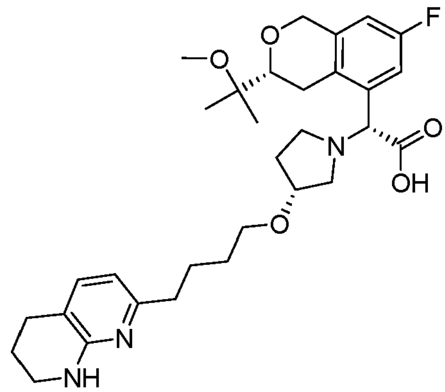,

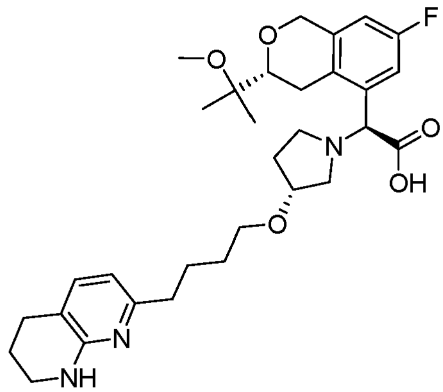,

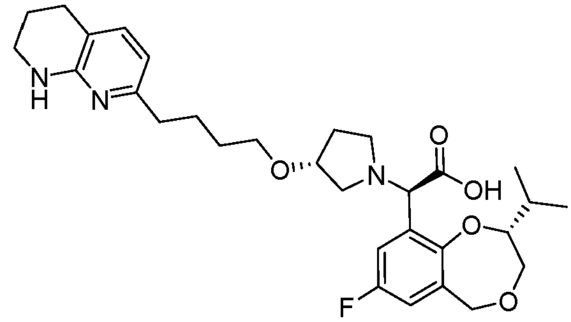,

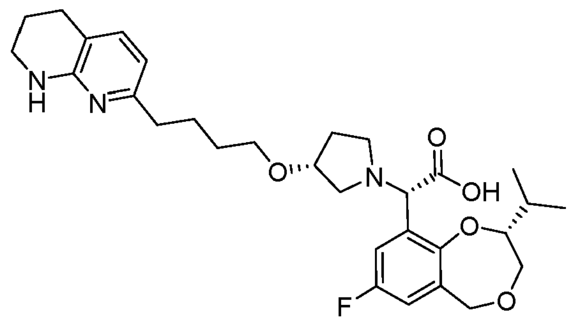,

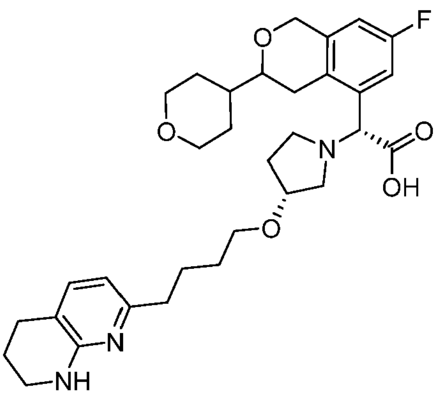,

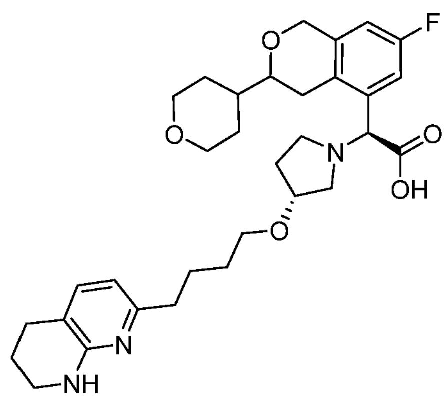,

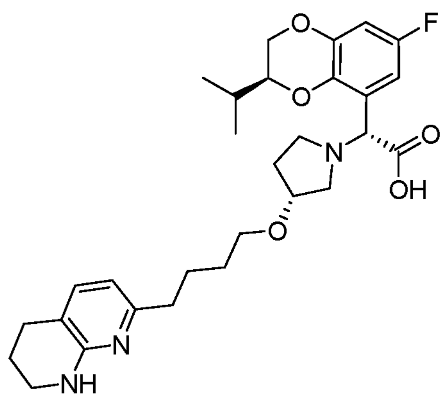,

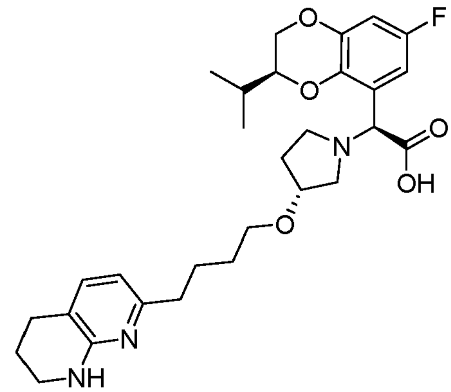

,

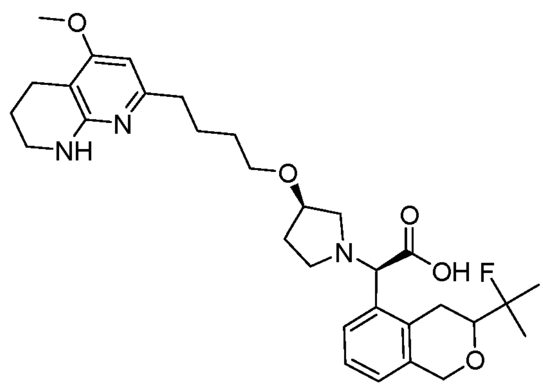

,

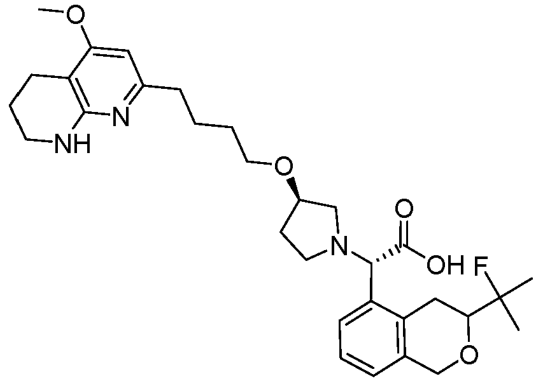

,

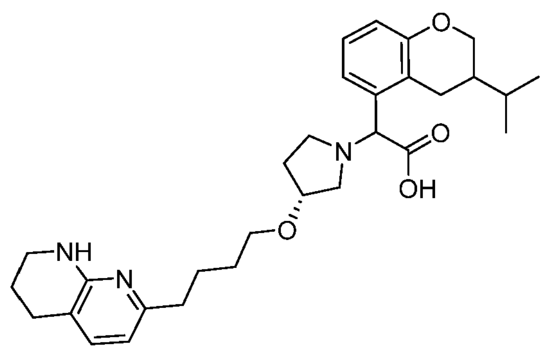

,

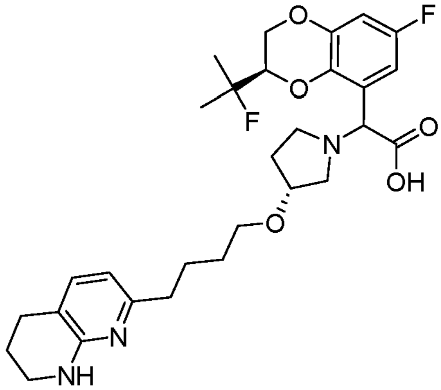

,

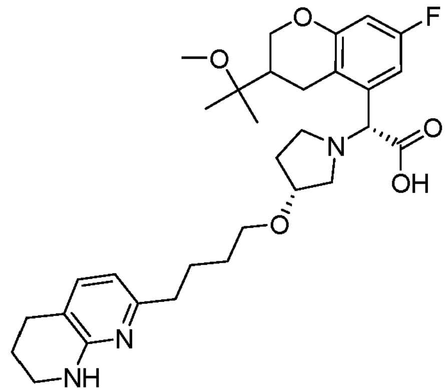

,

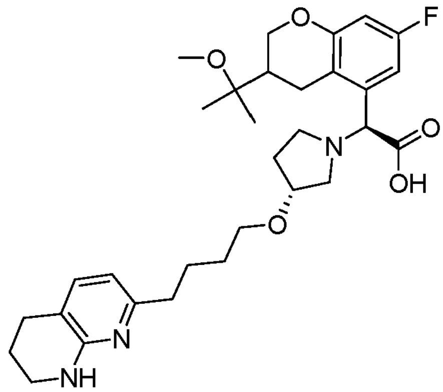

,

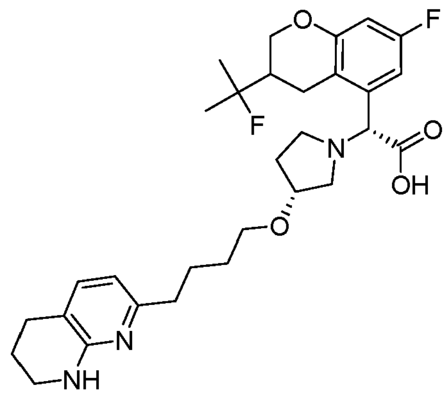
,
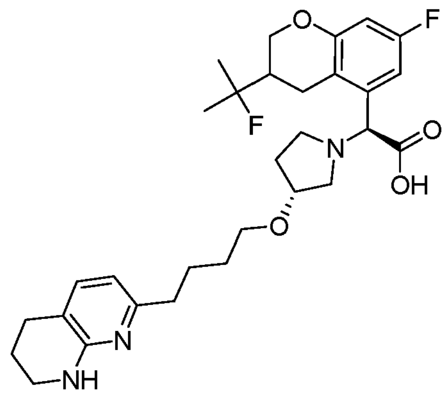
,

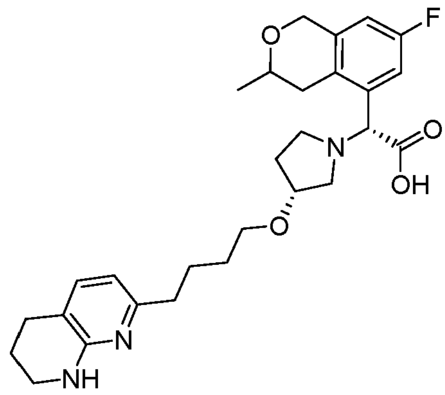
,
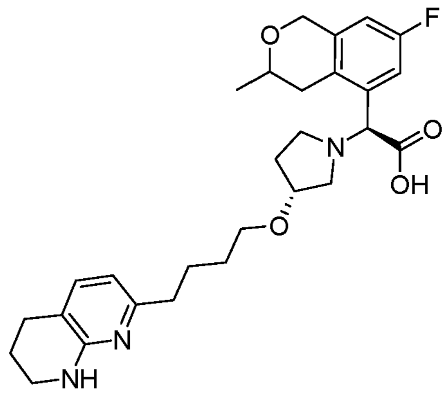
, and

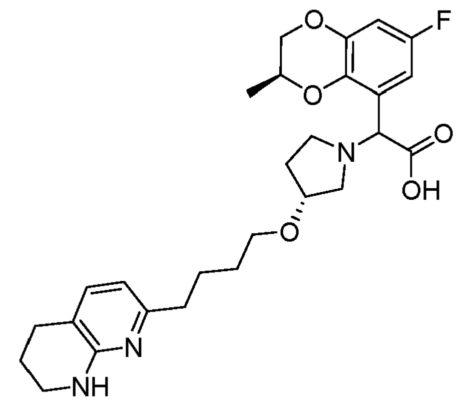
;

or a pharmaceutically acceptable salt thereof; or
selected from the group consisting of:

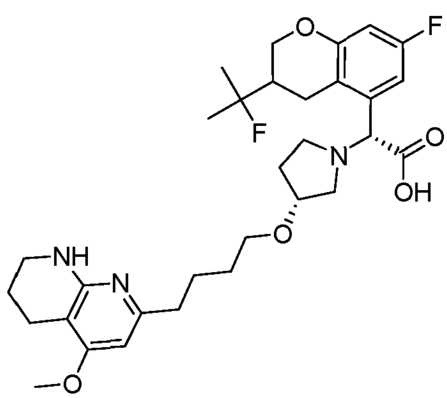
,
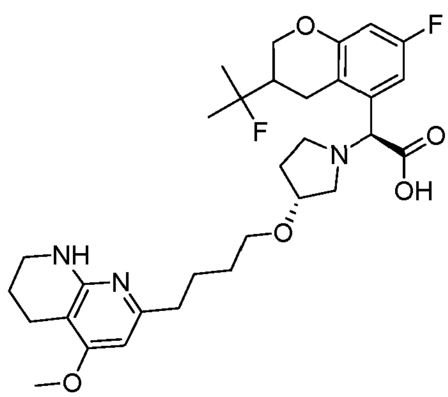
,

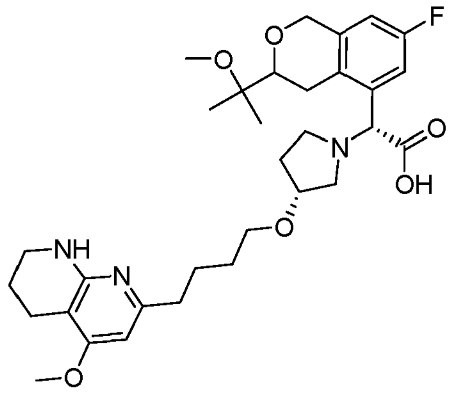
,
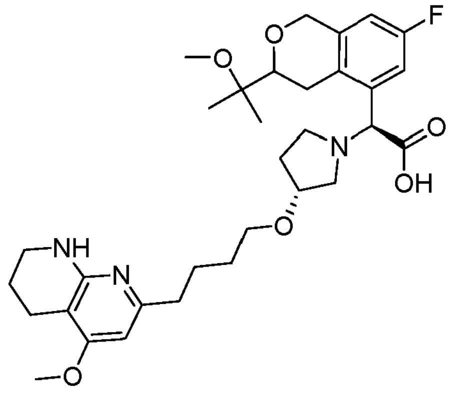
,

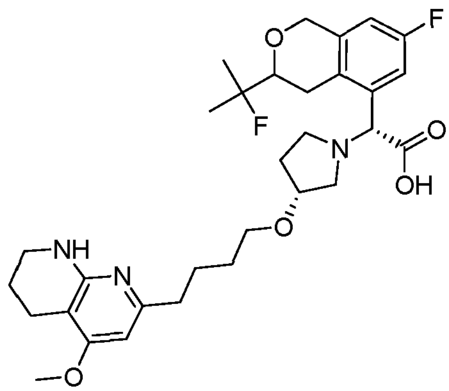
,
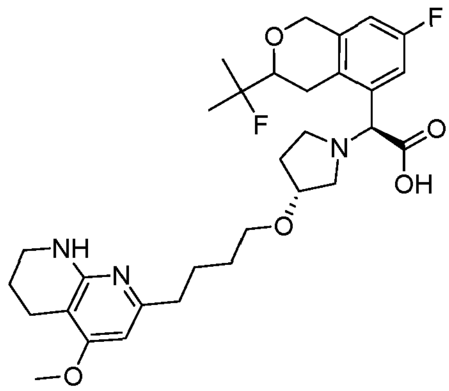
,

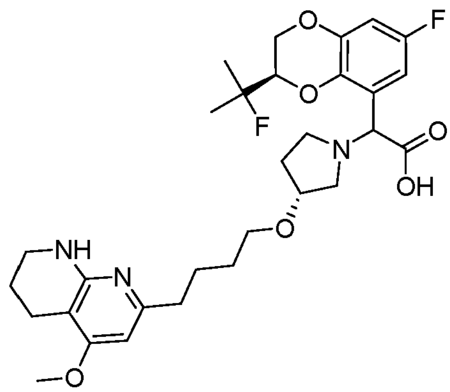
,
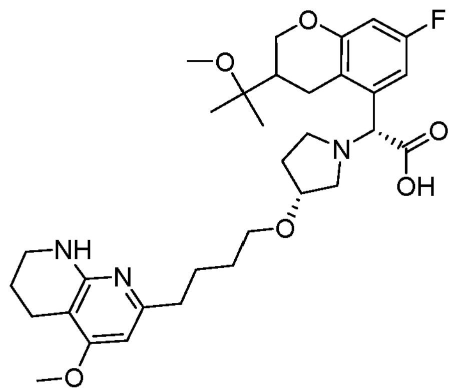
,

and

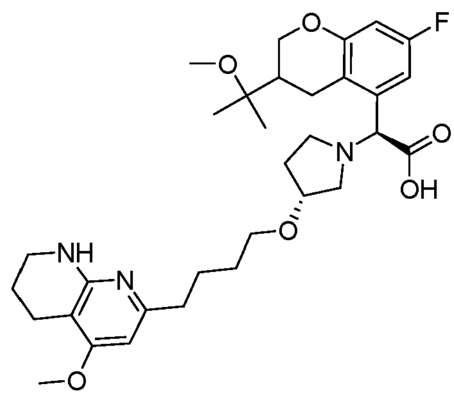
;

or a pharmaceutically acceptable salt thereof; or selected from the group consisting of:

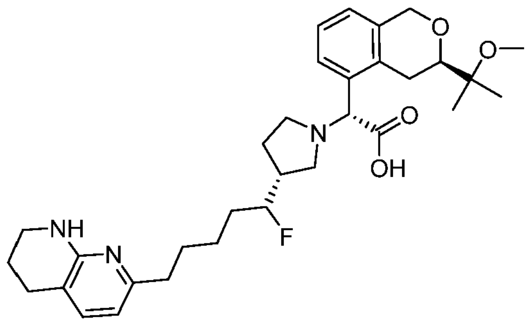

,

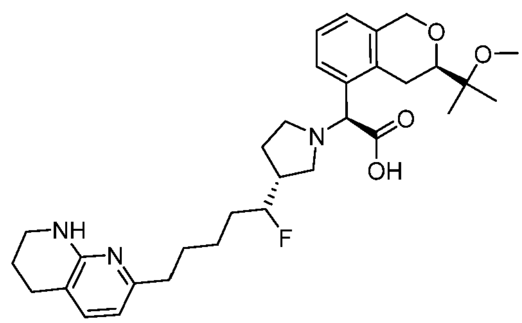

,

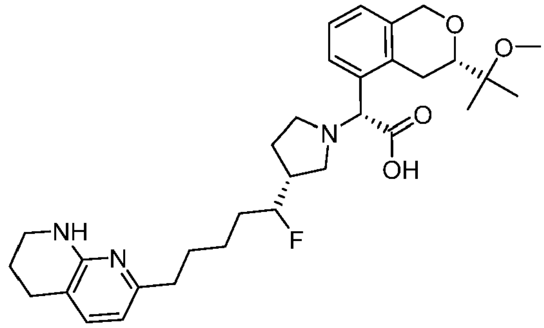

,

and

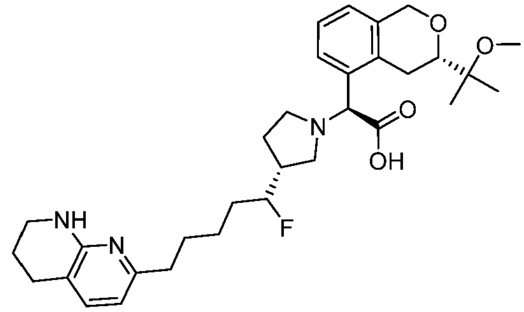

;

or a pharmaceutically acceptable salt thereof; or selected from the group consisting of:

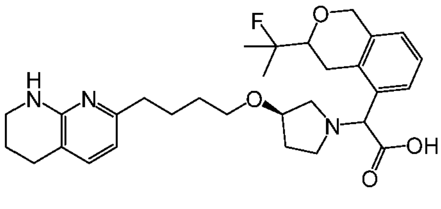

,

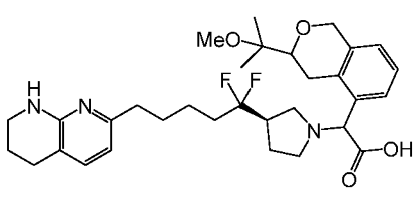

,

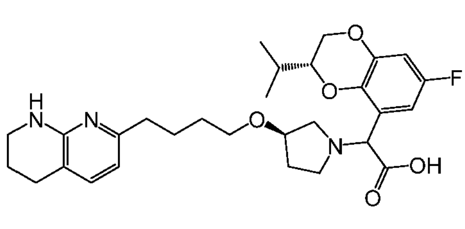

,

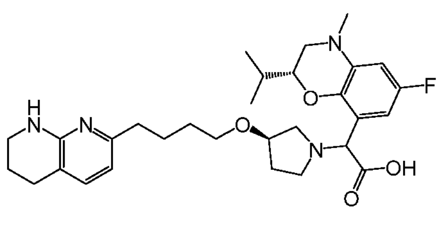

,

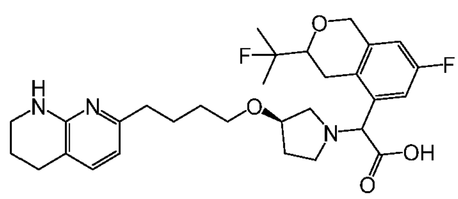
,
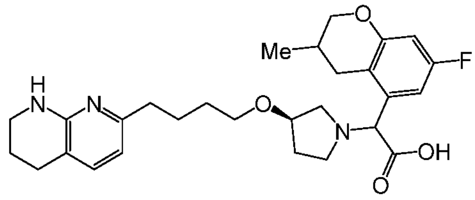
,

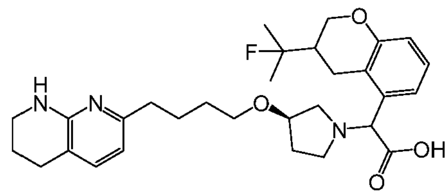
, and
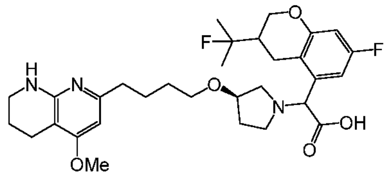
;

or a pharmaceutically acceptable salt thereof; or
selected from the group consisting of:

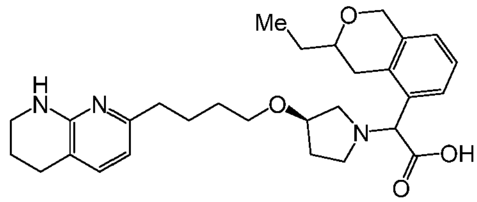
,
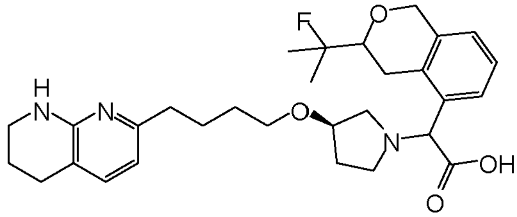
,

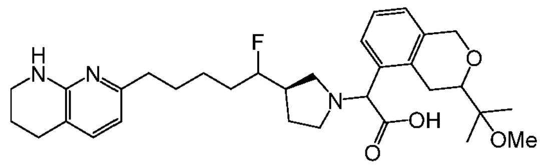
,
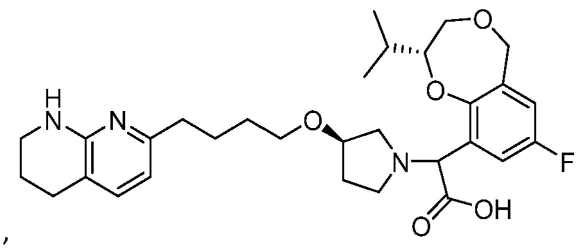
,

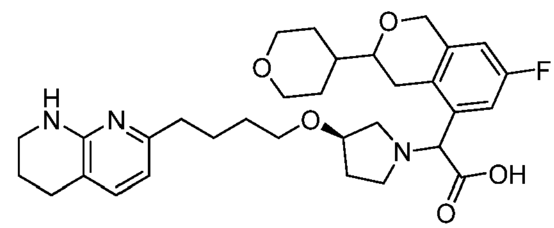
,
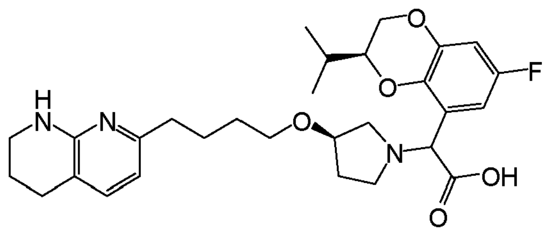
,

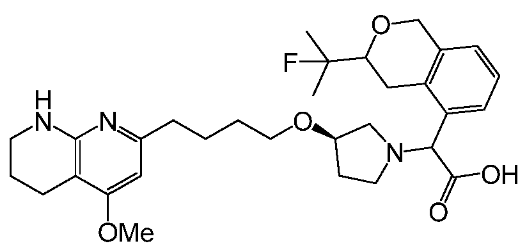
,
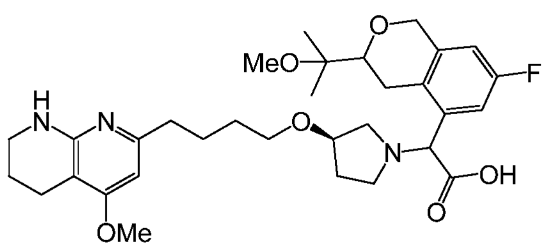
,

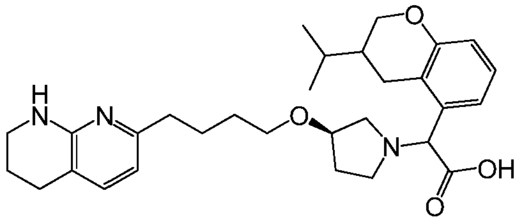, 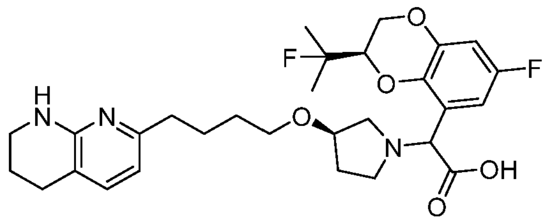,

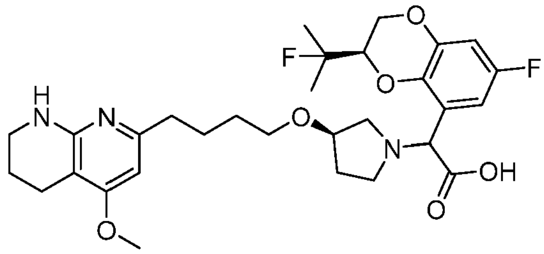, 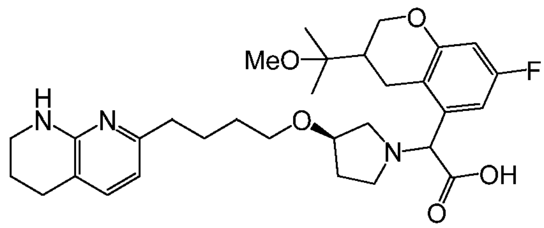,

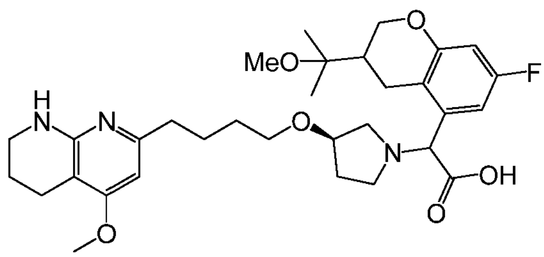, 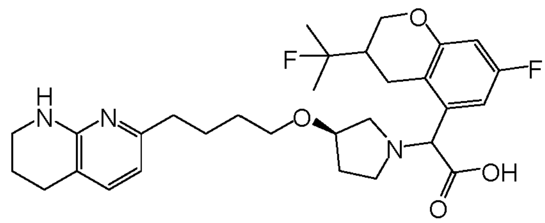,

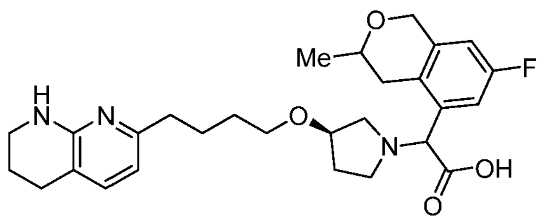, and 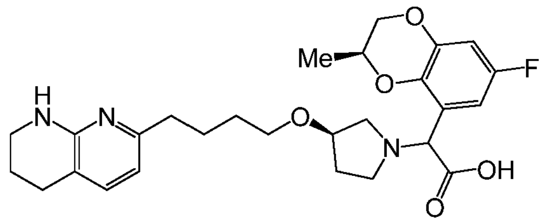;

or a pharmaceutically acceptable salt thereof.

**2.** The compound of claim 1 selected from the group consisting of:

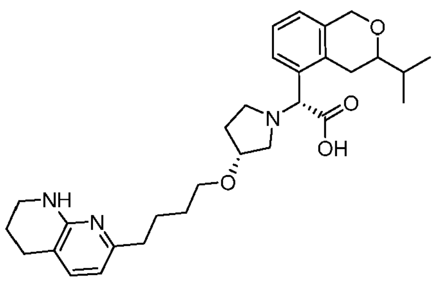, 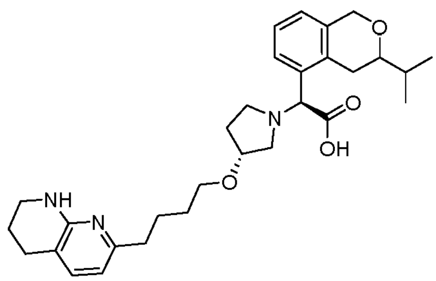,

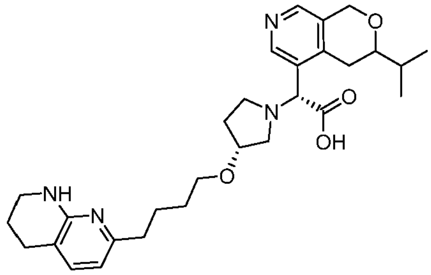, 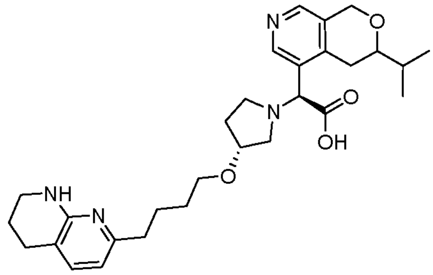,

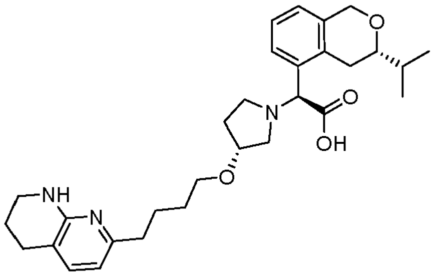, 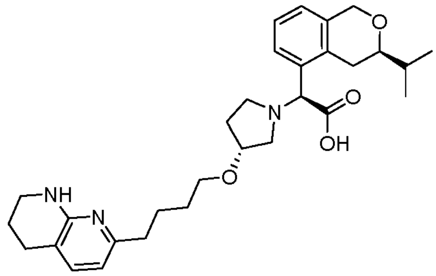,

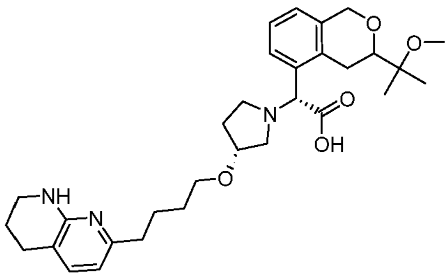, and 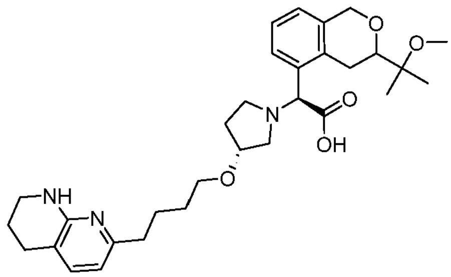;

or a pharmaceutically acceptable salt thereof.

**3.** The compound of claim 1 selected from the group consisting of:

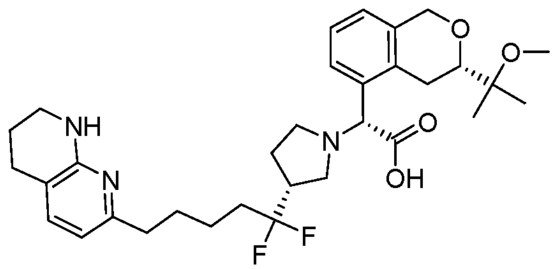,

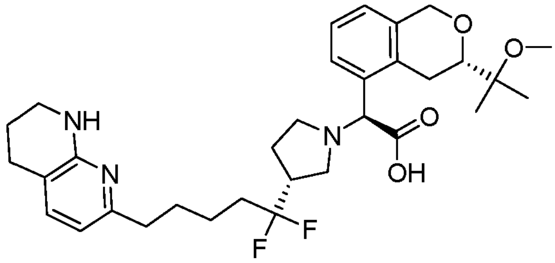,

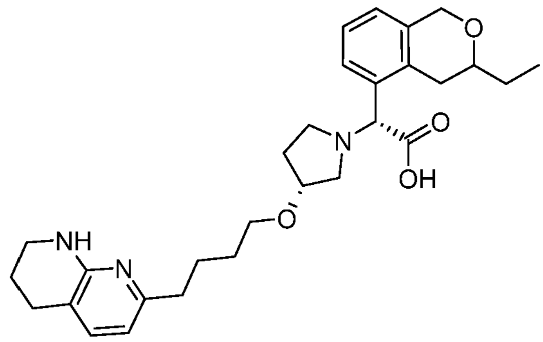

,

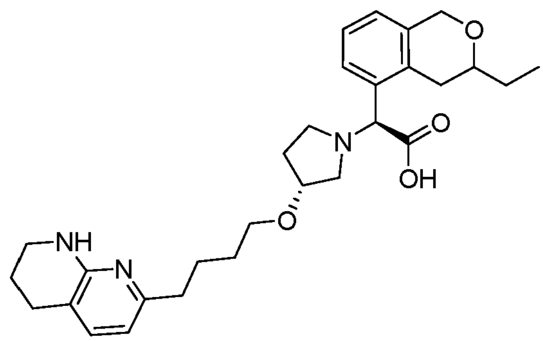

,

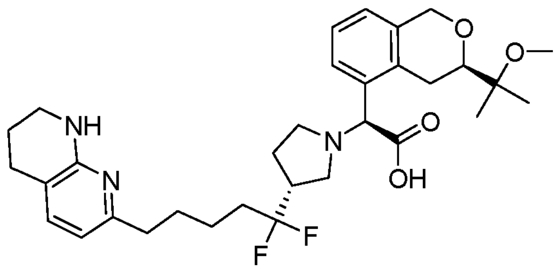

,

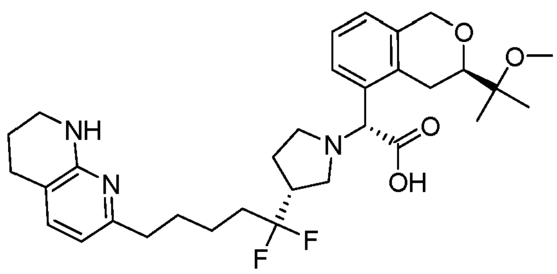

,

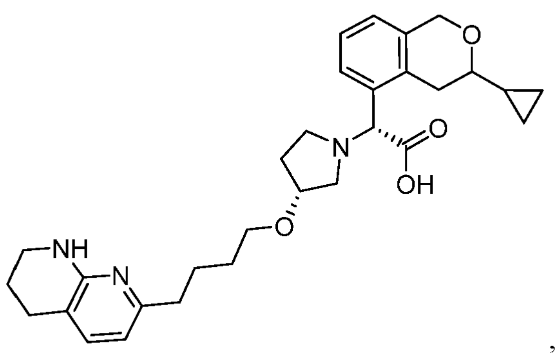

,

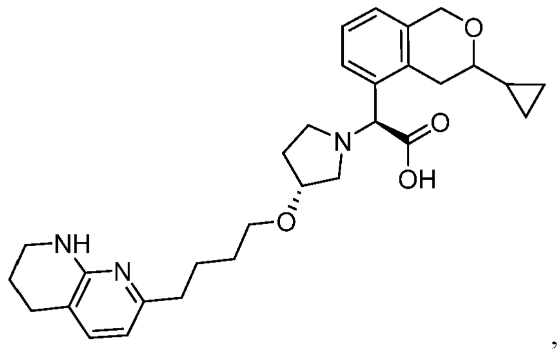

,

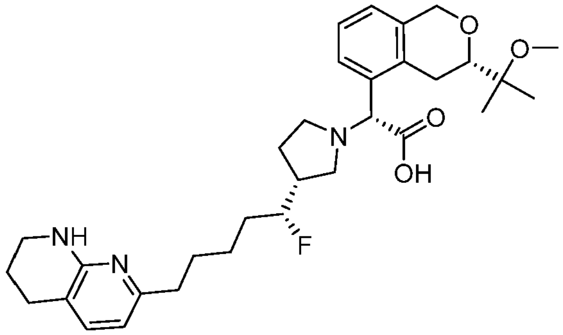

,

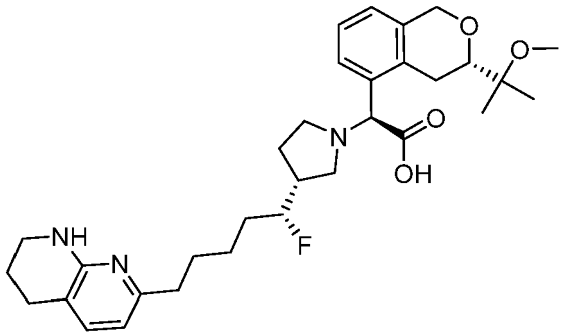

,

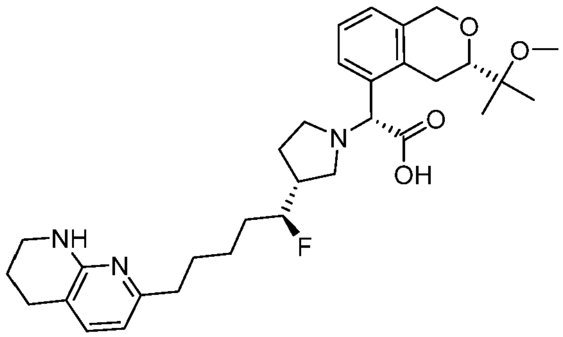

,

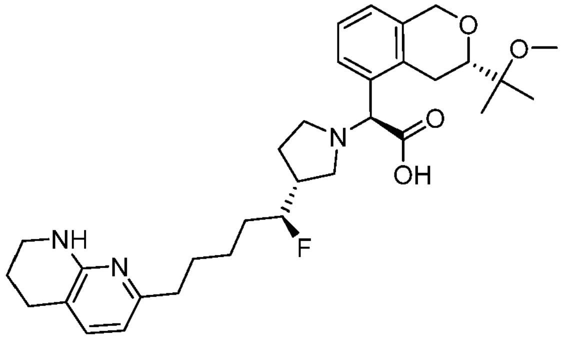

,

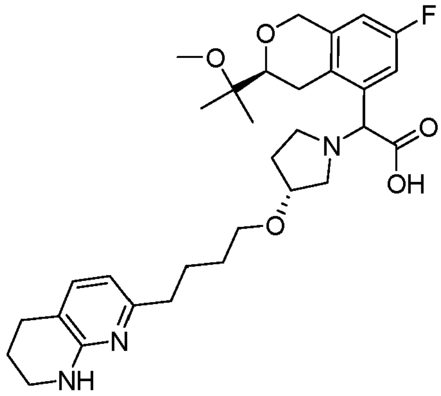

,

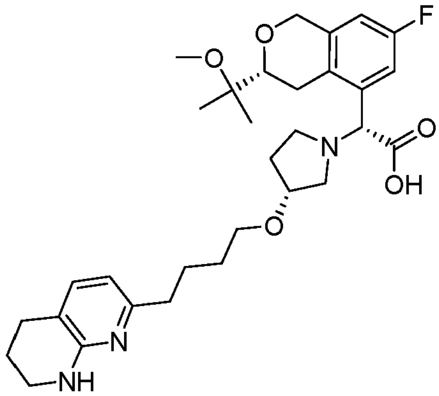

,

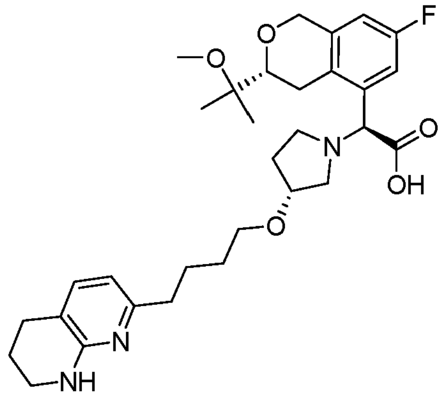

,

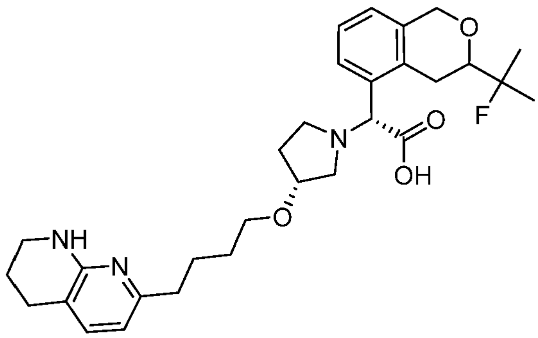

,

and

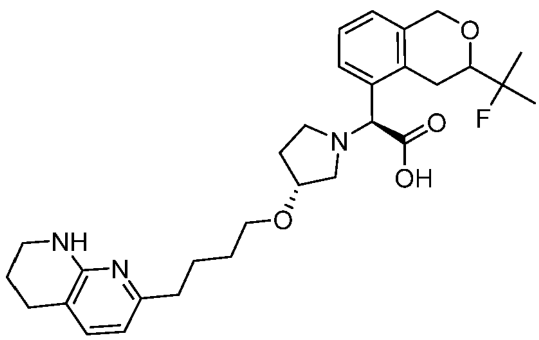

;

or a pharmaceutically acceptable salt thereof.

**4.** The compound of claim 1 selected from the group consisting of:

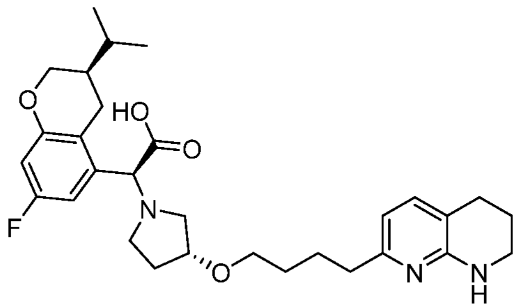, 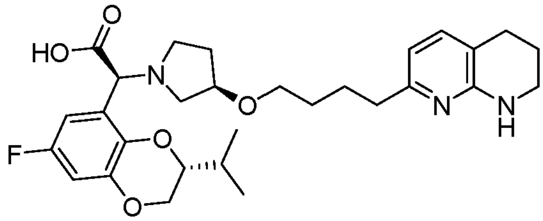,

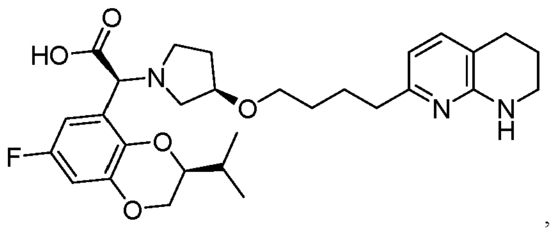, 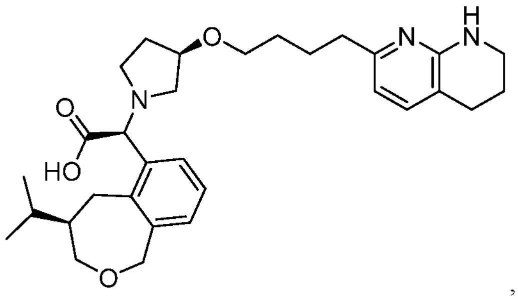,

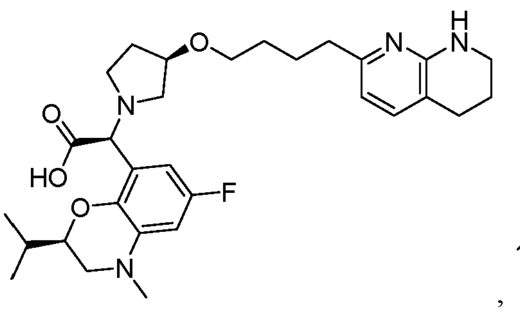, 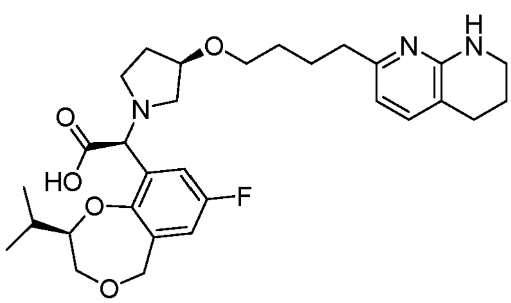,

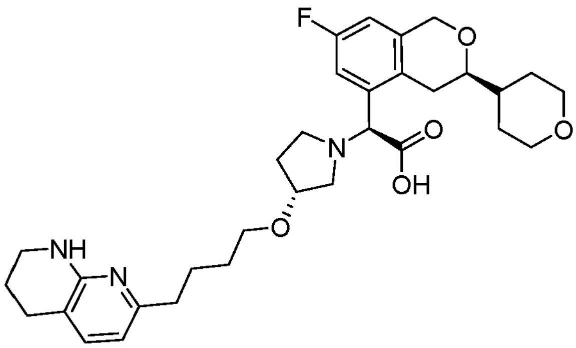, and 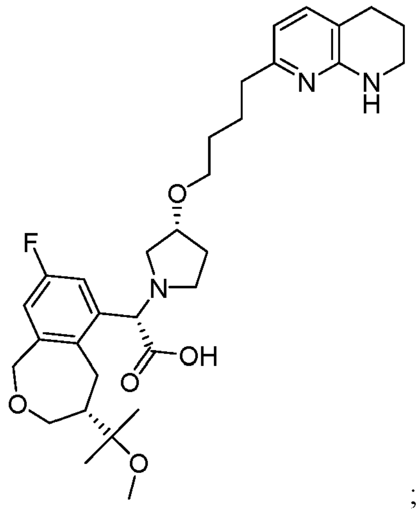;

or a pharmaceutically acceptable salt thereof.

5. The compound of claim 1 selected from the group consisting of:

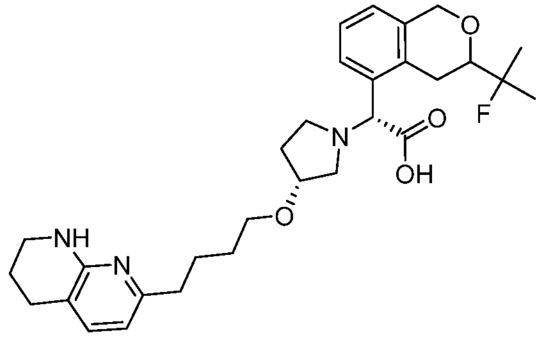,
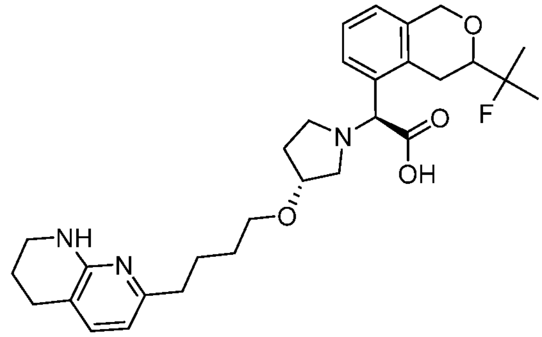,

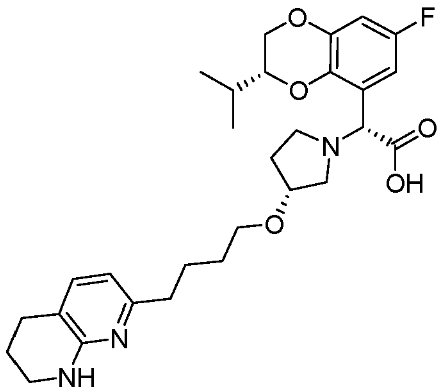,
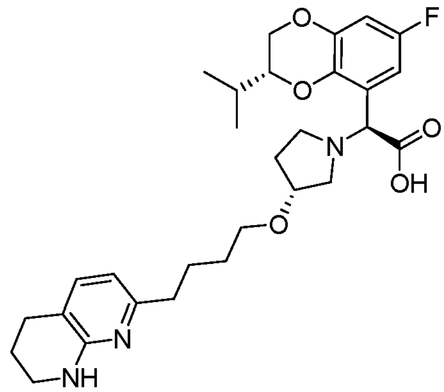,

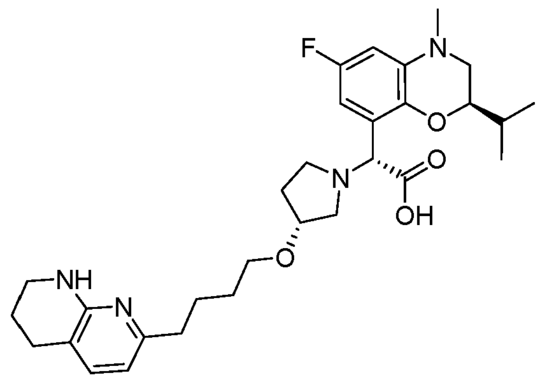,

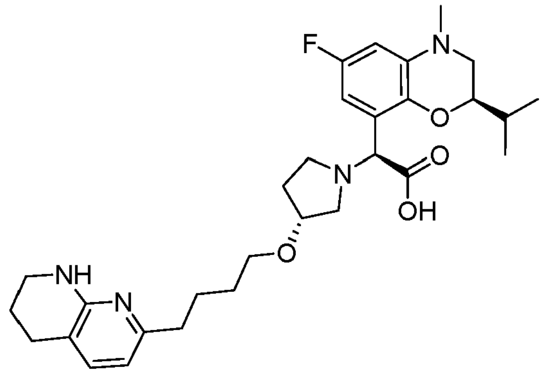,
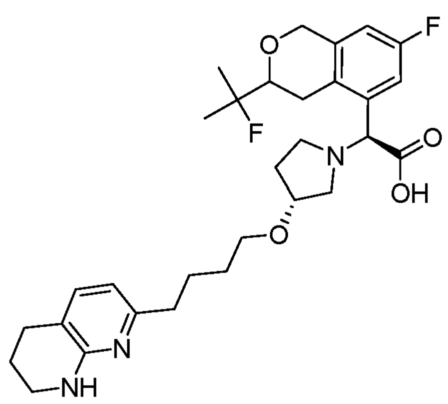,

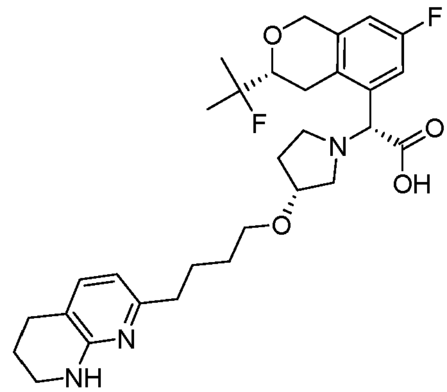
,
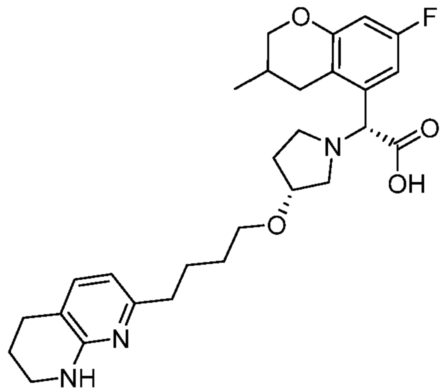
,
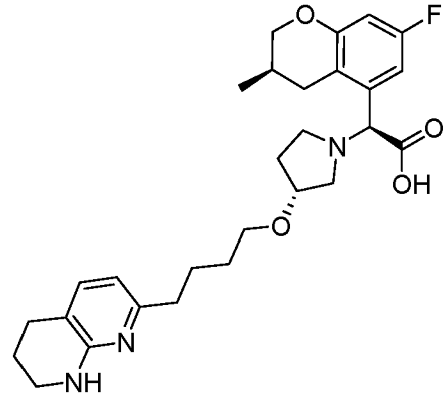
,
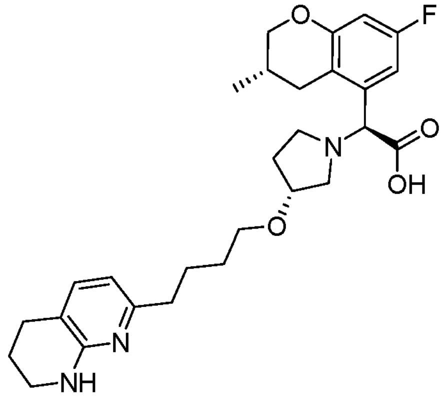
,
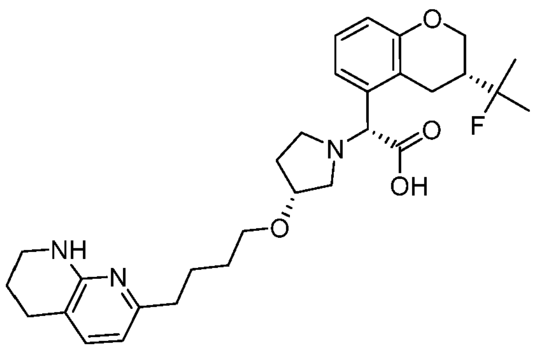
,
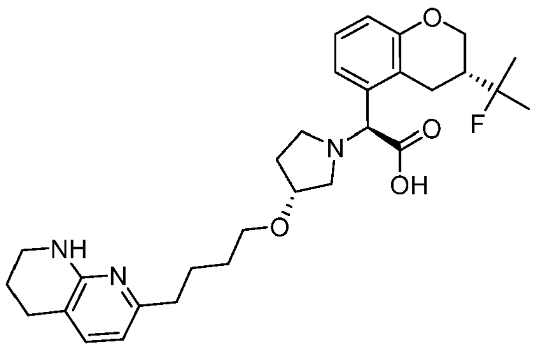
,

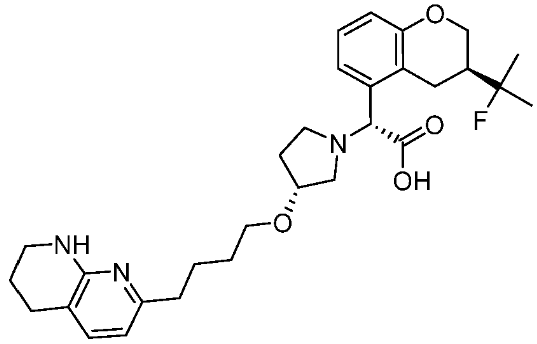

,

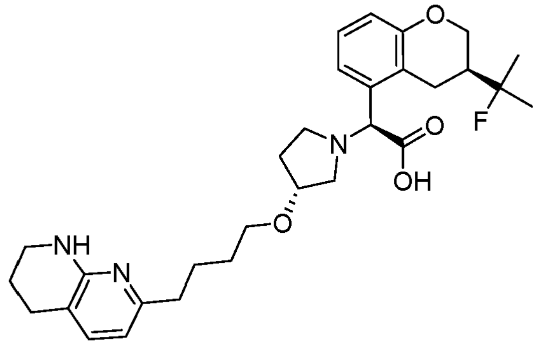

,

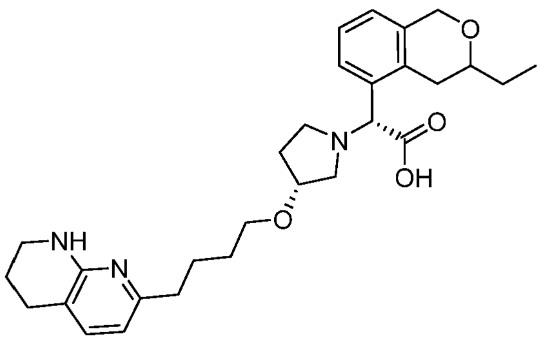

,

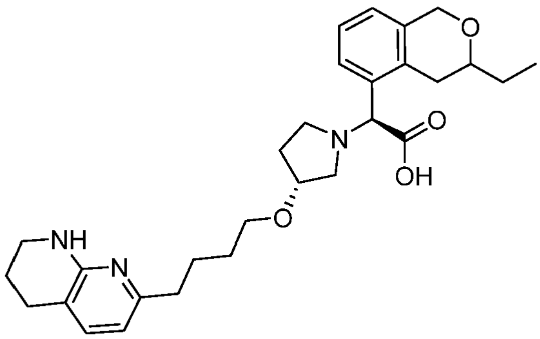

,

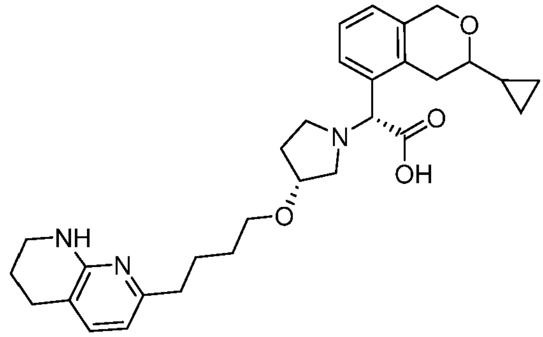

,

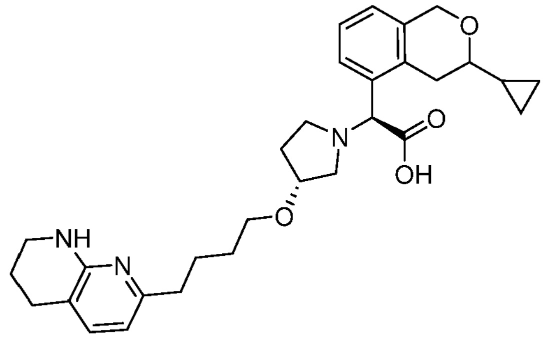

,

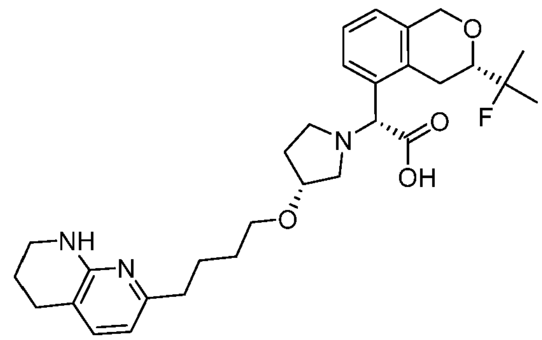

,

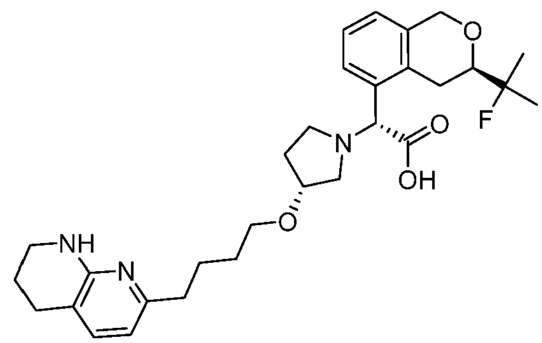

,

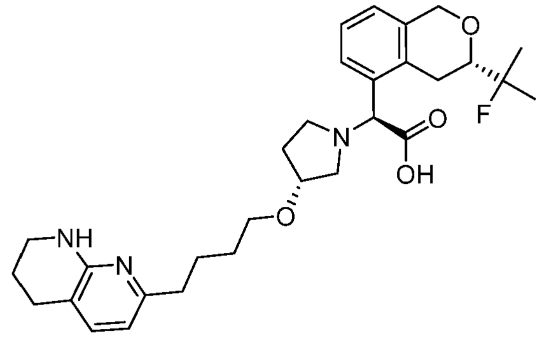

,

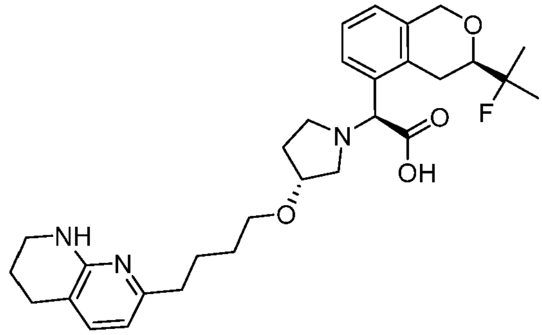

,

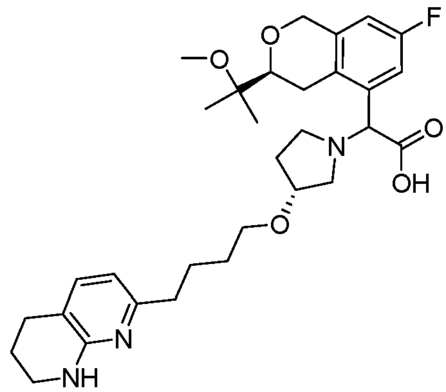

,

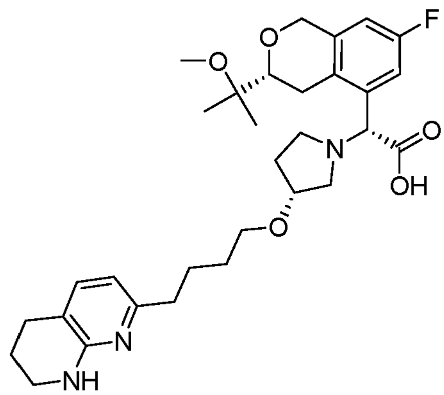

,

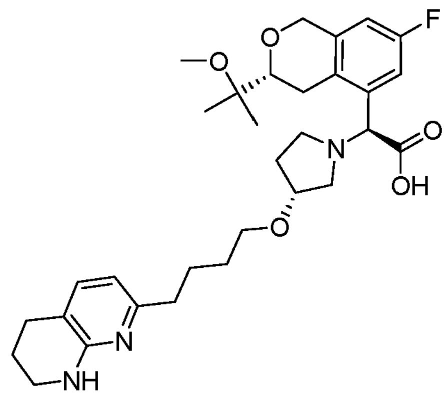

,

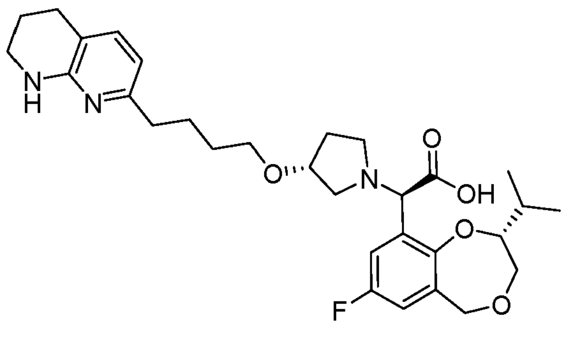

,

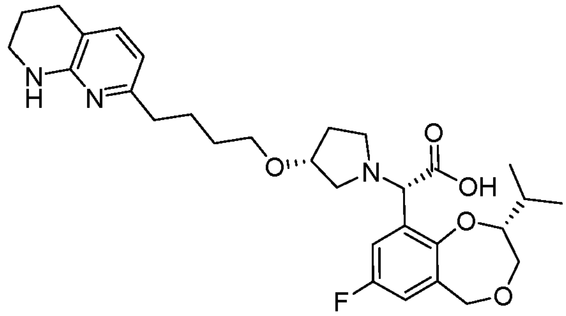, 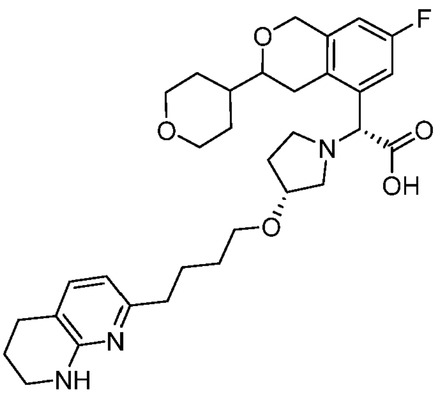,

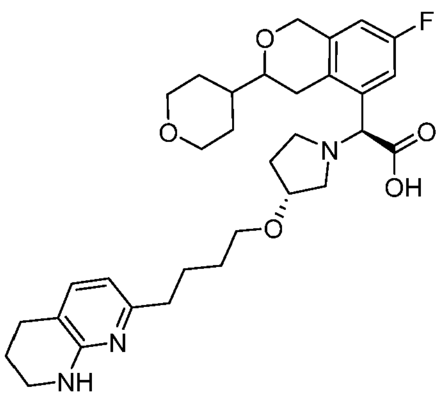, 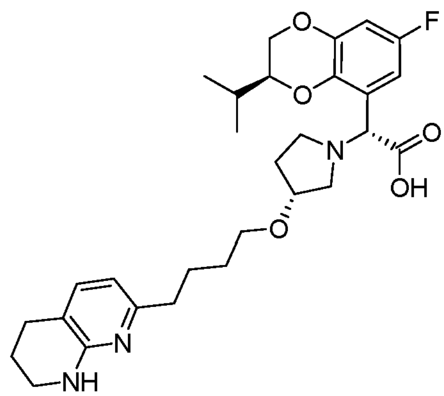,

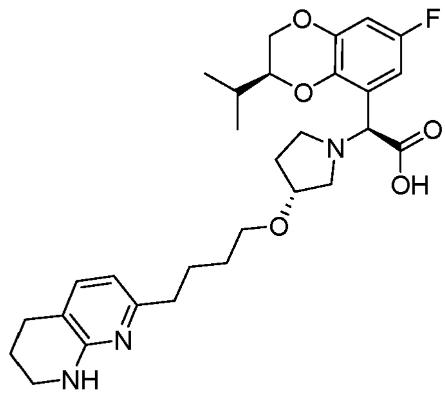, 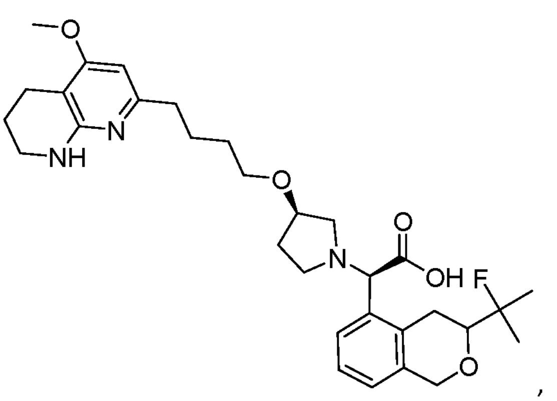,

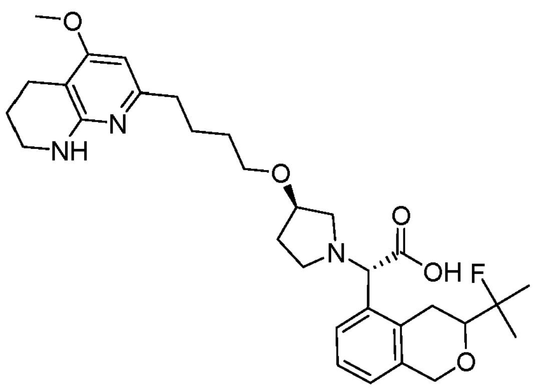,

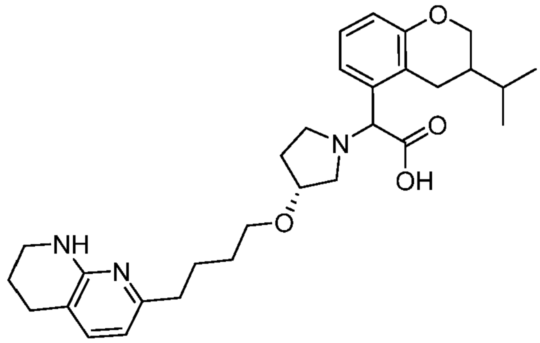

,

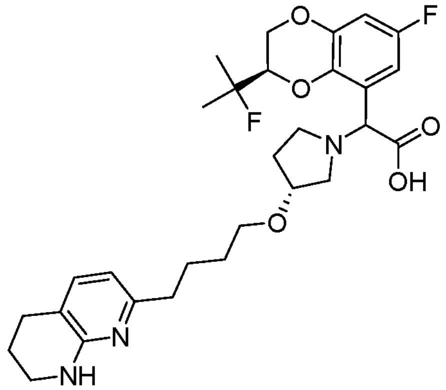

,

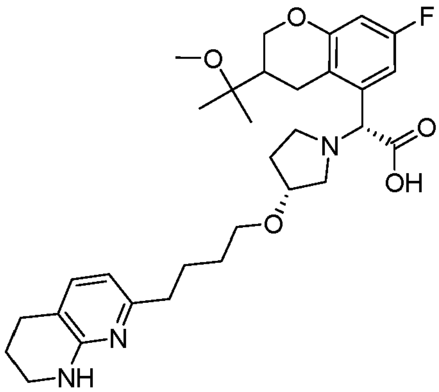

,

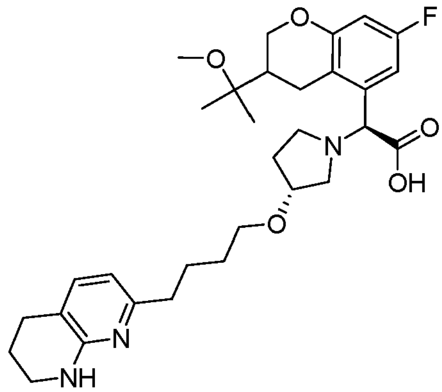

,

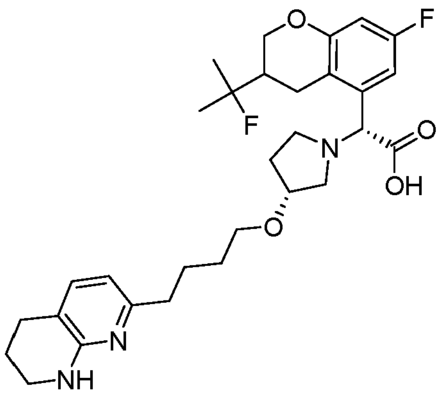

,

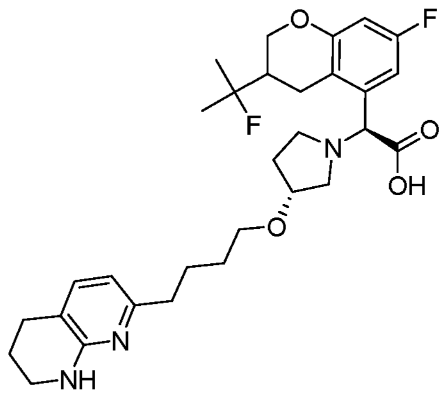

,

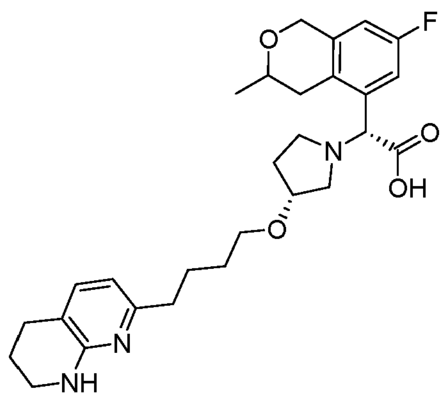

,

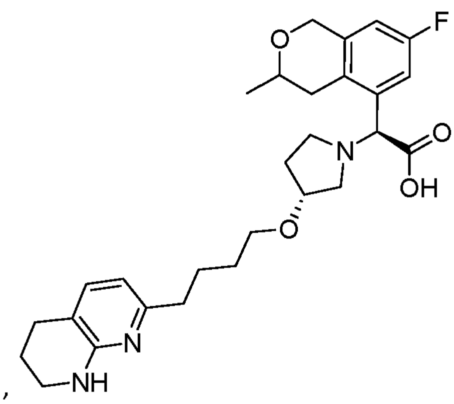

,

and

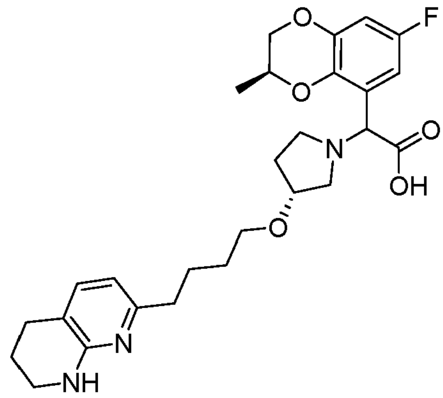

;

or a pharmaceutically acceptable salt thereof.

**6.** The compound of claim 1 selected from the group consisting of:

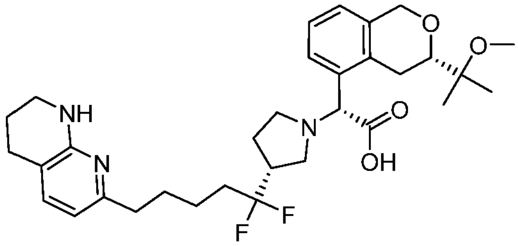

,

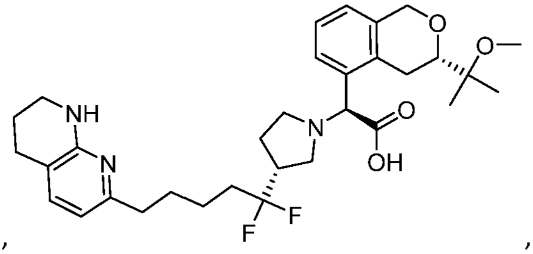

,

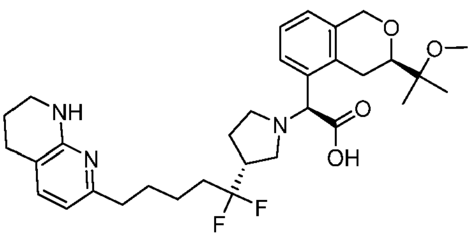

, and

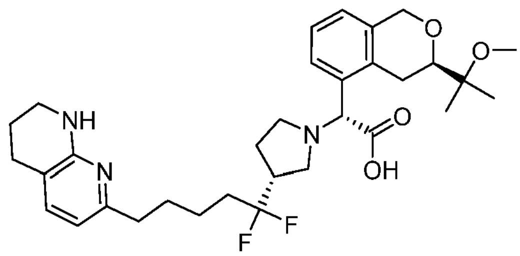

;

or a pharmaceutically acceptable salt thereof.

**7.** The compound of claim 1 selected from the group consisting of:

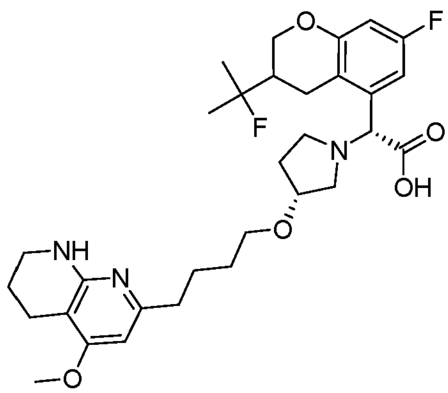

,

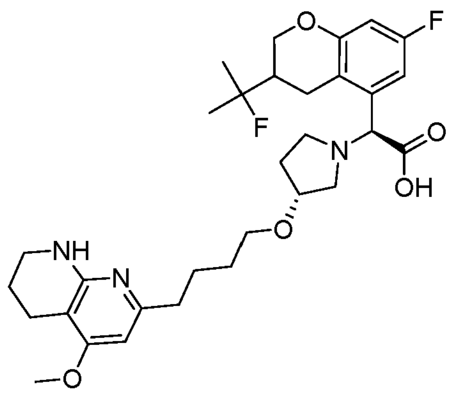

,

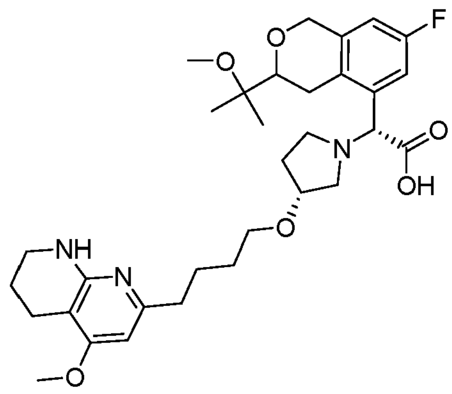,
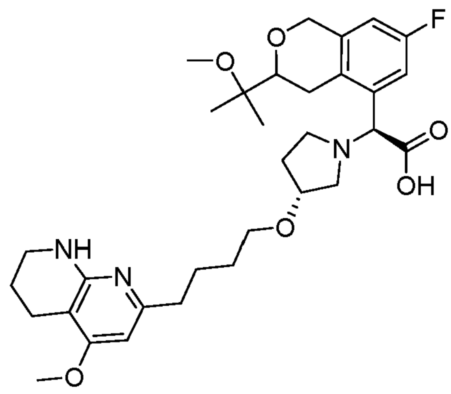,

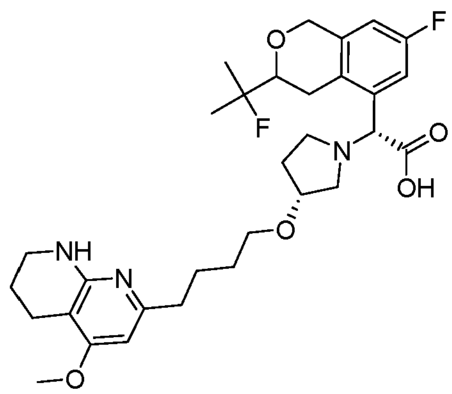,
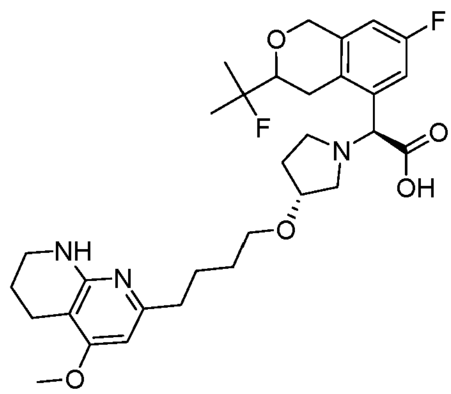,

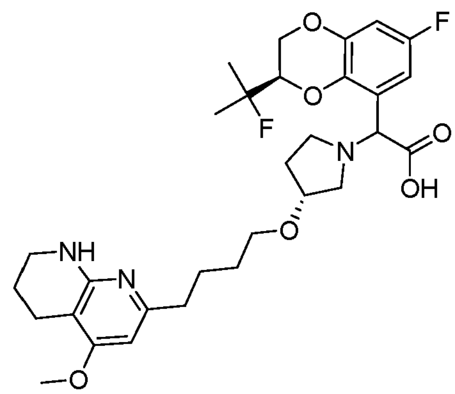,
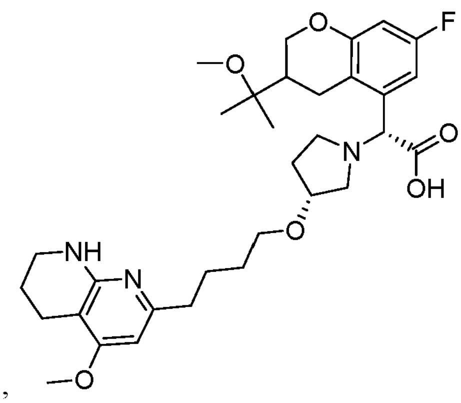,

and

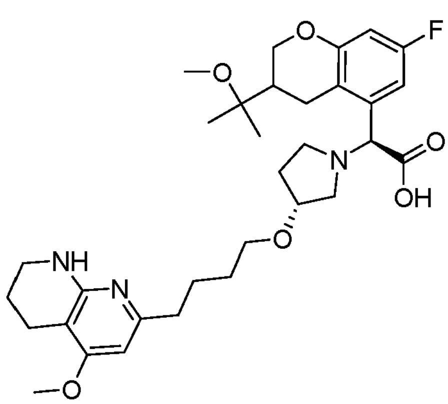;

or a pharmaceutically acceptable salt thereof.

**8.** The compound of claim 1 selected from the group consisting of:

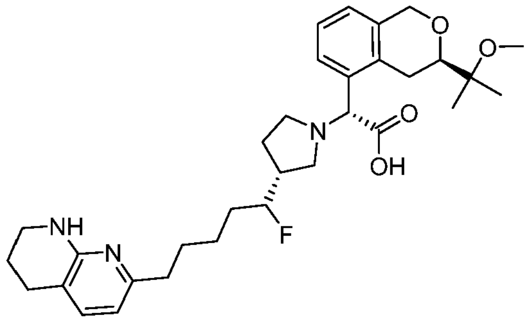,
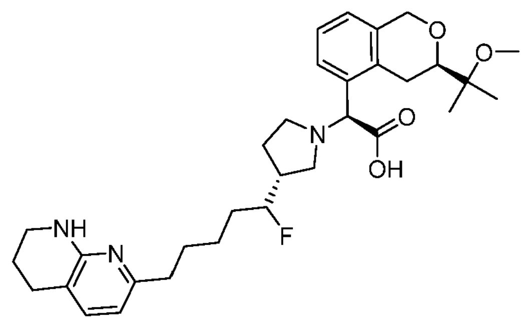,

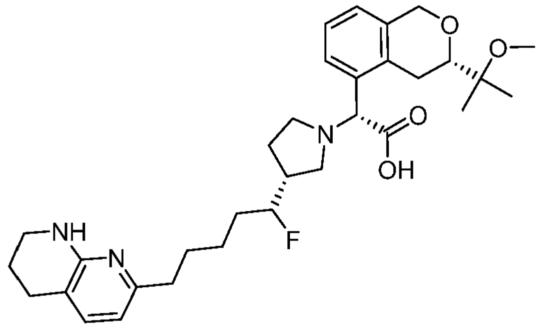,

and

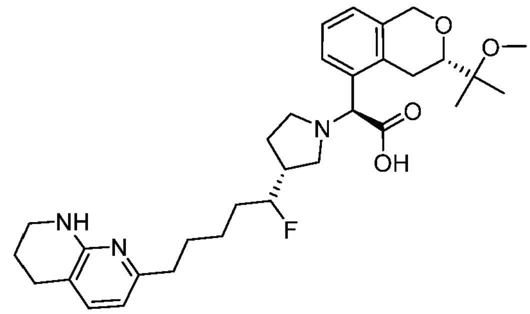;

or a pharmaceutically acceptable salt thereof.

**9.** The compound of claim 1 selected from the group consisting of:

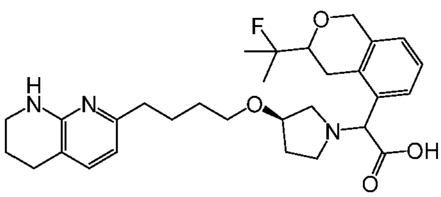,
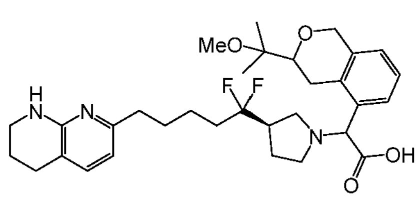,

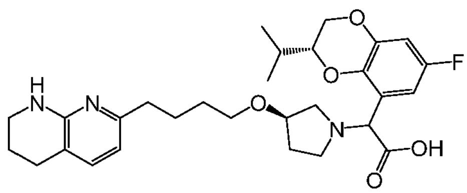, 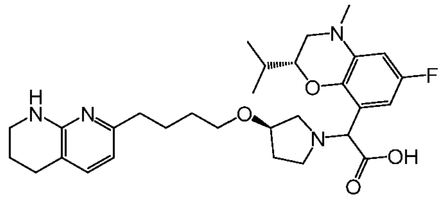,

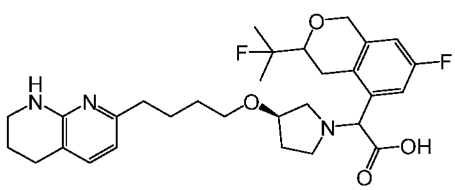, 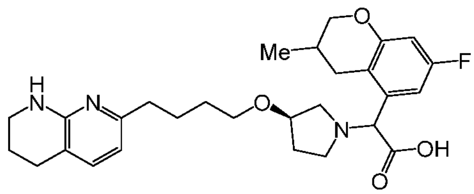,

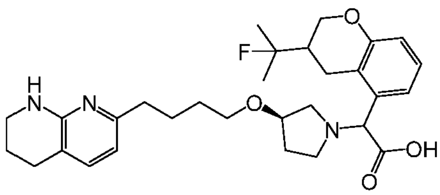, and 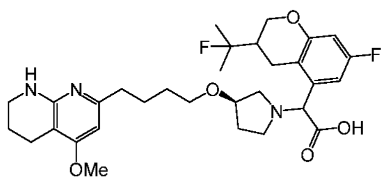;

or a pharmaceutically acceptable salt thereof.

**10.** The compound of claim 1 selected from the group consisting of:

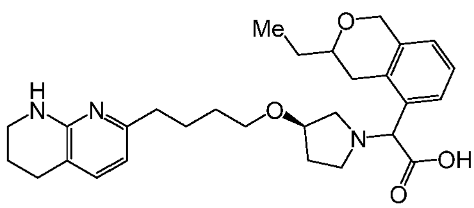, 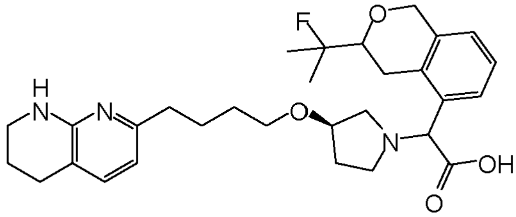,

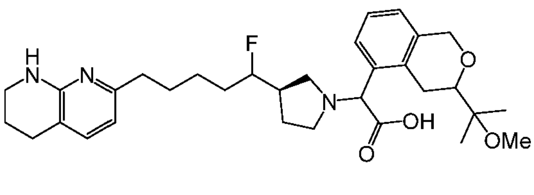, 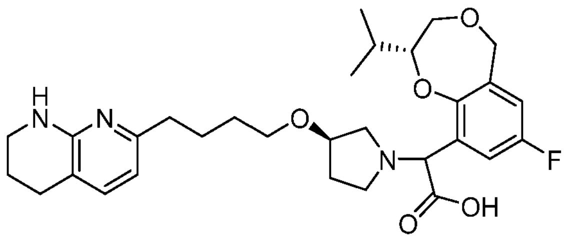,

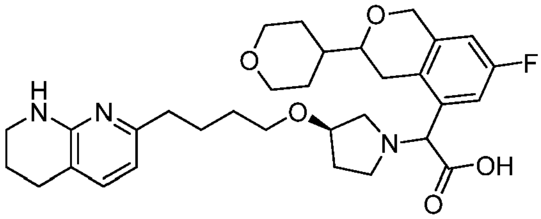, 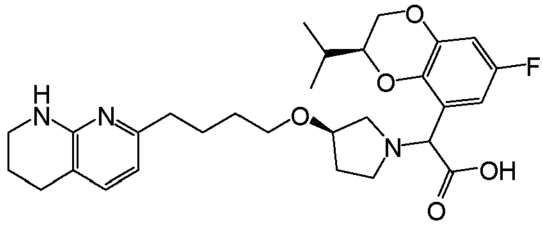,

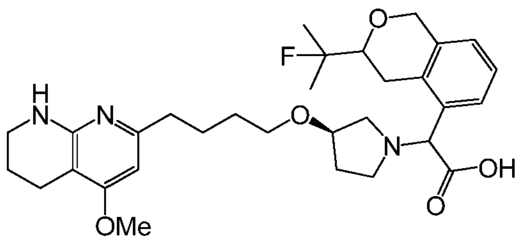,
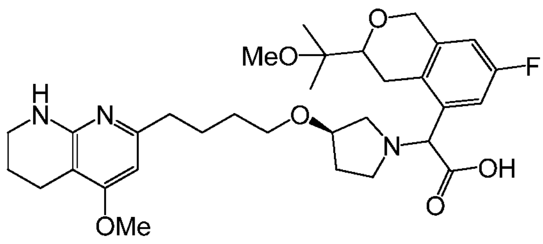,
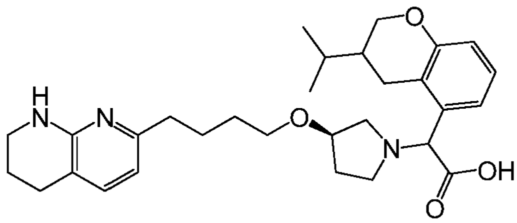,
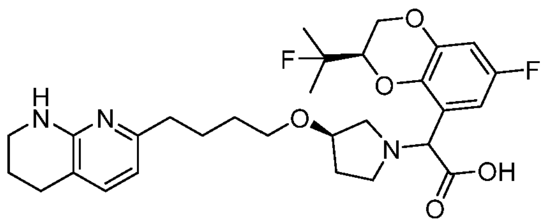,
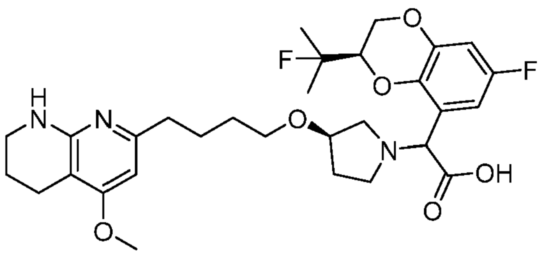,
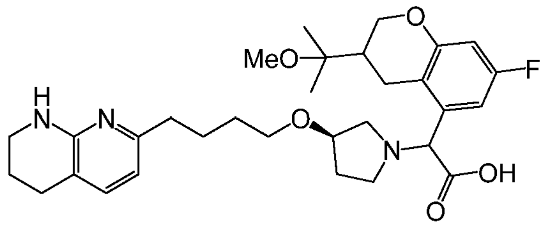,
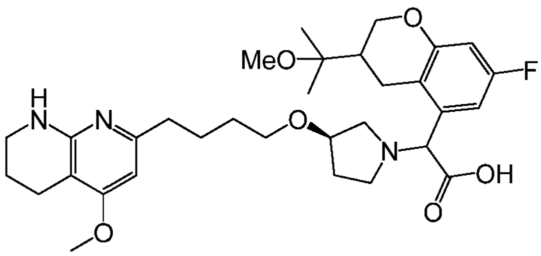,
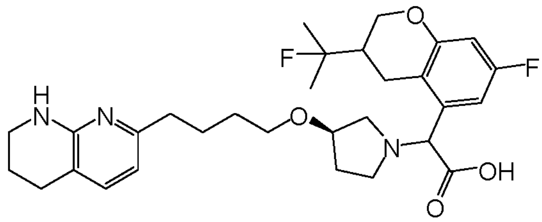,
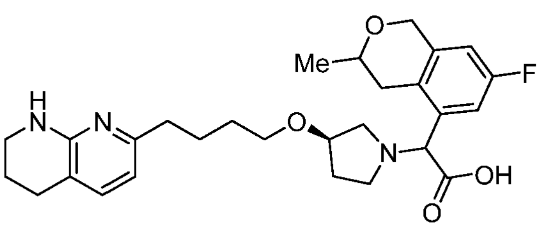, and
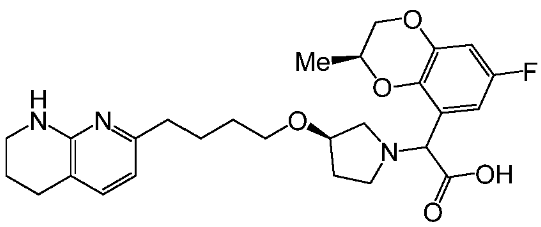;

or a pharmaceutically acceptable salt thereof.

## Patentansprüche

**1.** Verbindung, ausgewählt aus der Gruppe bestehend aus:

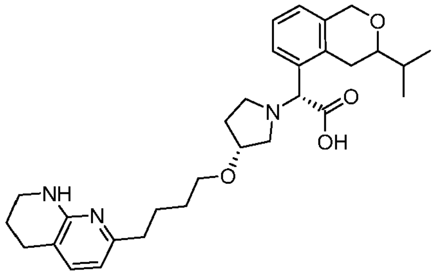

,

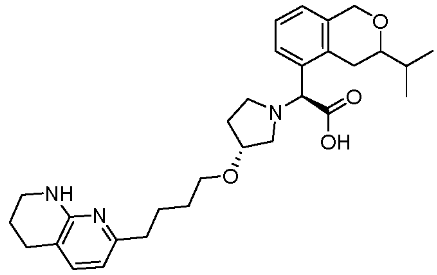

,

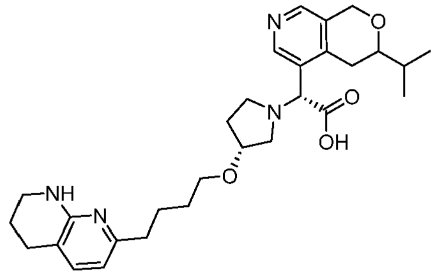

,

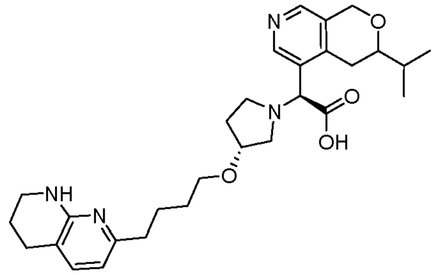

,

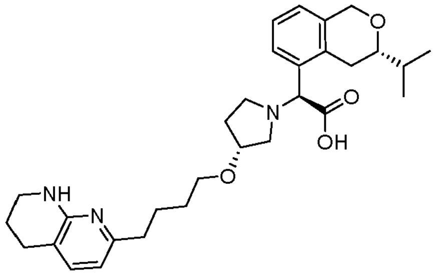

,

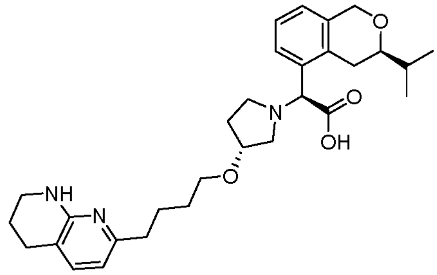

,

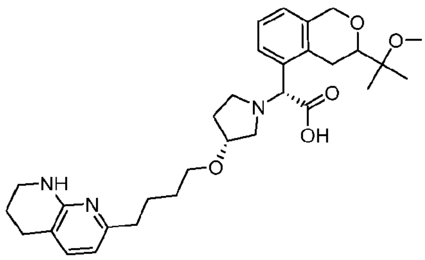

und

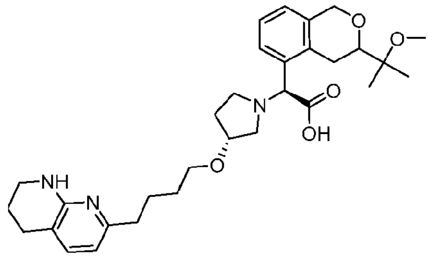

,

oder

pharmazeutisch unbedenkliches Salz davon, oder ausgewählt aus der Gruppe bestehend aus:

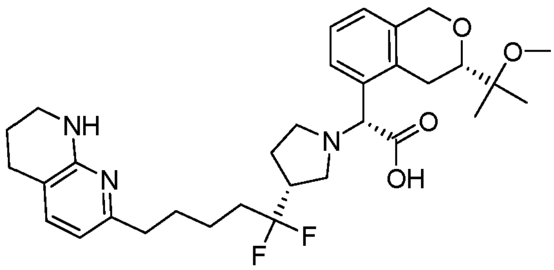

,

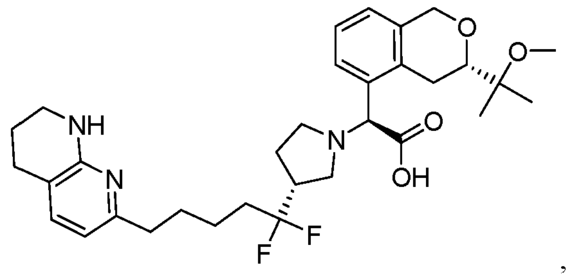

,

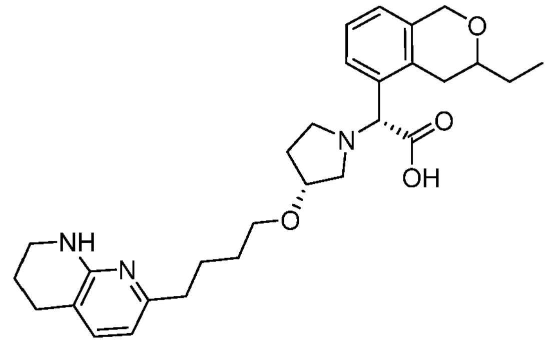

,

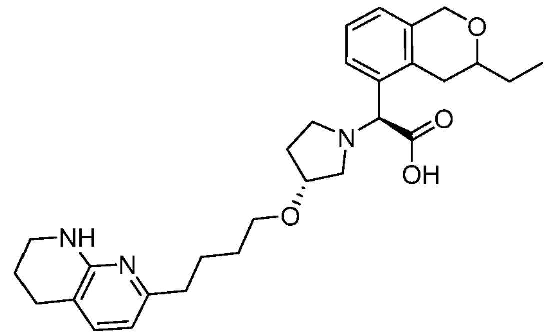

,

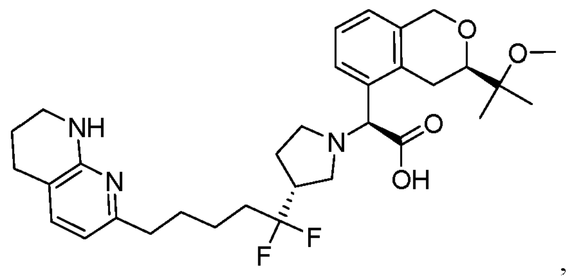

,

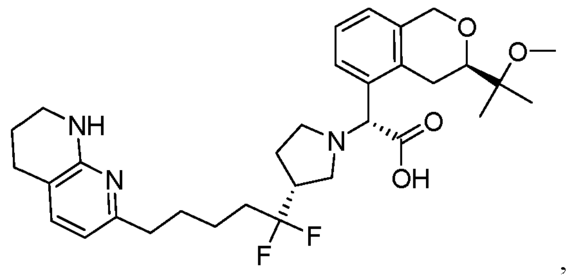

,

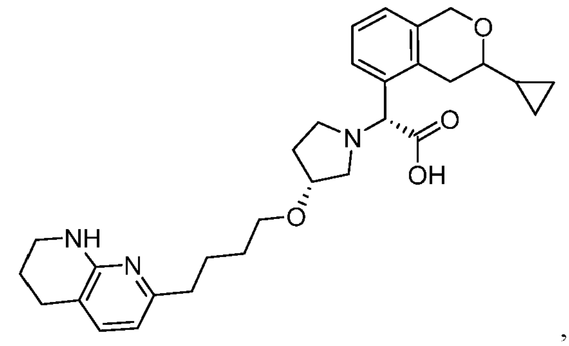

,

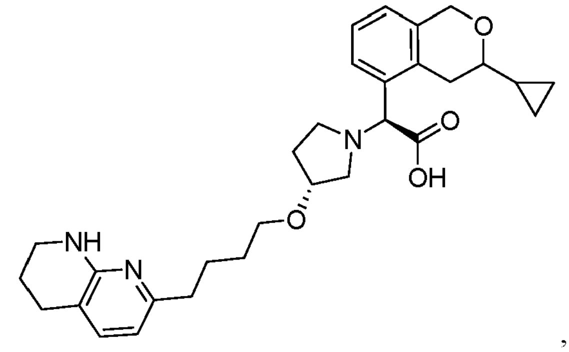

,

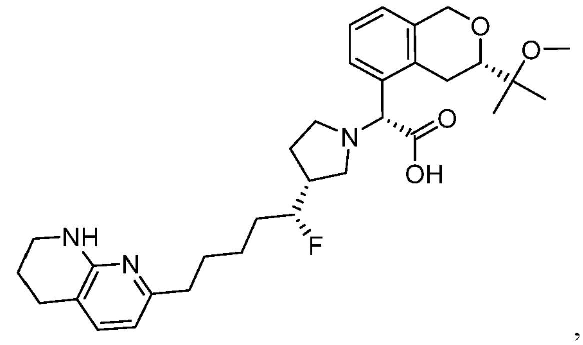

,

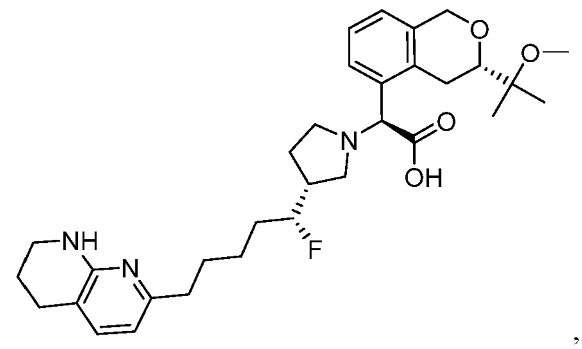

,

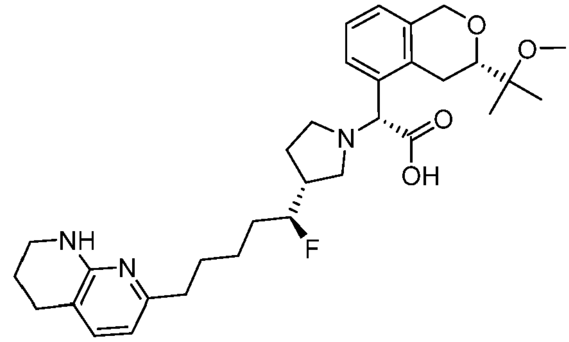

,

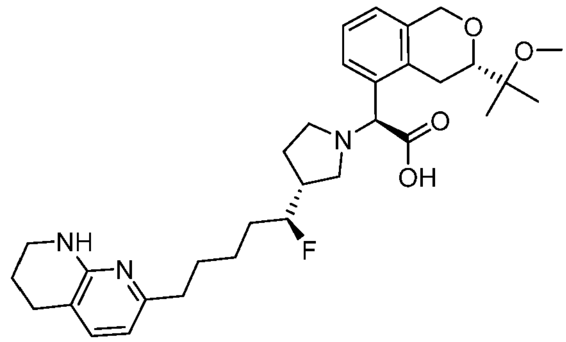

,

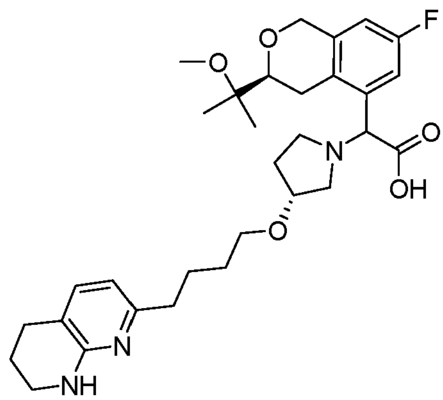

,

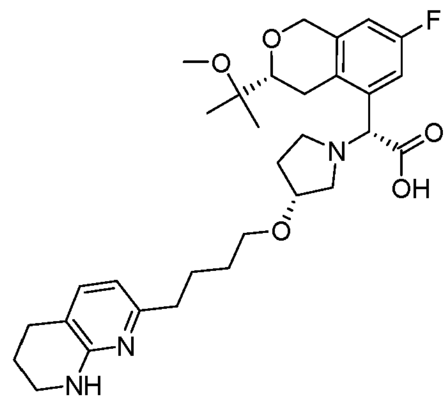

,

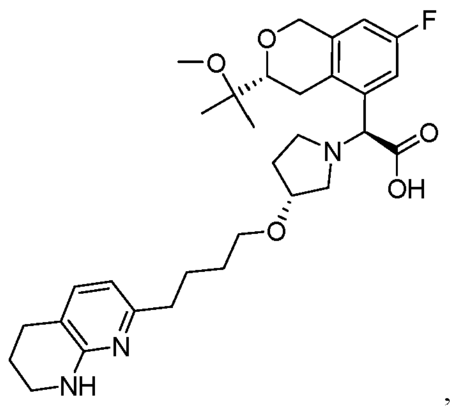

,

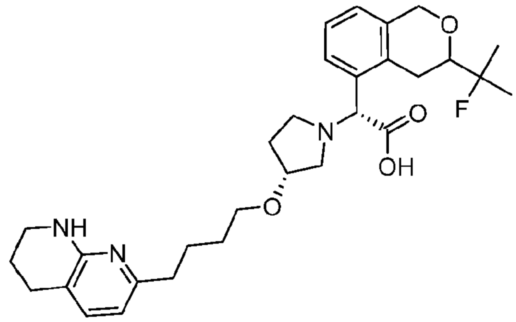

und

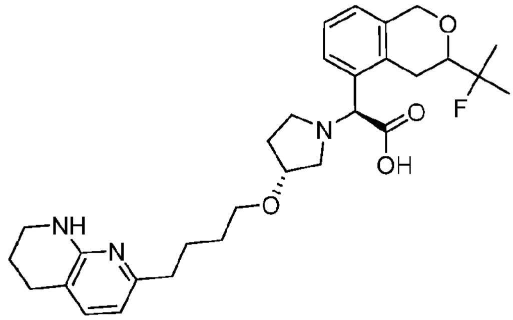

,

oder pharmazeutisch unbedenkliches Salz davon, oder ausgewählt aus der Gruppe bestehend aus:

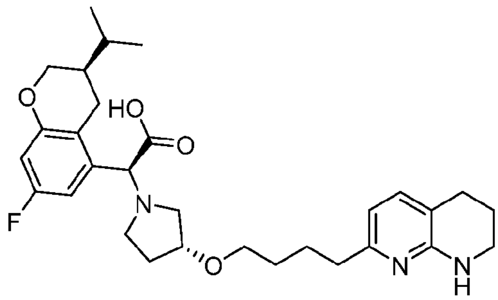

,

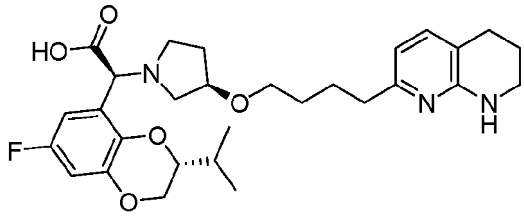

,

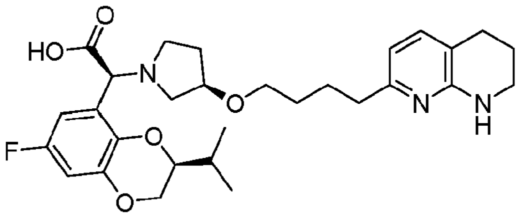

,

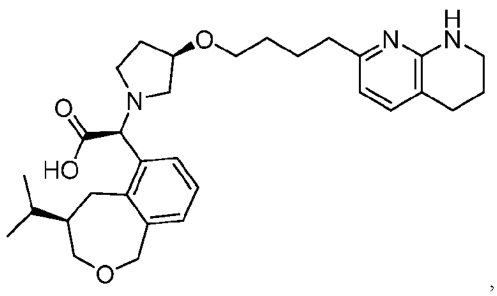

,

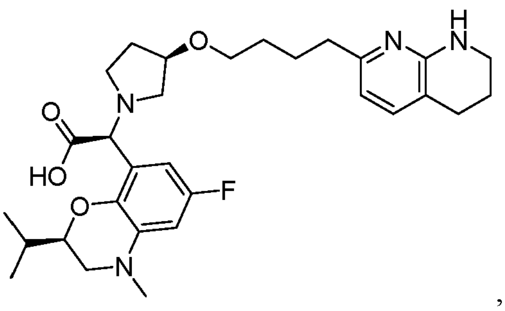

,

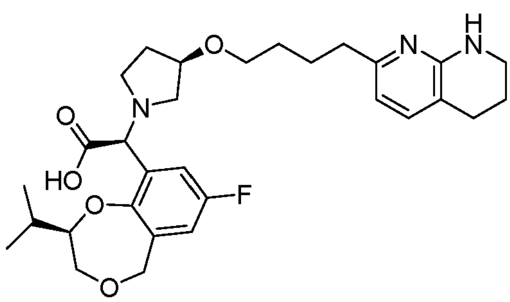

,

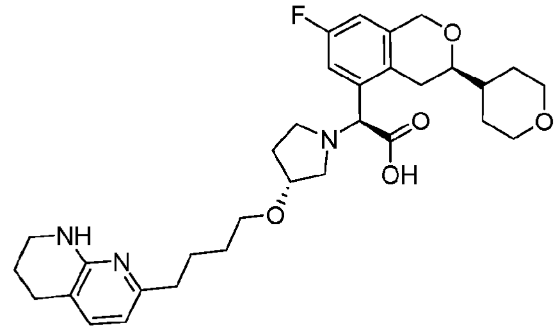

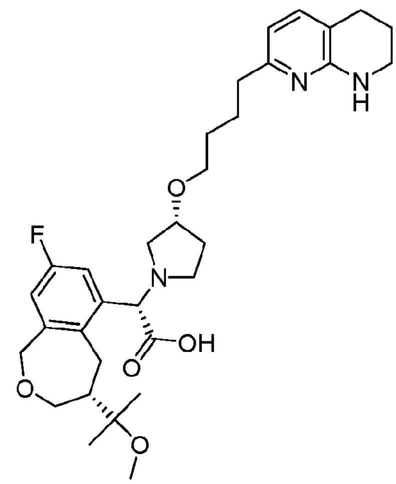

und ,

oder pharmazeutisch unbedenkliches Salz davon, oder ausgewählt aus der Gruppe bestehend aus:

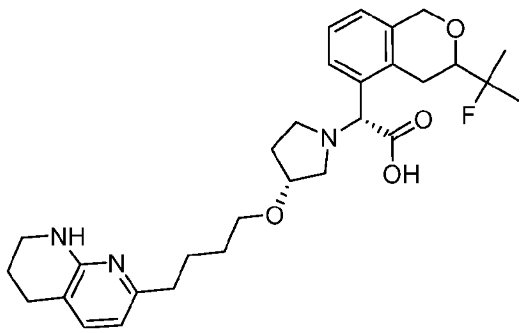

,

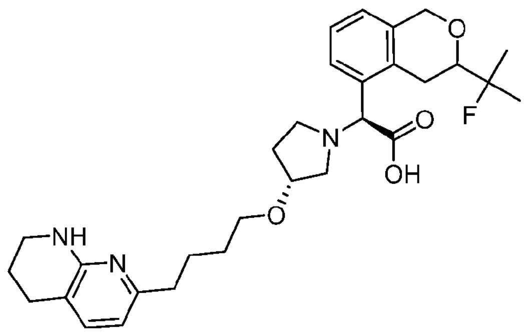

,

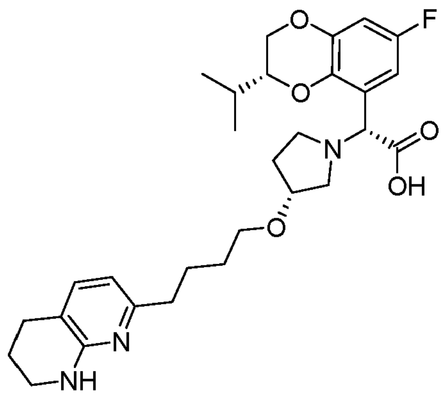

,

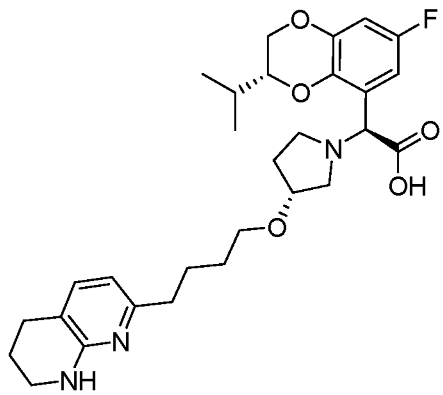

,

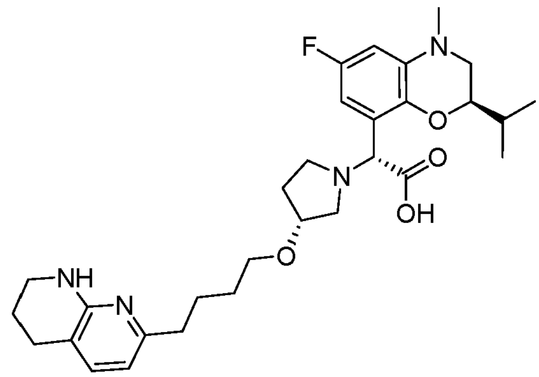

,

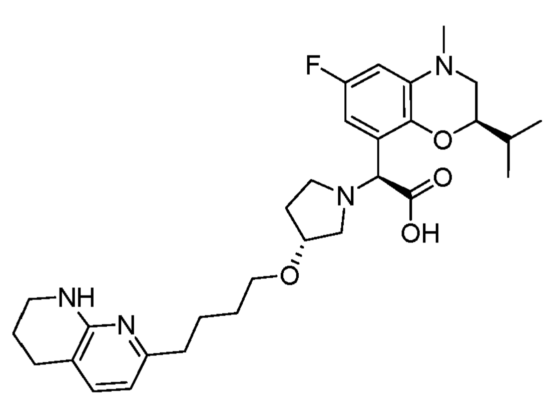

,

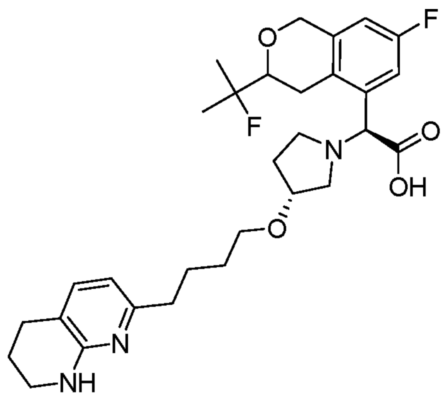

,

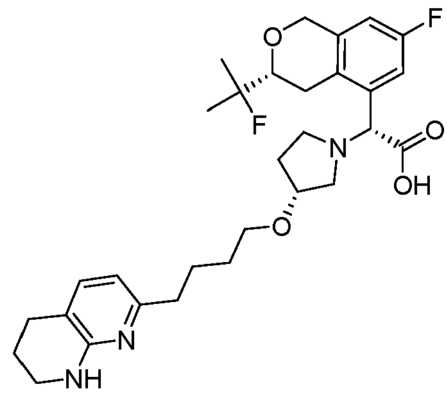

,

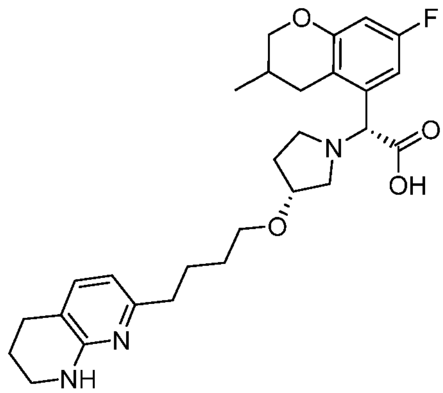

,

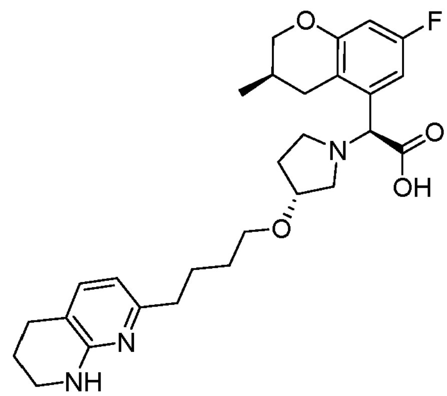

,

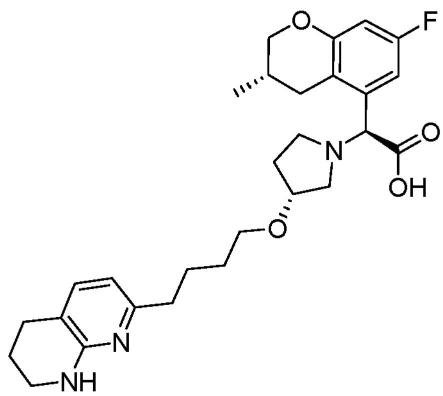

,

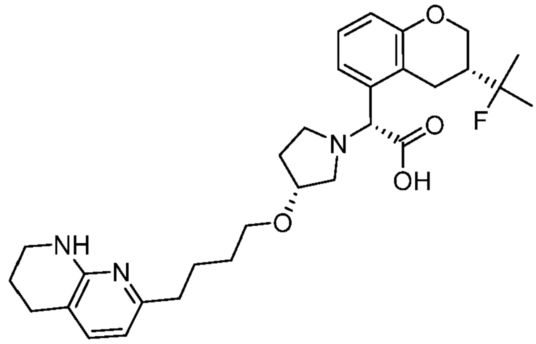

,

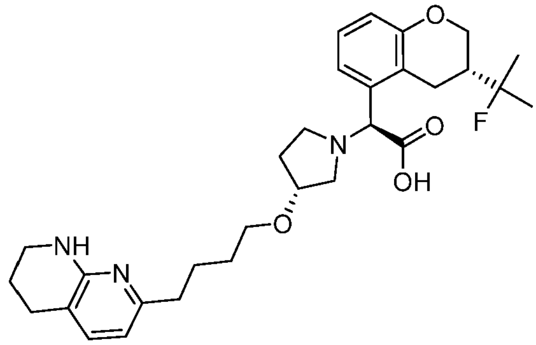

,

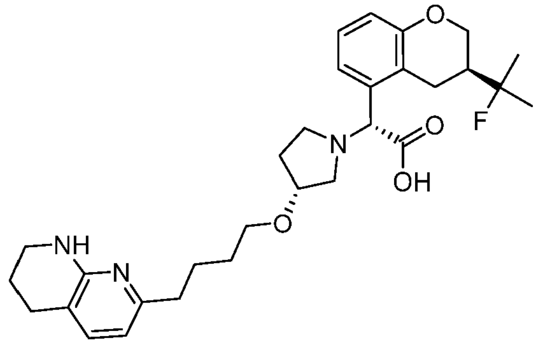

,

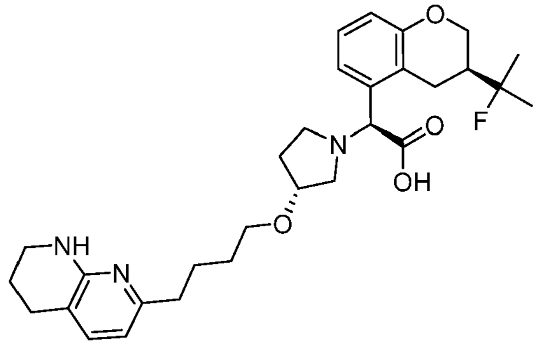

,

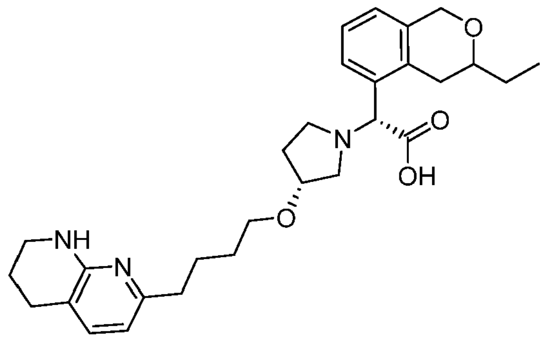

,

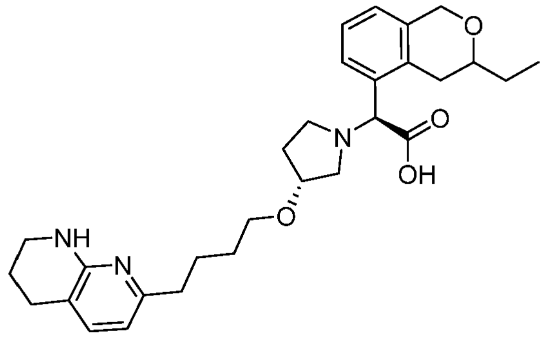

,

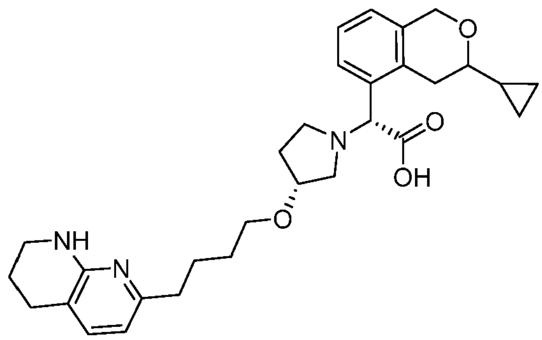

,

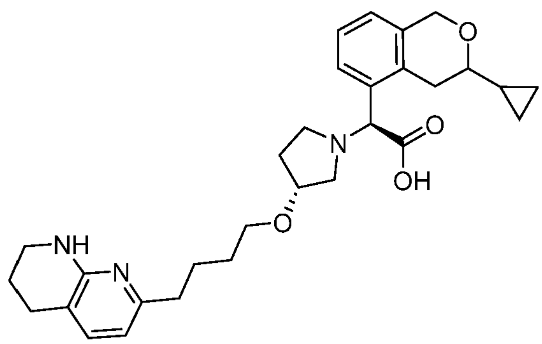

,

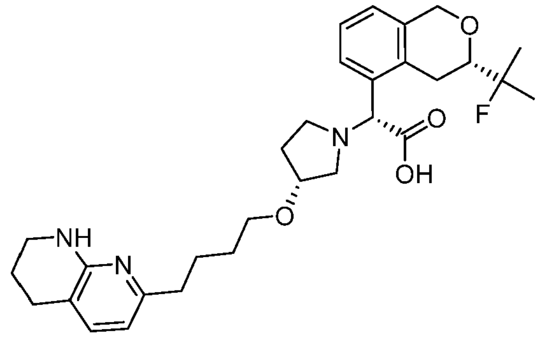

,

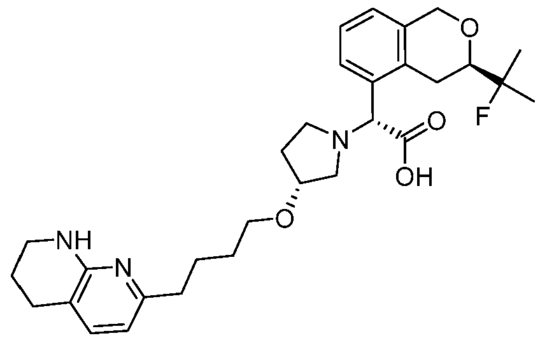

,

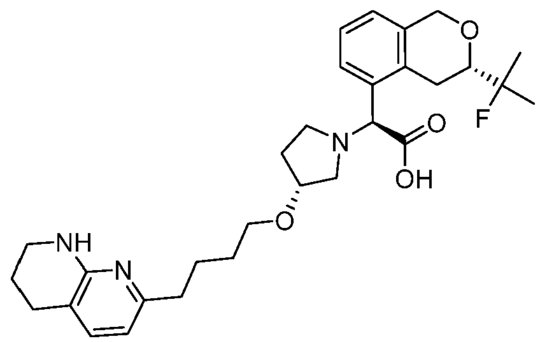

,

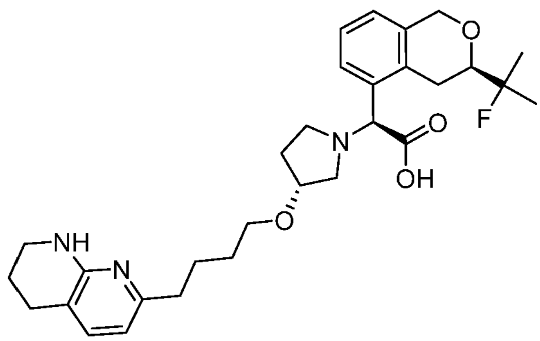

,

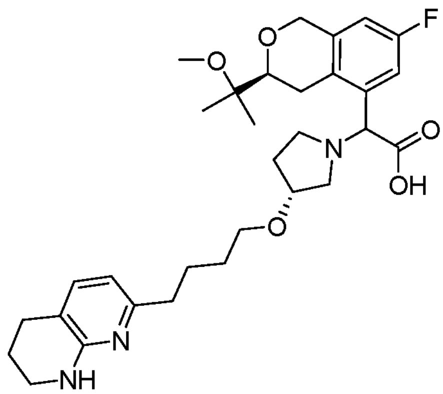

,

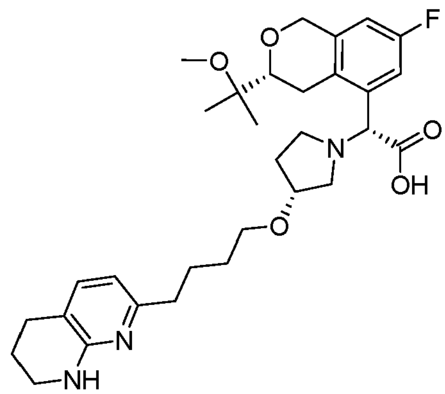

,

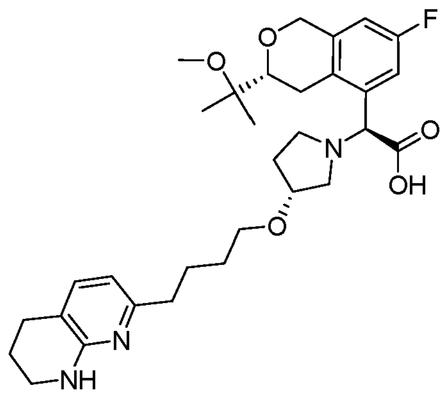

,

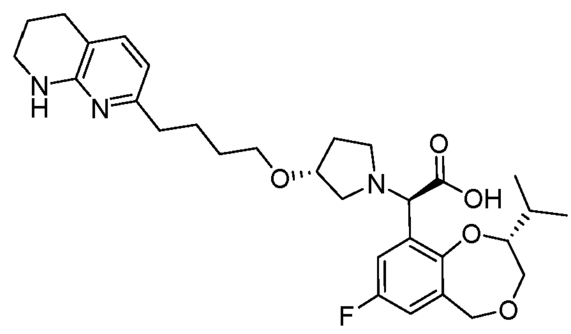

,

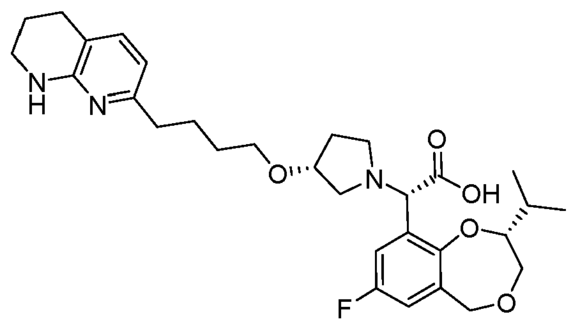

,

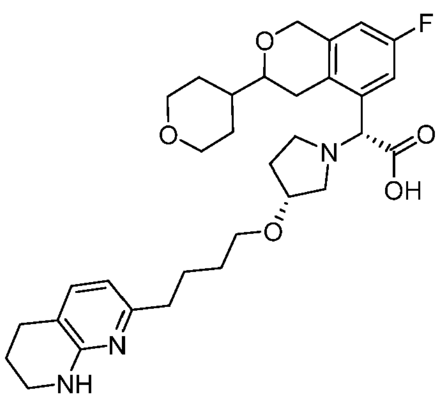

,

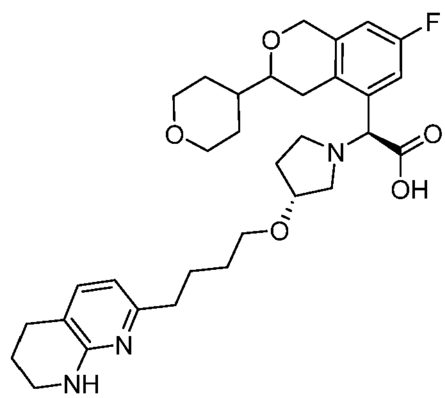

,

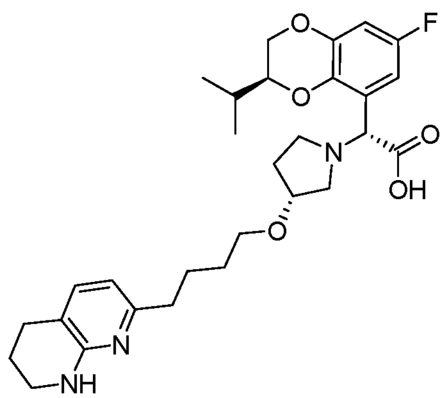

,

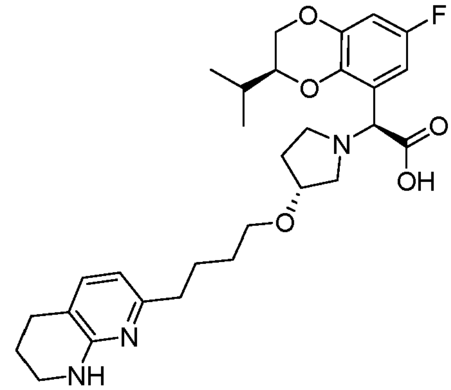

,

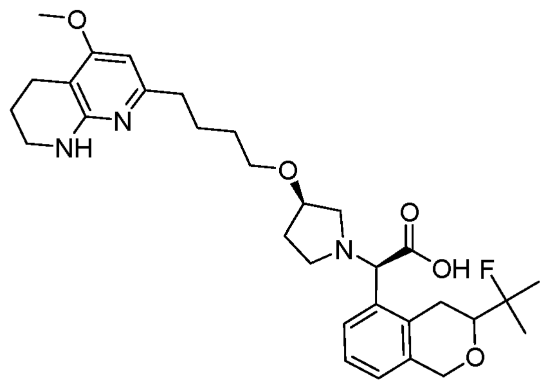

,

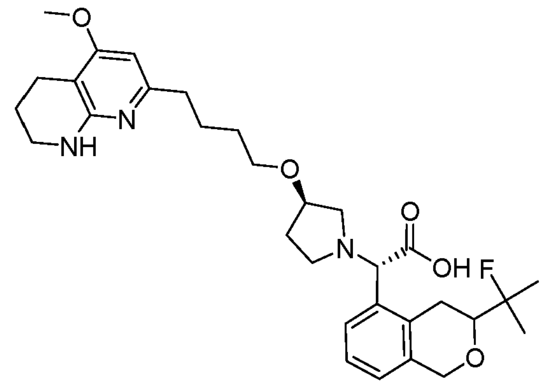

,

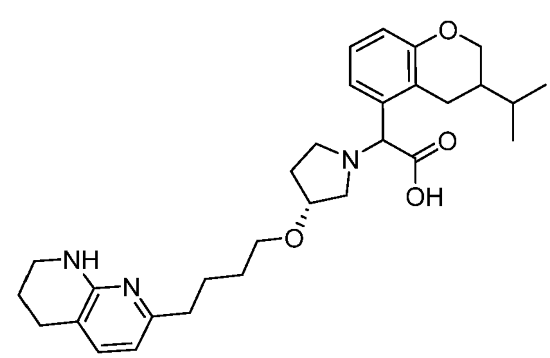

,

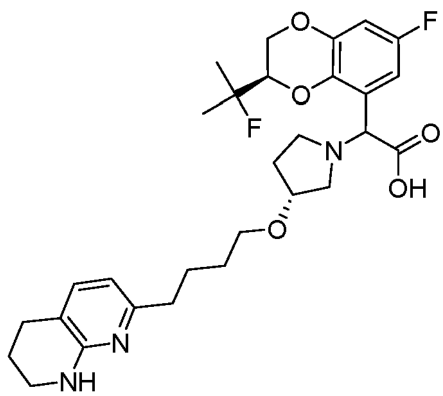

,

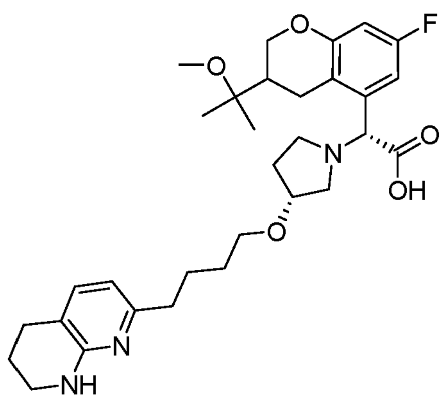

,

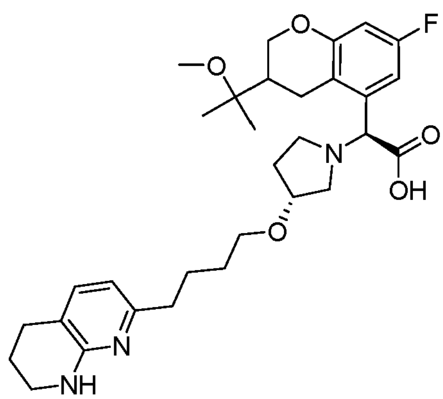

,

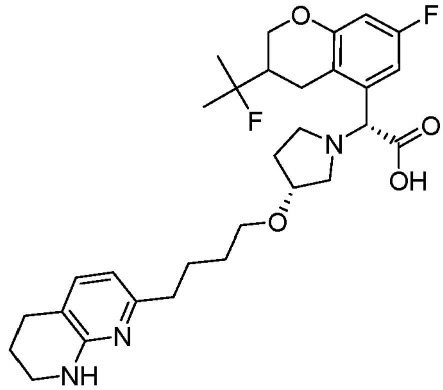
,
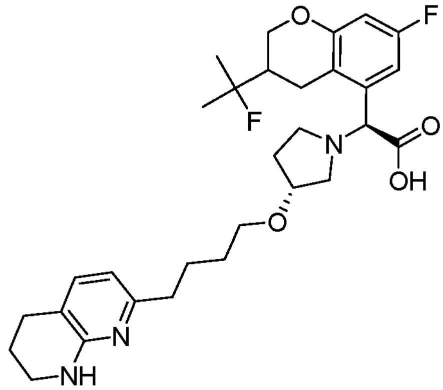
,

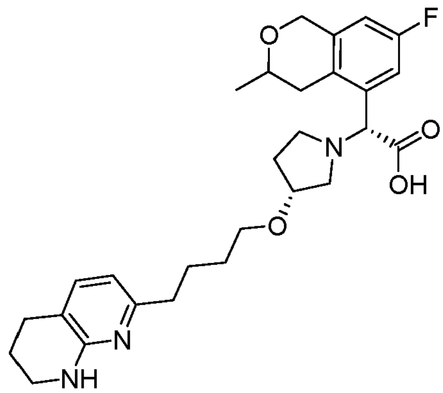
,
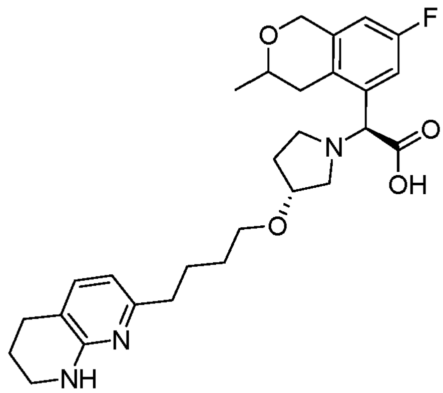
,

und

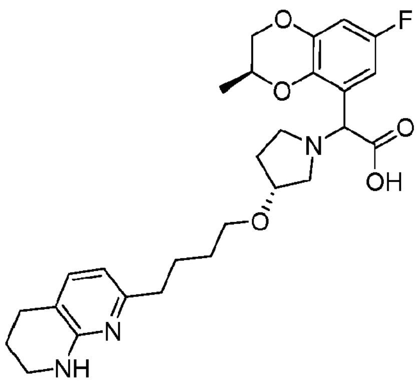
,

oder pharmazeutisch unbedenkliches Salz davon, oder
ausgewählt aus der Gruppe bestehend aus:

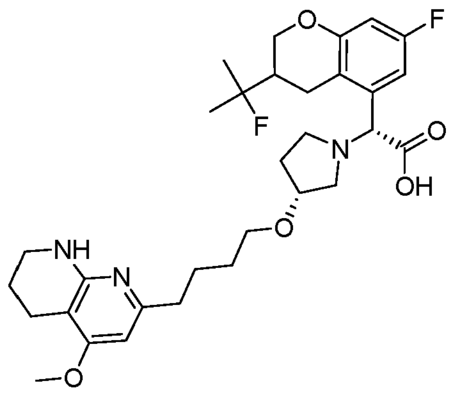,
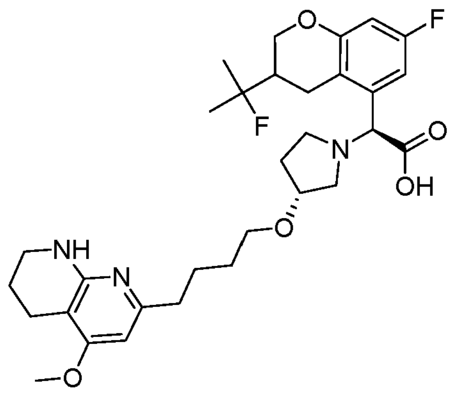,

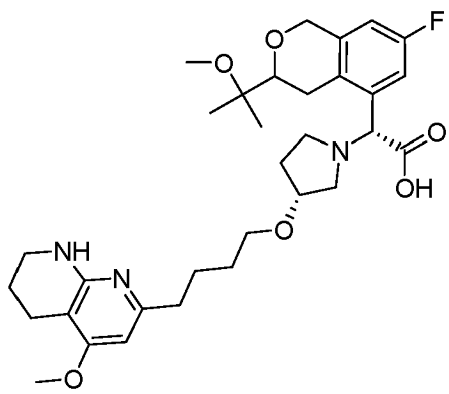,
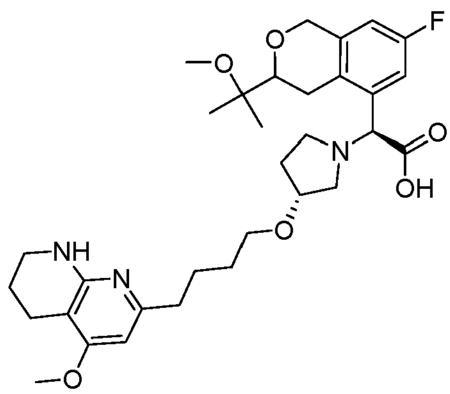,

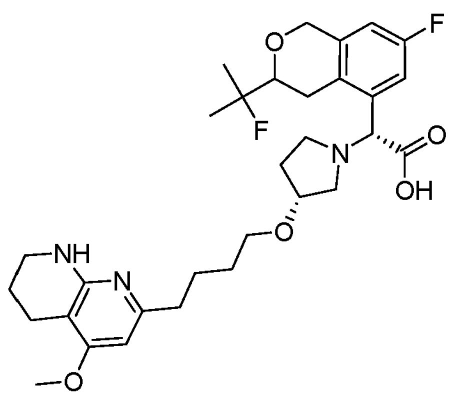,
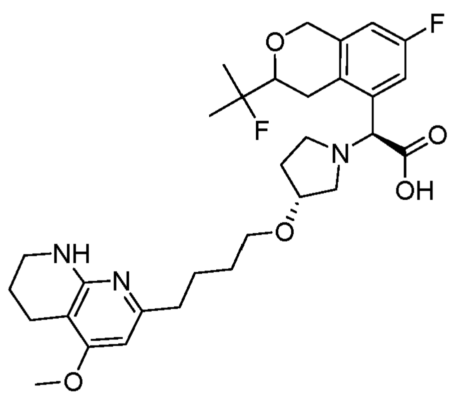,

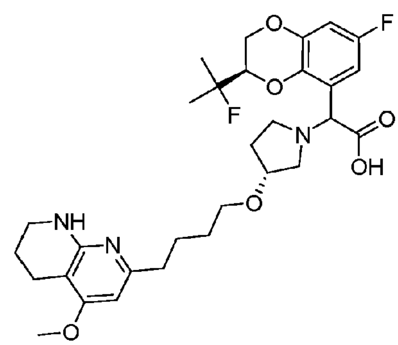,
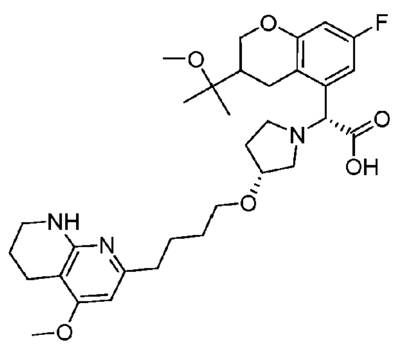

und

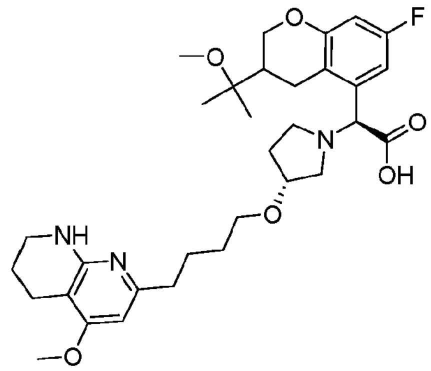

,

oder pharmazeutisch unbedenkliches Salz davon, oder
ausgewählt aus der Gruppe bestehend aus:

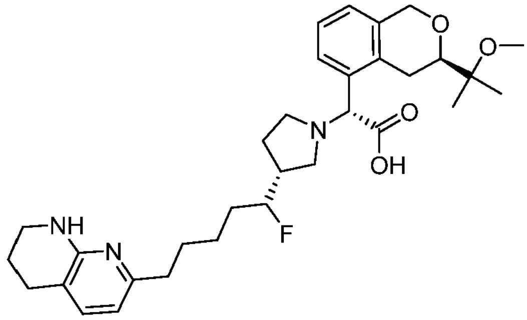

,

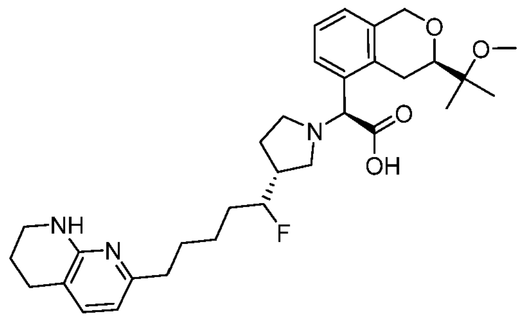

,

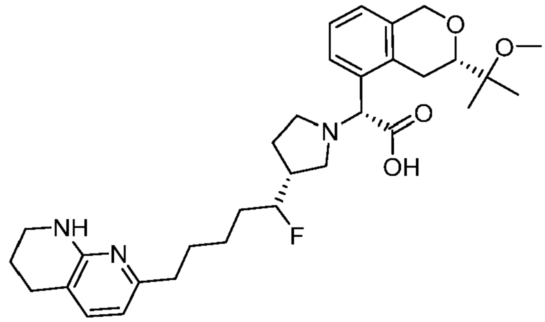

und

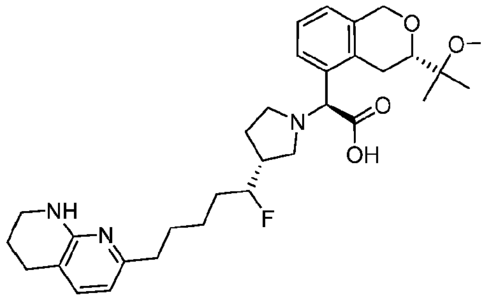

,

oder pharmazeutisch unbedenkliches Salz davon, oder
ausgewählt aus der Gruppe bestehend aus:

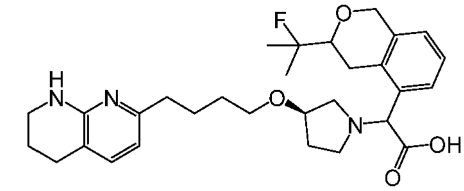 , 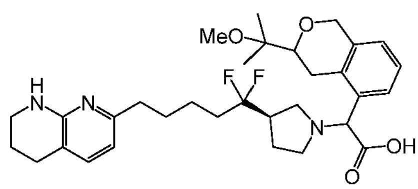 ,

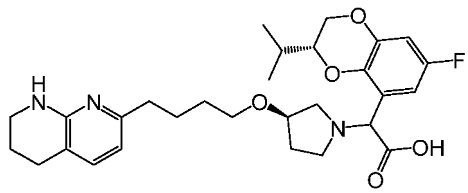 , 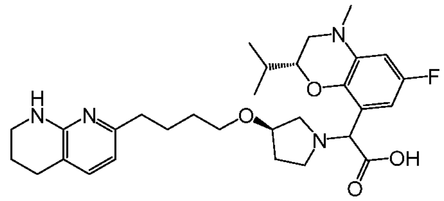 ,

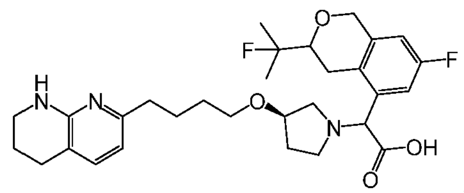 , 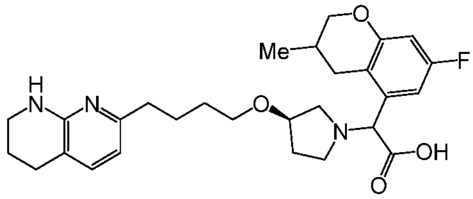 ,

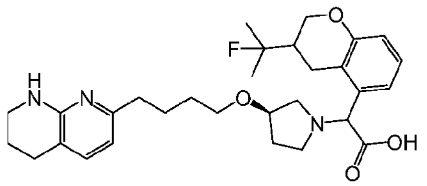 und 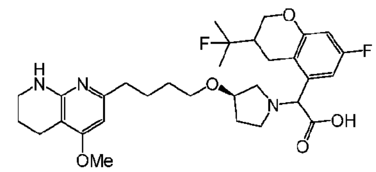 ,

oder pharmazeutisch unbedenkliches Salz davon, oder ausgewählt aus der Gruppe bestehend aus:

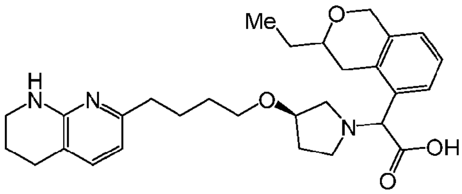 , 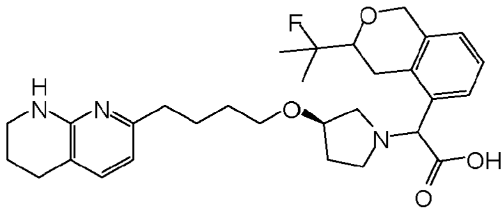 ,

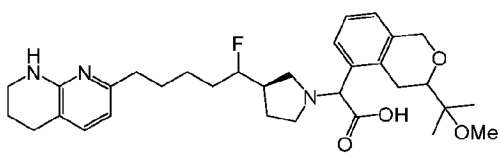 , 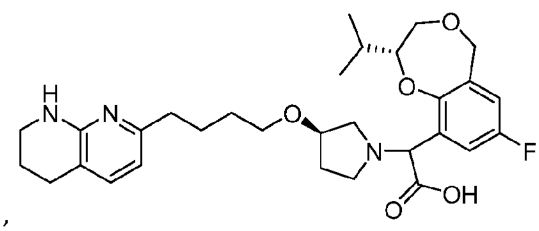 ,

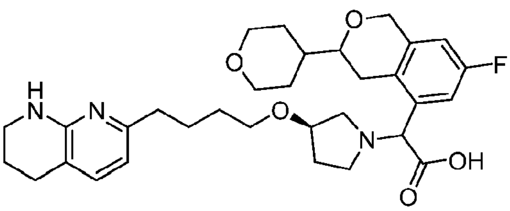 , 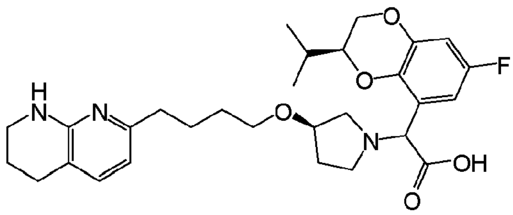 ,

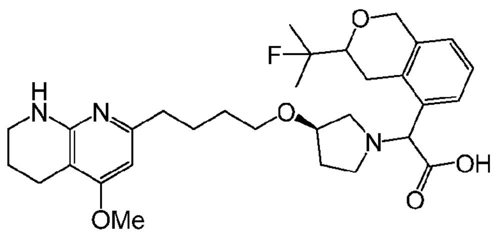,
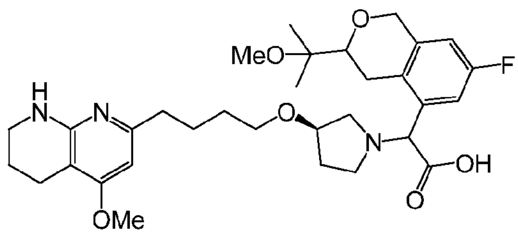,

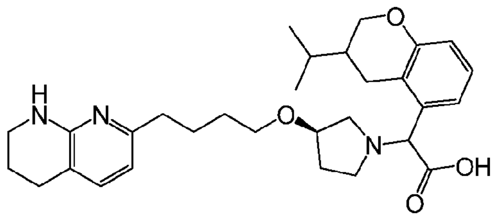,
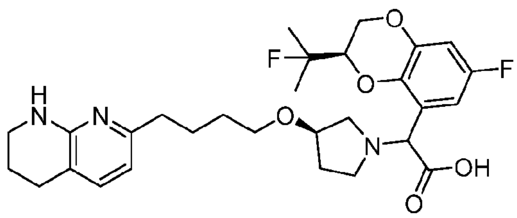,

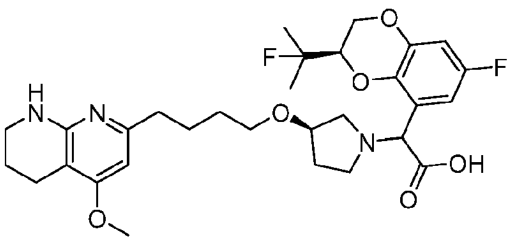,
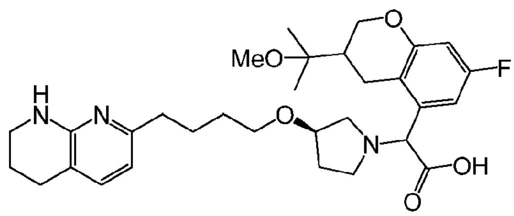,

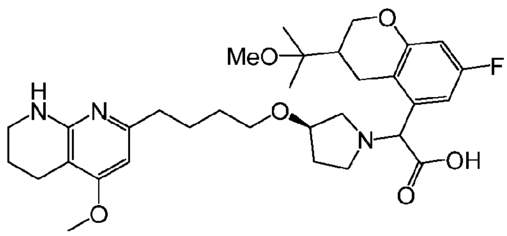,
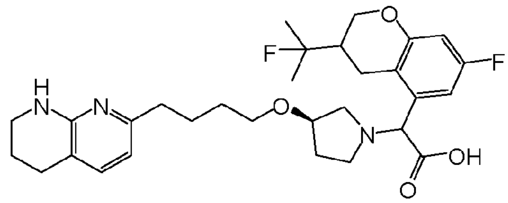,

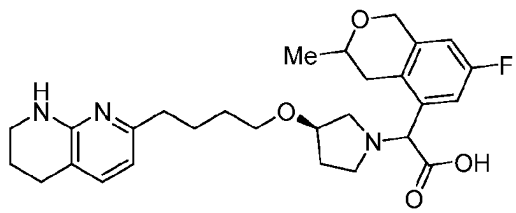

und

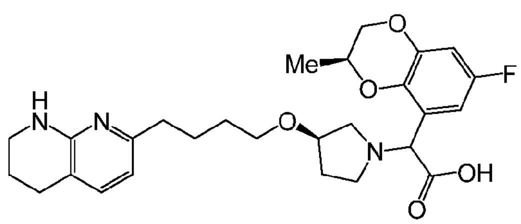,

oder pharmazeutisch unbedenkliches Salz davon.

**2.** Verbindung nach Anspruch 1, ausgewählt aus der Gruppe bestehend aus:

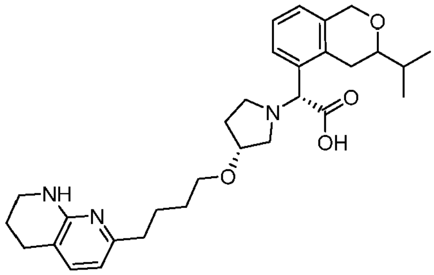, 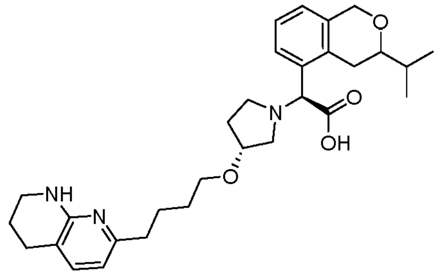,

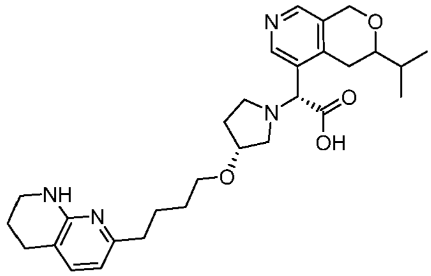, 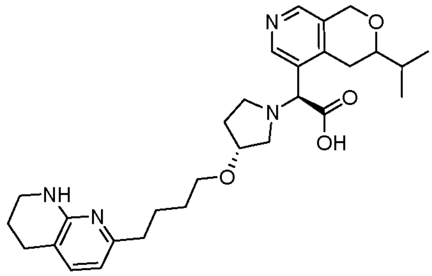,

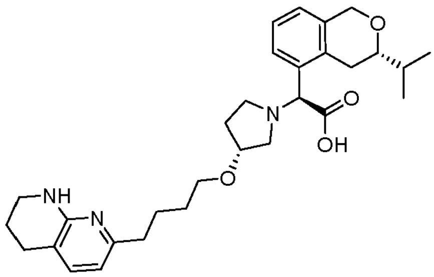, 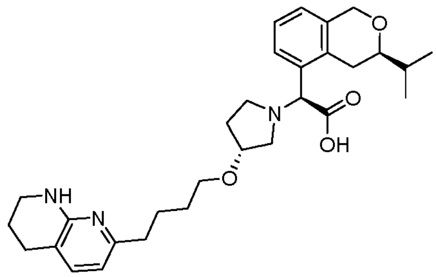,

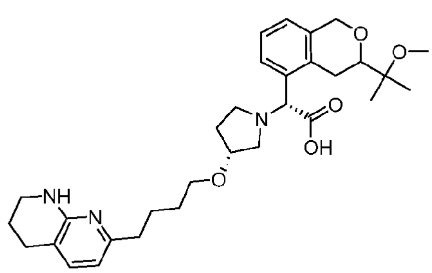 und 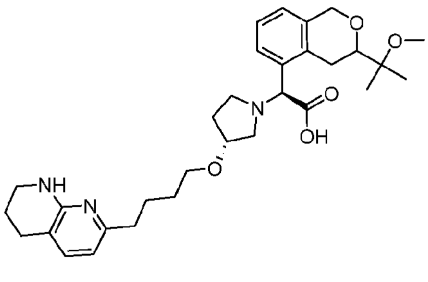,

oder pharmazeutisch unbedenkliches Salz davon.

**3.** Verbindung nach Anspruch 1, ausgewählt aus der Gruppe bestehend aus:

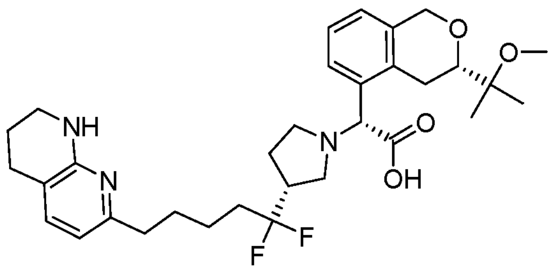,

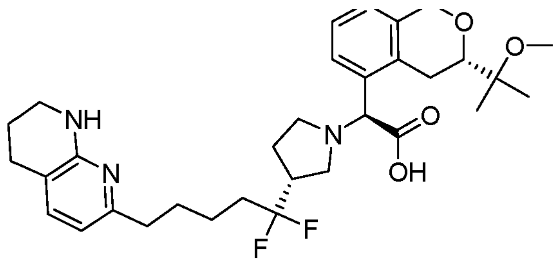

,

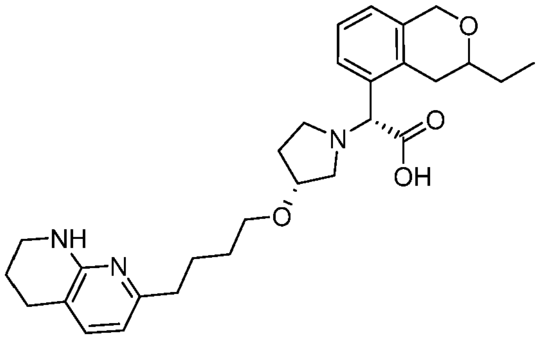

,

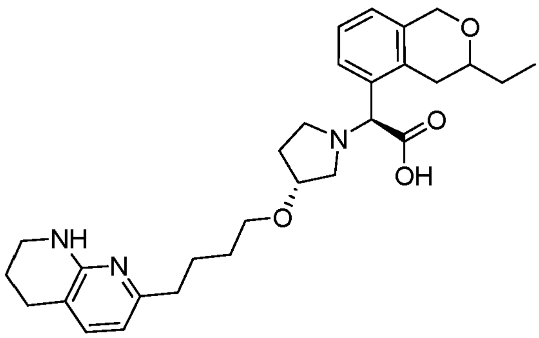

,

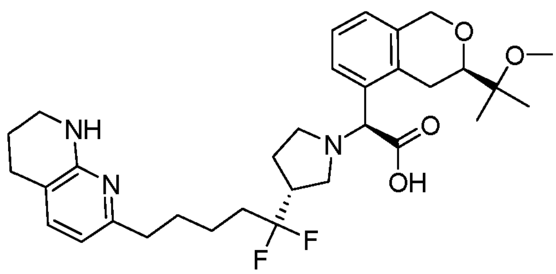

,

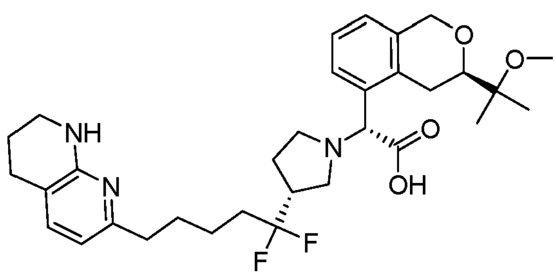

,

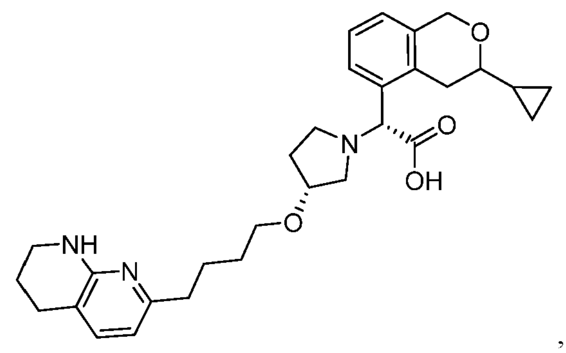

,

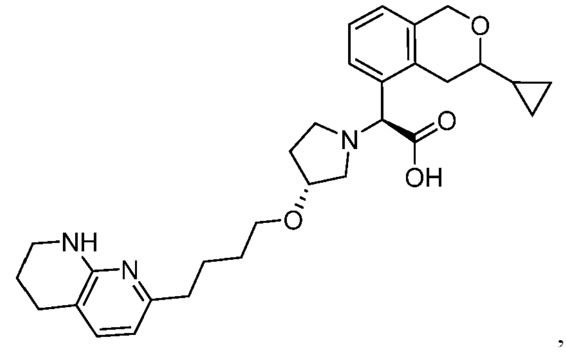

,

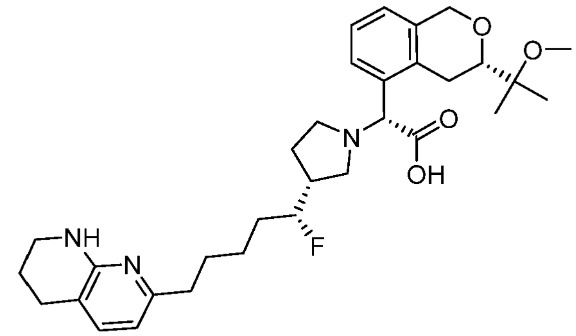

,

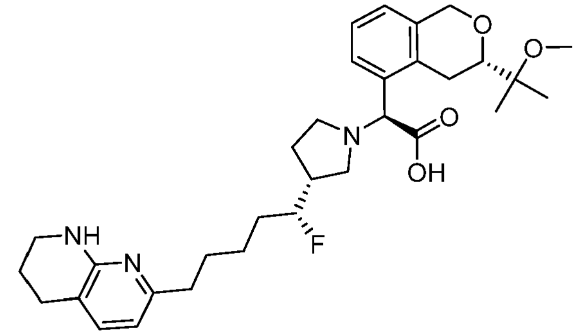

,

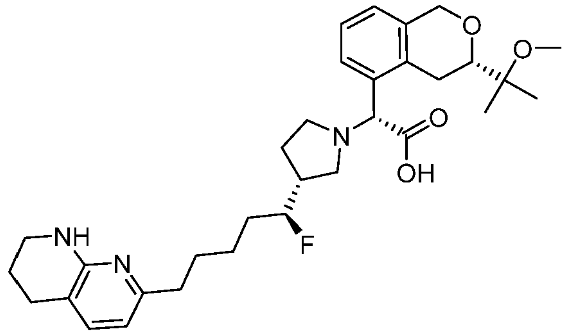

,

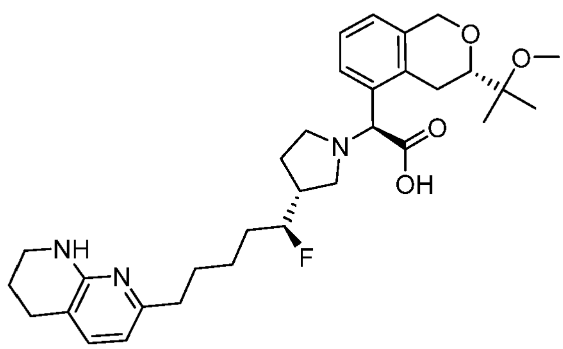

,

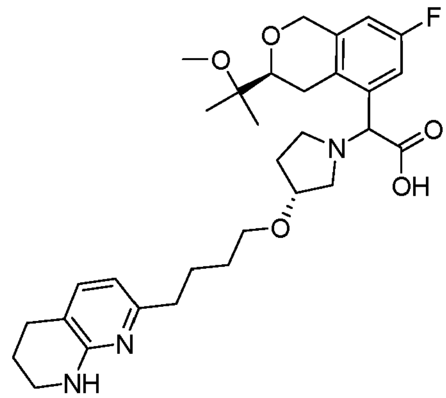

,

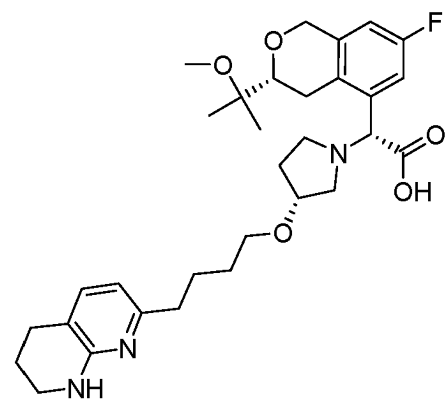

,

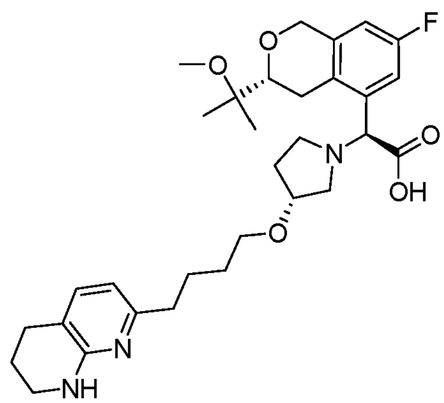

,

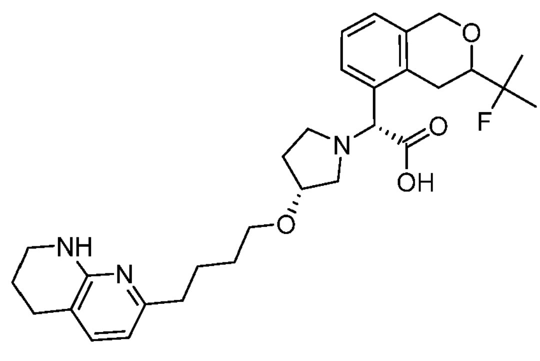

und

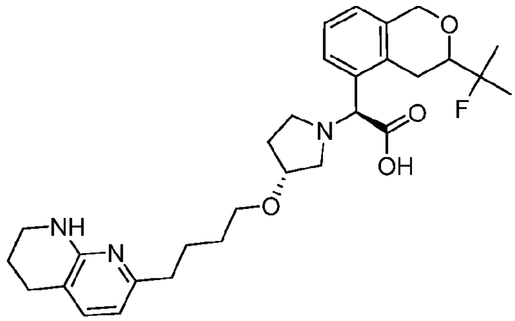

,

oder pharmazeutisch unbedenkliches Salz davon.

**4.** Verbindung nach Anspruch 1, ausgewählt aus der Gruppe bestehend aus:

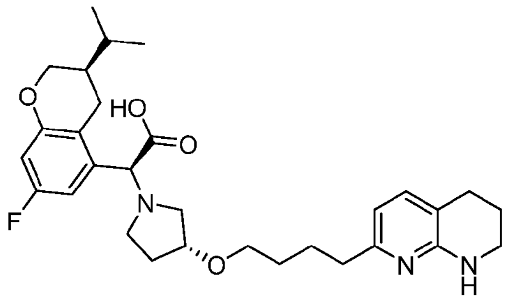

,

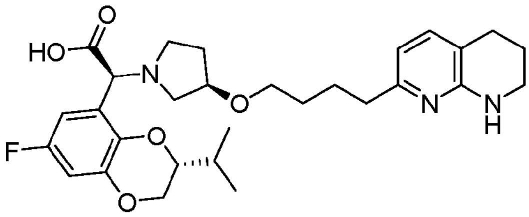

,

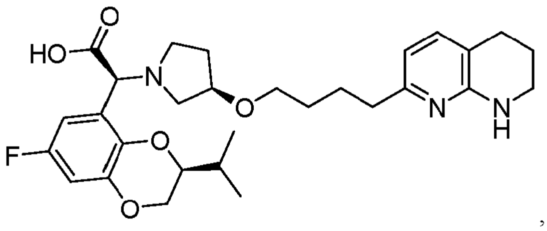

,

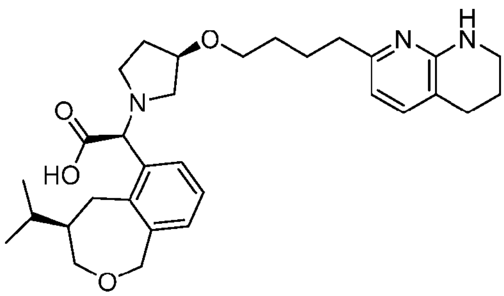

,

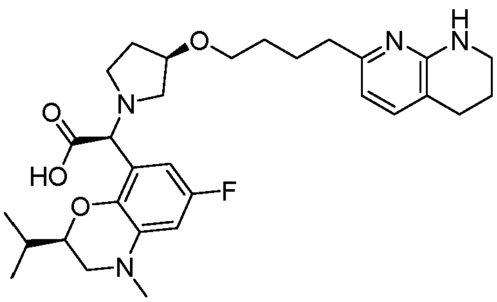

,

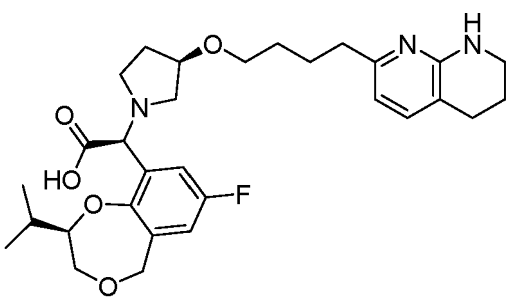

,

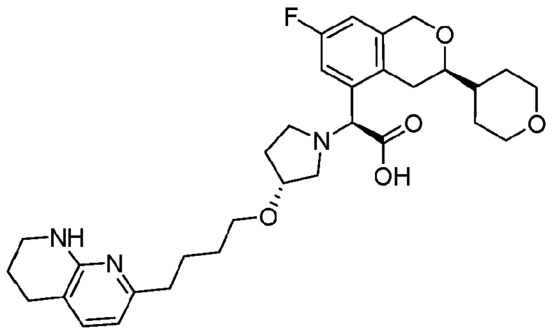

und

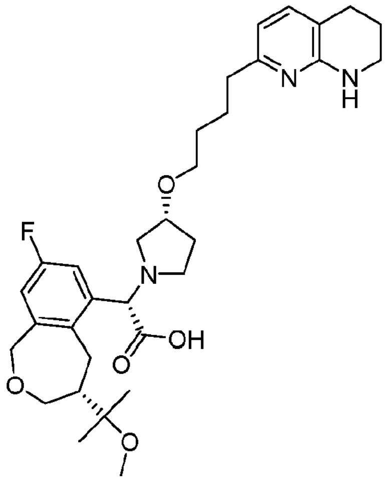

, oder pharmazeutisch unbedenkliches Salz davon.

**5.** Verbindung nach Anspruch 1, ausgewählt aus der Gruppe bestehend aus:

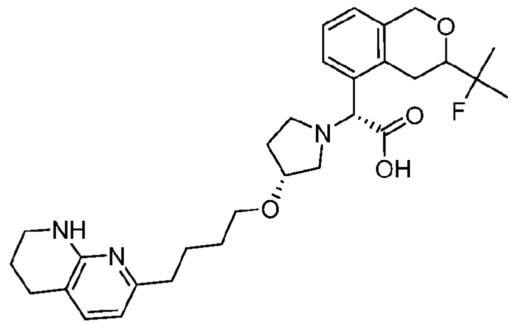

,

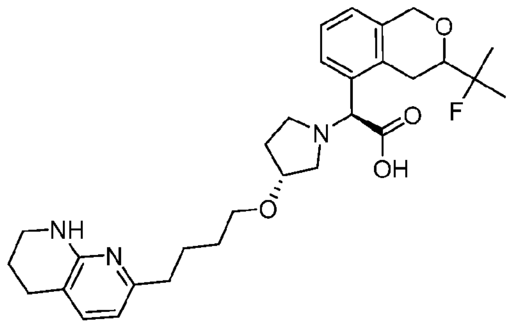

,

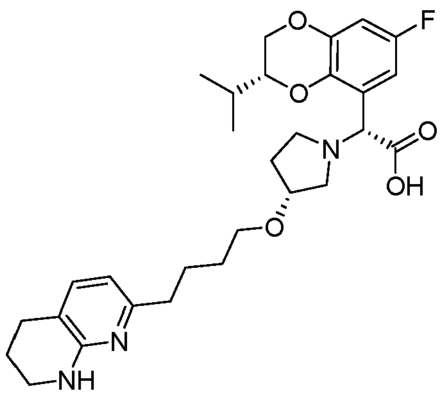

,

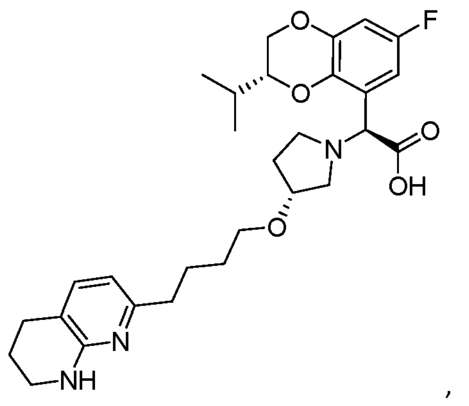

,

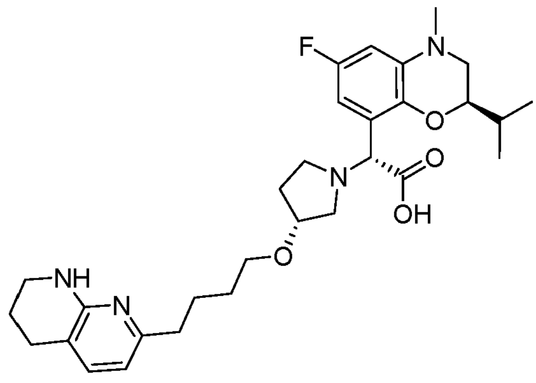

,

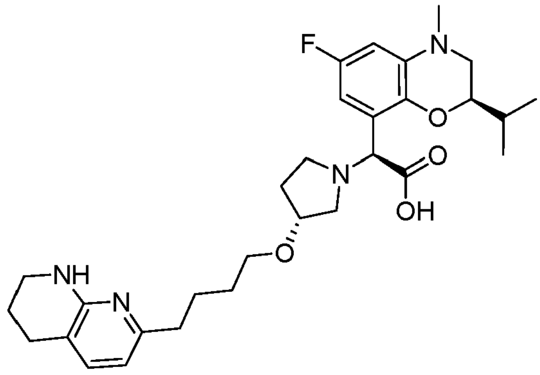

,

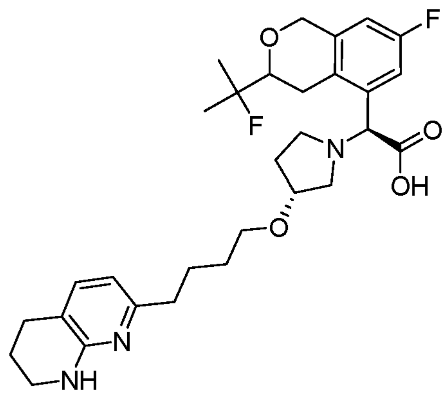

,

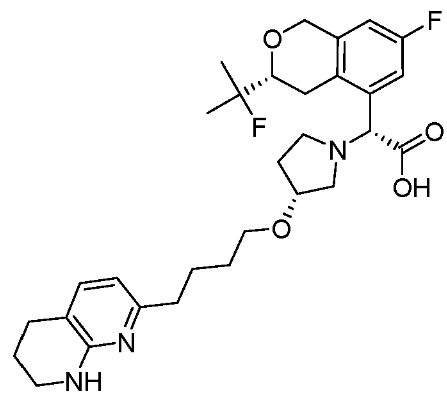

,

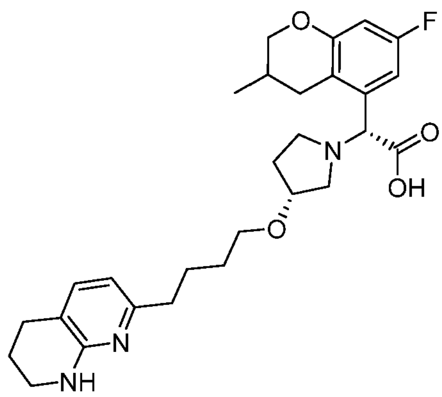

,

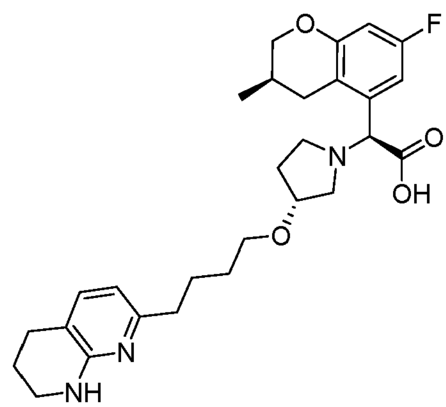

,

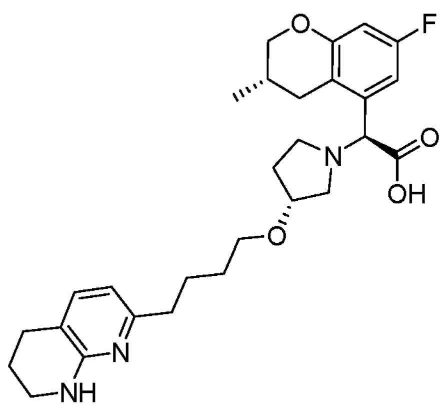

,

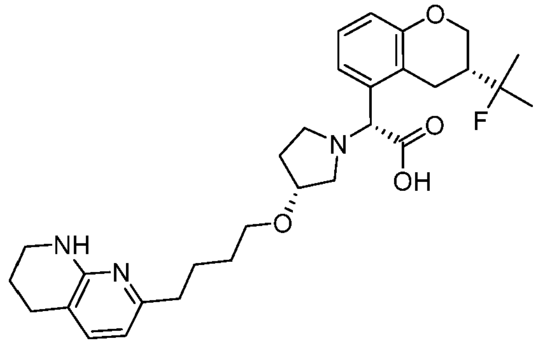

,

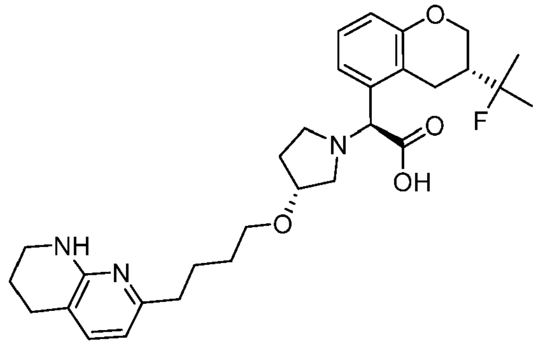

,

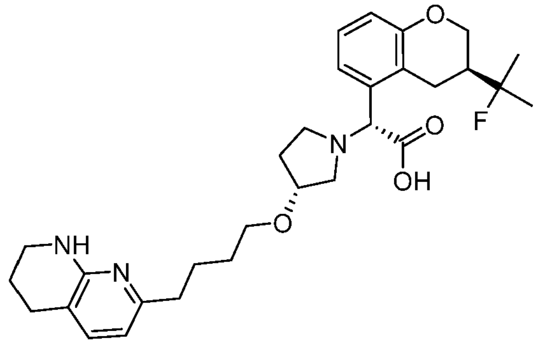

,

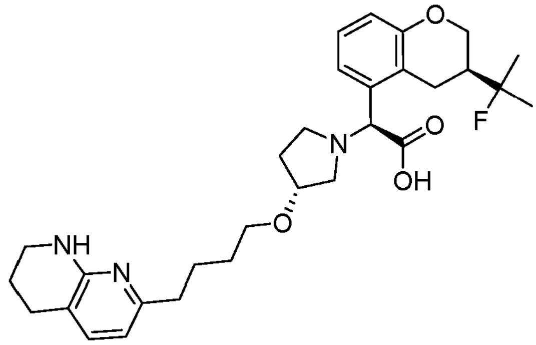

,

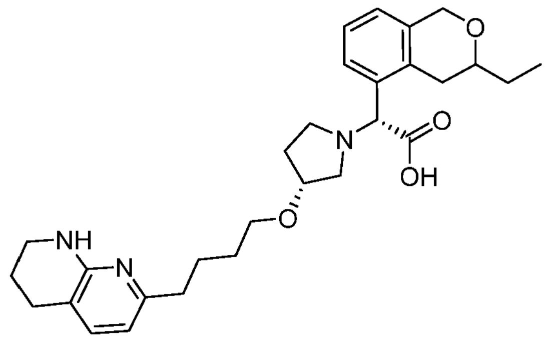

,

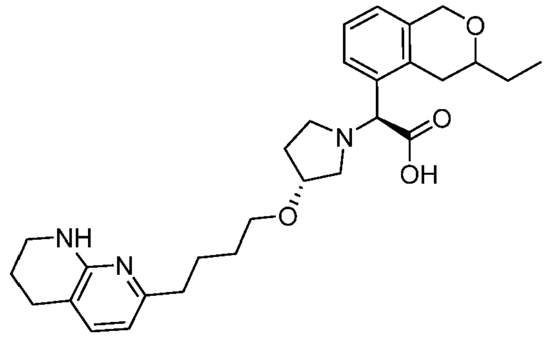

,

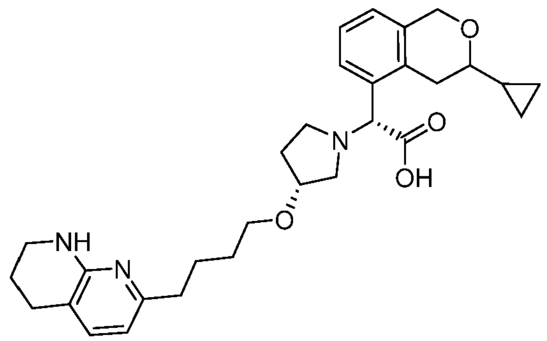

,

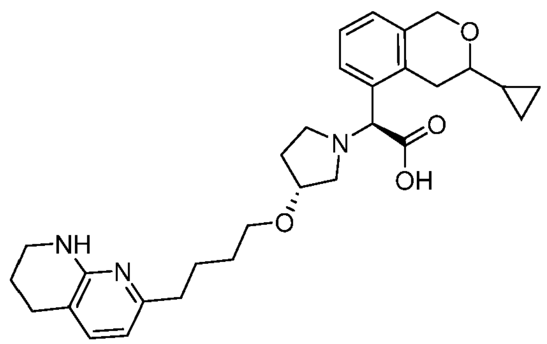

,

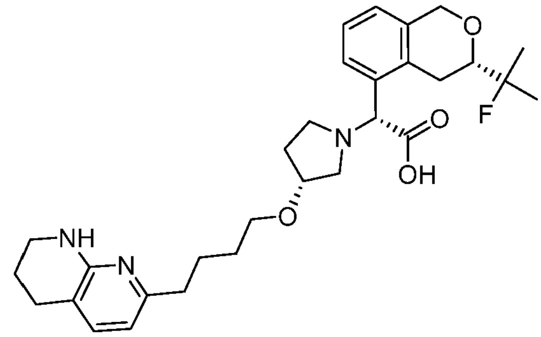

,

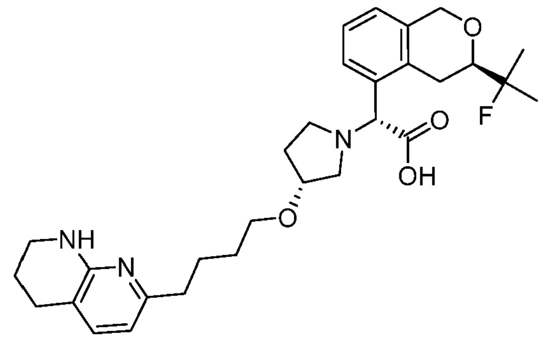

,

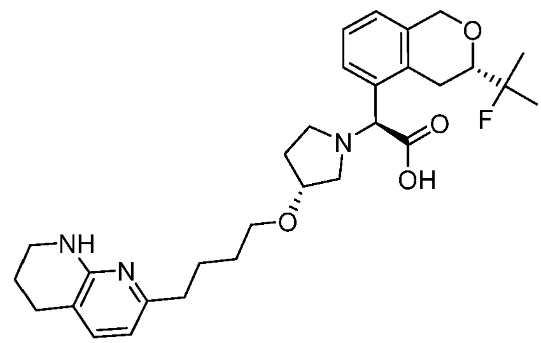

,

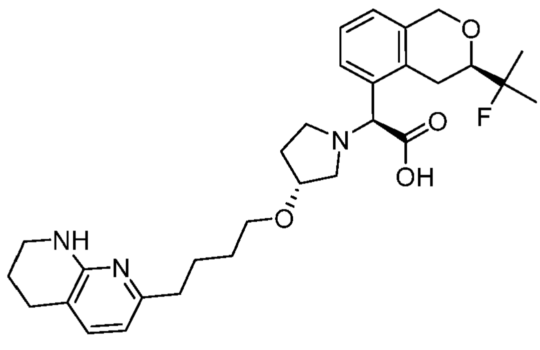

,

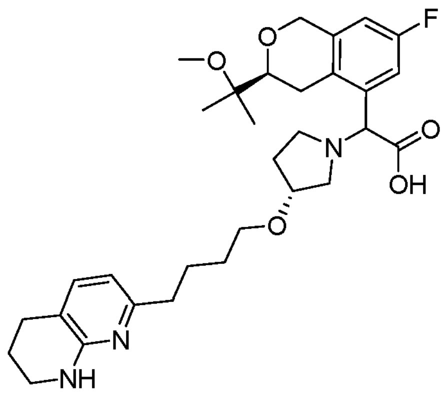

,

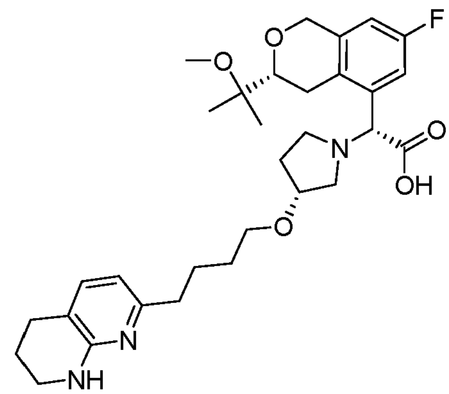,

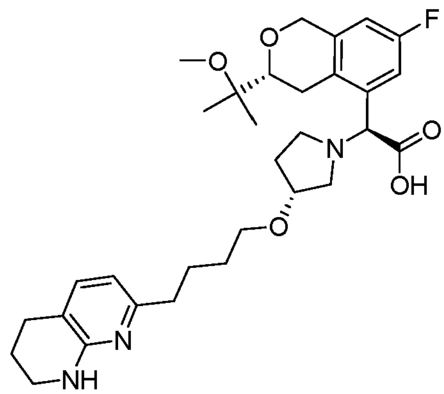,

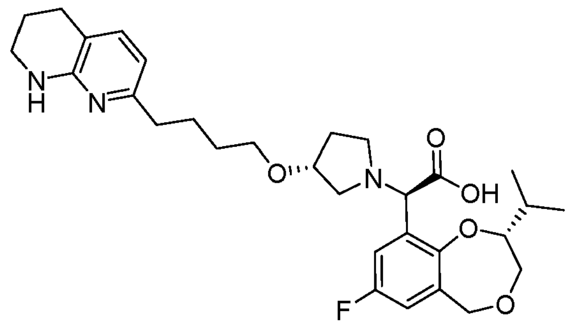,

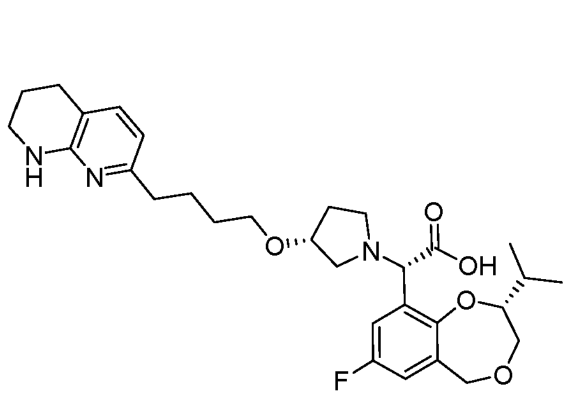,

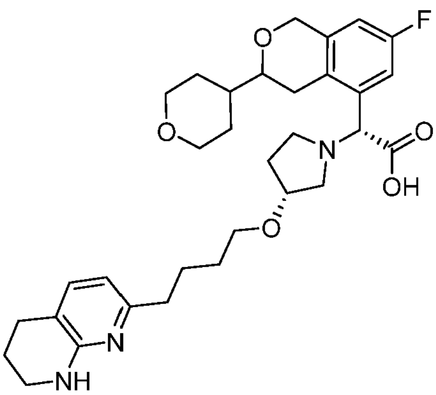,

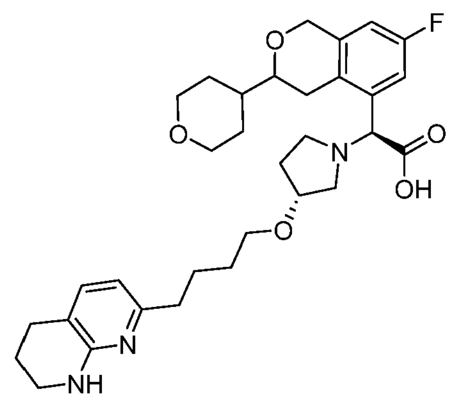,

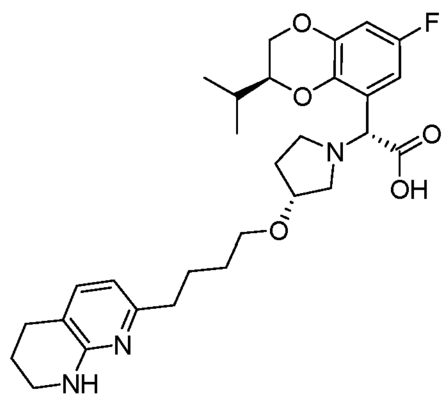,

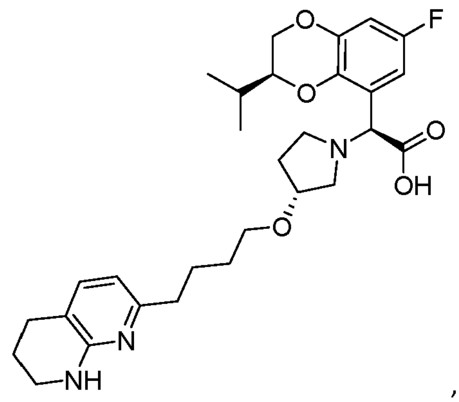

,

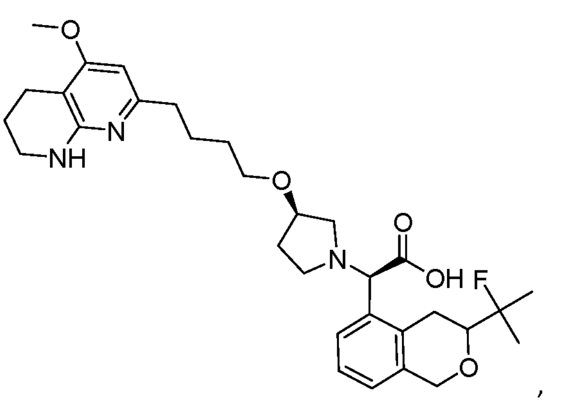

,

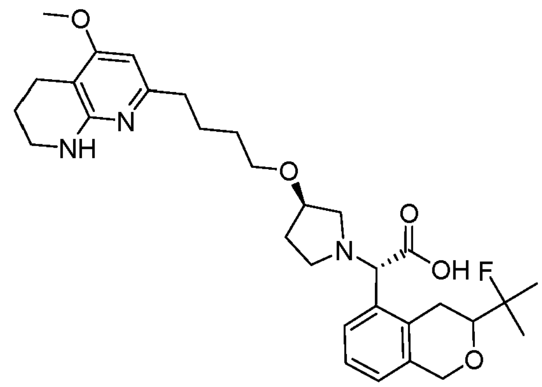

,

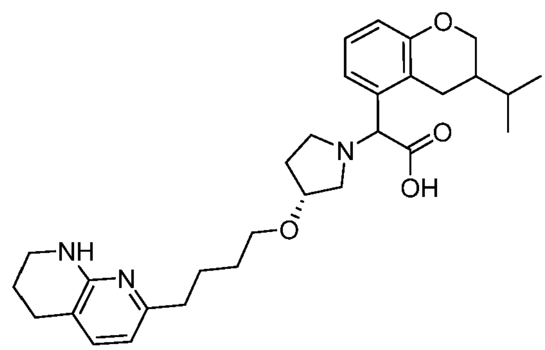

,

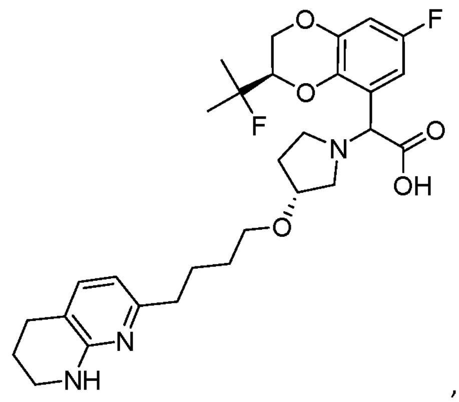

,

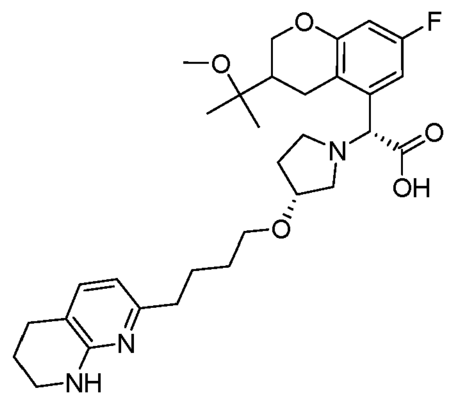

,

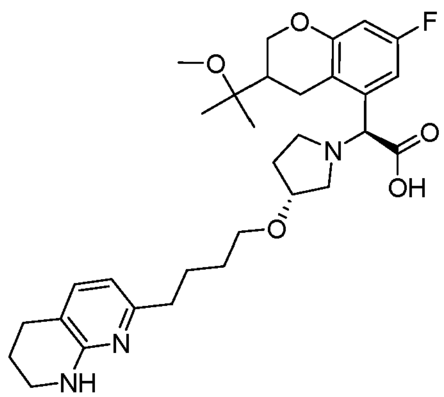

,

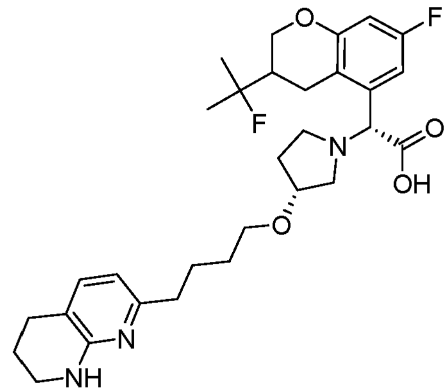,
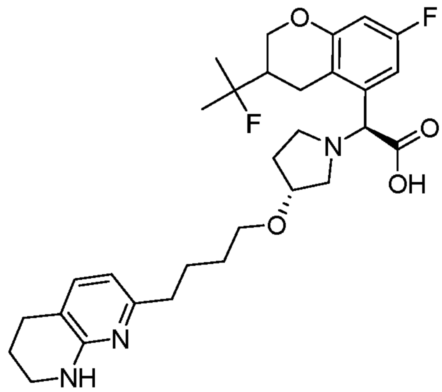,

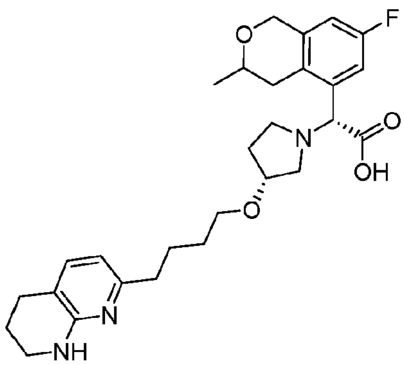,
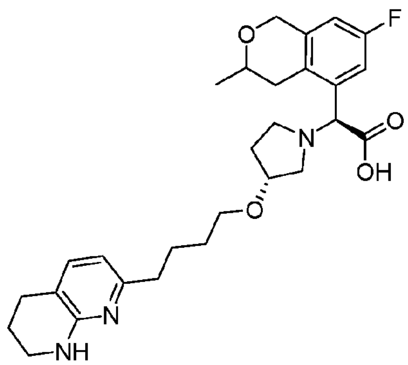

und

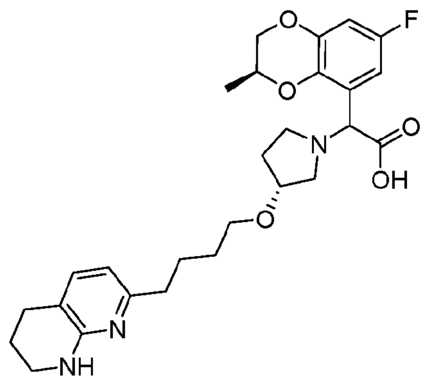,

oder pharmazeutisch unbedenkliches Salz davon.

**6.** Verbindung nach Anspruch 1, ausgewählt aus der Gruppe bestehend aus:

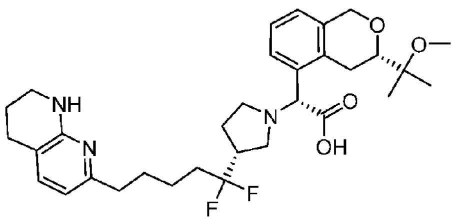,
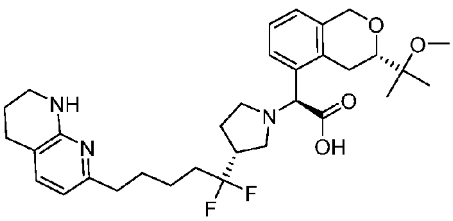,

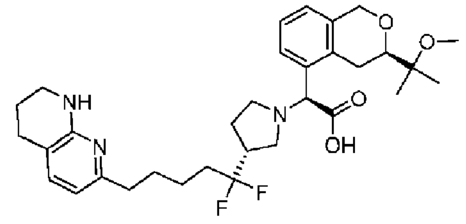

und

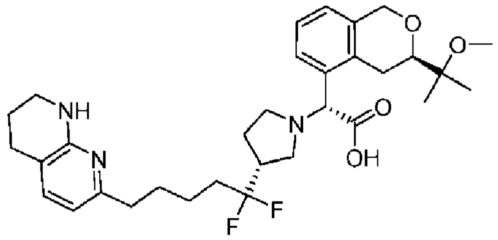

,

oder pharmazeutisch unbedenkliches Salz davon.

7. Verbindung nach Anspruch 1, ausgewählt aus der Gruppe bestehend aus:

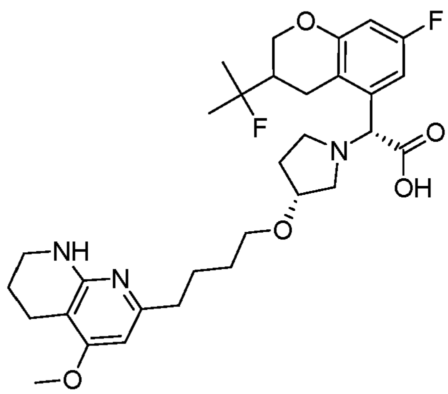

,

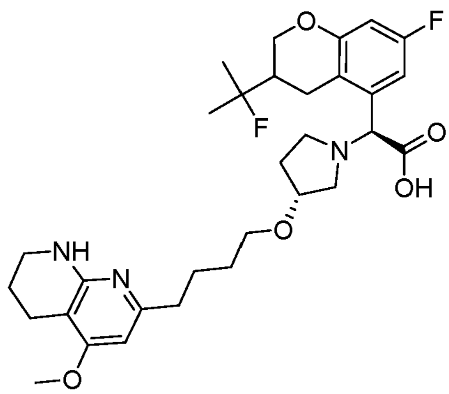

,

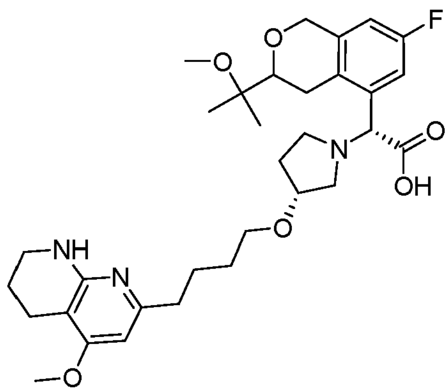

,

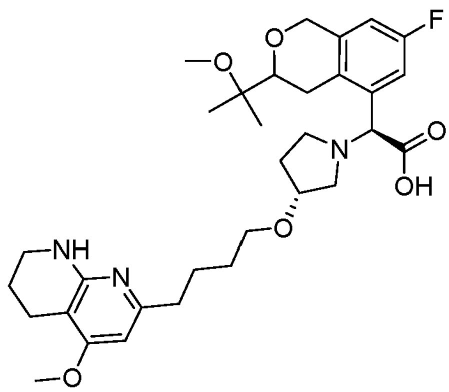

,

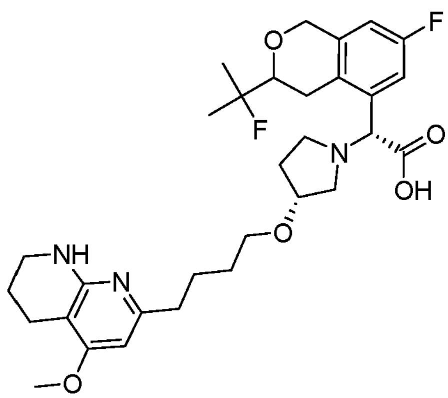

,

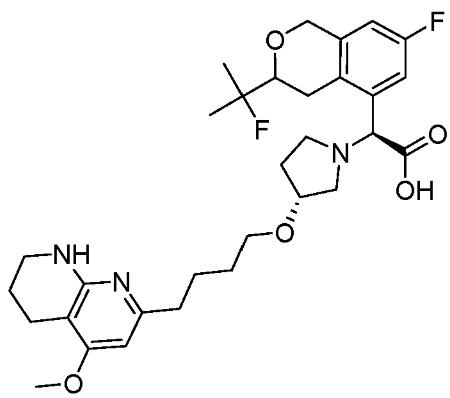

,

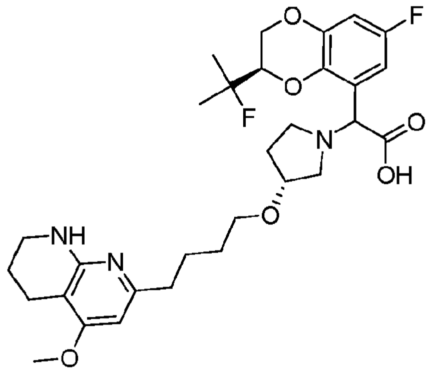

,

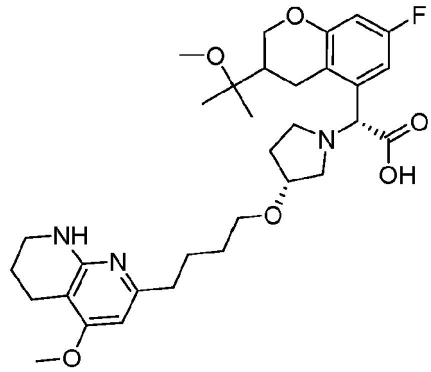

und

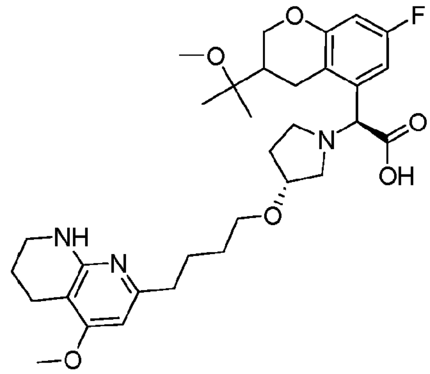

,

oder pharmazeutisch unbedenkliches Salz davon.

**8.** Verbindung nach Anspruch 1, ausgewählt aus der Gruppe bestehend aus:

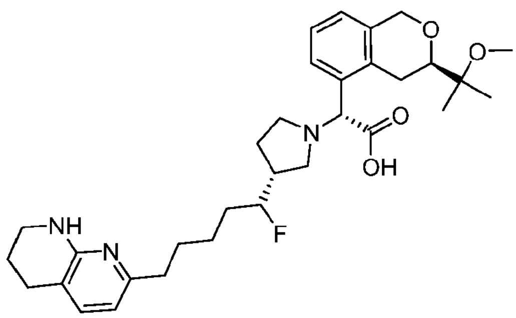

,

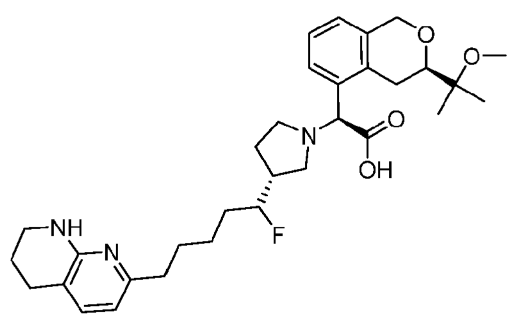

,

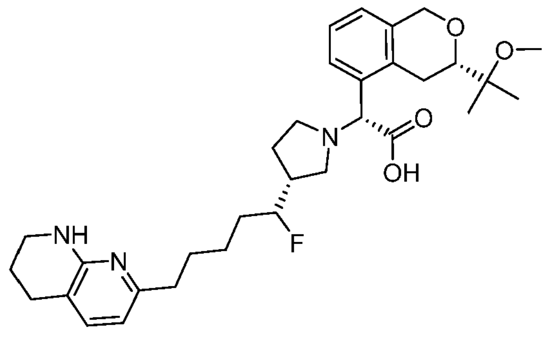

und

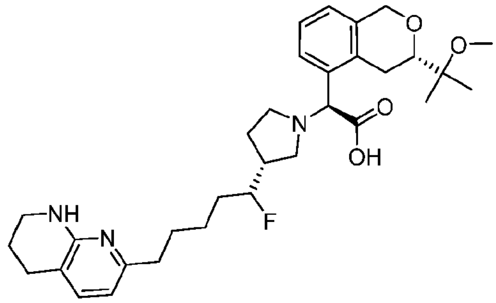

,

oder pharmazeutisch unbedenkliches Salz davon.

**9.** Verbindung nach Anspruch 1, ausgewählt aus der Gruppe bestehend aus:

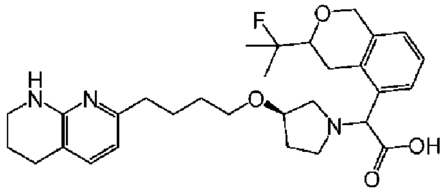 , 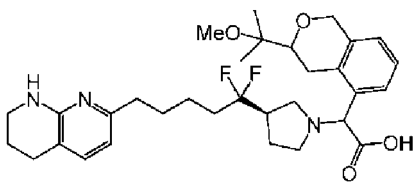 ,

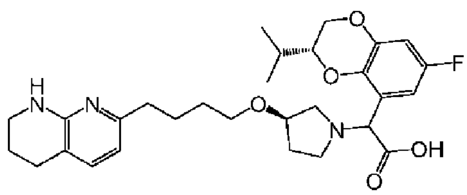 , 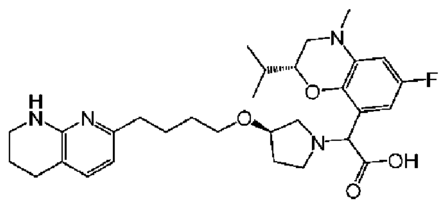 ,

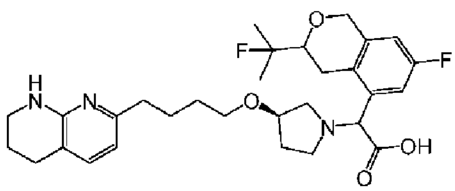 , 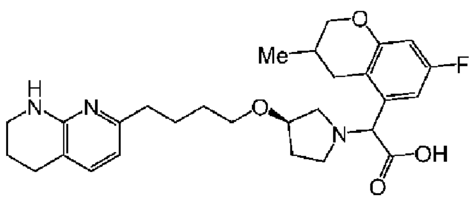 ,

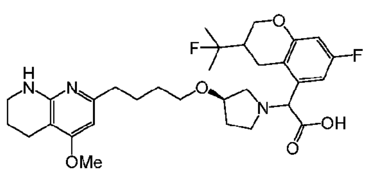 und 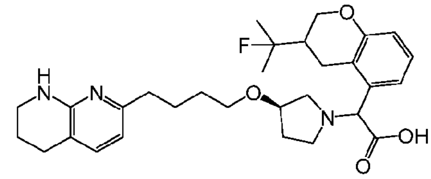 ,

oder pharmazeutisch unbedenkliches Salz davon.

**10.** Verbindung nach Anspruch 1, ausgewählt aus der Gruppe bestehend aus:

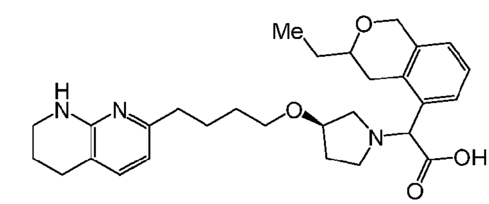,
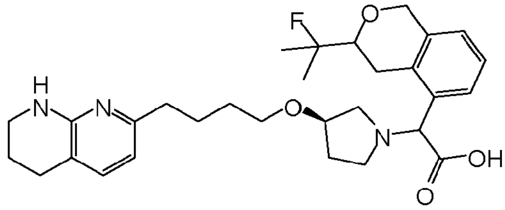,
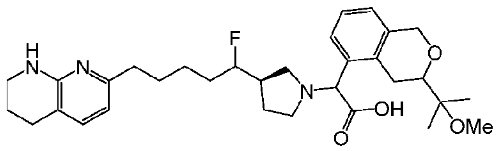,
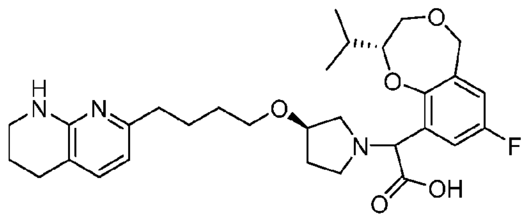,
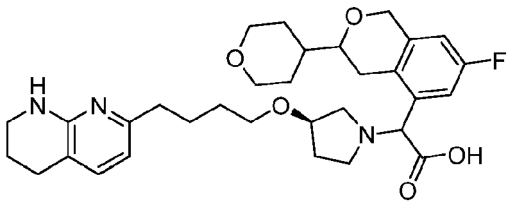,
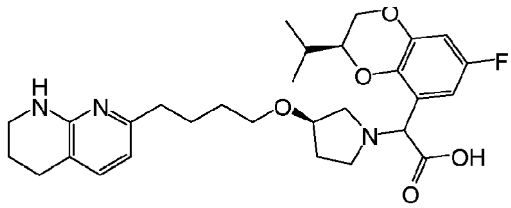,
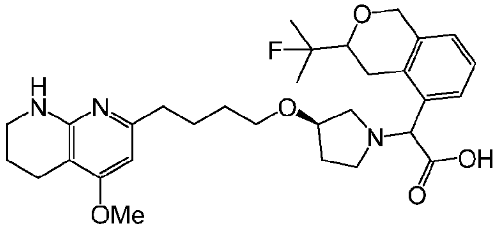,
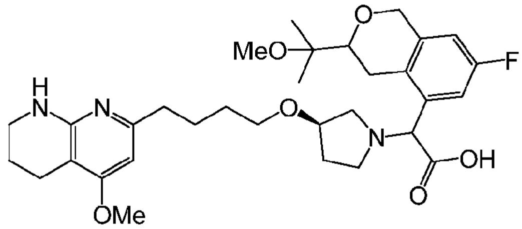,
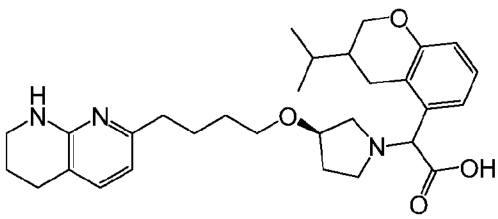,
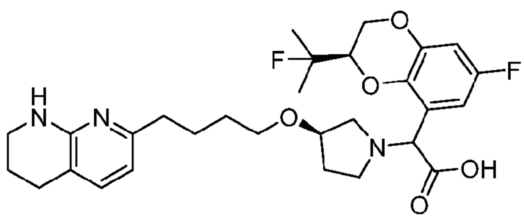,
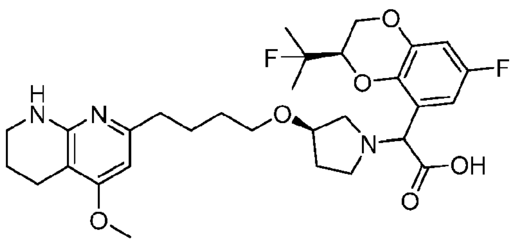,
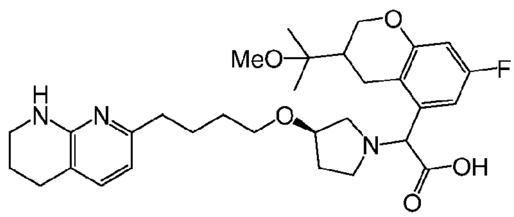,

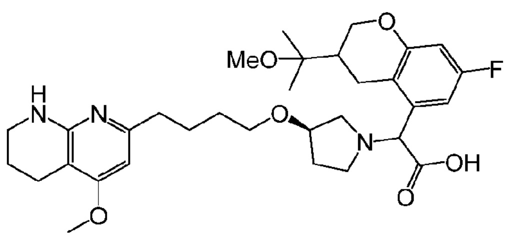
,
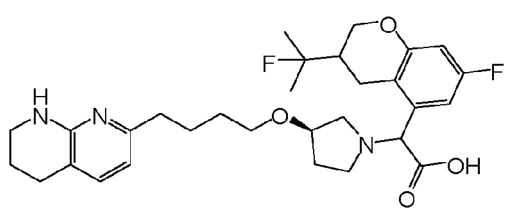
,

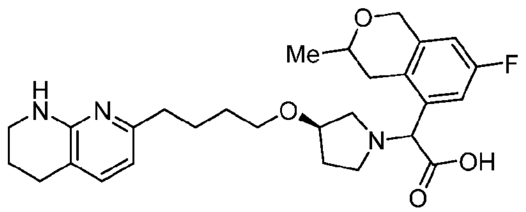

und

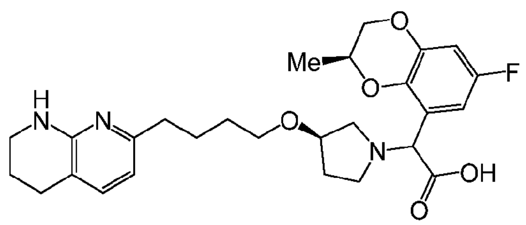
,

oder pharmazeutisch unbedenkliches Salz davon.

**Revendications**

1. Composé choisi dans le groupe constitué par :

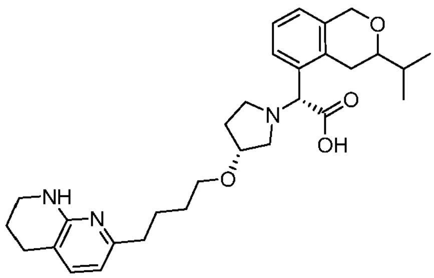
,
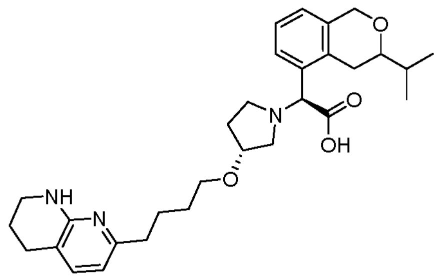
,

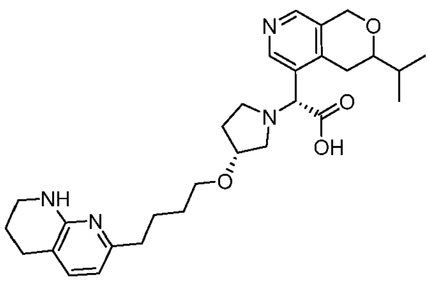
,
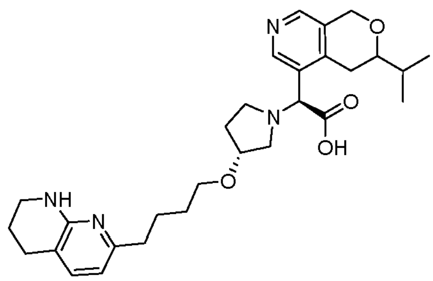
,

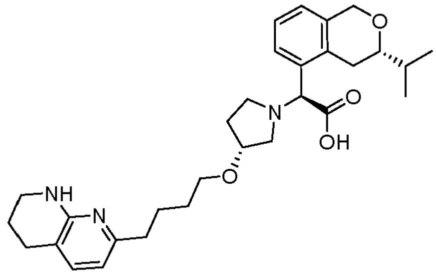
,
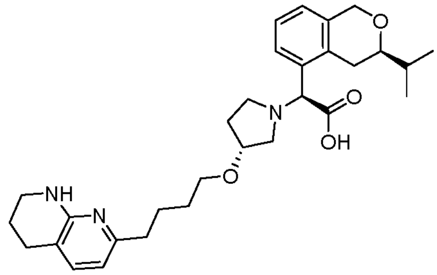
,

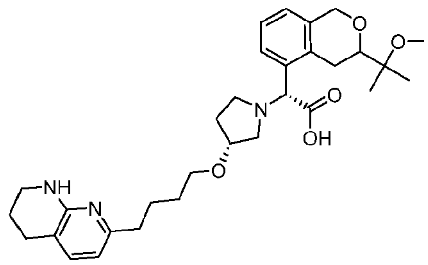
et
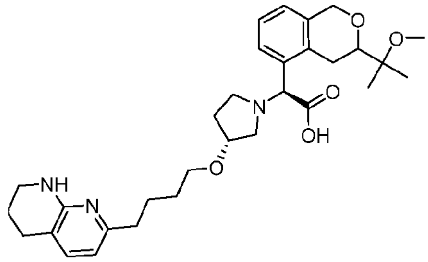
;

ou sel pharmaceutiquement acceptable correspondant ; ou choisi dans le groupe constitué par :

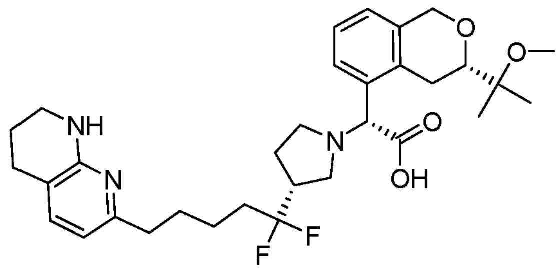
,

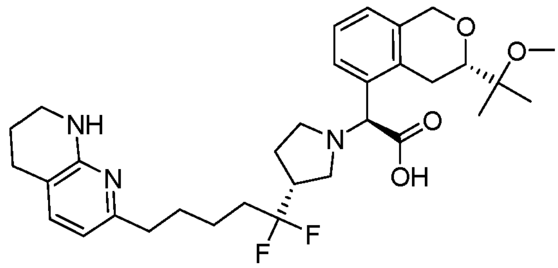
,

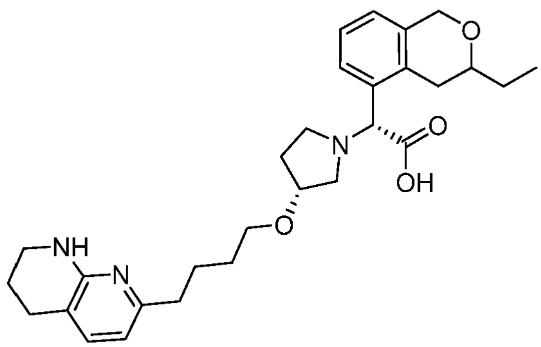
,

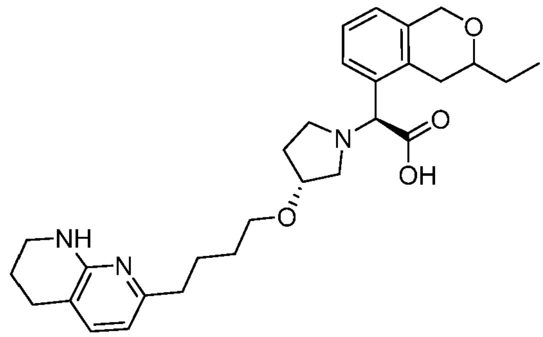

,

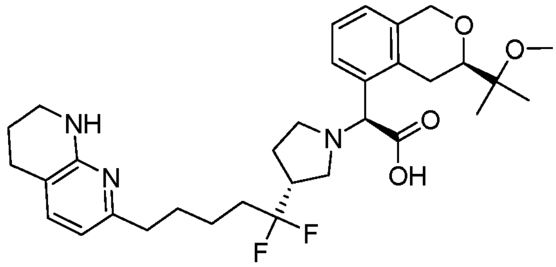

,

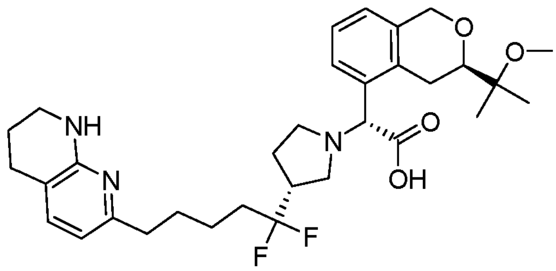

,

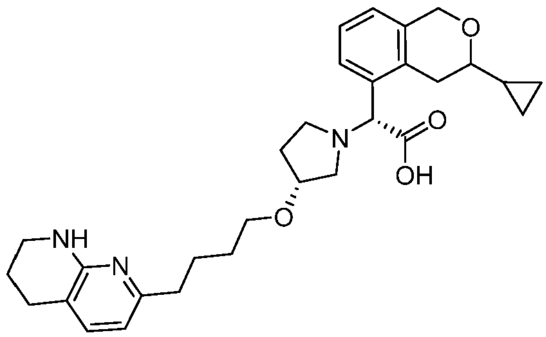

,

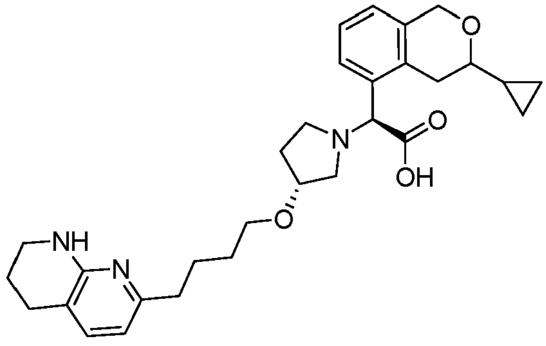

,

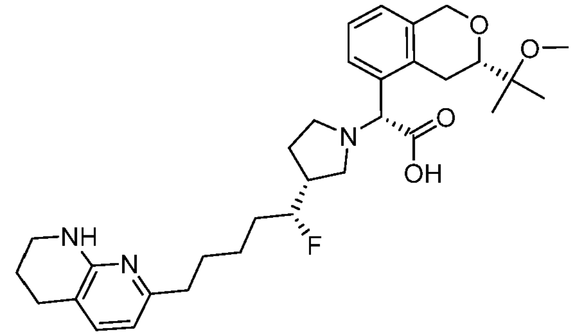

,

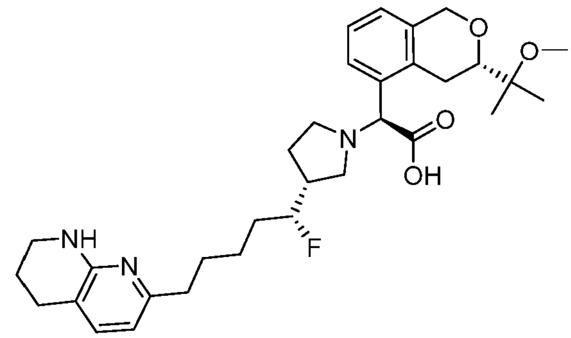

,

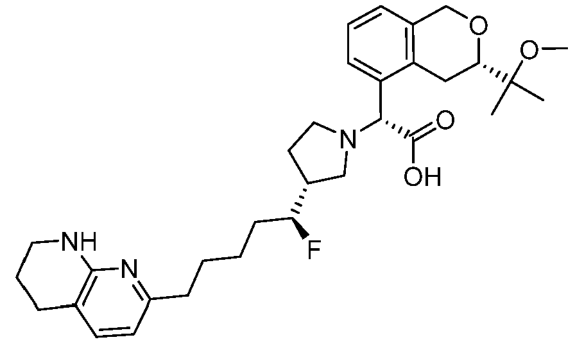

,

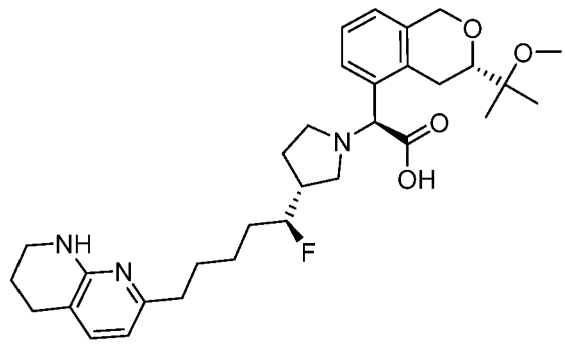

,

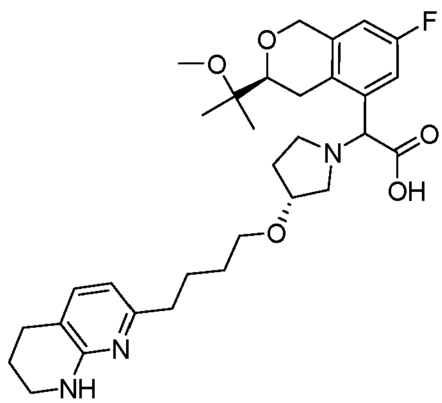

,

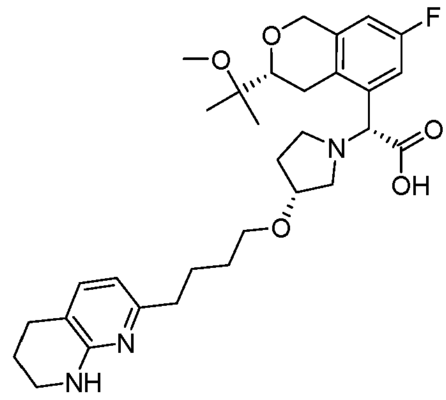,
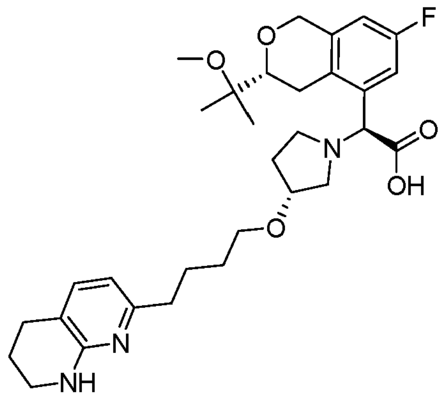,

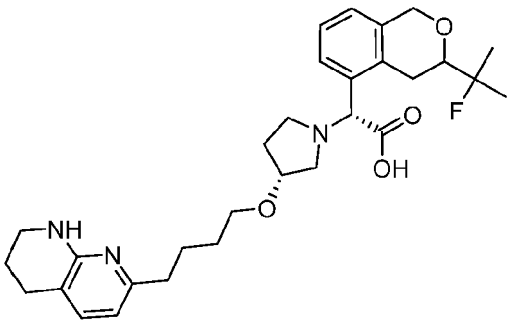

et

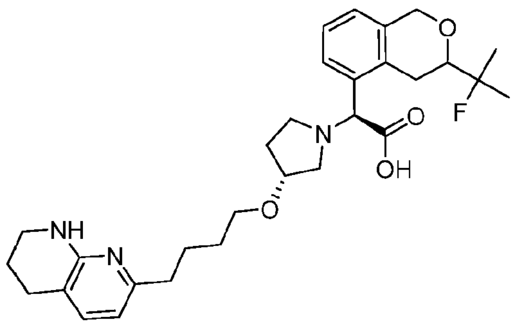;

ou sel pharmaceutiquement acceptable correspondant ; ou

choisi dans le groupe constitué par :

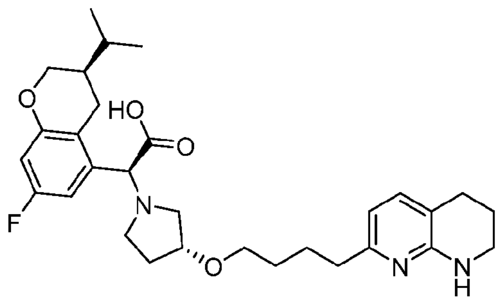,
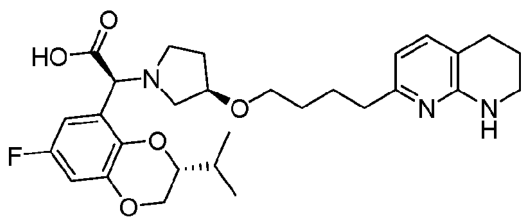,

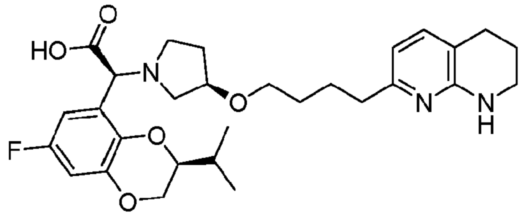
,
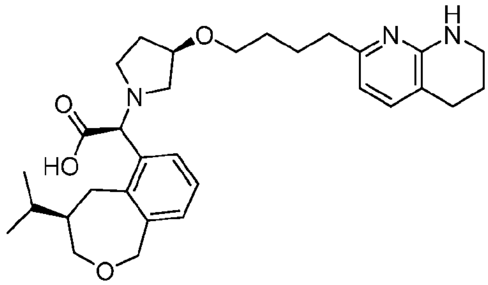
,
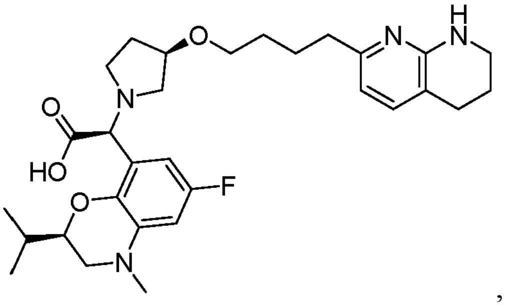
,
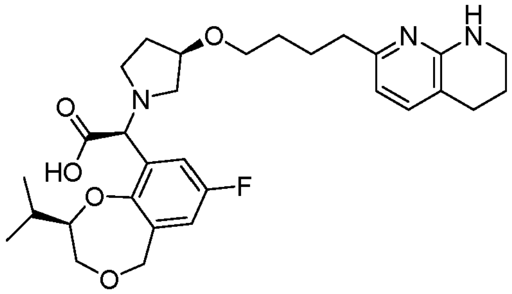
,
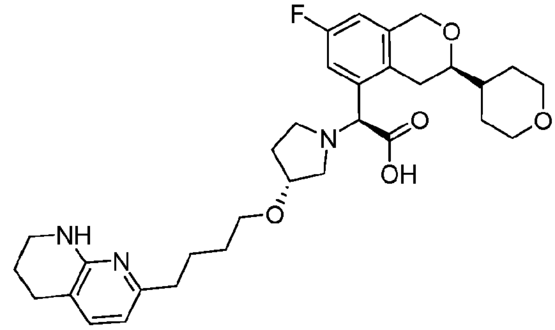
et
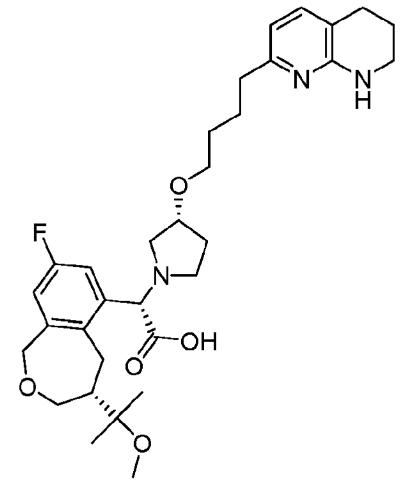
;

ou sel pharmaceutiquement acceptable correspondant ; ou choisi dans le groupe constitué par :

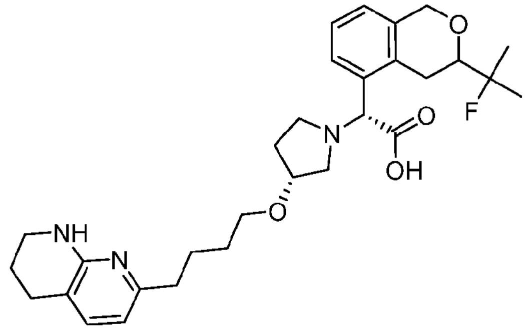
,
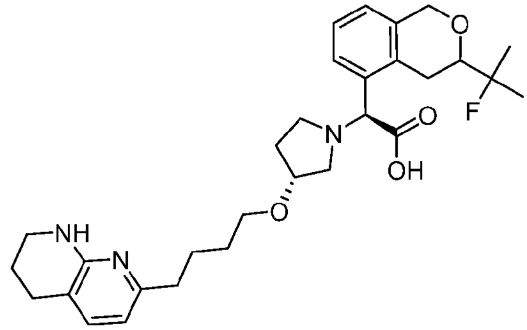
,

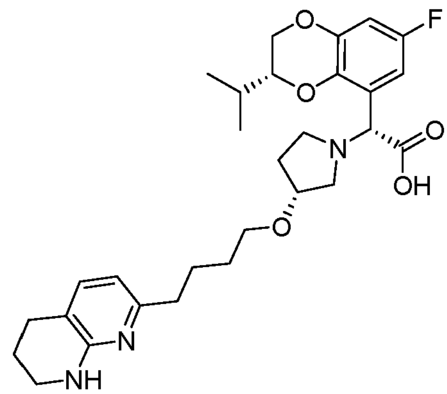 , 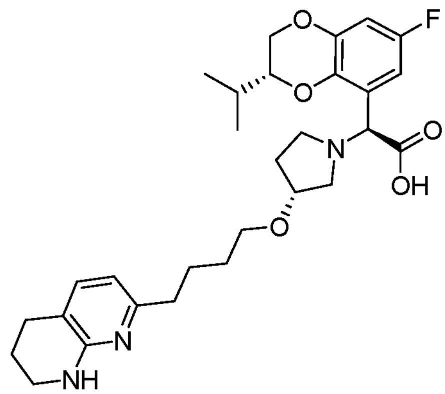 ,

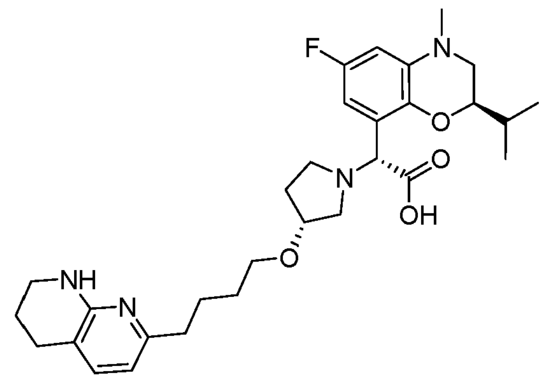 ,

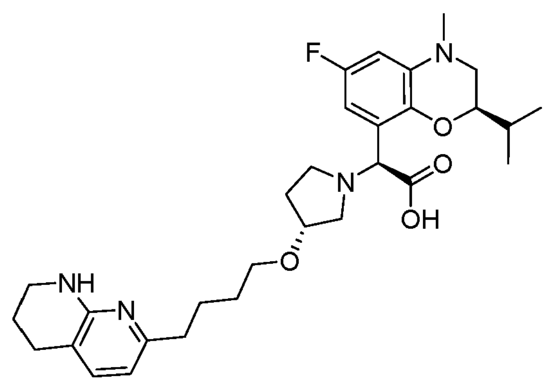 , 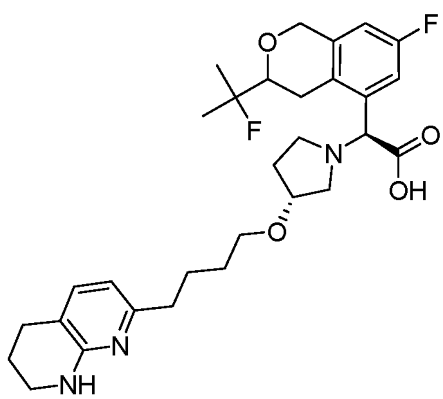 ,

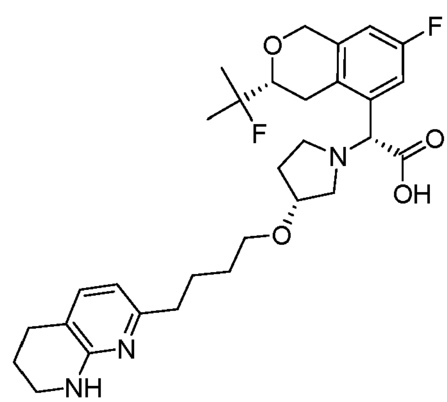 , 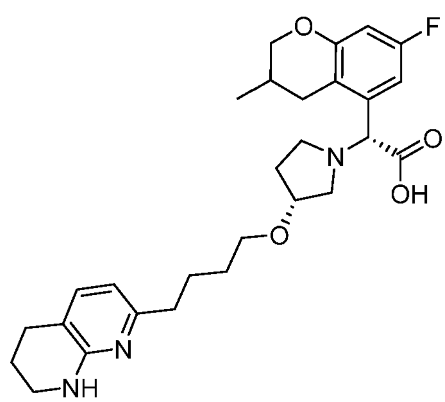 ,

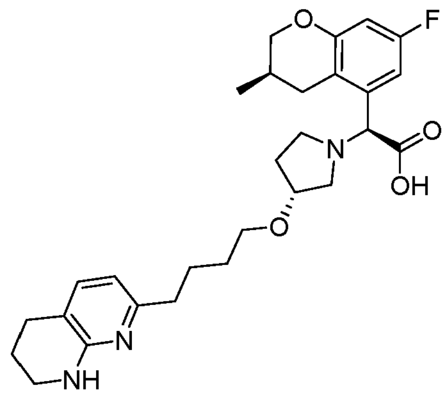

,

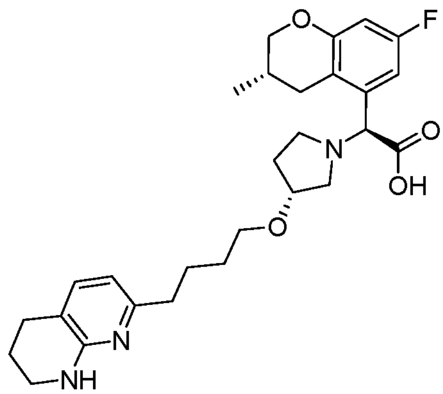

,

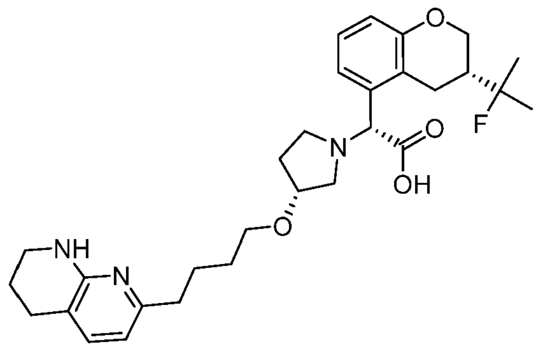

,

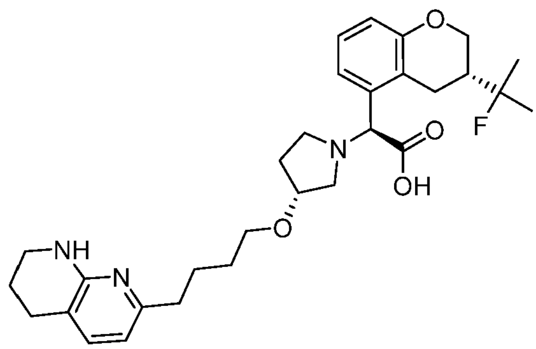

,

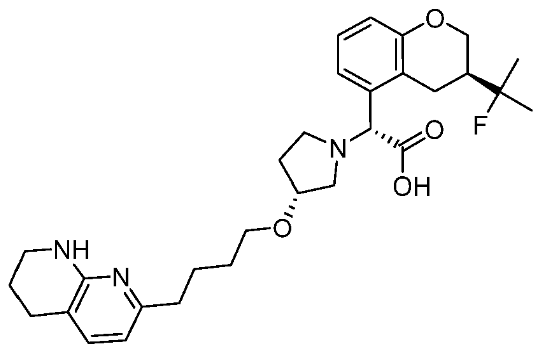

,

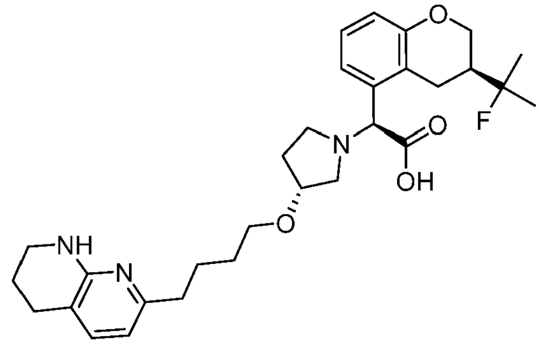

,

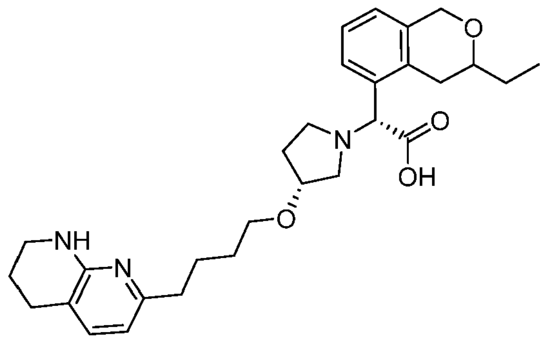

,

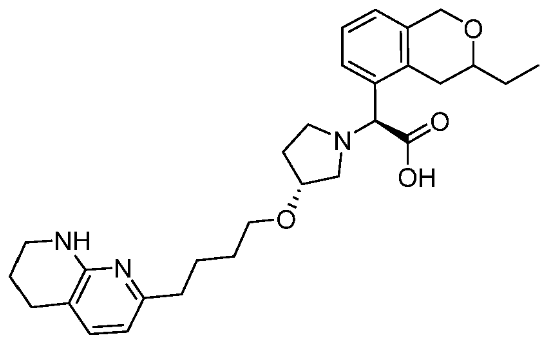

,

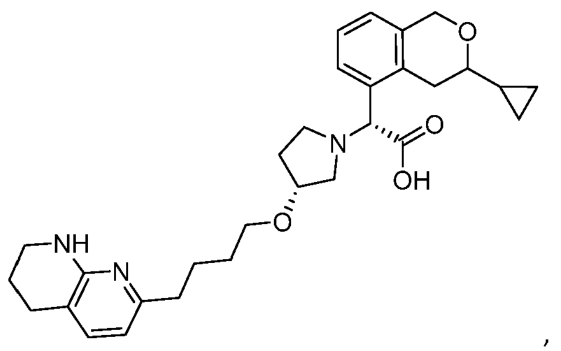

,

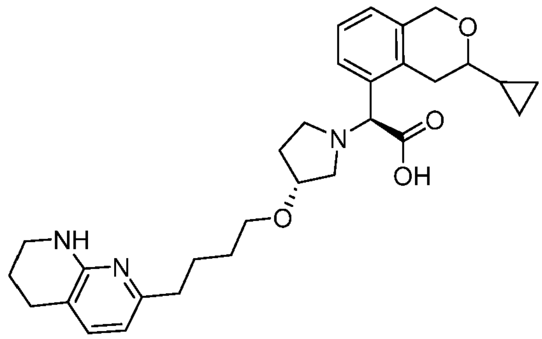

,

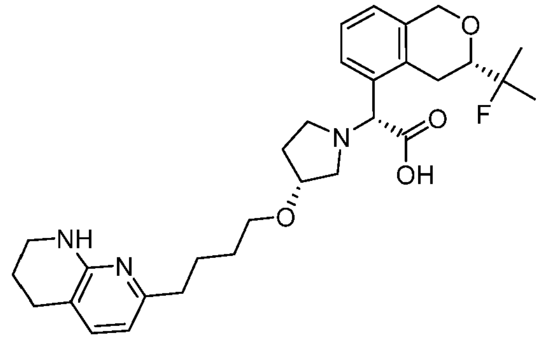

,

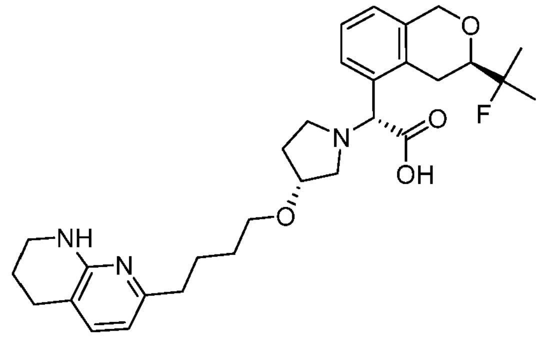

,

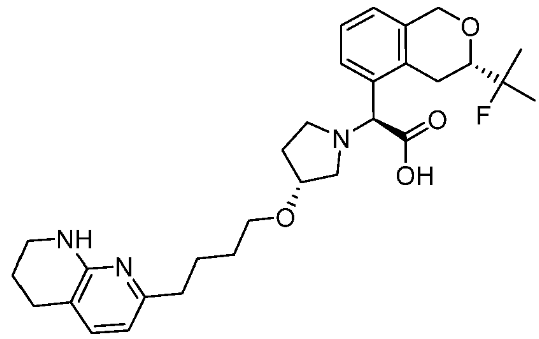

,

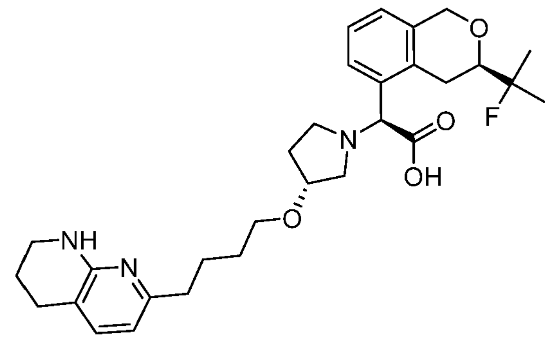,

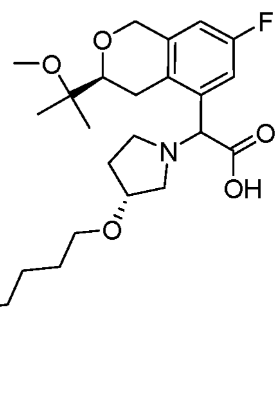,

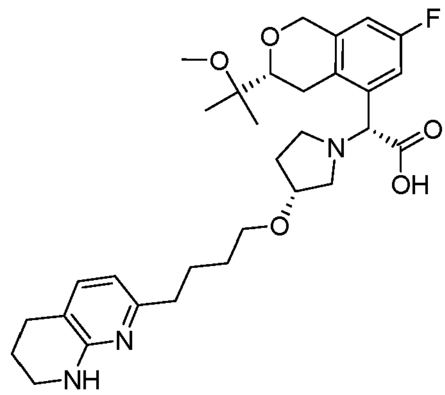,

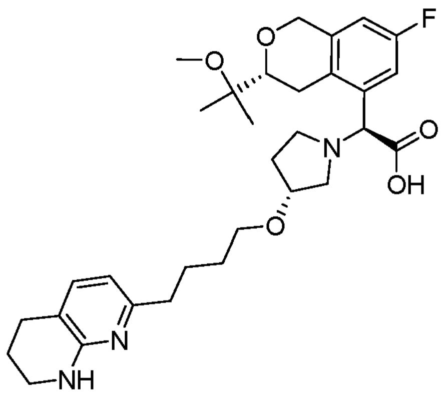,

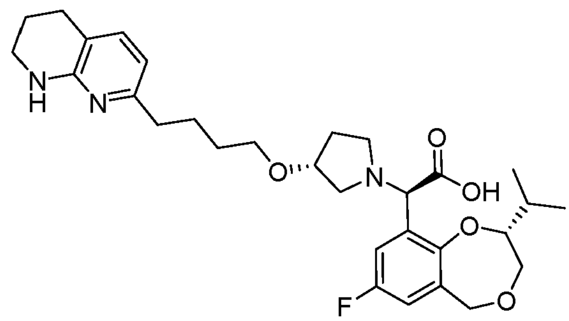,

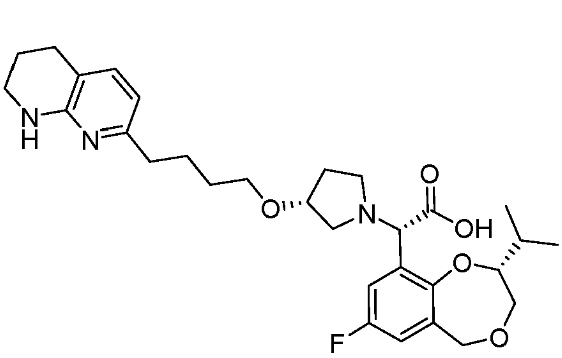,

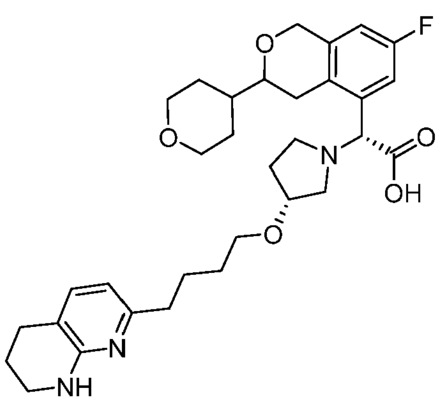,

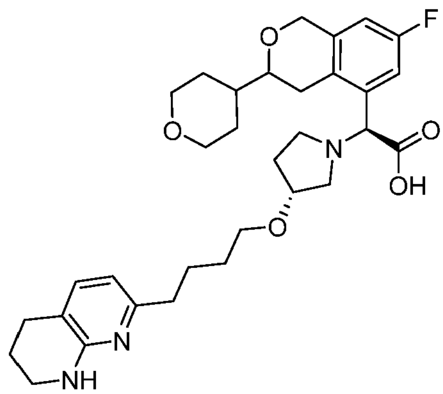

,

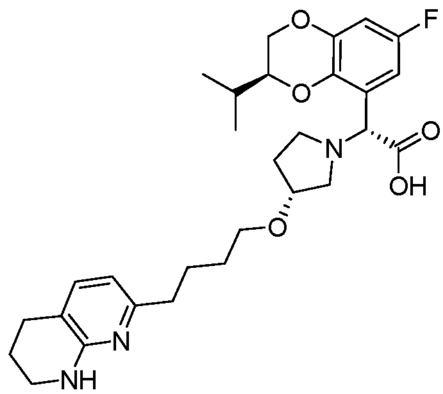

,

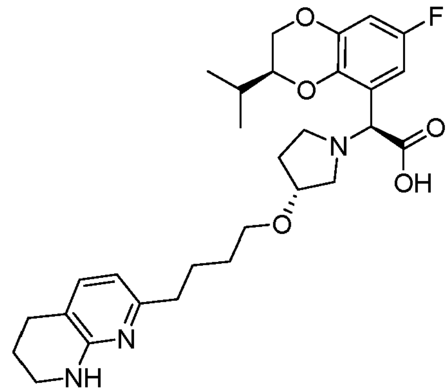

,

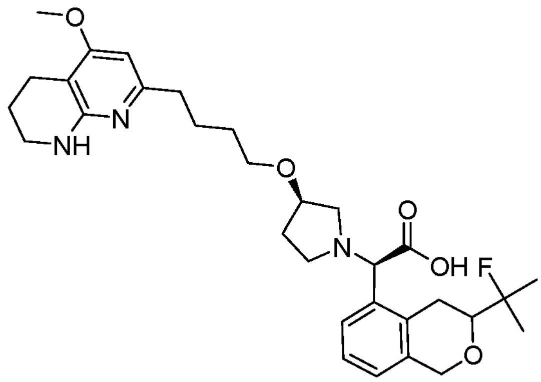

,

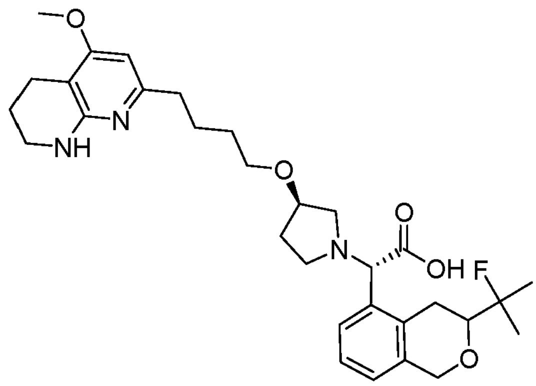

,

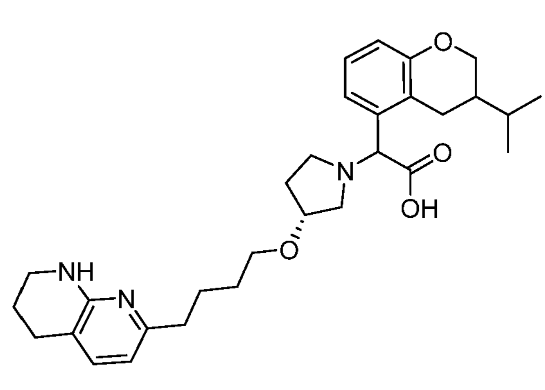

,

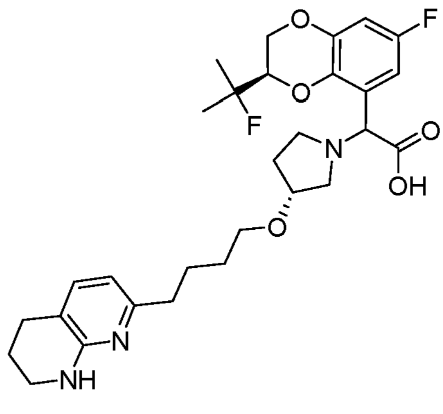

,

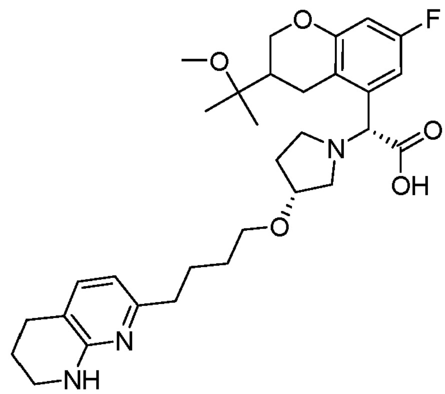

,

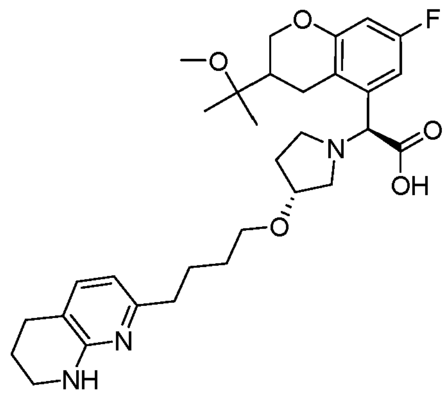

,

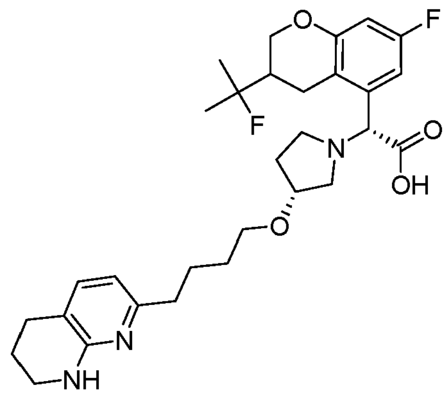

,

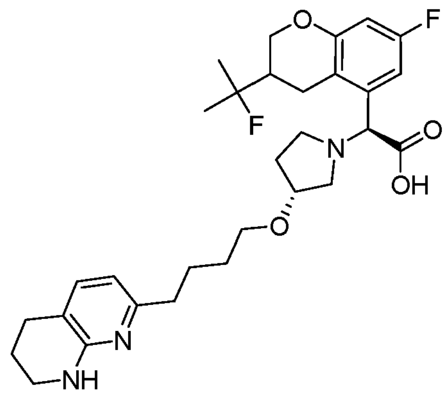

,

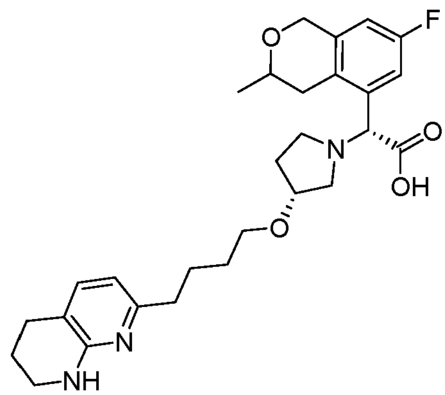

,

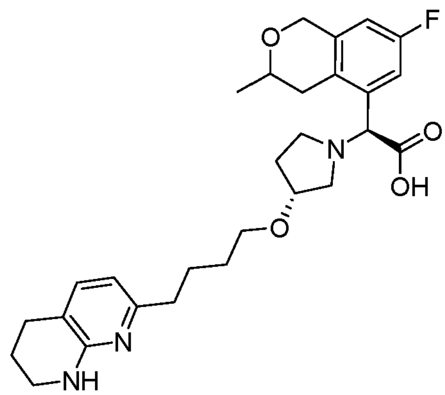

,

et

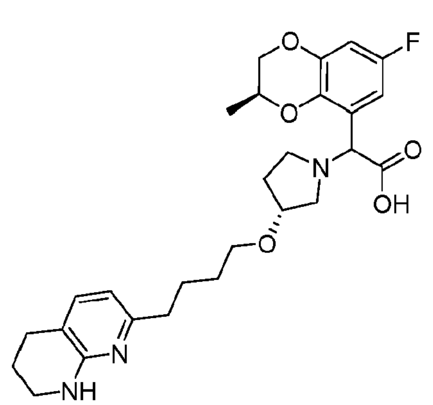

;

ou sel pharmaceutiquement acceptable correspondant ; ou choisi dans le groupe constitué par :

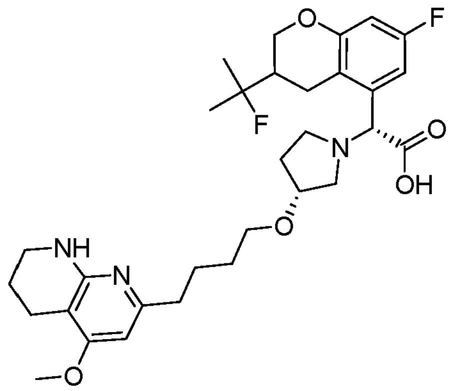
,
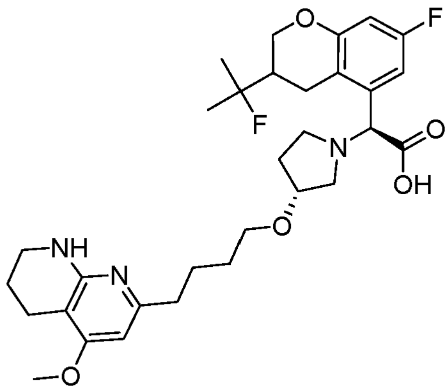
,
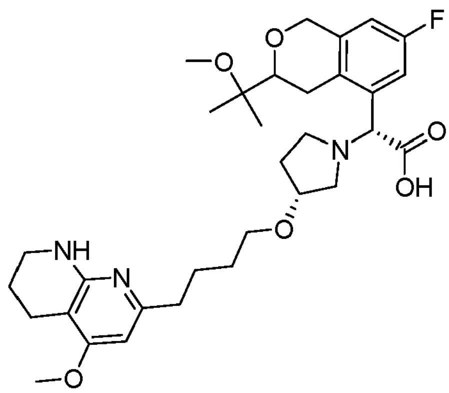
,
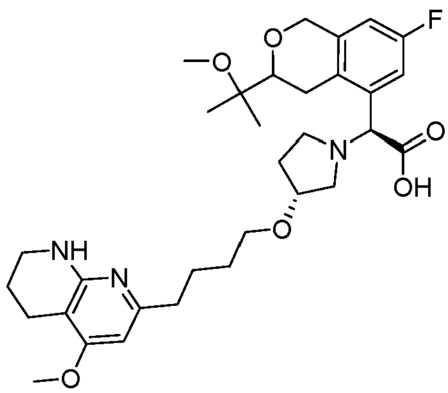
,
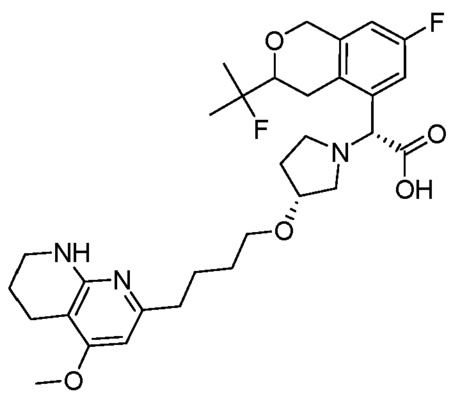
,
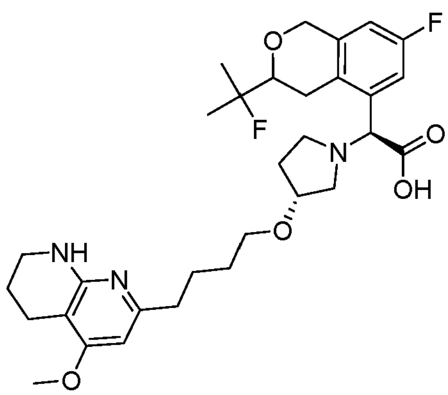
,
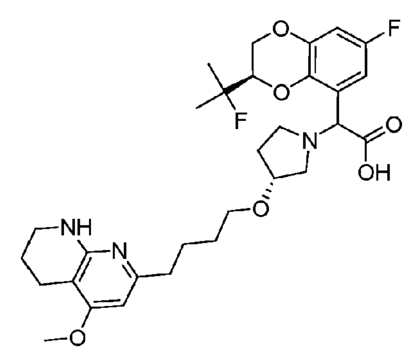
,
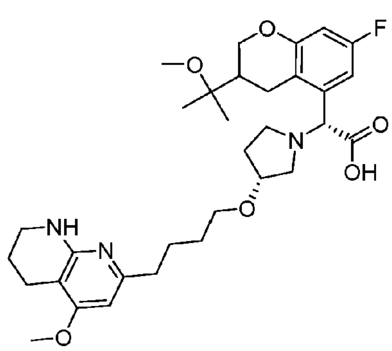

et

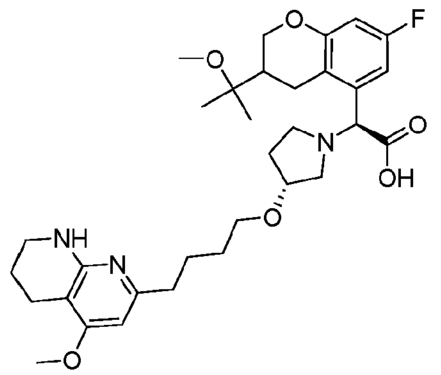

;

ou sel pharmaceutiquement acceptable correspondant ; ou choisi dans le groupe constitué par :

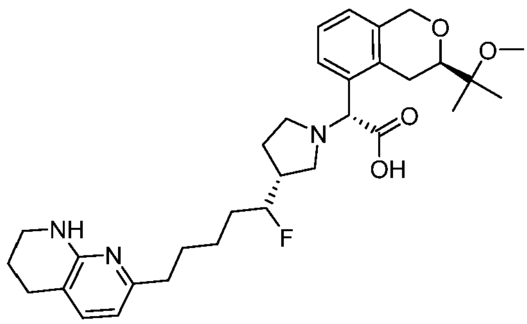

,

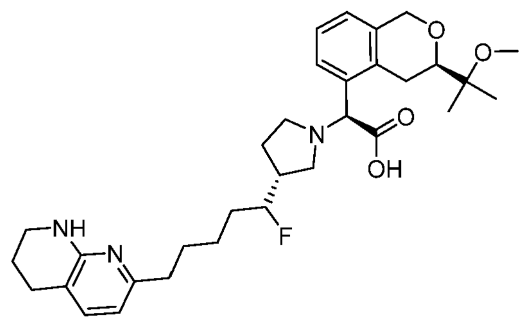

,

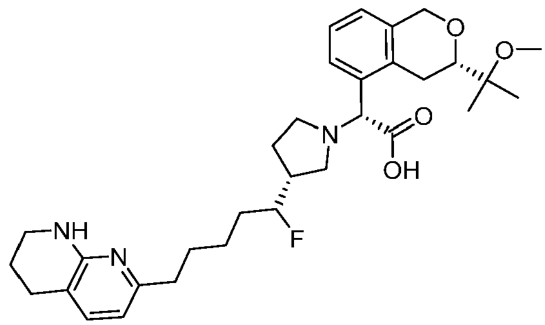

et

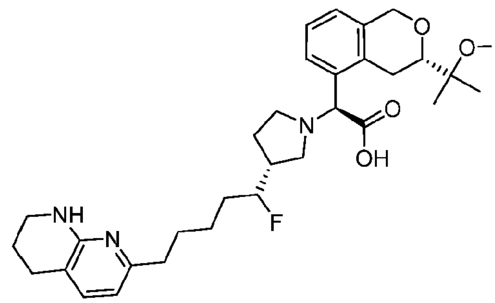

;

ou sel pharmaceutiquement acceptable correspondant ; ou choisi dans le groupe constitué par :

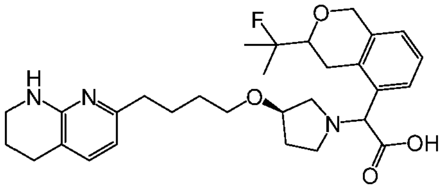, 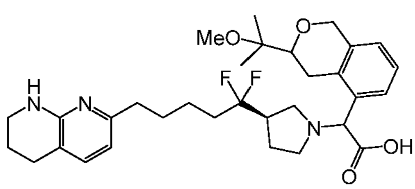,

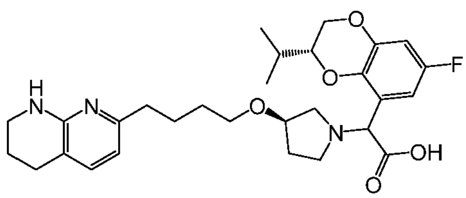, 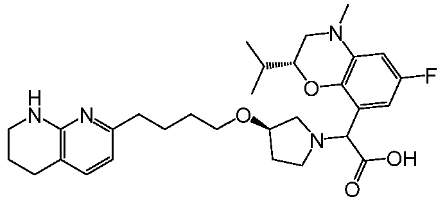,

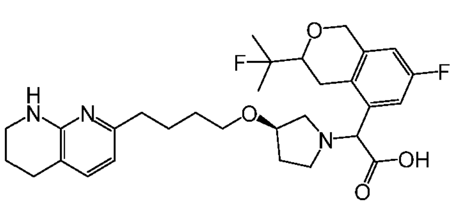, 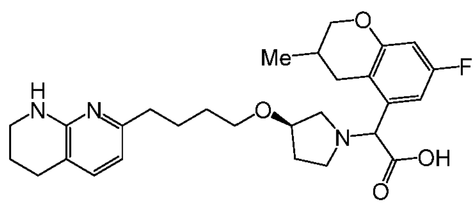,

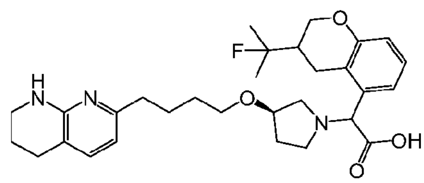 et 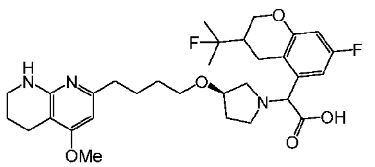 ;

ou sel pharmaceutiquement acceptable correspondant ; ou
choisi dans le groupe constitué par :

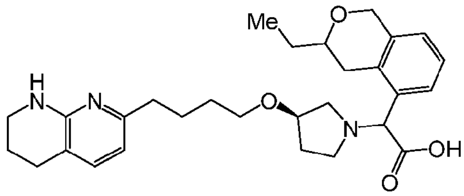, 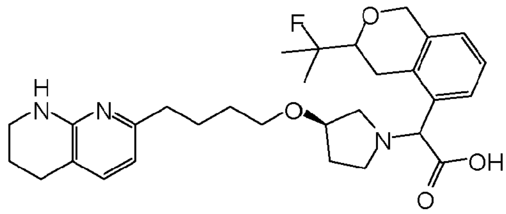,

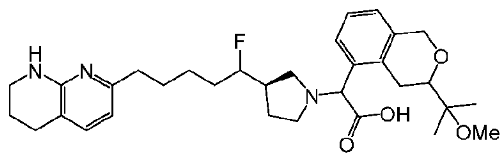, 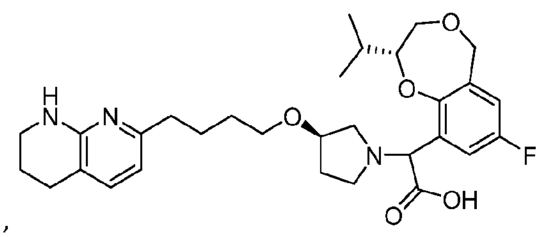,

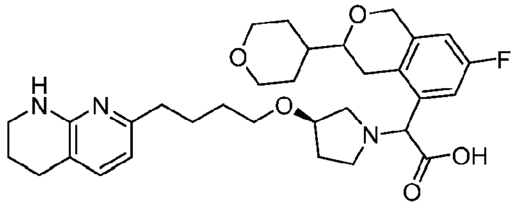,
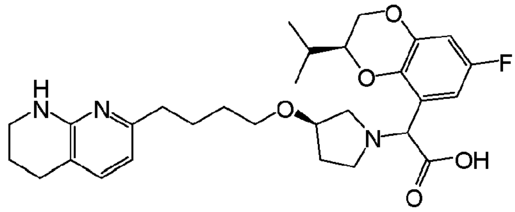,

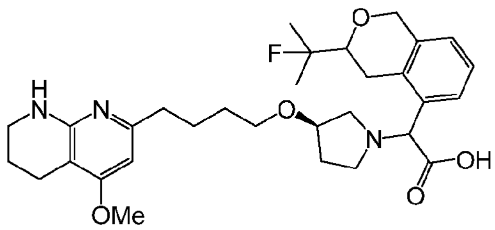,
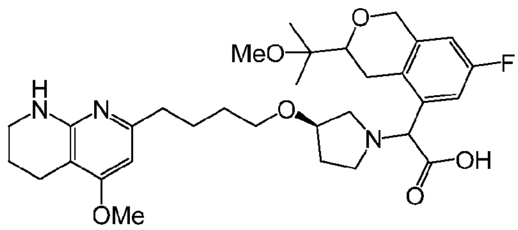,

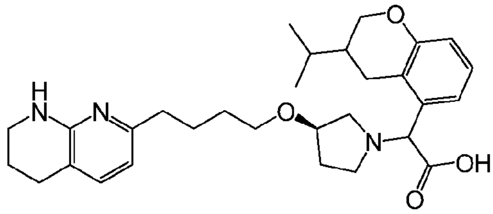,
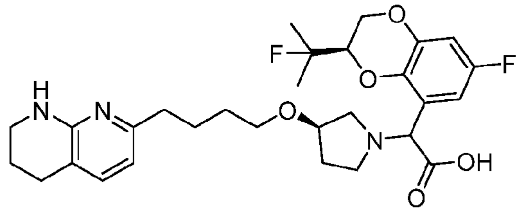,

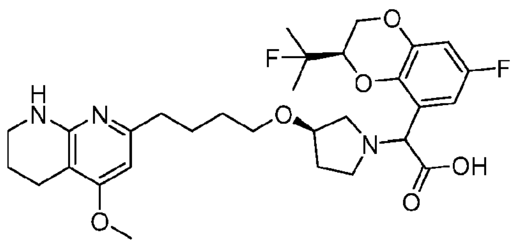,
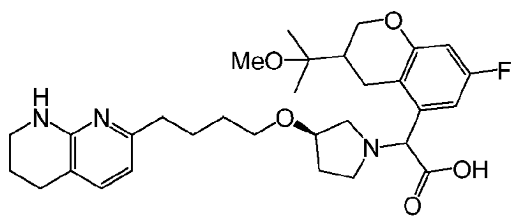,

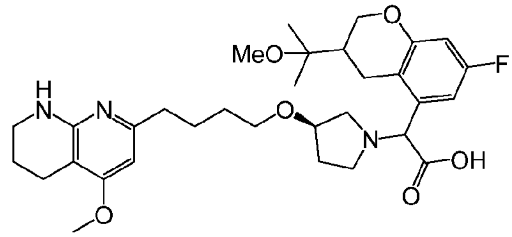,
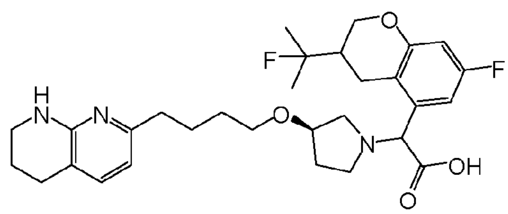,

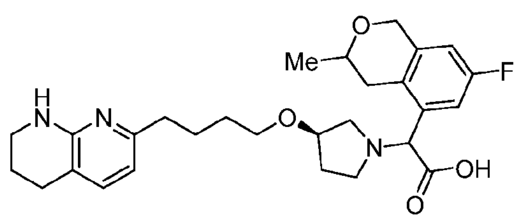

et

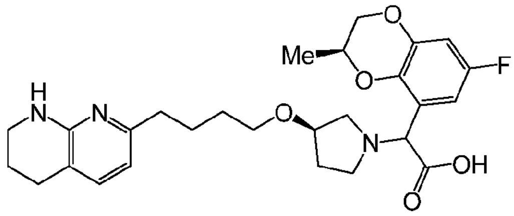

;

ou sel pharmaceutiquement acceptable correspondant.

**2.** Composé selon la revendication 1 choisi dans le groupe constitué par :

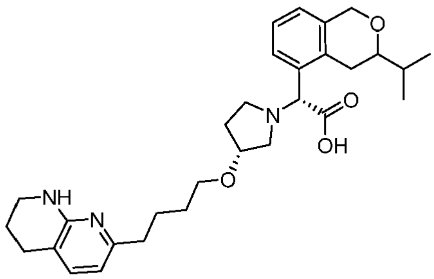

,

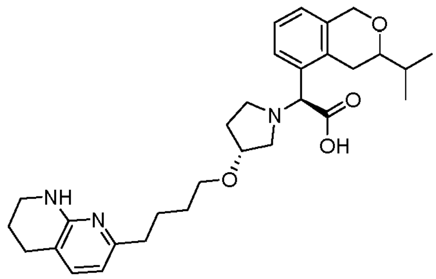

,

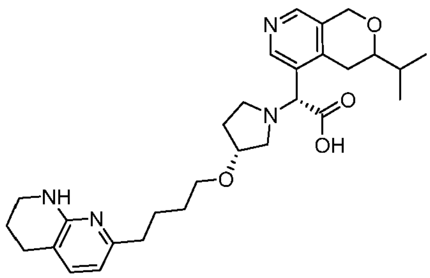

,

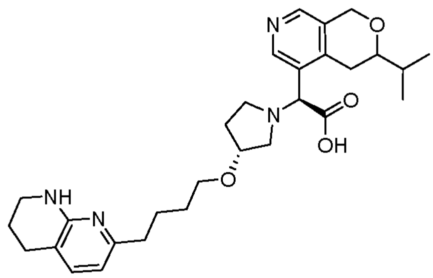

,

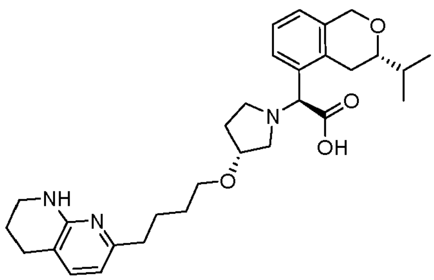

,

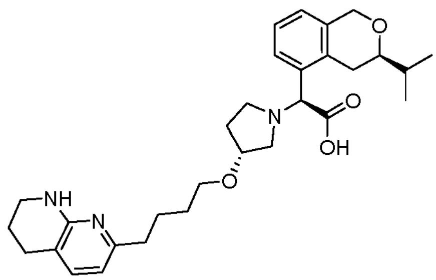

,

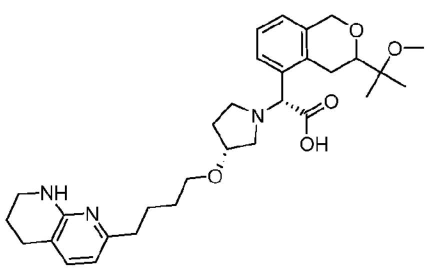

et

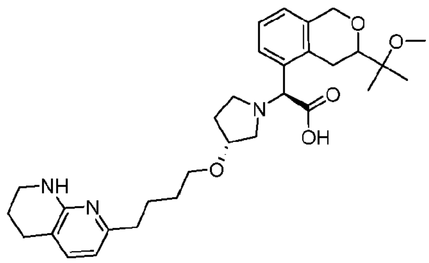

;

ou sel pharmaceutiquement acceptable correspondant.

**3.** Composé selon la revendication 1 choisi dans le groupe constitué par :

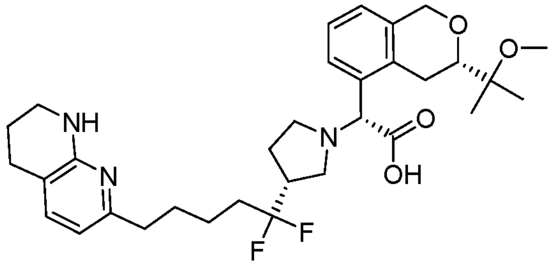

,

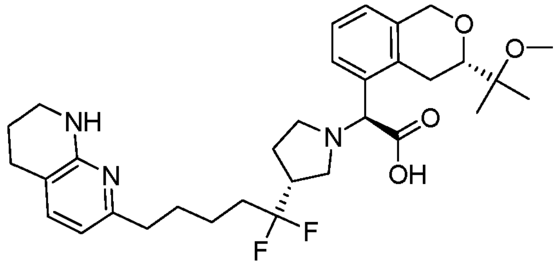

,

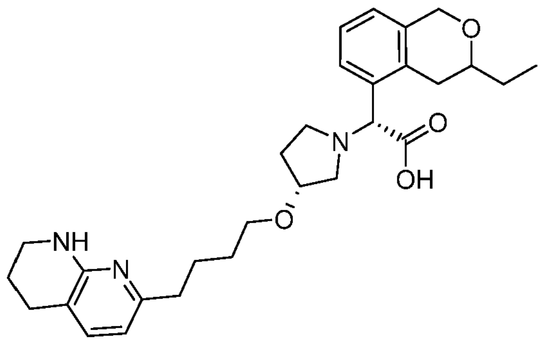

.

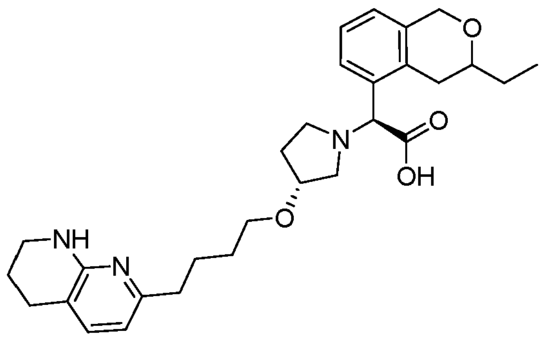

,

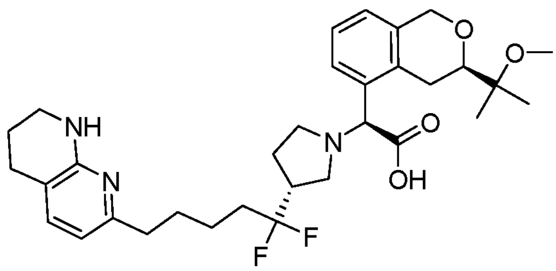

,

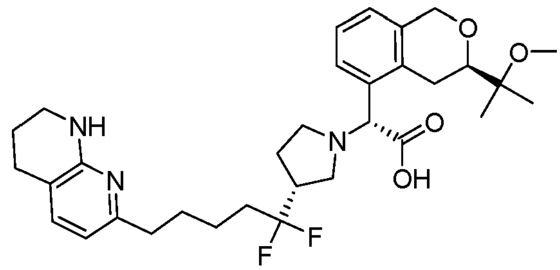

,

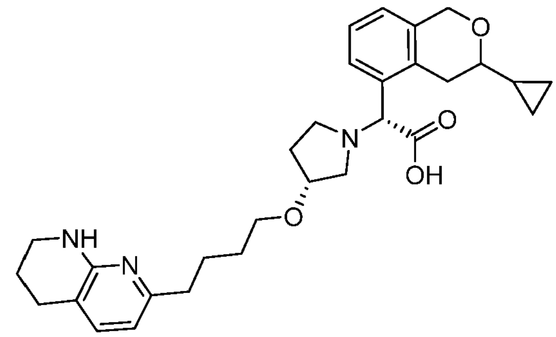

,

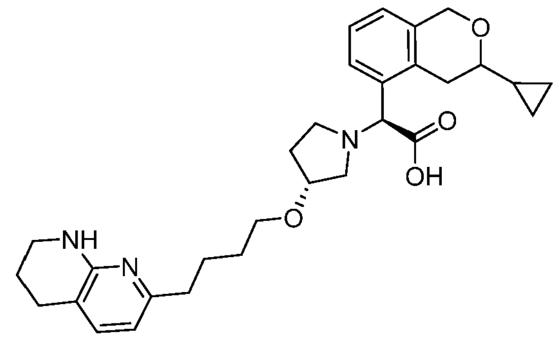

,

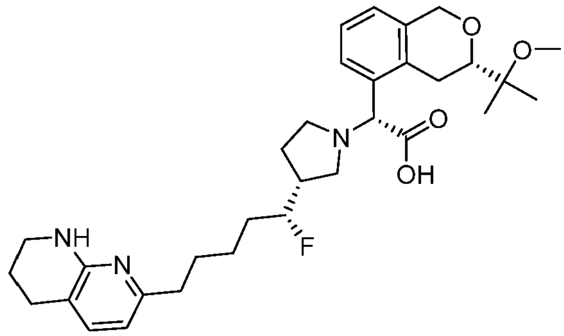

,

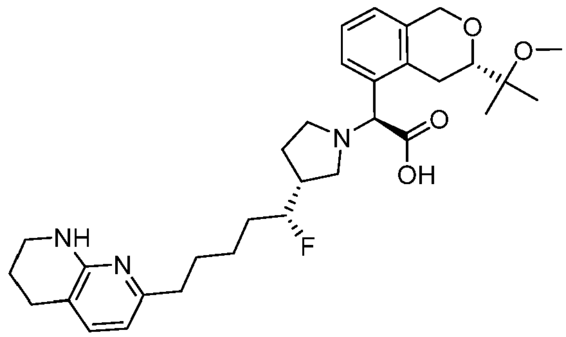

,

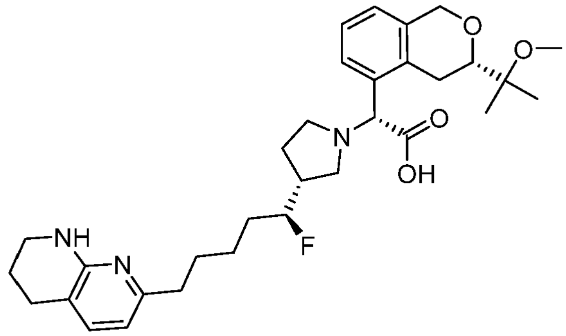

,

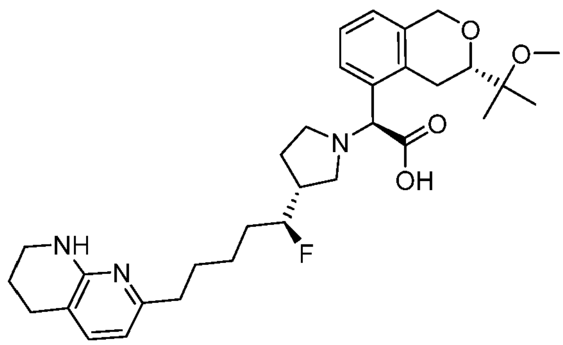

,

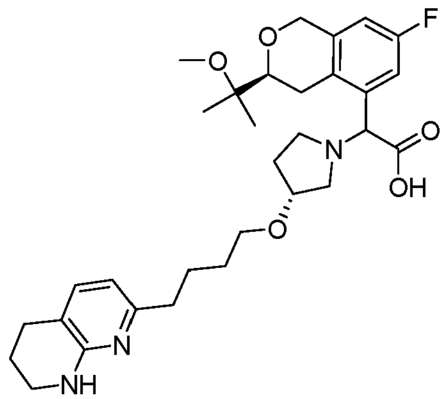

,

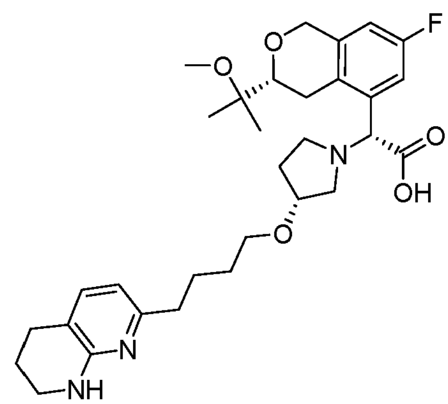

,

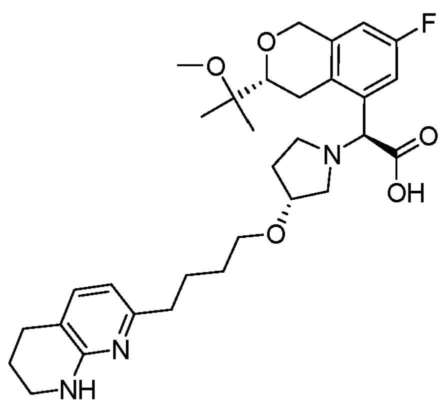

,

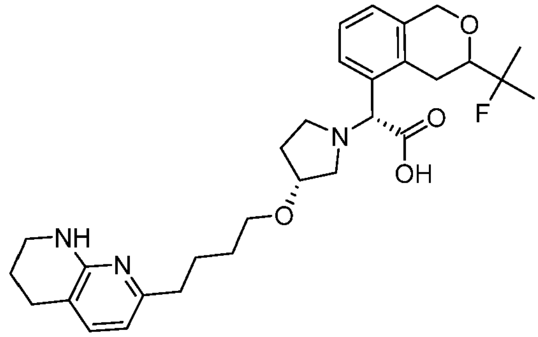

et

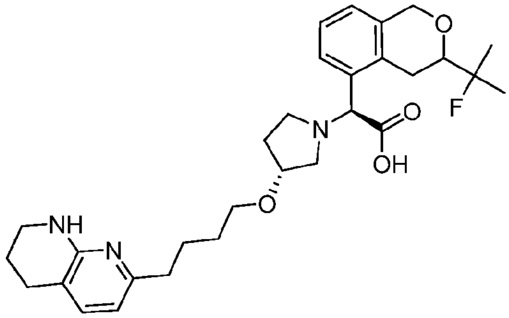

;

ou sel pharmaceutiquement acceptable correspondant.

**4.** Composé selon la revendication 1 choisi dans le groupe constitué par :

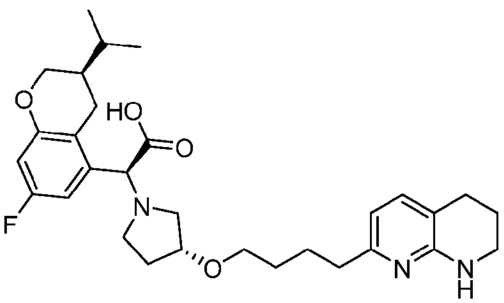

,

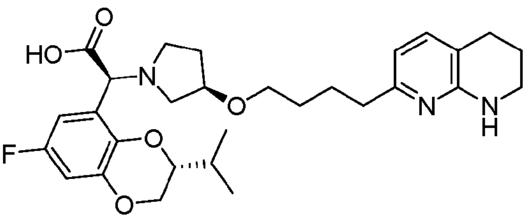

,

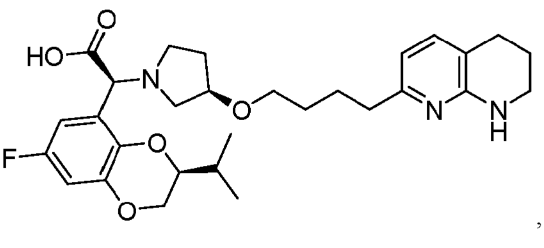

,

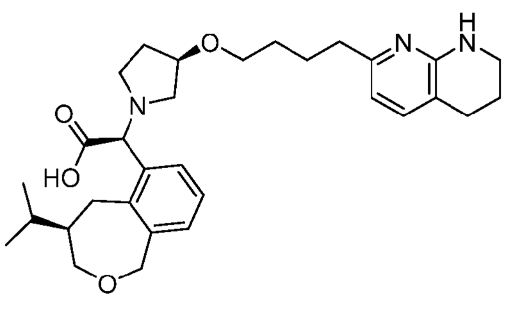

,

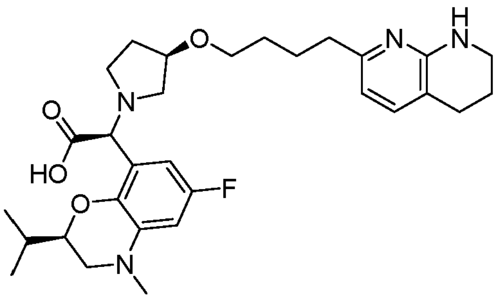
,
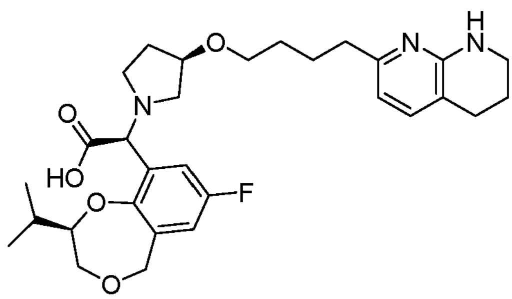
,

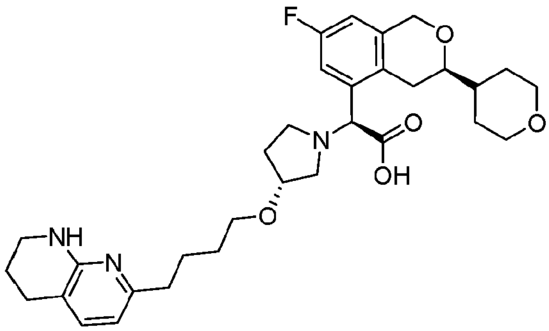

et

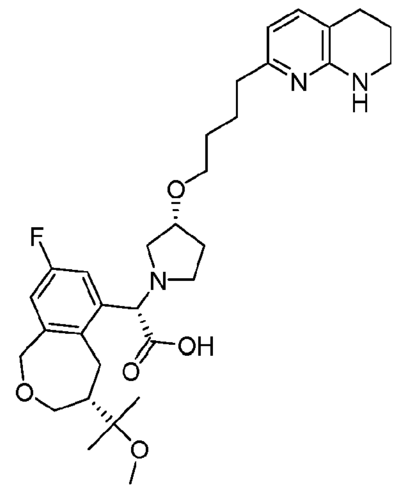
;

ou sel pharmaceutiquement acceptable correspondant.

**5.** Composé selon la revendication 1 choisi dans le groupe constitué par :

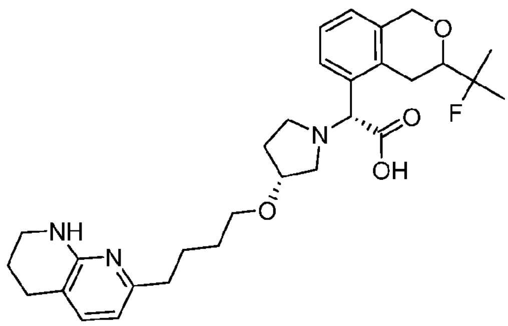
,
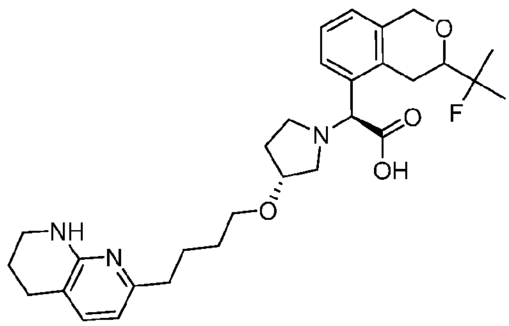
,

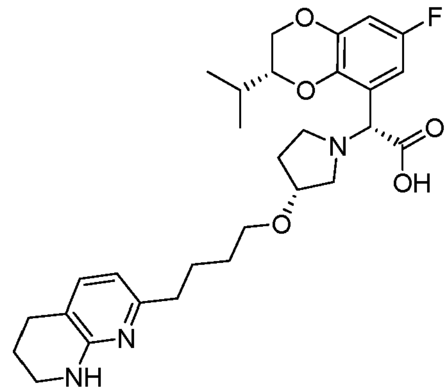

,

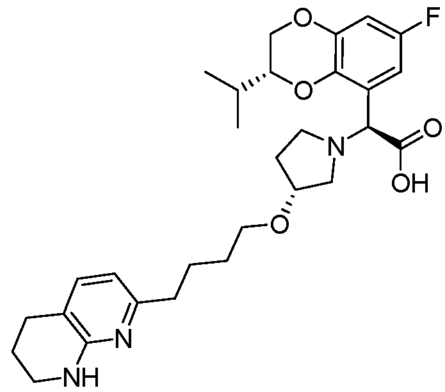

,

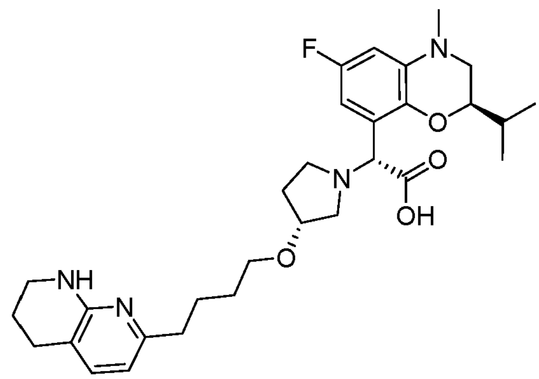

,

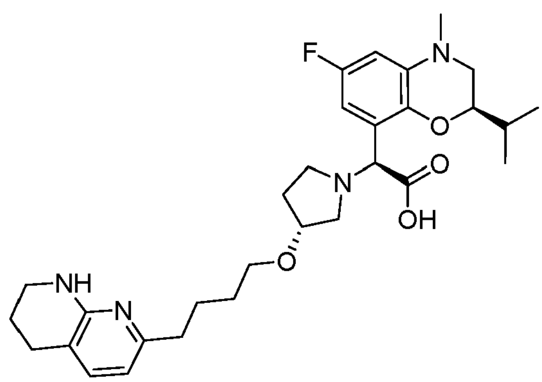

,

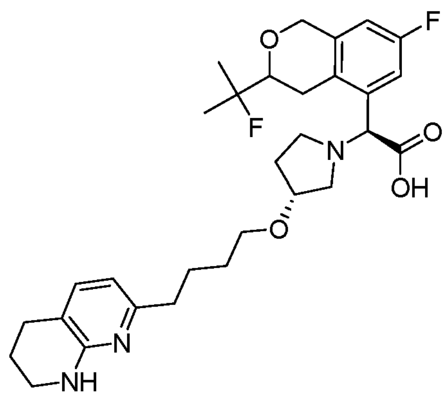

,

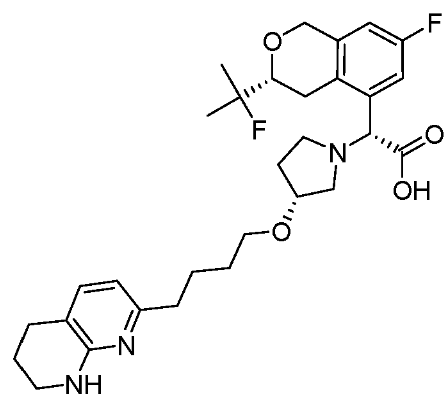

,

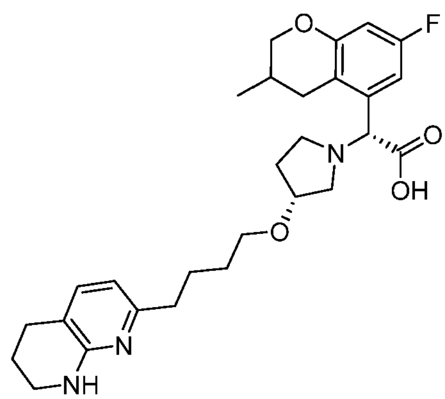

,

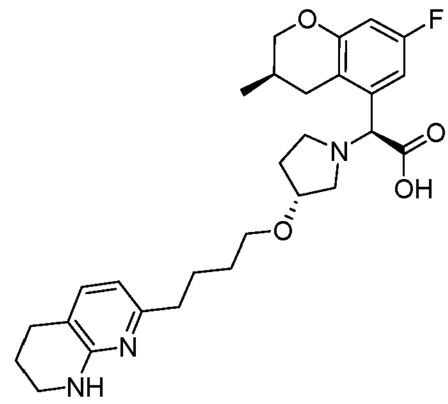 ,

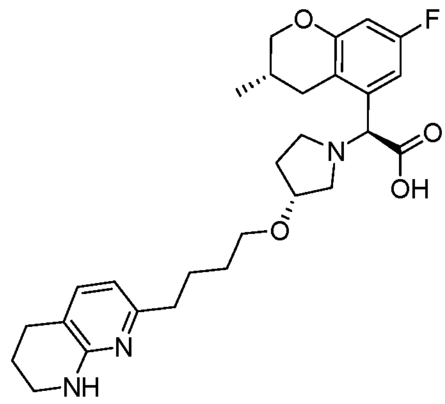 ,

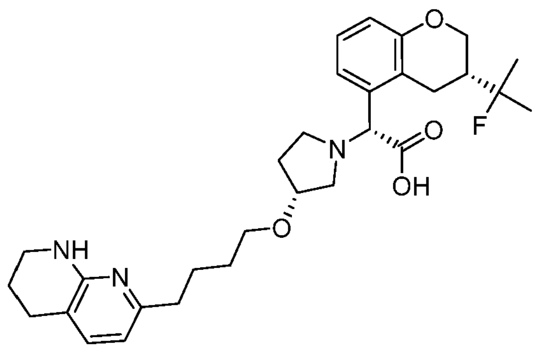 ,

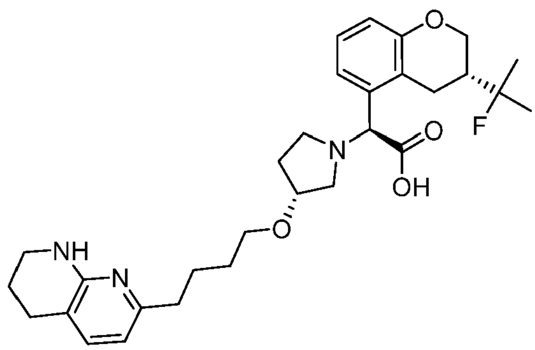 ,

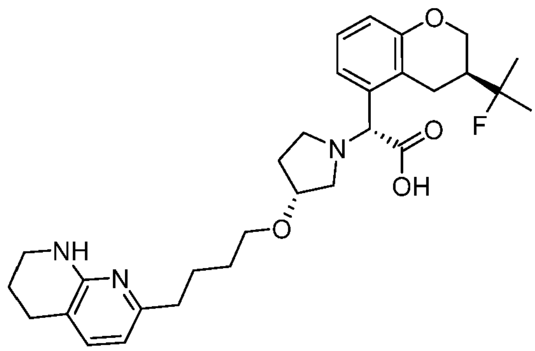 ,

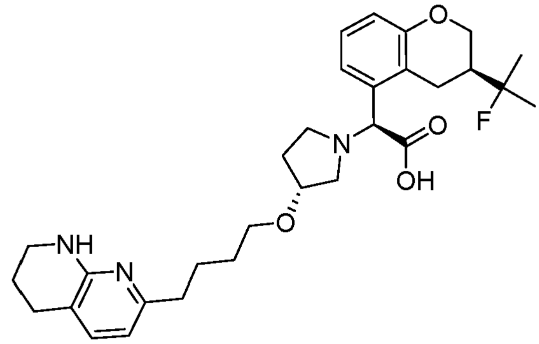

,

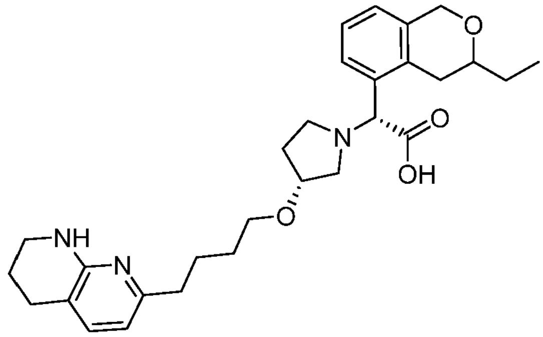

,

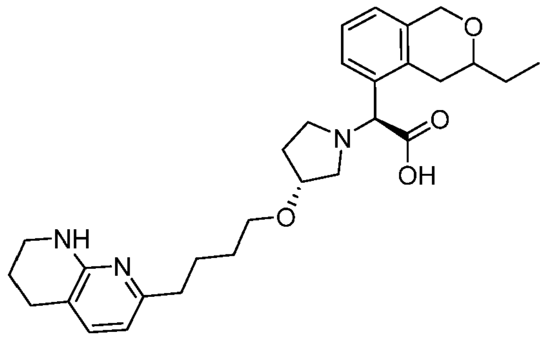

,

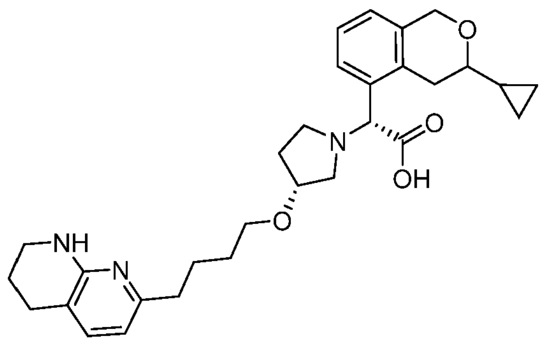

,

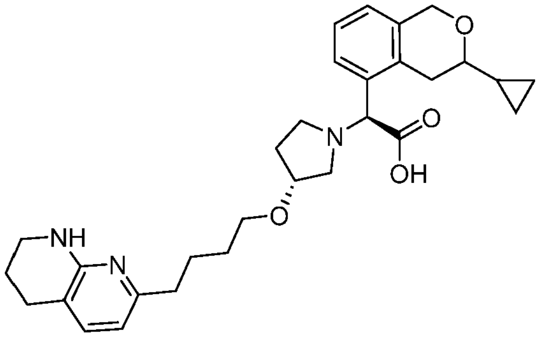

,

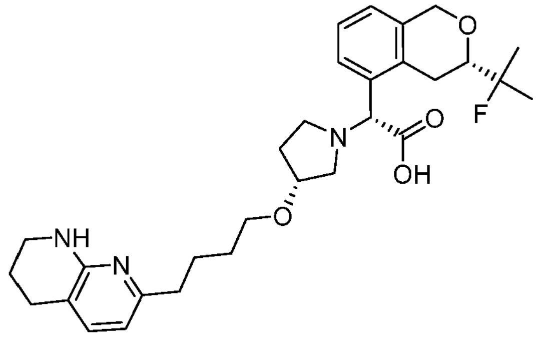

,

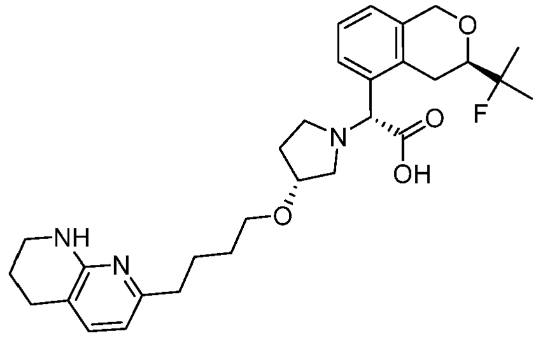

,

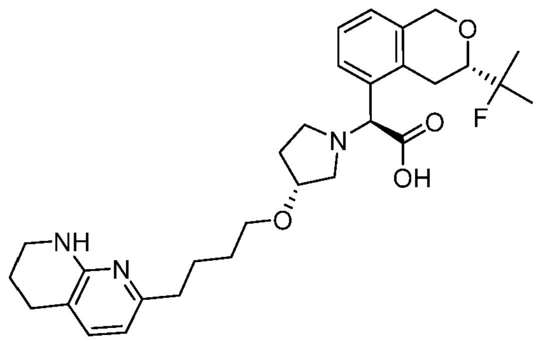

,

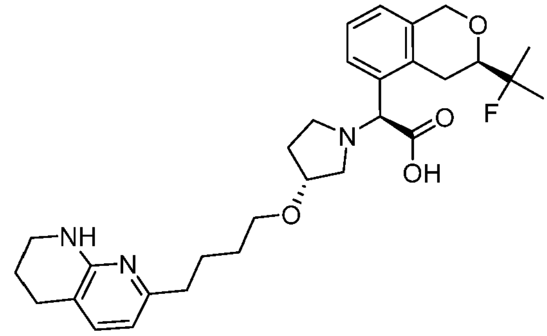

,

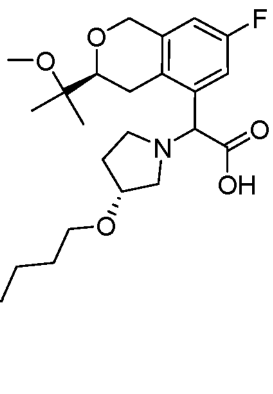

,

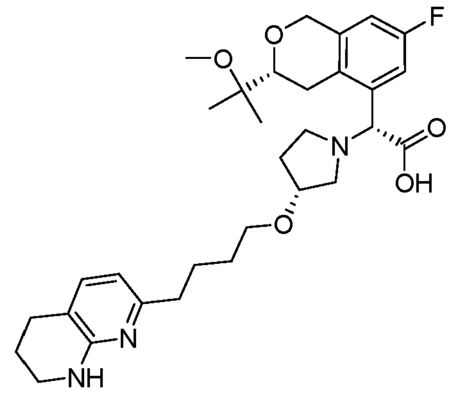

,

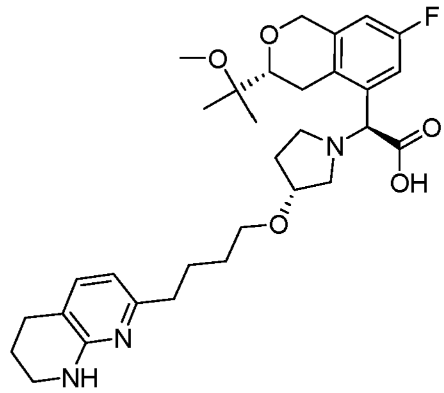

,

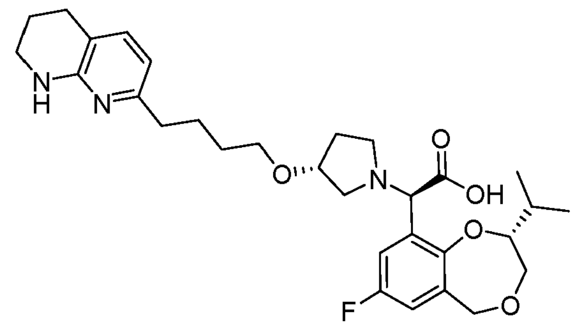

,

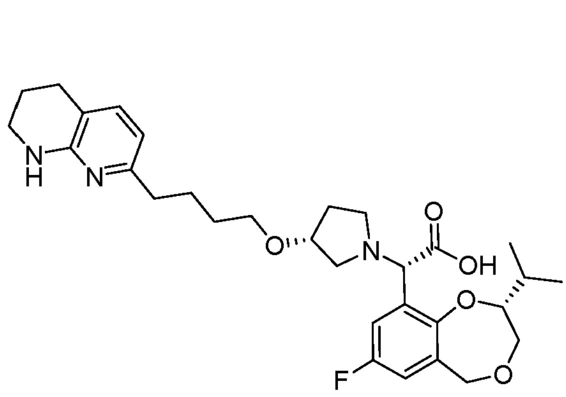

,

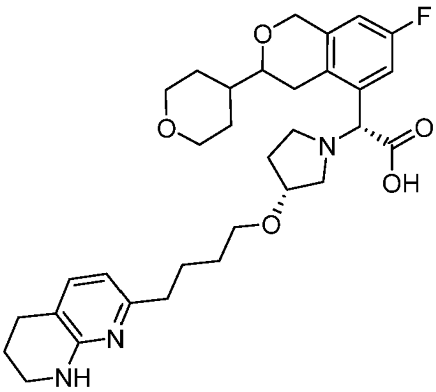

,

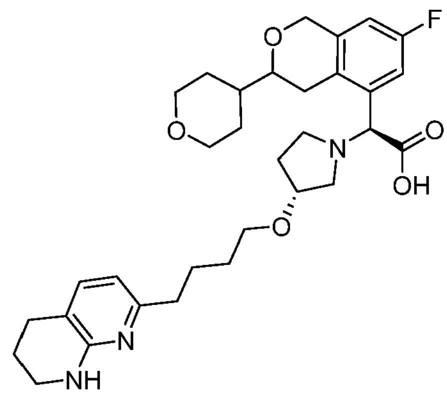

,

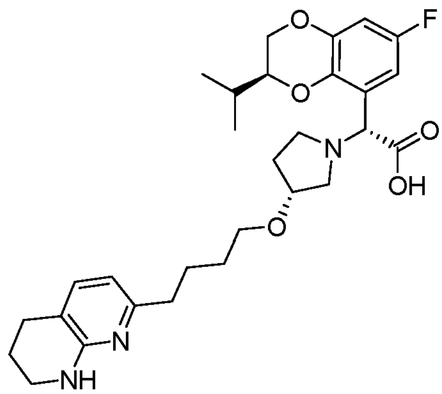

,

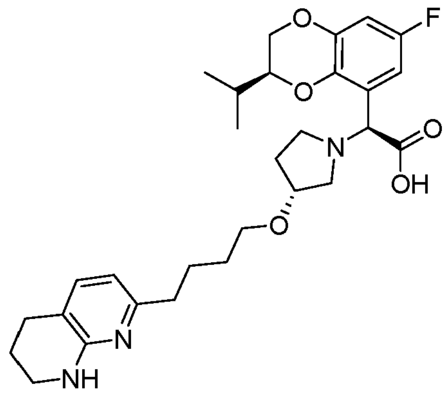

,

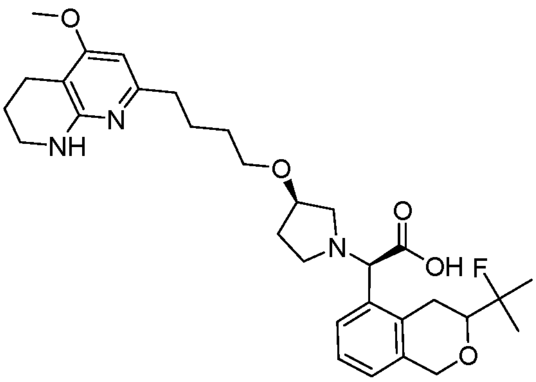

,

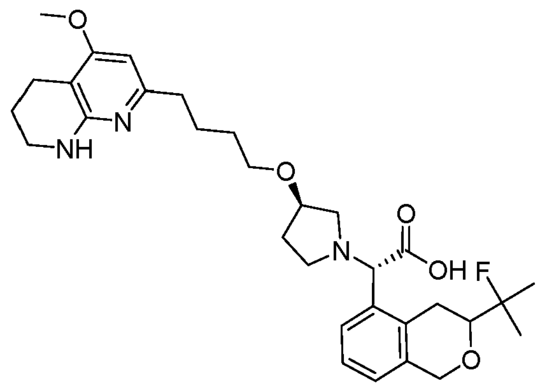

,

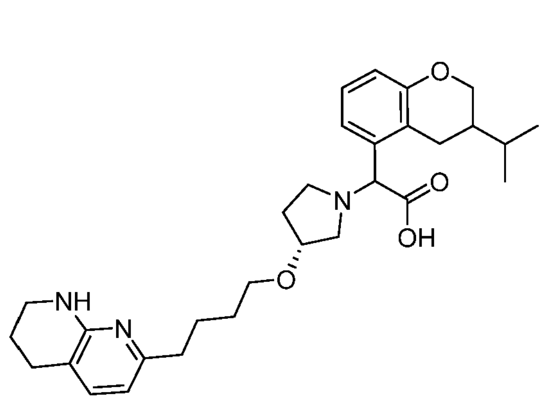

,

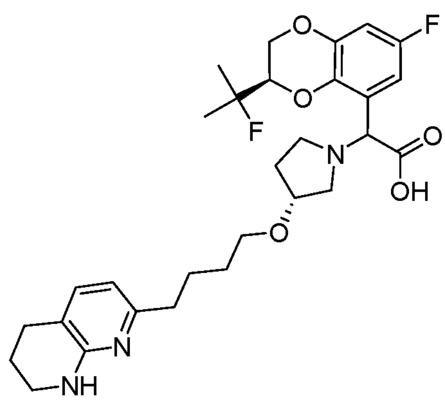

,

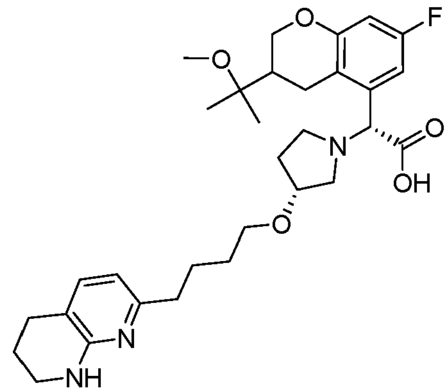,
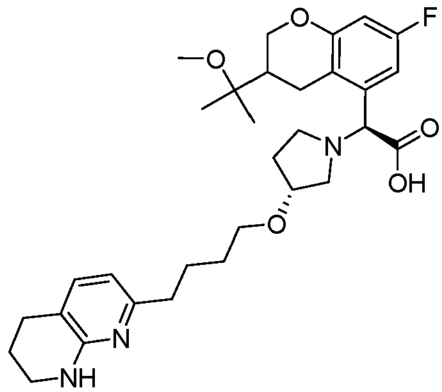,

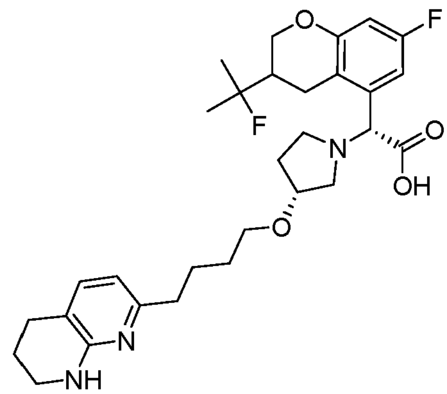,
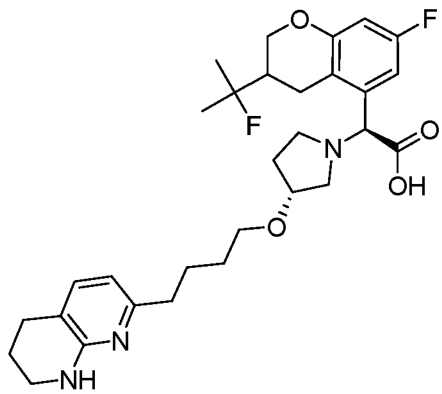,

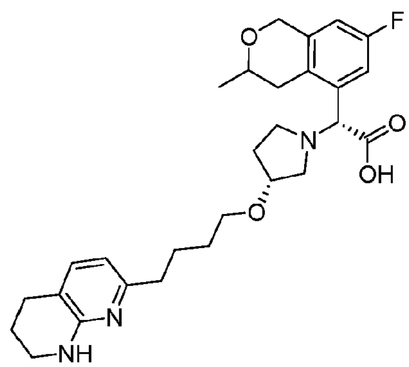,
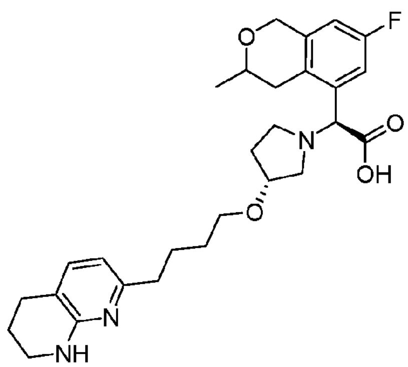

et

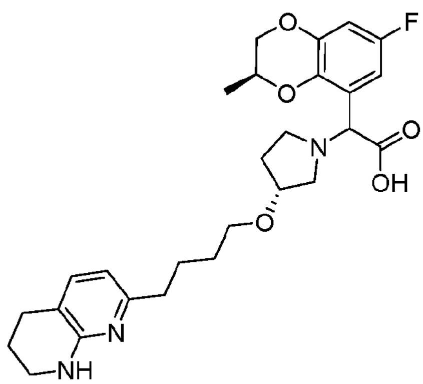;

ou sel pharmaceutiquement acceptable correspondant.

**6.** Composé selon la revendication 1 choisi dans le groupe constitué par :

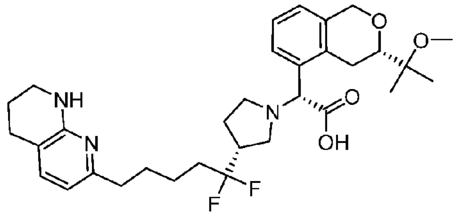,
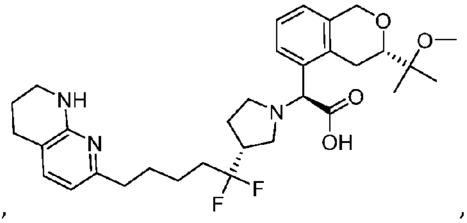,
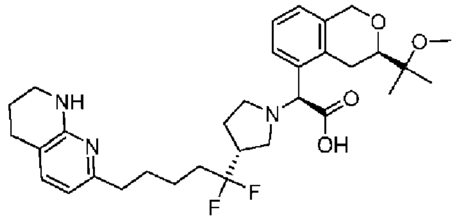
et
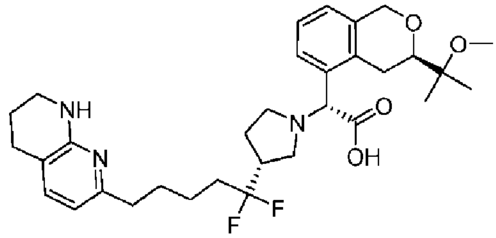;

ou sel pharmaceutiquement acceptable correspondant.

**7.** Composé selon la revendication 1 choisi dans le groupe constitué par :

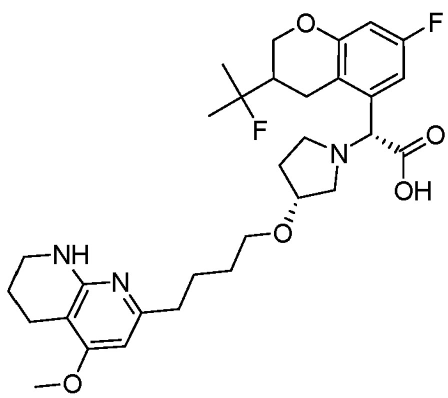,
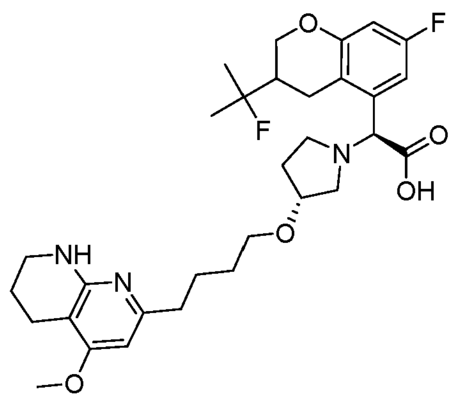,
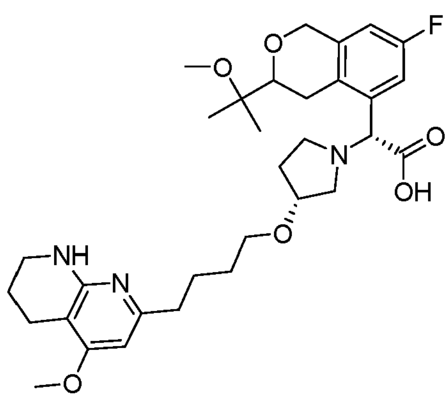,
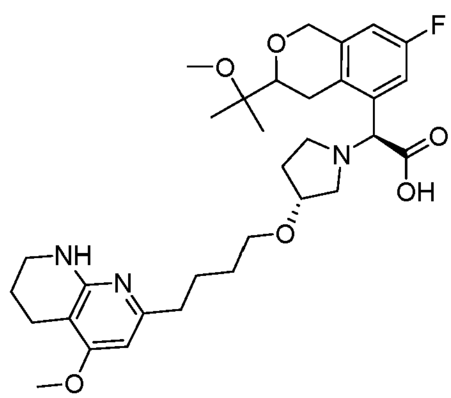,

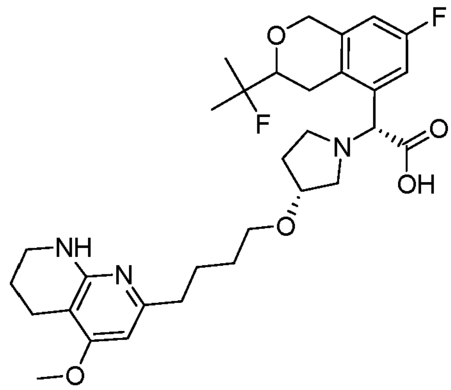,
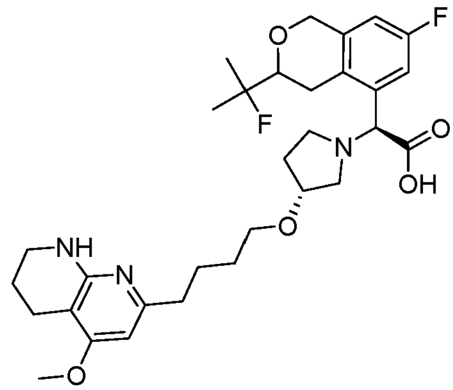,

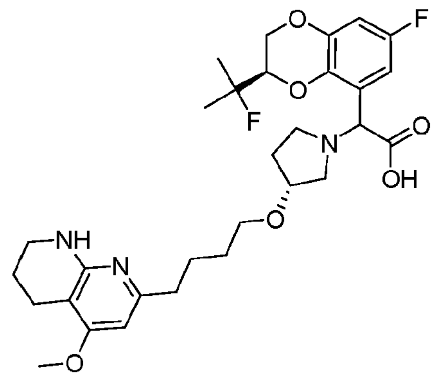,
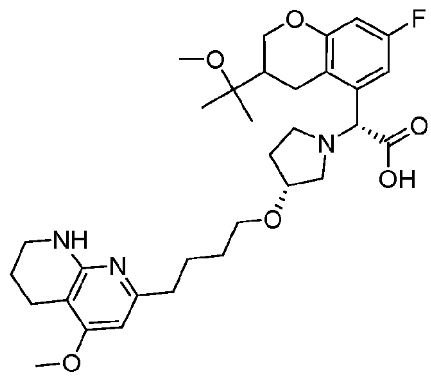

et

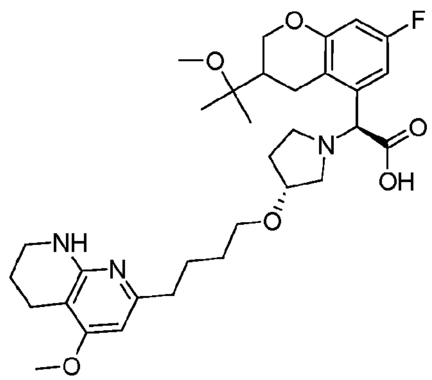;

ou sel pharmaceutiquement acceptable correspondant.

**8.** Composé selon la revendication 1 choisi dans le groupe constitué par :

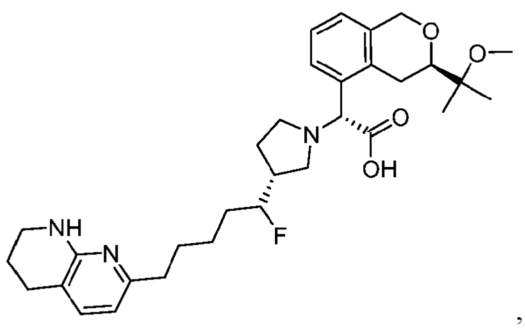,
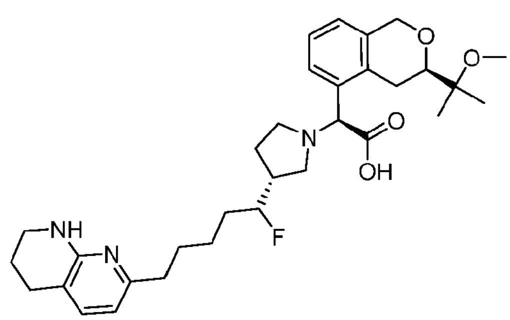,

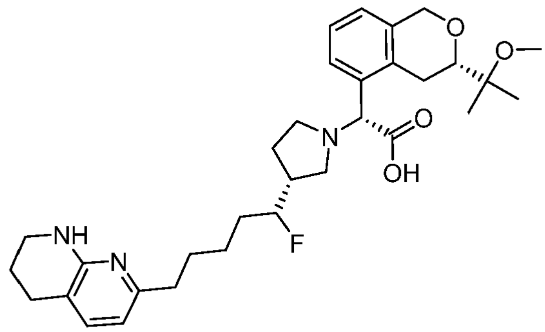

et

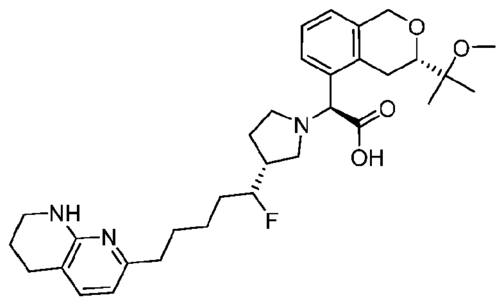

;

ou sel pharmaceutiquement acceptable correspondant.

**9.** Composé selon la revendication 1 choisi dans le groupe constitué par :

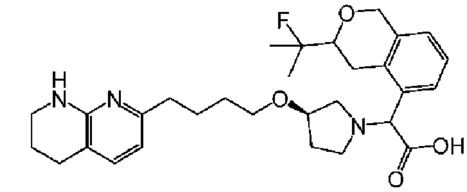
,
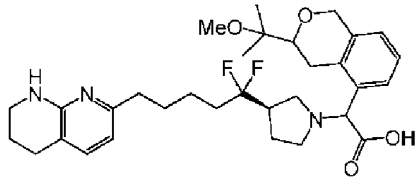
,

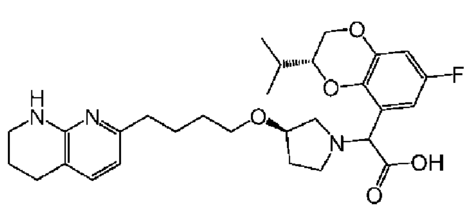
,
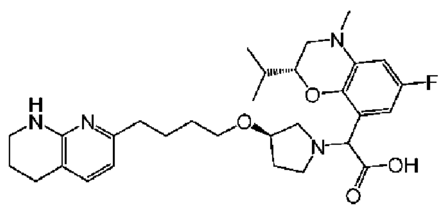
,

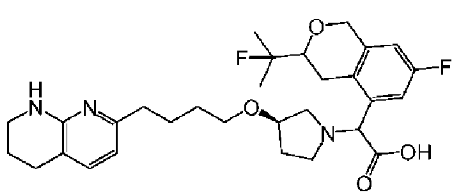
,
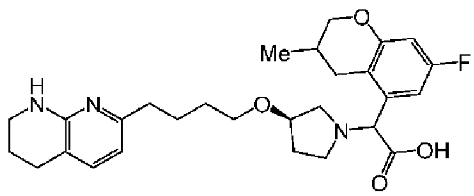
,

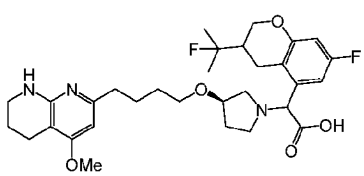 et 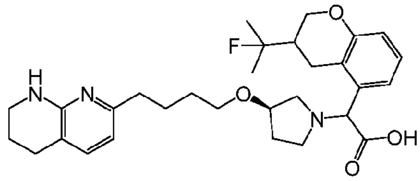 ;

ou sel pharmaceutiquement acceptable correspondant.

**10.** Composé selon la revendication 1 choisi dans le groupe constitué par :

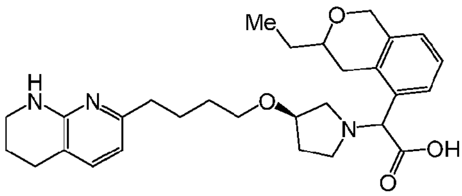 , 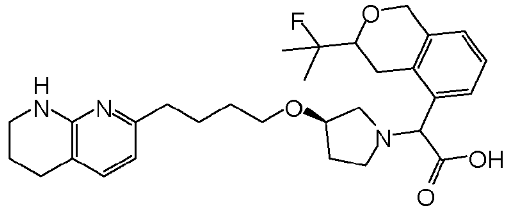 ,

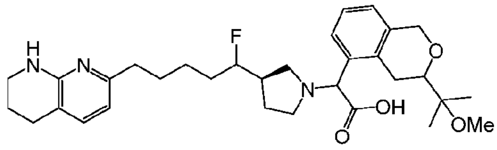 , 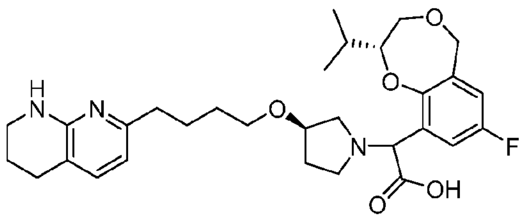 ,

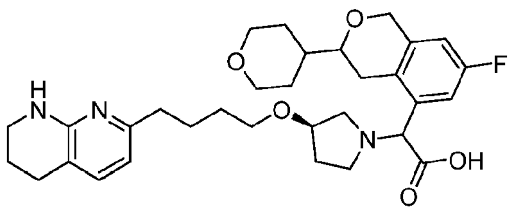 , 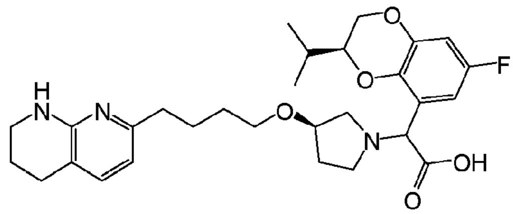 ,

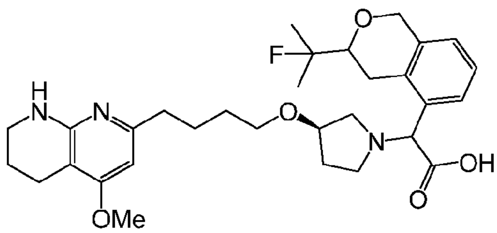 , 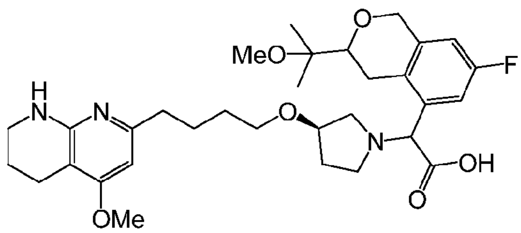 ,

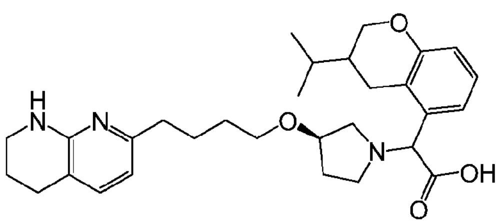 , 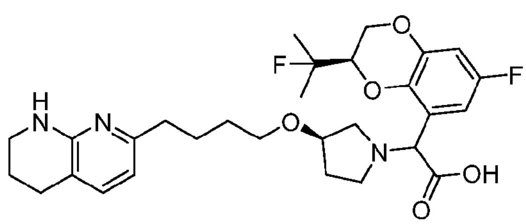 ,

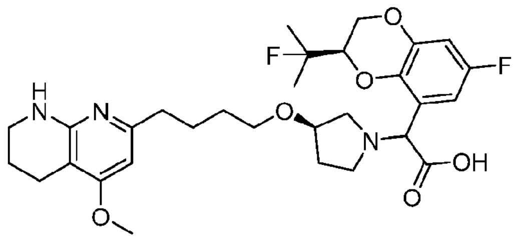
,
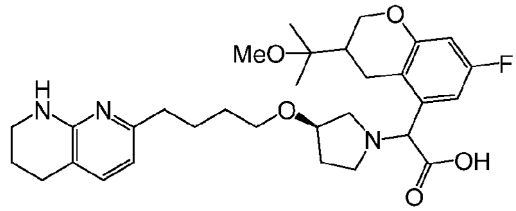
,

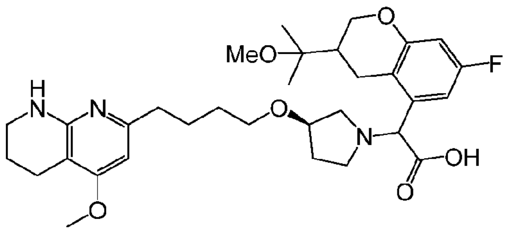
,
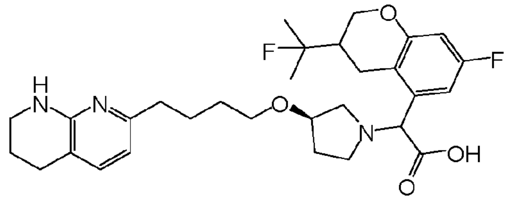
,

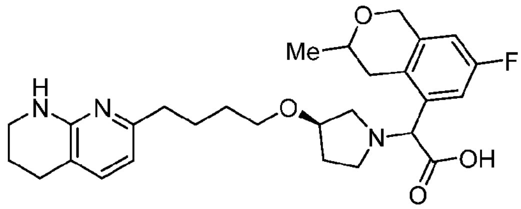

et

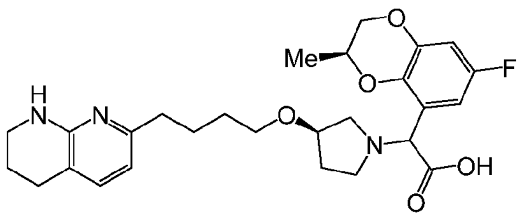
;

ou sel pharmaceutiquement acceptable correspondant.

**Figure 1**

αvβ6 $IC_{50}$:

A: $IC_{50}$ <0.01 μM; B: 0.01 μM < $IC_{50}$ <0.1 μM; and C: 0.1 μM < $IC_{50}$ <1 μM.

| Compound | αvβ6 $IC_{50}$ | Compound | αvβ6 $IC_{50}$ | Compound | αvβ6 $IC_{50}$ |
|---|---|---|---|---|---|
| 1-E1 | B | 10-A-E2 | A | 19-E1 | A |
| 1-E2 | A | 10-B-E1 | A | 19-E2 | C |
| 2-E1 | B | 10-B-E2 | B | 20-E1 | A |
| 2-E2 | B | 11-E1 | A | 20-E2 | C |
| 3-E1 | C | 11-E2 | B | 21-E1 | B |
| 3-E2 | A | 12-E1 | A | 21-E2 | A |
| 4-E1 | A | 12-E2 | B | 22 | A |
| 4-E2 | B | 13-E2 | B | 23-E1 | B |
| 5-A-E1 | C | 14-B-E1 | A | 23-E2 | A |
| 5-A-E2 | A | 14-B-E2 | A | 24 | A |
| 5-B-E1 | C | 15-E1 | B | 25 | A |
| 5-B-E2 | A | 15-E2 | C | 26-E1 | B |
| 6-E1 | A | 15-E3 | A | 26-E2 | A |
| 6-E2 | C | 16-A-E1 | C | 27-E1 | A |
| 7-E1 | A | 16-A-E2 | A | 27-E2 | A |
| 7-E2 | C | 16-B-E1 | A | 28-E1 | A |
| 8-E1 | B | 16-B-E2 | B | 28-E2 | B |
| 8-E2 | A | 17-E1 | C | 29-E1 | A |
| 9-E2 | A | 17-E2 | A | 29-E2 | B |
| 9-E3 | A | 18-E1 | B | 30 | A |
| 10-A-E1 | C | 18-E2 | A | | |

**Figure 2**

MDCK-MDR1 with PGP inh (A->B) [10^6 cm/s]

A: >20; B: 15-20; C: 10-<15; D: 5-<10; E: <5; NT: not tested

| Compound | MDCK-MDR1 w/PGP inh (A-B) | Compound | MDCK-MDR1 w/PGP inh (A-B) | Compound | MDCK-MDR1 w/PGP inh (A-B) |
|---|---|---|---|---|---|
| 1-E1 | NT | 10-A-E2 | C | 19-E1 | C |
| 1-E2 | C | 10-B-E1 | D | 19-E2 | NT |
| 2-E1 | E | 10-B-E2 | NT | 20-E1 | E |
| 2-E2 | NT | 11-E1 | A | 20-E2 | NT |
| 3-E1 | NT | 11-E2 | NT | 21-E1 | NT |
| 3-E2 | E | 12-E1 | E | 21-E2 | C |
| 4-E1 | D | 12-E2 | NT | 22 | B |
| 4-E2 | NT | 13-E2 | D | 23-E1 | NT |
| 5-A-E1 | NT | 14-B-E1 | E | 23-E2 | B |
| 5-A-E2 | D | 14-B-E2 | NT | 24 | NT |
| 5-B-E1 | NT | 15-E1 | NT | 25 | E |
| 5-B-E2 | E | 15-E2 | NT | 26-E1 | C |
| 6-E1 | D | 15-E3 | D | 26-E2 | C |
| 6-E2 | NT | 16-A-E1 | NT | 27-E1 | NT |
| 7-E1 | E | 16-A-E2 | NT | 27-E2 | B |
| 7-E2 | NT | 16-B-E1 | D | 28-E1 | B |
| 8-E1 | NT | 16-B-E2 | NT | 28-E2 | NT |
| 8-E2 | A | 17-E1 | NT | 29-E1 | D |
| 9-E2 | A | 17-E2 | E | 29-E2 | NT |
| 9-E3 | A | 18-E1 | NT | 30 | E |
| 10-A-E1 | NT | 18-E2 | B | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62724400 A
- WO 1999030709 A1
- US 6017926 A
- WO 2018089355 A1
- WO 2018160521 A2
- US 4522811 A


### Non-patent literature cited in the description


- **PERKINS JAMES J et al.** *Bioorganic & Medicinal Chemistry Letters*, 2003, vol. 13 (24), 4285-4288
- **BERGE et al.** Pharmaceutical Salts. *J. Pharm. Sci.*, 1977, vol. 66, 1-19
- Periodic Table of the Elements. Handbook of Chemistry and Physics, 1986-87